(19) Europäisches Patentamt
European Patent Office
Office européen des brevets

(11) EP 3 617 202 B1

(12) EUROPEAN PATENT SPECIFICATION

(45) Date of publication and mention of the grant of the patent:
**05.11.2025 Bulletin 2025/45**

(21) Application number: **19192772.2**

(22) Date of filing: **21.08.2019**

(51) International Patent Classification (IPC):
***C07D 403/10*** (2006.01) ***C07D 403/14*** (2006.01)
***C07D 417/14*** (2006.01) ***C07D 491/02*** (2006.01)
***C07D 495/02*** (2006.01) ***H10K 85/60*** (2023.01)

(52) Cooperative Patent Classification (CPC):
**C07D 403/10; C07D 403/14; C07D 417/14; C07D 491/02; C07D 495/02; H10K 85/654; H10K 85/657; H10K 85/6572;** H10K 50/11

(54) **HETEROCYCLIC COMPOUND AND ORGANIC LIGHT-EMITTING DEVICE**

HETEROCYCLISCHE VERBINDUNG UND ORGANISCHE LICHTEMITTIERENDE VORRICHTUNG

COMPOSÉ HÉTÉROCYCLIQUE ET DISPOSITIF ÉLECTROLUMINESCENT ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2018 KR 20180100569**

(43) Date of publication of application:
**04.03.2020 Bulletin 2020/10**

(73) Proprietor: **Samsung Electronics Co., Ltd.**
**Gyeonggi-do 16677 (KR)**

(72) Inventors:
- **JEON, Soonok**
**16678 Gyeonggi-do (KR)**
- **CHUNG, Yeonsook**
**16678 Gyeonggi-do (KR)**
- **LEE, Hasup**
**16678 Gyeonggi-do (KR)**
- **SIM, Myungsun**
**16678 Gyeonggi-do (KR)**
- **IHN, Sooghang**
**16678 Gyeonggi-do (KR)**
- **KOISHIKAWA, Yasushi**
**16678 Gyeonggi-do (KR)**

(74) Representative: **Elkington and Fife LLP**
**Prospect House**
**8 Pembroke Road**
**Sevenoaks, Kent TN13 1XR (GB)**

(56) References cited:
**EP-A1- 1 829 871** **EP-A1- 3 476 915**
**WO-A1-2017/171420** **WO-A1-2019/086667**
**WO-A1-2019/101594** **CN-A- 107 954 922**
**CN-A- 107 987 009** **KR-A- 20170 113 808**
**KR-A- 20170 134 264** **US-A1- 2017 186 962**
**US-A1- 2017 200 902** **US-A1- 2018 145 262**

- **YUANYUAN WANG ET AL: "A series of new bipolar CBP derivatives with introduction of a electron-deficient moiety for efficient green organic light-emitting diodes", ORGANIC ELECTRONICS., vol. 61, 26 April 2018 (2018-04-26), NL, pages 142 - 150, XP055646445, ISSN: 1566-1199, DOI: 10.1016/j.orgel.2018.04.040**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 617 202 B1

## Description

### FIELD OF THE INVENTION

One or more embodiments relate to a heterocyclic compound and an organic light-emitting device including the same.

### BACKGROUND OF THE INVENTION

Organic light-emitting devices (OLEDs) are self-emission devices that produce full-color images, and that also have wide viewing angles, high contrast ratios, short response times, and excellent characteristics in terms of brightness, driving voltage, and response speed, compared to the devices in the art.

In an example, an organic light-emitting device includes an anode, a cathode, and an organic layer disposed between the anode and the cathode, wherein the organic layer includes an emission layer. A hole transport region may be disposed between the anode and the emission layer, and an electron transport region may be disposed between the emission layer and the cathode. Holes provided from the anode may move toward the emission layer through the hole transport region, and electrons provided from the cathode may move toward the emission layer through the electron transport region. Carriers, such as holes and electrons, recombine in the emission layer to produce excitons. These excitons transit from an excited state to a ground state, thereby generating light.

Various types of organic light emitting devices are known. However, there still remains a need in OLEDs having low driving voltage, high efficiency, high brightness, and long lifespan.

EP 1 829 871 relates to organic compounds and charge transporting materials, and organic electroluminescent devices using the organic compounds.

CN 107 987 009 discloses a carbazole derivative which can be applied in the field of an organic electroluminescence device, an organic solar battery, an organic film transistor or an organic photoreceptor.

CN 107 954 922 discloses a diphenyl dicarbazole derivative which can be applied to fields such as organic electroluminescence devices, organic solar cells, organic film transistors or organic photoreceptors.

In Y. Wang et al., "A series of new bipolar CBP derivatives with introduction of a electron-deficient moiety for efficient green organic light-emitting diodes", Organic Electronics Volume 61, October 2018, Pages 142-150, three CBP derivatives host materials were designed and synthesized by introducing pyridine, benzimidazole and triazine groups.

US 2017/186962 discloses organic electroluminescent materials and their applications in organic photoelectric apparatus.

US 2018/145262 discloses carbazole- or indolocarbazole-containing donor acceptor type compounds with an ortho substitution on the aromatic group that is connected to carbazole. The compounds can be used in host applications or as delayed fluorescent emitters.

KR 2017 0113808 discloses a thermal activated delayed fluorescent material and an organic light-emitting device containing the same.

### SUMMARY OF THE INVENTION

Aspects of the present disclosure provide a novel heterocyclic compound and an organic light-emitting device including the same.

Additional aspects will be set forth in part in the description which follows and, in part, will be apparent from the description, or may be learned by practice of the presented embodiments.

The present invention provides a heterocyclic compound in accordance with claim 1.

An embodiment of the present invention provides an organic light-emitting device including:

a first electrode;
a second electrode; and
an organic layer disposed between the first electrode and the second electrode,
wherein the organic layer includes an emission layer and the organic layer includes at least one heterocyclic compound represented by Formula 1.

### BRIEF DESCRIPTION OF THE DRAWING

These and/or other aspects will become apparent and more readily appreciated from the following description of the embodiments, taken in conjunction with the FIGURE which is a schematic diagram of an organic light-emitting device according to an embodiment.

DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly in contact with the other element or intervening elements may be present therebetween. In contrast, when an element is referred to as being "directly on" another element, there are no intervening elements present.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

The terminology used herein is for the purpose of describing particular embodiments only and is not intended to be limiting. As used herein, the singular forms "a," "an," and "the" are intended to include the plural forms as well, unless the context clearly indicates otherwise.

The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, 5% of the stated value.

In an embodiment, a heterocyclic compound is provided. The heterocyclic compound is represented by Formula 1 below:

Formula 1

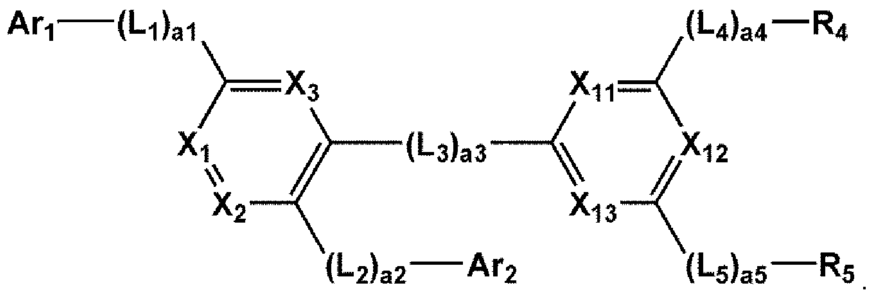

In Formula 1, $X_1$ is N or $C(R_1)$, $X_2$ is N or $C(R_2)$, and $X_3$ is N or $C(R_3)$, i) wherein one selected from $X_1$ to $X_3$ is N, and the others thereof are not N; or ii) each of $X_1$ to $X_3$ is not N.

In an embodiment,

$X_1$ may be $C(R_1)$, $X_2$ may be $C(R_2)$, and $X_3$ may be $C(R_3)$;
$X_1$ may be N, $X_2$ may be $C(R_2)$, and $X_3$ may be $C(R_3)$; or
$X_1$ may be $C(R_1)$, $X_2$ may be N, and $X_3$ may be $C(R_3)$, but embodiments of the present disclosure are not limited thereto.

$R_1$ to $R_3$ are each independently hydrogen, deuterium, a $C_1$-$C_{20}$ alkyl group (for example, a methyl group, an ethyl group, a propyl group, a butyl group, or a pentyl group), a $C_1$-$C_{20}$ alkyl group substituted with at least one deuterium, a $C_1$-$C_{20}$ alkoxy group (for example, a methoxy group, an ethoxy group, a propoxy group, a butoxy group, or a pentoxy group), or a $C_1$-$C_{20}$ alkoxy group substituted with at least one deuterium.

In Formula 1,

$L_1$ and $L_2$ are a single bond.

A group represented by $*-(L_3)_{a3}-*'$ is a single bond. In a group represented by $*-(L_3)_{a3}-*'$, * indicates a binding site to a 6-membered ring on the left side in Formula 1, and *' indicates a binding site to a 6-membered ring on the right side in Formula 1.

A group represented by

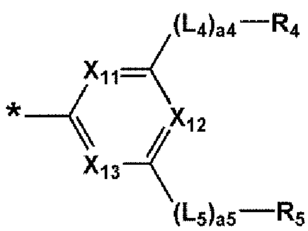

is a group represented by one selected from Formulae A1 to A38.

* in a group represented by

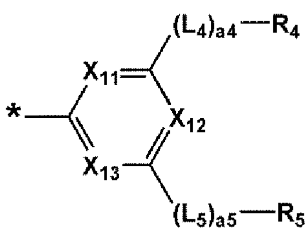

indicates a binding site to a neighboring atom.

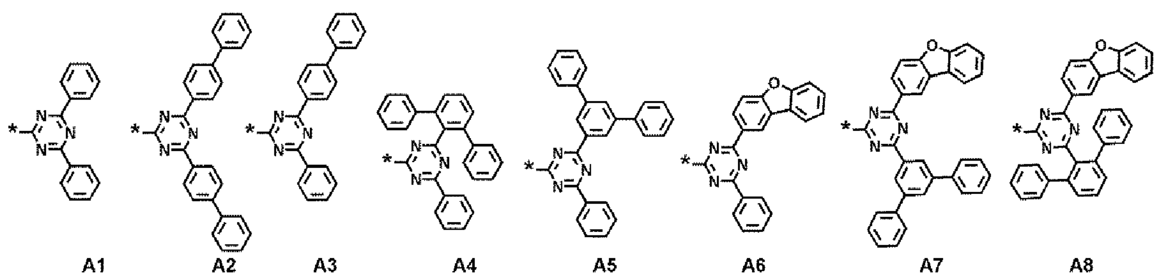

A1 A2 A3 A4 A5 A6 A7 A8

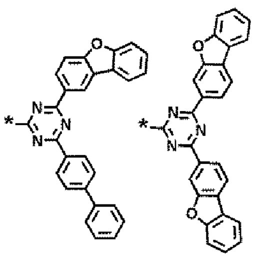
A9 A10

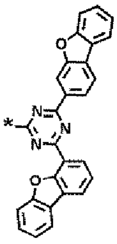
A11

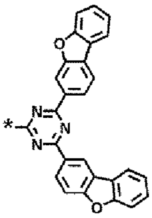
A12

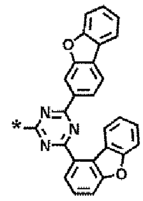
A13

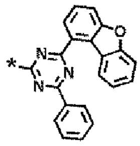
A14

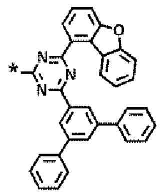
A15

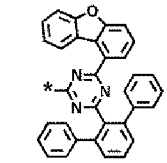
A16

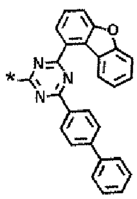
A17

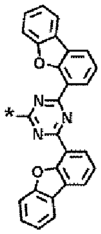
A18

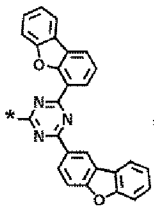
A19

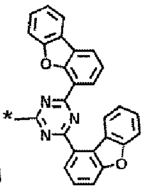
A20

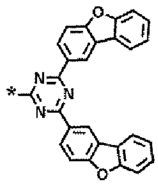
A21

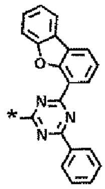
A22

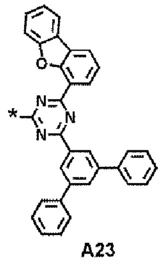
A23

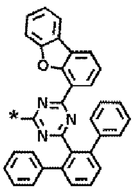
A24

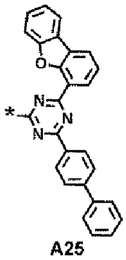
A25

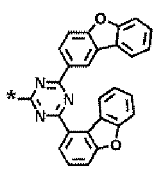
A26

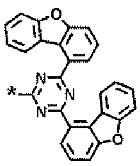
A27

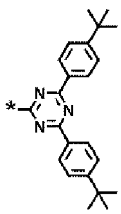
A28

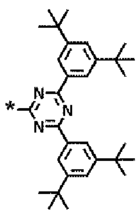
A29

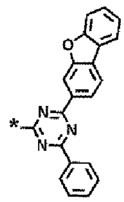
A30

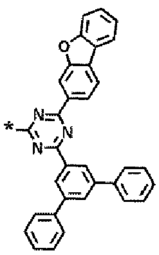
A31

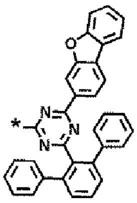
A32

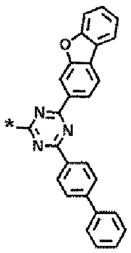
A33

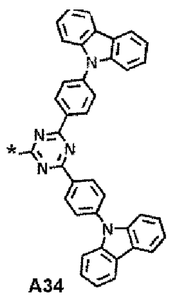
A34

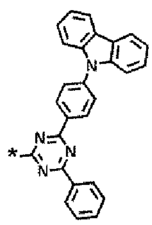
A35

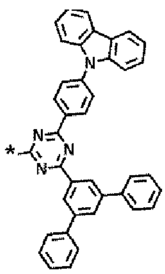
A36

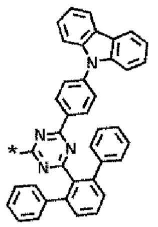
A37

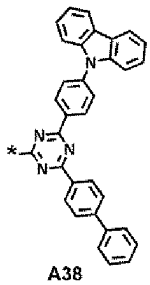
A38

, wherein, in the formulae above, * and *' each indicate a binding site to a neighboring atom.

$Ar_1$ and $Ar_2$ are each independently a group represented by one selected from Formulae D'-1 to D'-7 and D002 to D005, D015 to D0027, D0029 to D0032 and D0034 to D279:

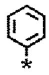 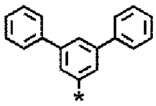 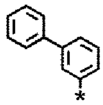 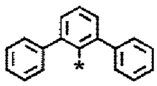 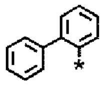 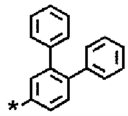 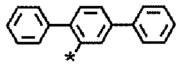

D'-1 D'-2 D'-3 D'-4 D'-5 D'-6 D'-7

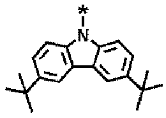 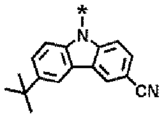 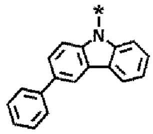 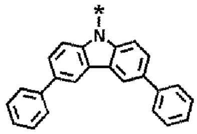

D002 D003 D004 D005

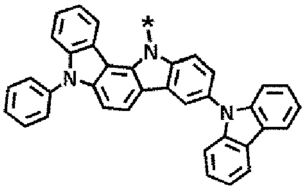 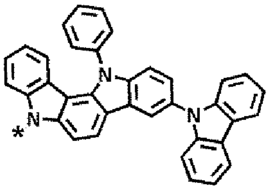 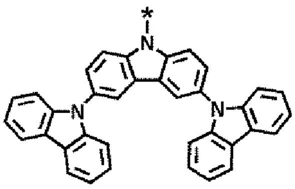

**D015** **D016** **D017**

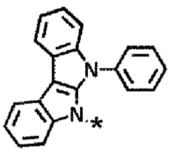 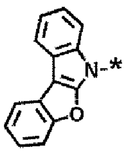 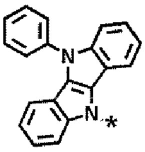 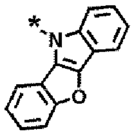 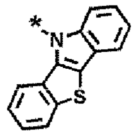 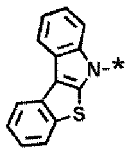

**D018** **D019** **D020** **D021** **D022** **D023**

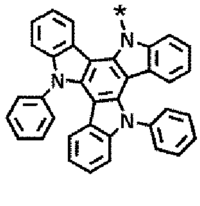 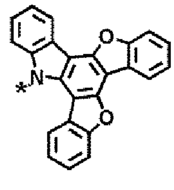 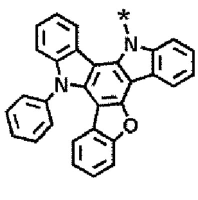 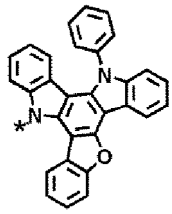

**D024** **D025** **D026** **D027**

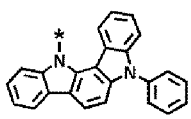 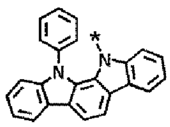 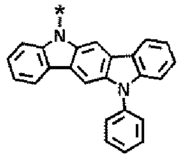 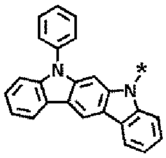

D029 D030 D031 D032

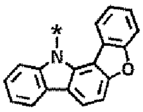 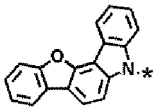 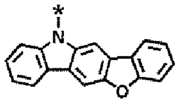 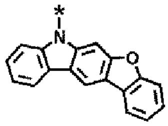 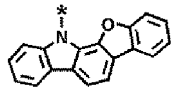 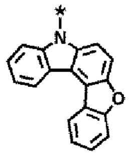

D034 D035 D036 D037 D038 D039

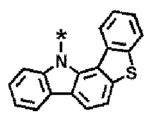
D040

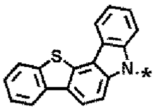
D041

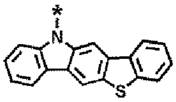
D042

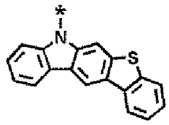
D043

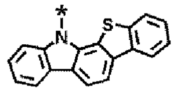
D044

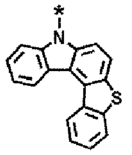
D045

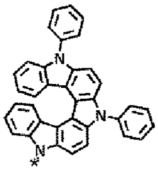
D046

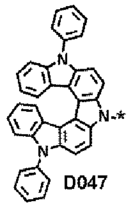
D047

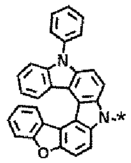
D048

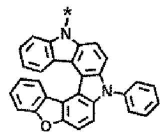
D049

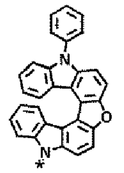
D050

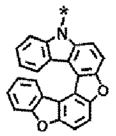
D051

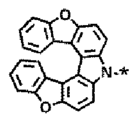
D052

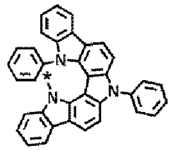
D053

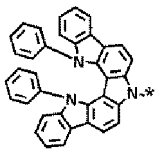
D054

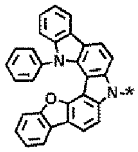
D055

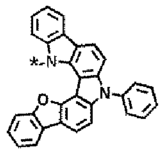
D056

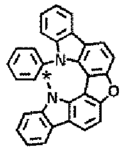
D057

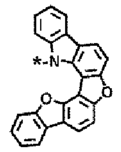
D058

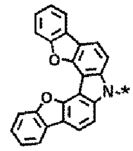
D059

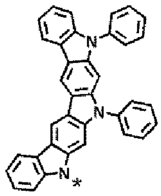
D060

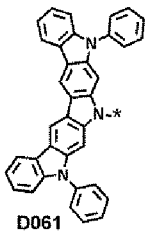
D061

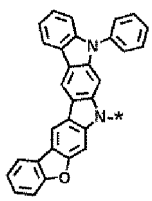
D062

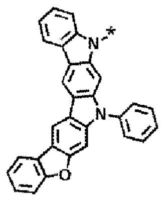
D063

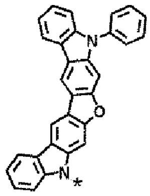
D064

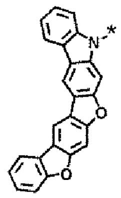
D065

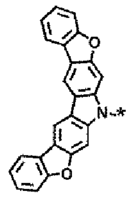
D066

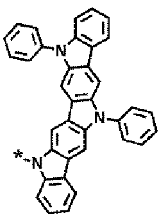
D067

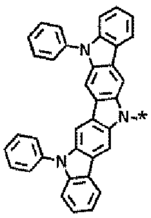
D068

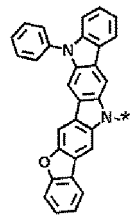
D069

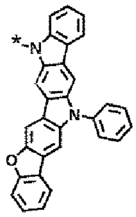
D070

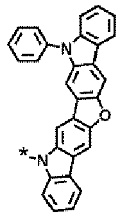
D071

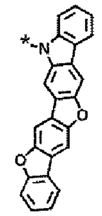
D072

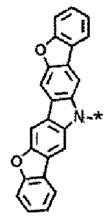
D073

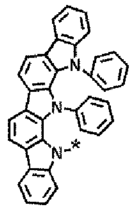
D074

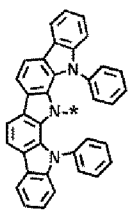
D075

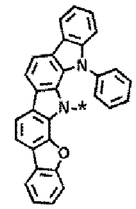
D076

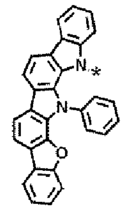
D077

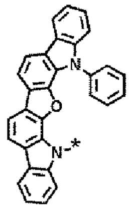
D078

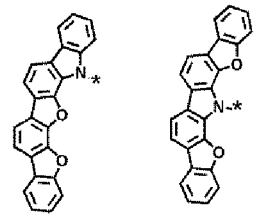
D079

D080

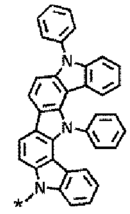
D081

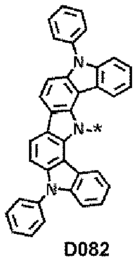
D082

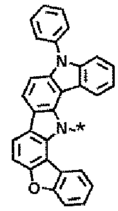
D083

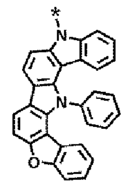
D084

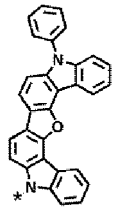
D085

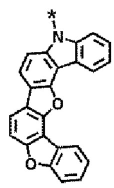
D086

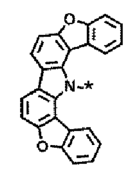
D087

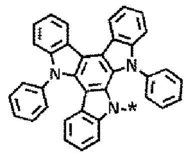
D088

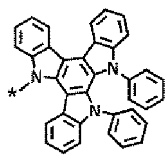
D089

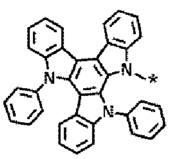
D090

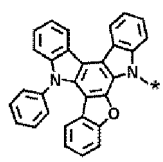
D091

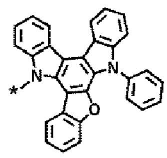
D092

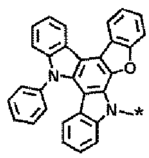
D093

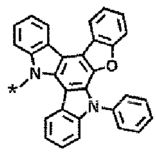
D094

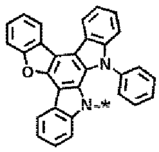
D095

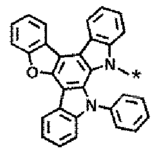
D096

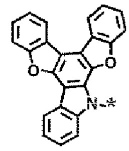
D097

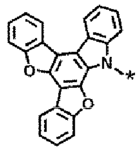
D098

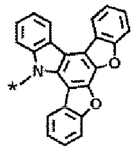
D099

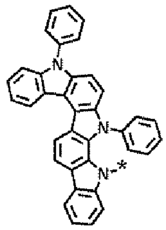
D100

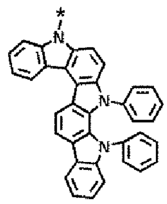
D101

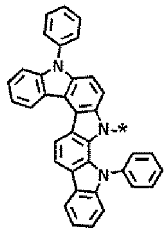
D102

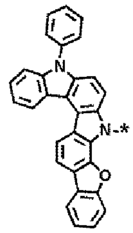
D103

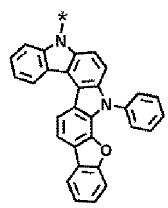
D104

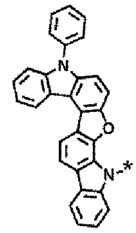
D105

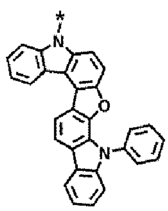
D106

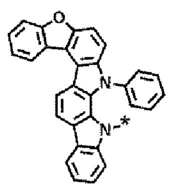
D107

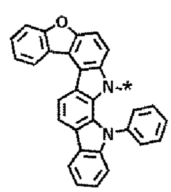
D108

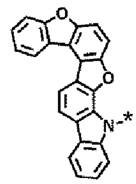
D109

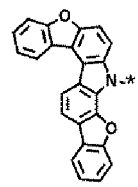
D110

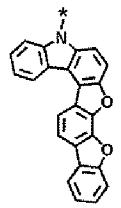
D111

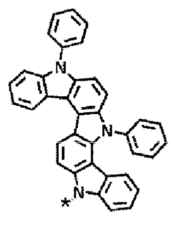
D112

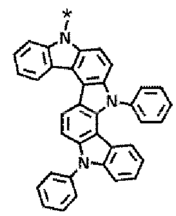
D113

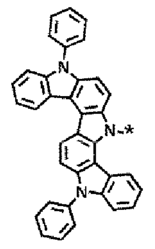
D114

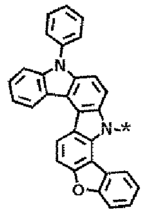
D115

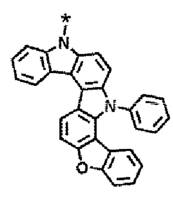
D116

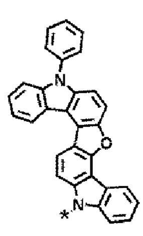
D117

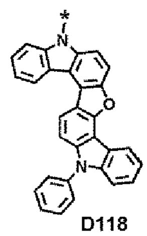
D118
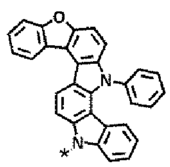
D119
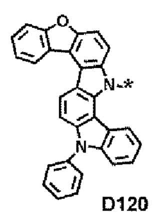
D120
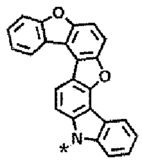
D121
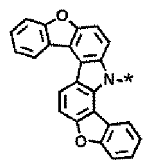
D122
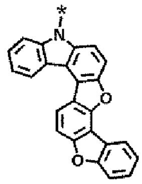
D123

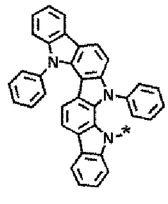
D124
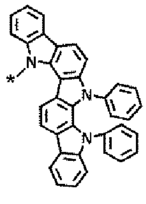
D125
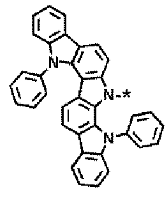
D126
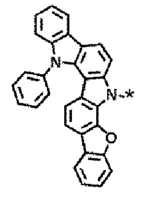
D127
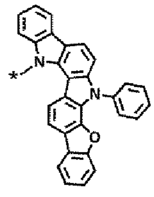
D128
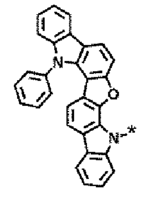
D129

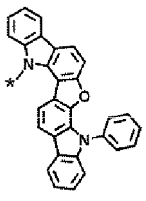
D130
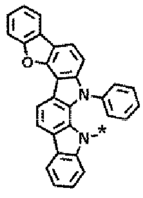
D131
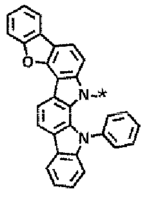
D132
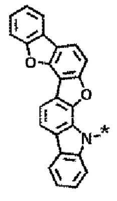
D133
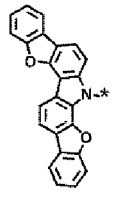
D134
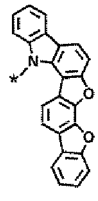
D135

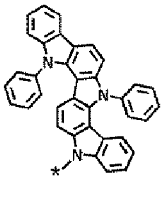
D136
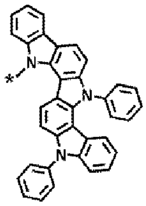
D137
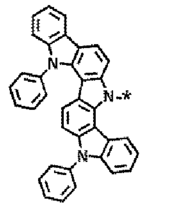
D138
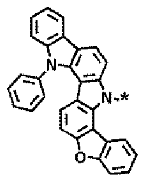
D139
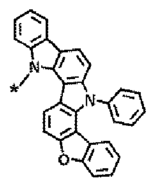
D140
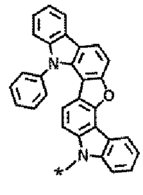
D141

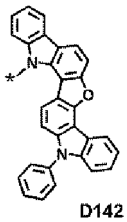
D142
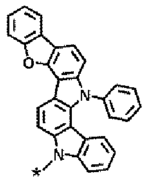
D143
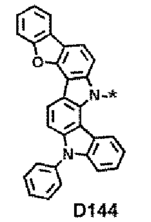
D144
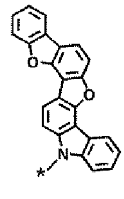
D145
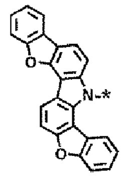
D146
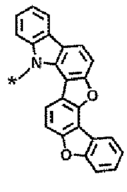
D147

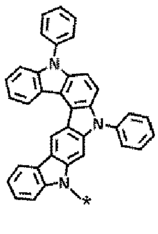
D148
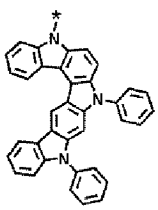
D149
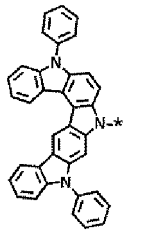
D150
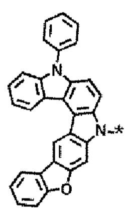
D151
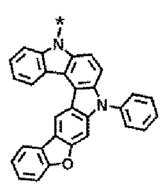
D152
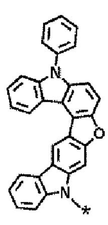
D153

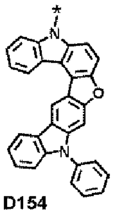
D154

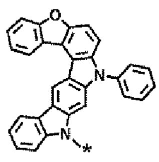
D155

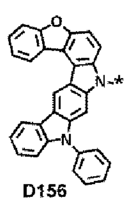
D156

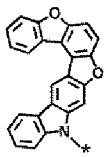
D157

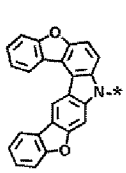
D158

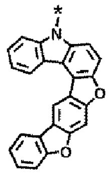
D159

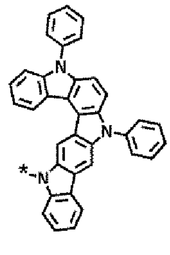
D160

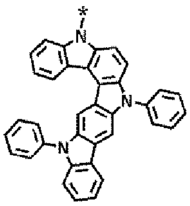
D161

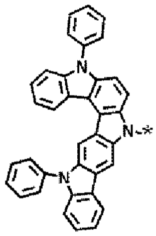
D162

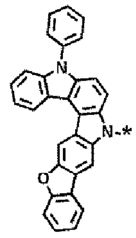
D163

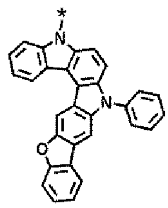
D164

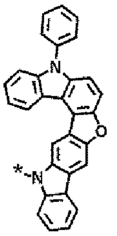
D165

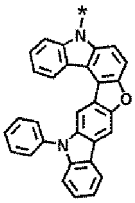
D166

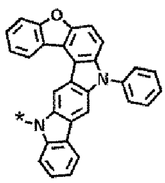
D167

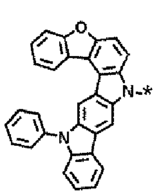
D168

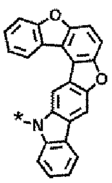
D169

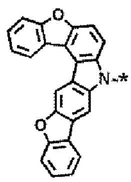
D170

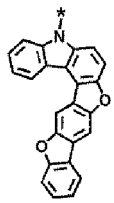
D171

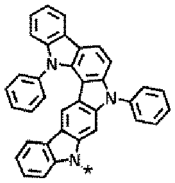
D172

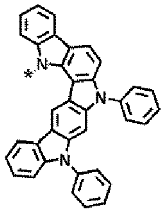
D173

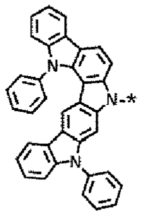
D174

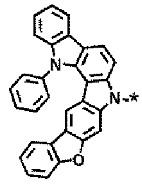
D175

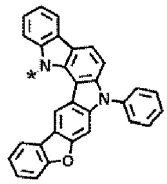
D176

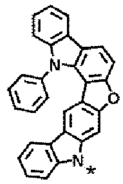
D177

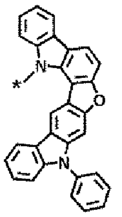
D178

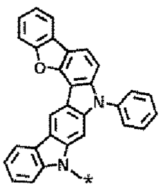
D179

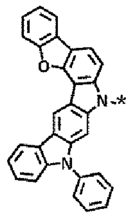
D180

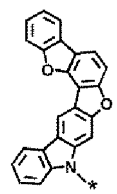
D181

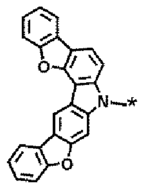
D182

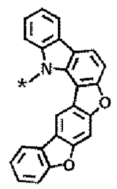
D183

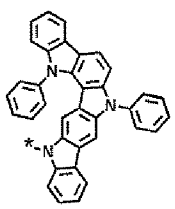
D184

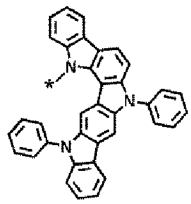
D185

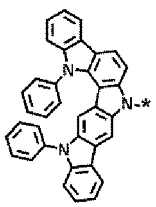
D186

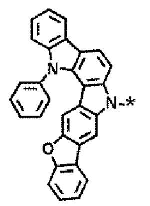
D187

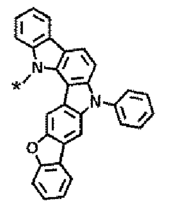
D188

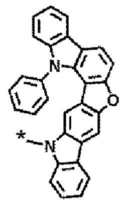
D189

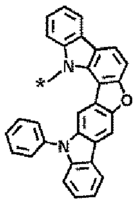
D190

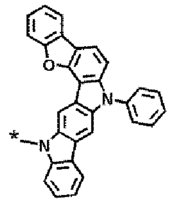
D191

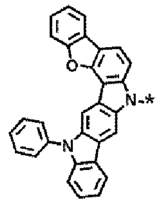
D192

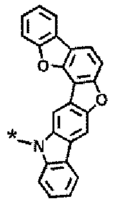
D193

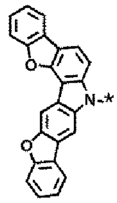
D194

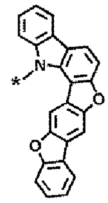
D195

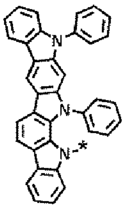
D196

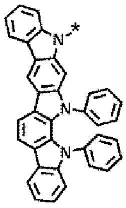
D197

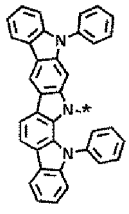
D198

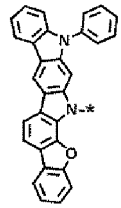
D199

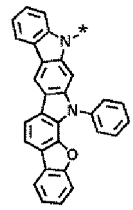
D200

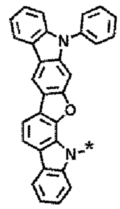
D201

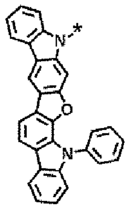
D202

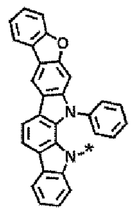
D203

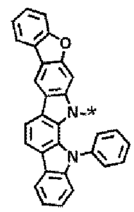
D204

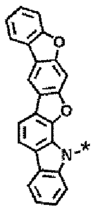
D205

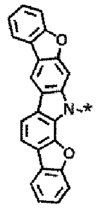
D206

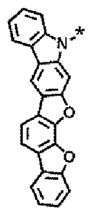
D207

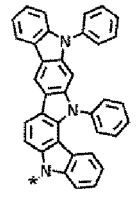
D208

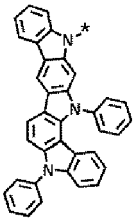
D209

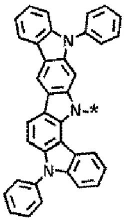
D210

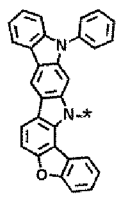
D211

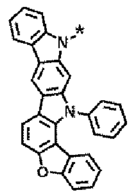
D212

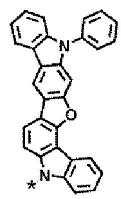
D213

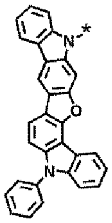
D214

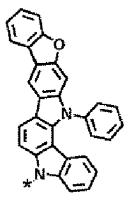
D215

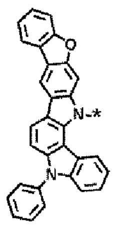
D216

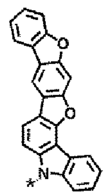
D217

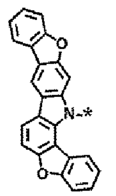
D218

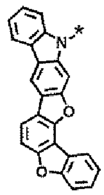
D219

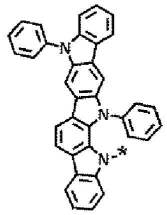
D220

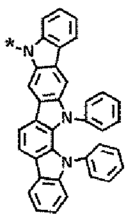
D221

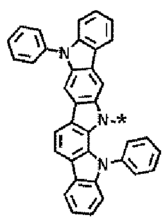
D222

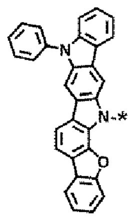
D223

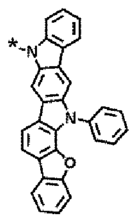
D224

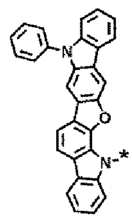
D225

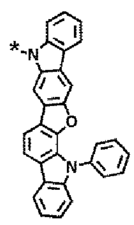
D226

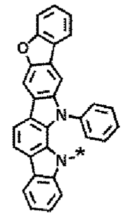
D227

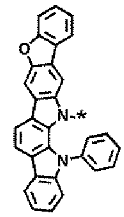
D228

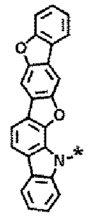
D229

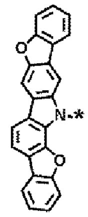
D230

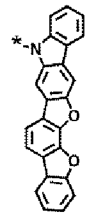
D231

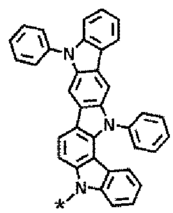
D232

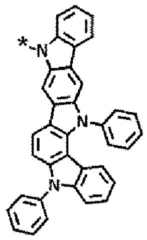
D233

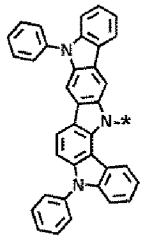
D234

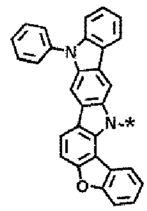
D235

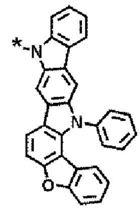
D236

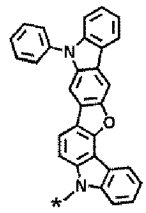
D237

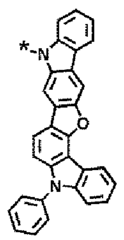
D238

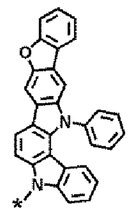
D239

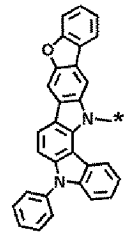
D240

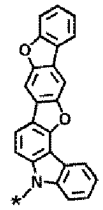
D241

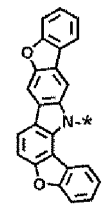
D242

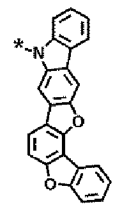
D243

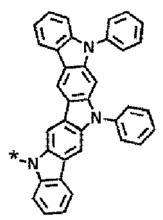
D244

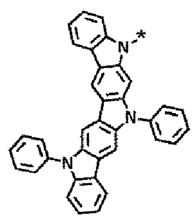
D245

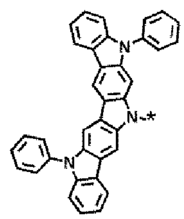
D246

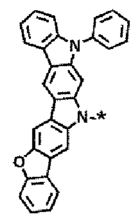
D247

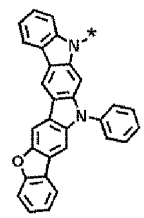
D248

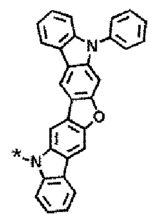
D249

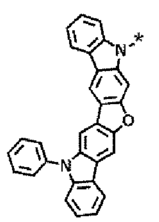
D250

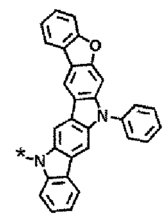
D251

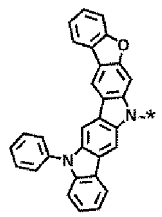
D252

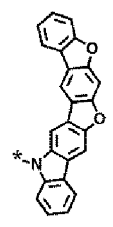
D253

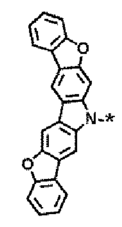
D254

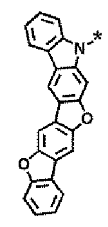
D255

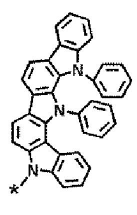
D256

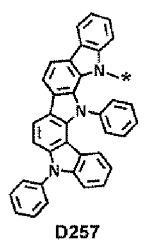
D257

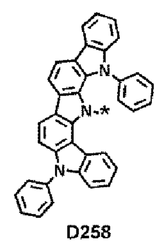
D258

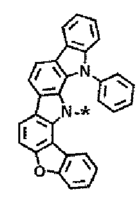
D259

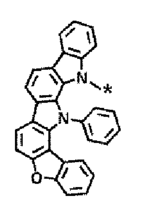
D260

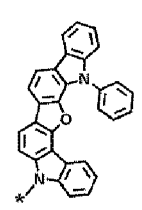
D261

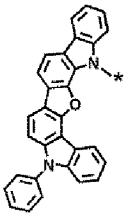
D262
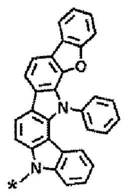
D263
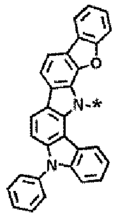
D264
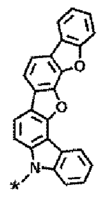
D265
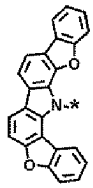
D266
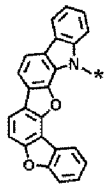
D267

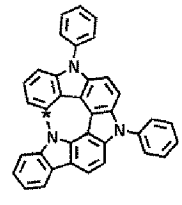
D268
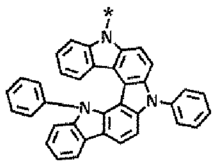
D269
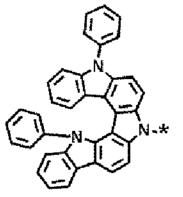
D270
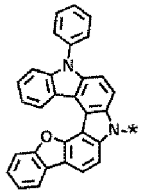
D271
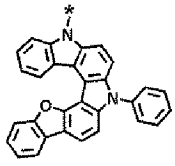
D272
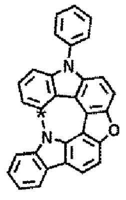
D273

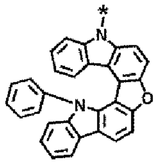
D274
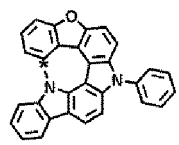
D275
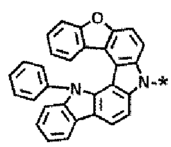
D276
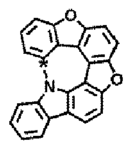
D277
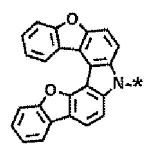
D278
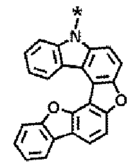
D279

In an embodiment, $Ar_1$ and $Ar_2$ in Formula 1 may be identical to each other.

In one or more embodiments, $Ar_1$ and $Ar_2$ in Formula 1 may be different from each other.

In Formula 1, $X_{11}$ to $X_{13}$ are each N.

In one or more embodiments, the heterocyclic compound may be one selected from Compounds 2-5, 10, 11, 13-28, 33, 34, 36-39, 44-57, 59-62, 67, 68, 70-85, 90, 91, 93-96, 101-114, 116-119, 124, 125, 127-142, 147-344, 346-349, 354, 355, 357-372, 377, 379-381, 386-401, 403-405, 410, 412-430, 435, 437-439, 444-459, 461-464, 469, 470, 472-479, 481-487, 492-1074, 1076, 1077 below. Compounds 1, 6-9, 12, 29-32, 35, 40-43, 58, 63-66, 69, 86-89, 92, 97-100, 115, 120-123, 126, 143-146, 345, 350-353, 356, 373-376, 378, 382-385, 402, 406-409, 411, 431-434, 436, 440-443, 460, 465-468, 471, 480, 488-491, 1075 and 1078 below do not form part of the invention:

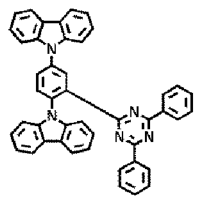
1
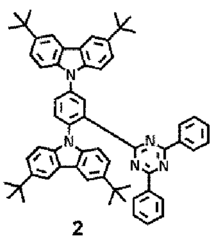
2
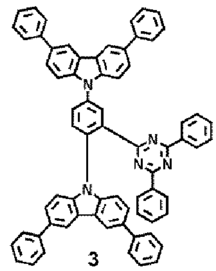
3
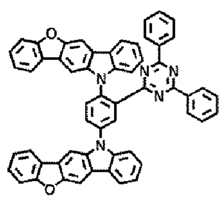
4
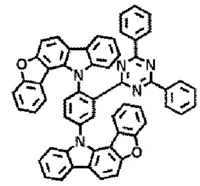
5

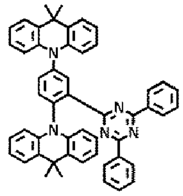
6
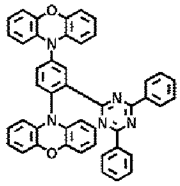
7
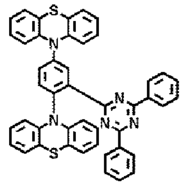
8
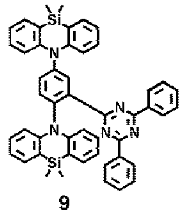
9
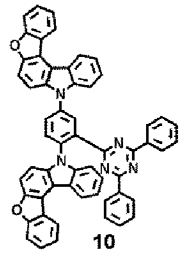
10
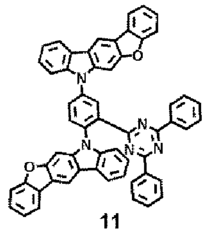
11

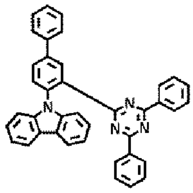
12

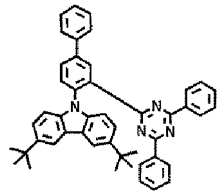
13

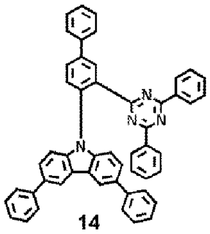
14

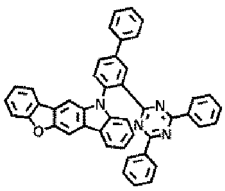
15

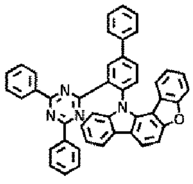
16

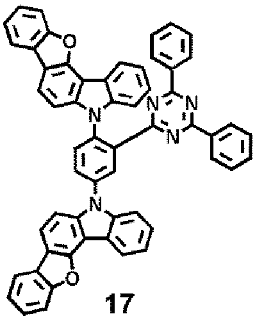
17

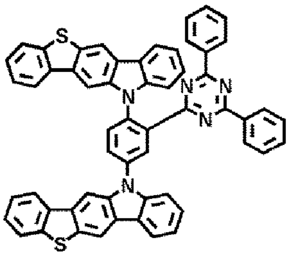
18

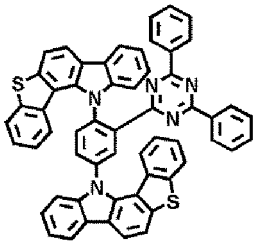
19

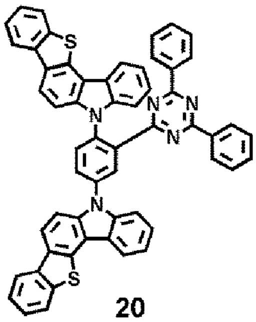
20

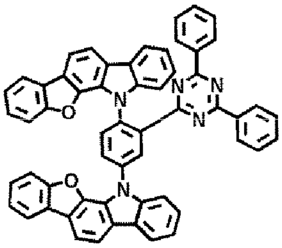
21

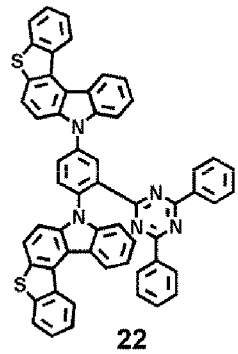
22

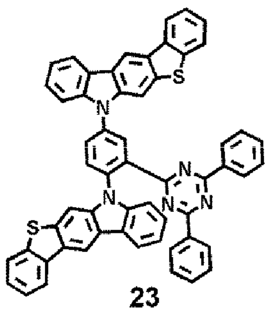
23

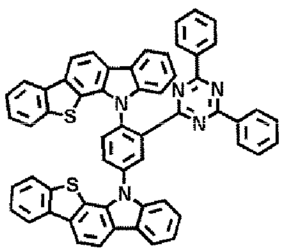
24

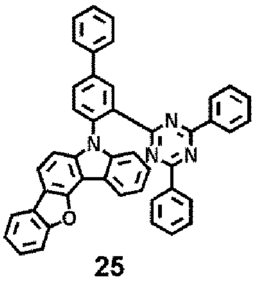
25

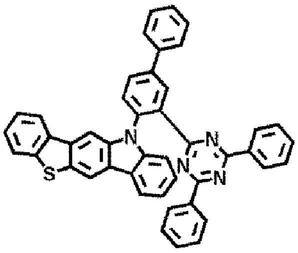
26

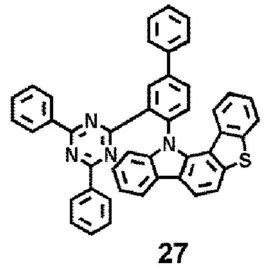
27

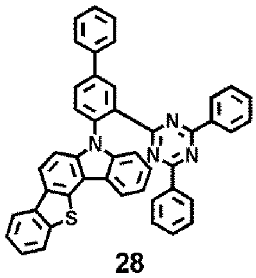
28

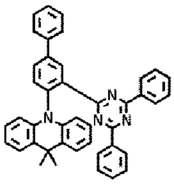
29

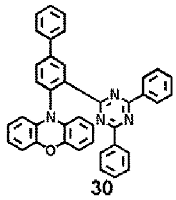
30

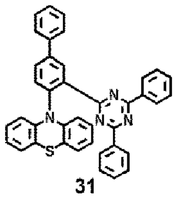
31

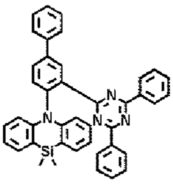
32

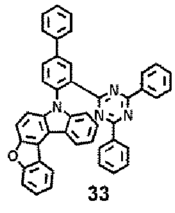
33

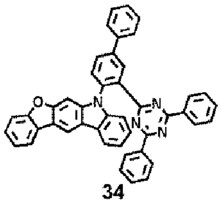
34

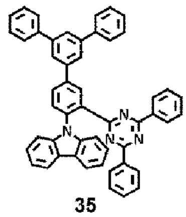
35

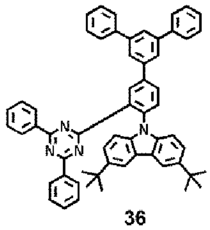
36

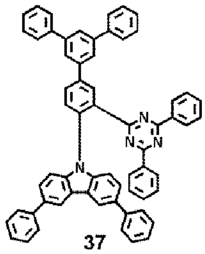
37

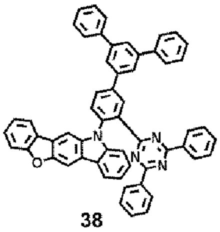
38

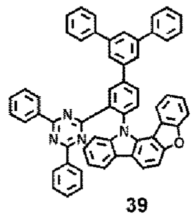
39

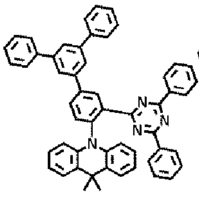
40

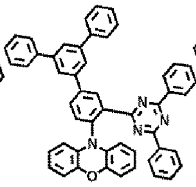
41

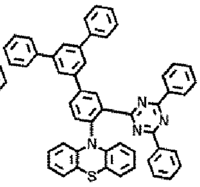
42

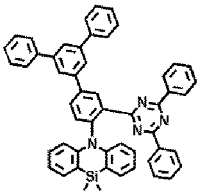
43

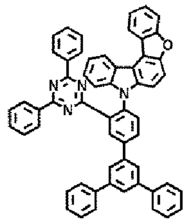
44

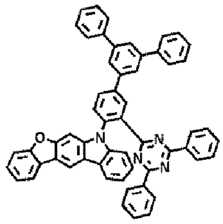
45

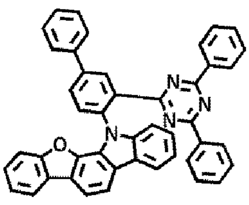
46

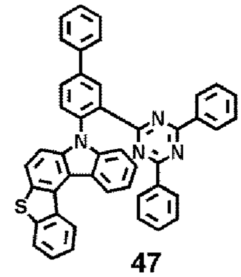
47

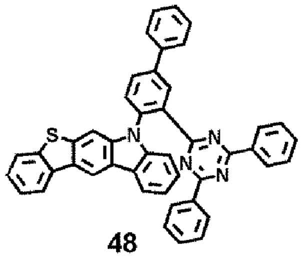
48

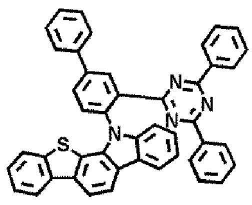
49

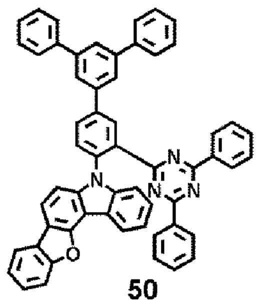
50

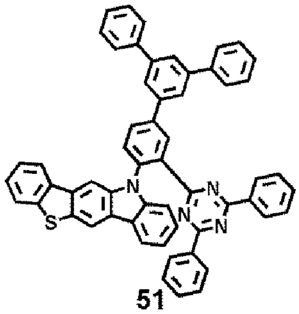
51

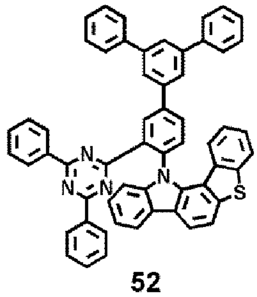
52

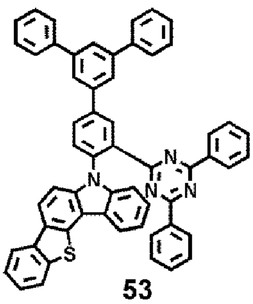
53

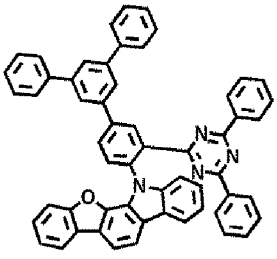
54

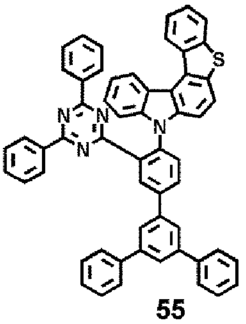
55

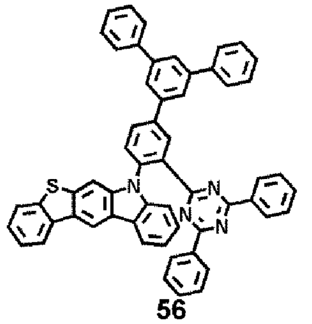
56

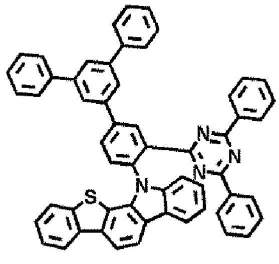
57

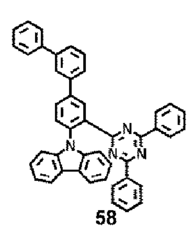
58

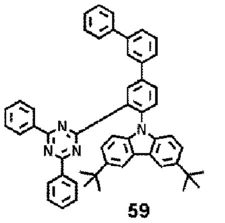
59

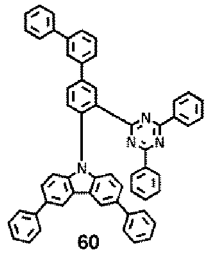
60

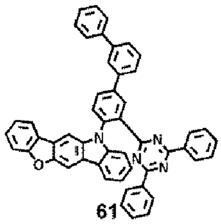
61

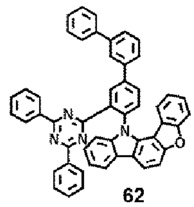
62

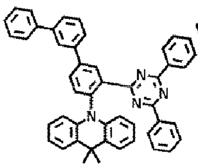
63

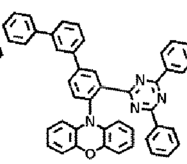
64

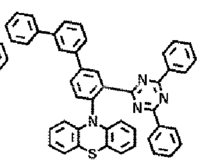
65

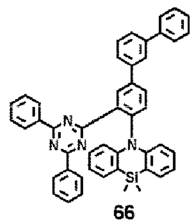
66

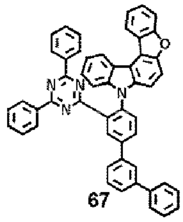
67

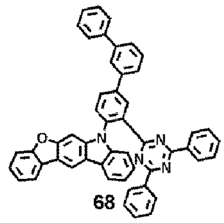
68

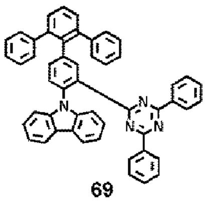
69

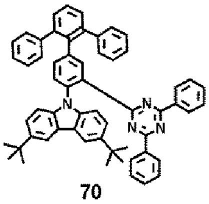
70

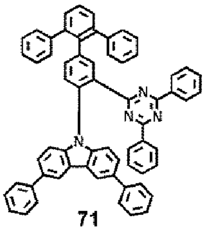
71

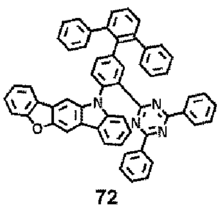
72

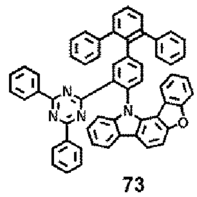
73

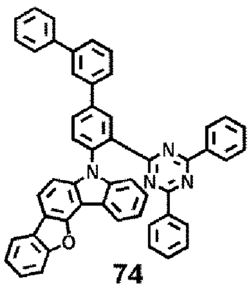
74

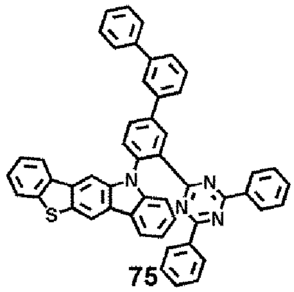
75

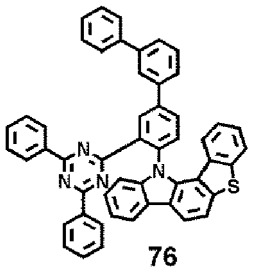
76

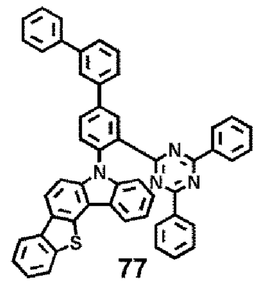
77

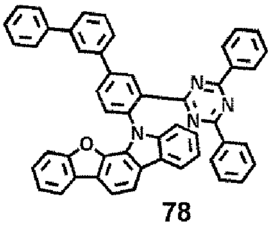
78

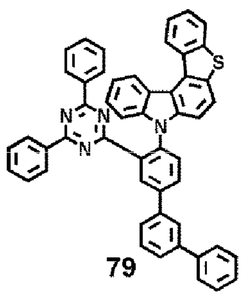
79

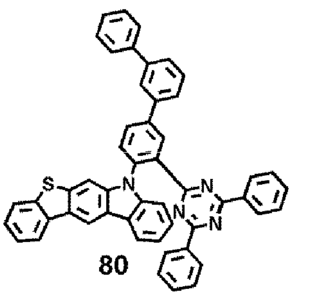
80

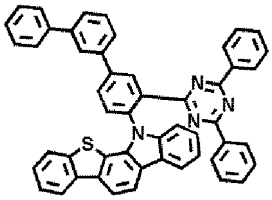
81

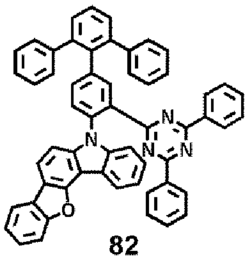
82

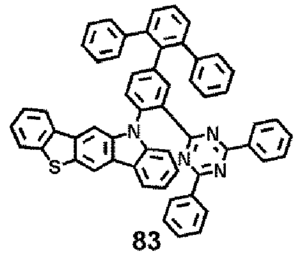
83

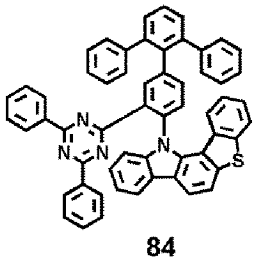
84

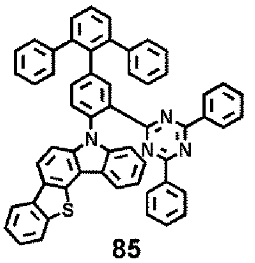
85

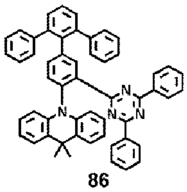
86
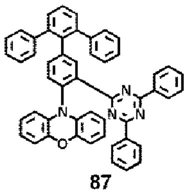
87
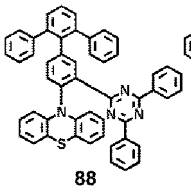
88
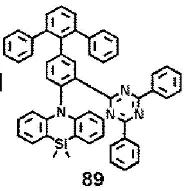
89
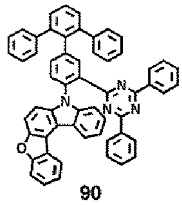
90
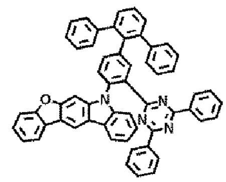
91

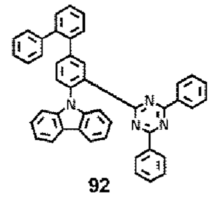
92
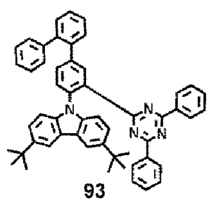
93
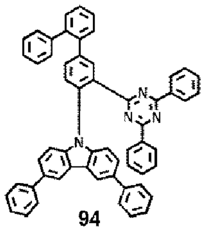
94
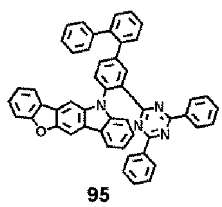
95
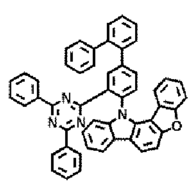
96

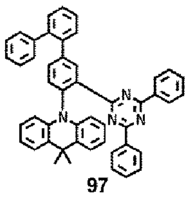
97
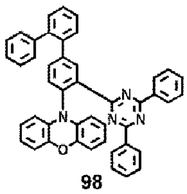
98
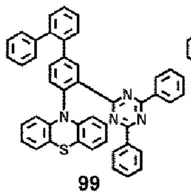
99
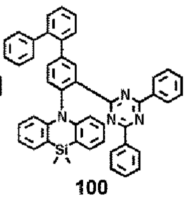
100
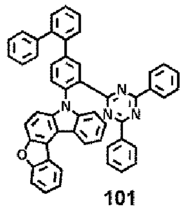
101
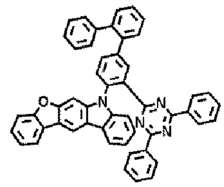
102

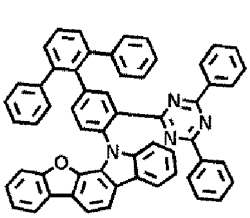
103
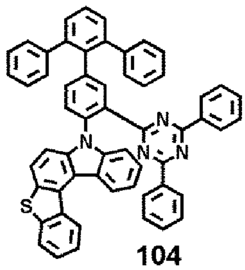
104
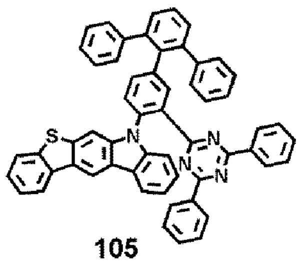
105
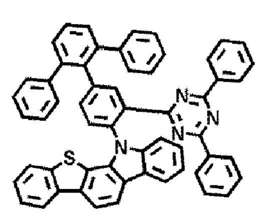
106

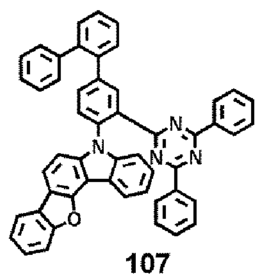
107
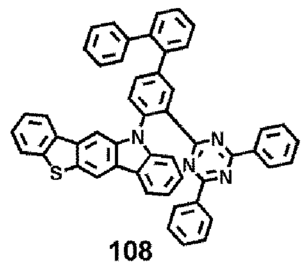
108
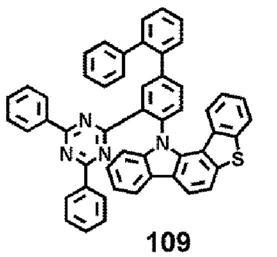
109
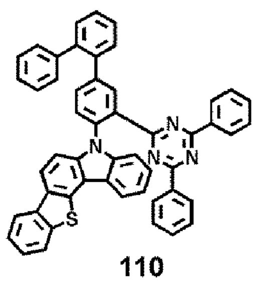
110

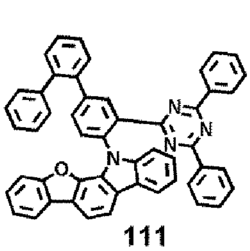
111
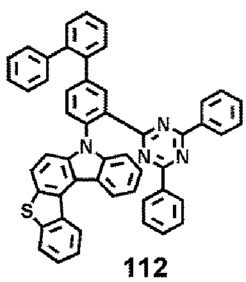
112
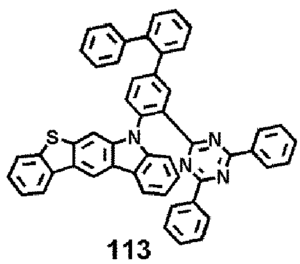
113
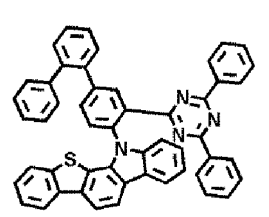
114

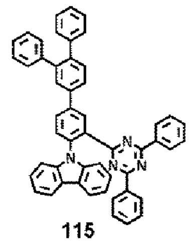
115
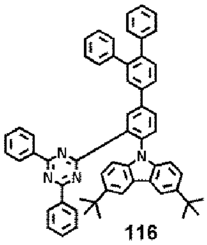
116
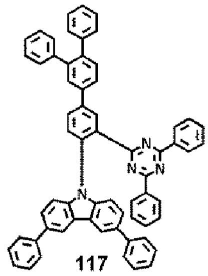
117
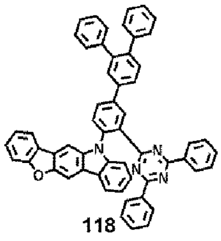
118
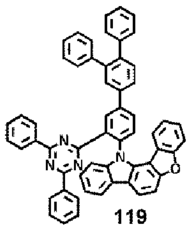
119

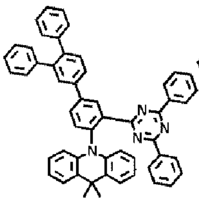
120
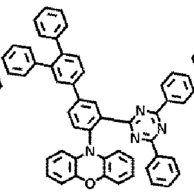
121
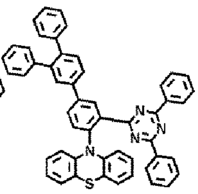
122
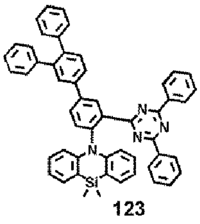
123
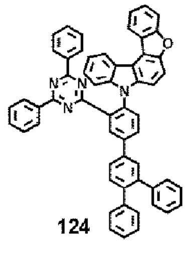
124
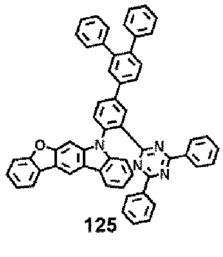
125

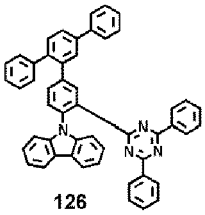
126
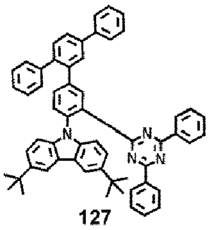
127
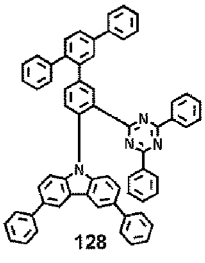
128
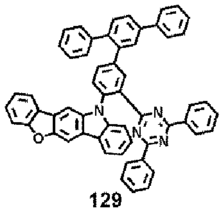
129
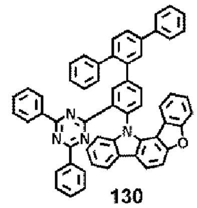
130

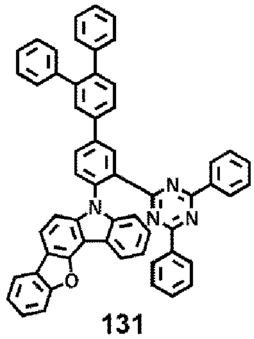
131
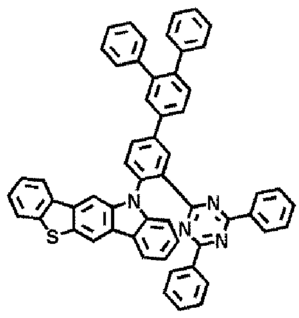
132
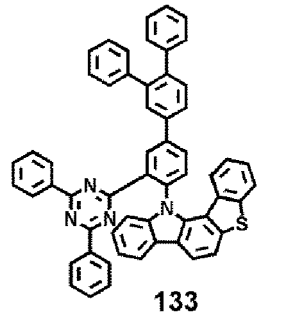
133
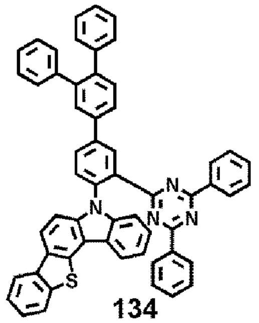
134

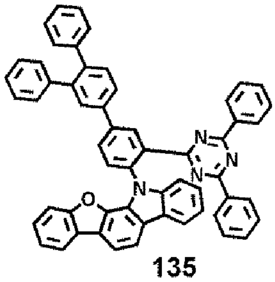
135
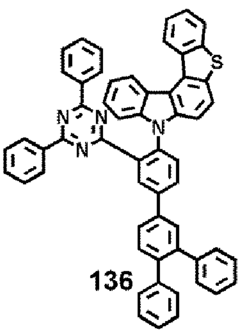
136
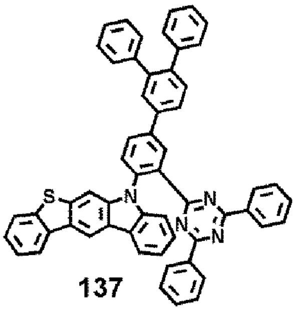
137
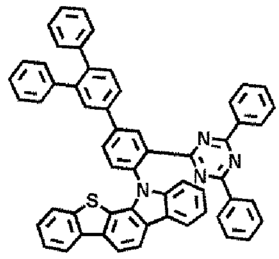
138

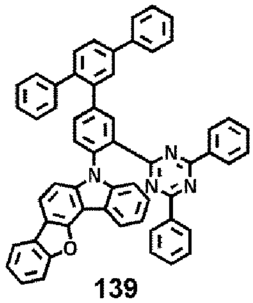
139

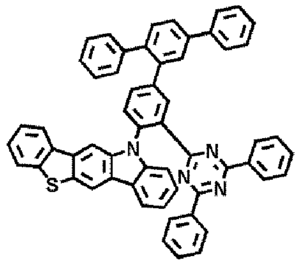
140

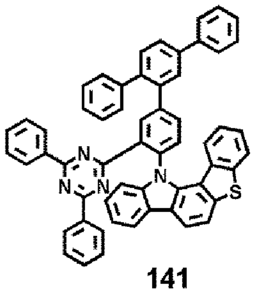
141

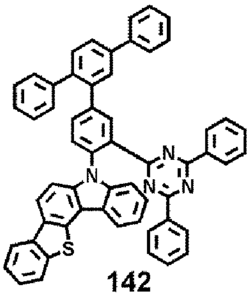
142

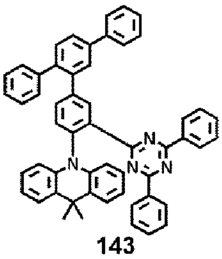
143

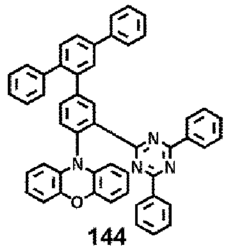
144

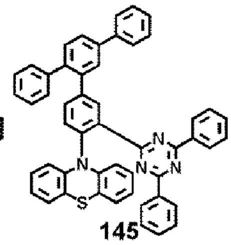
145

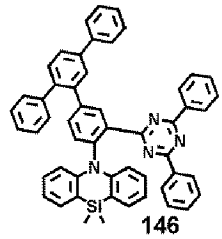
146

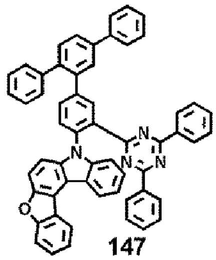
147

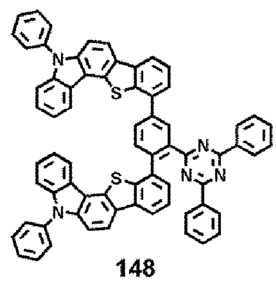
148

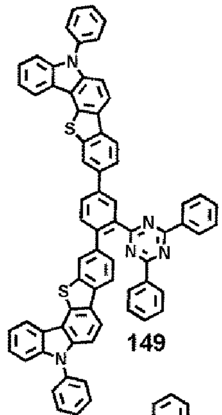
149

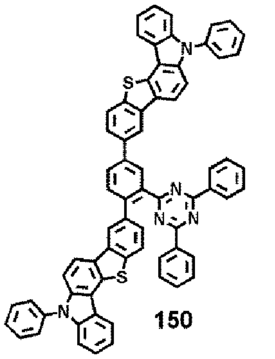
150

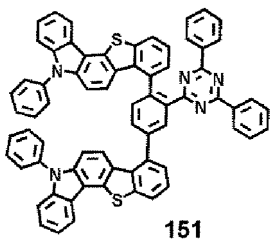
151

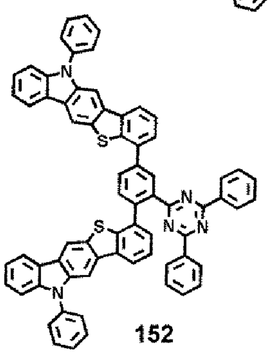
152

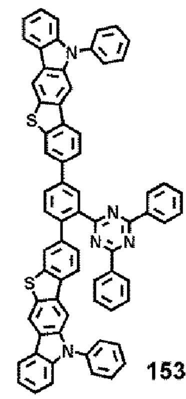
153

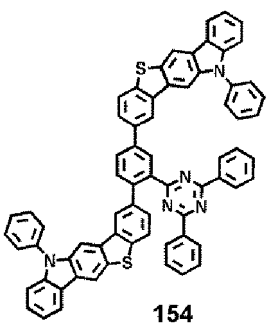
154

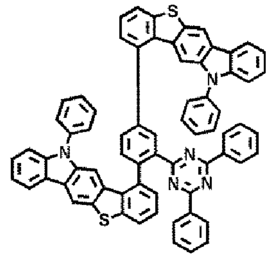
155

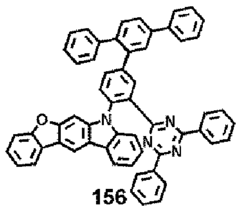
156

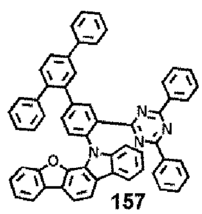
157

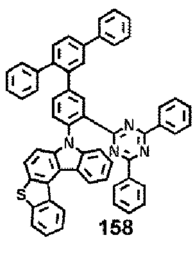
158

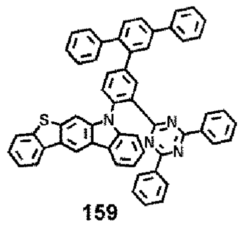
159

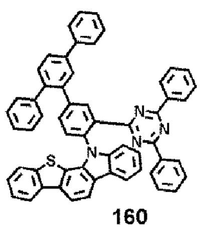
160

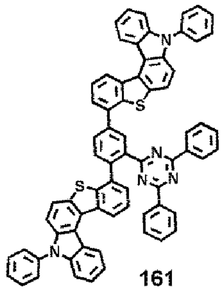
161

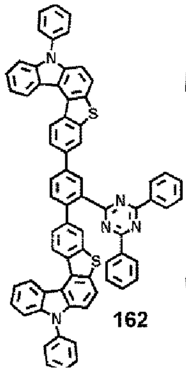
162

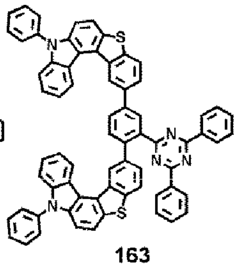
163

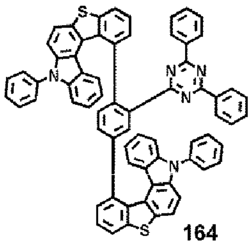
164

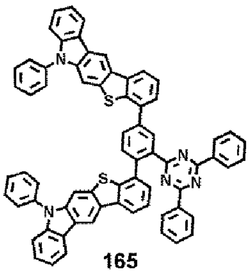
165

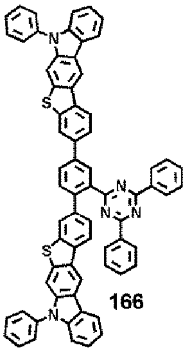
166

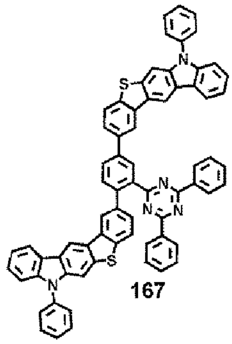
167

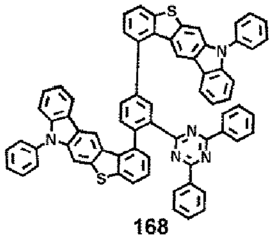
168

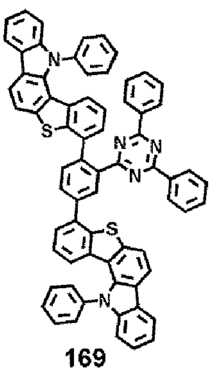
169

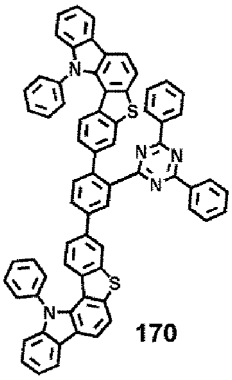
170

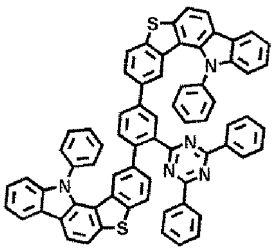
171

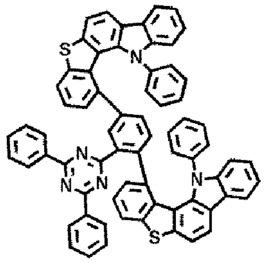
172

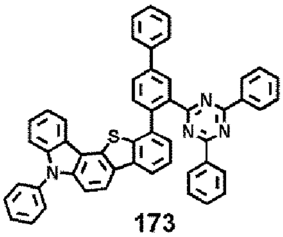
173

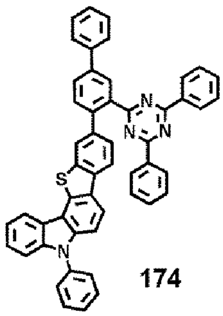
174

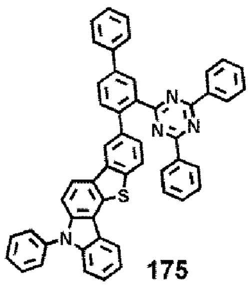
175

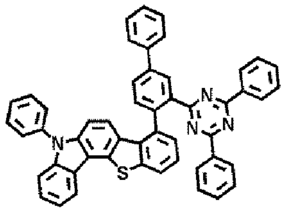
176

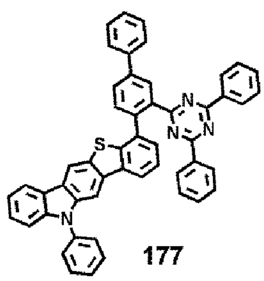
177

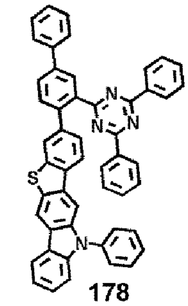
178

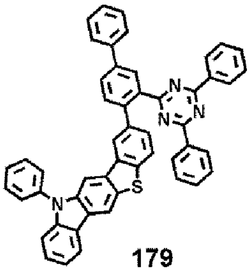
179

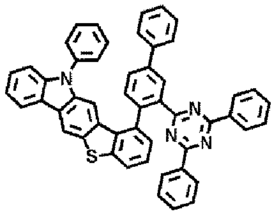
180

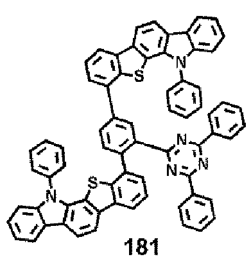
181

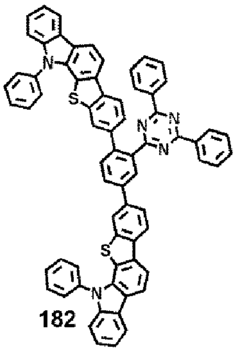
182

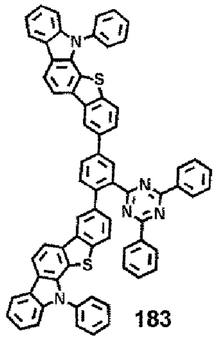
183

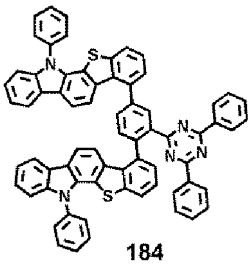
184

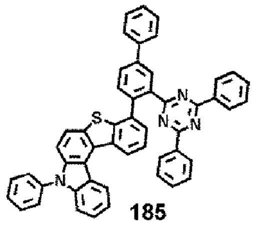
185

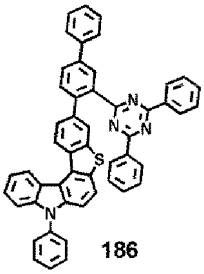
186

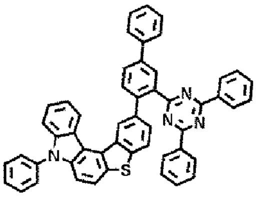
187

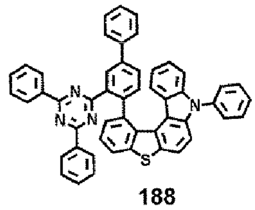
188

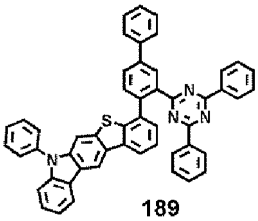
189

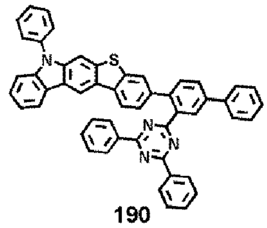
190

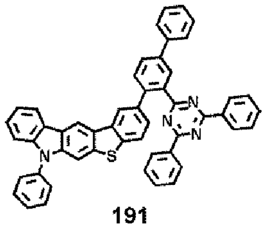
191

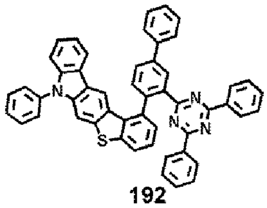
192

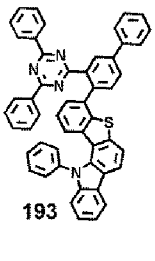
193

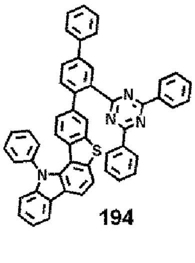
194

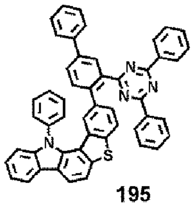
195

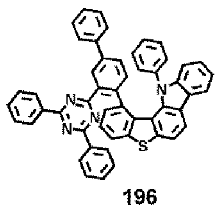
196

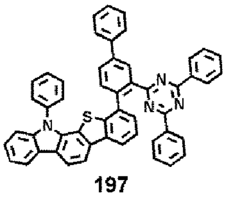
197

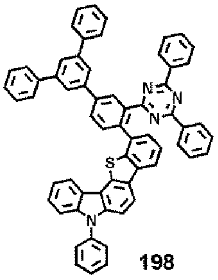
198

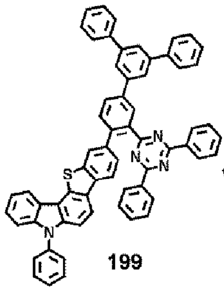
199

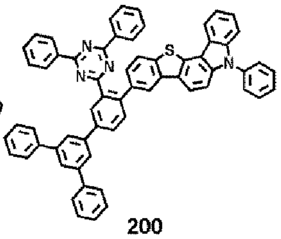
200

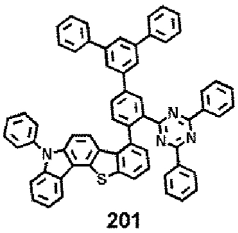
201

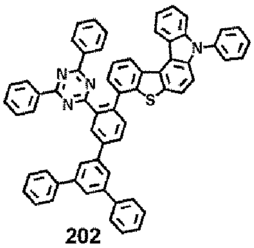
202

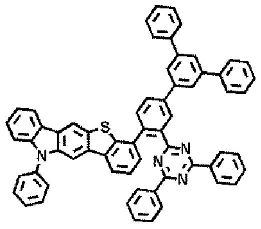
203

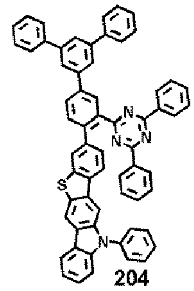
204

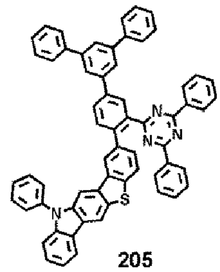
205

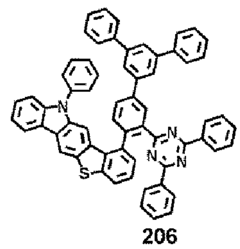
206

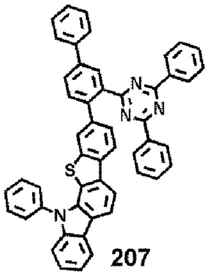
207

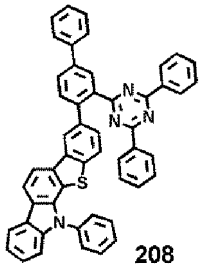
208

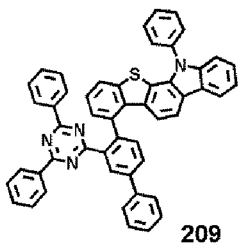
209

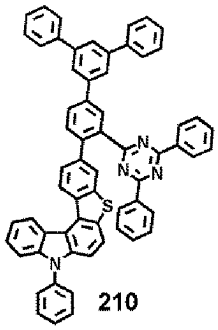
210

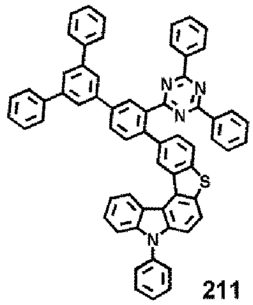
211

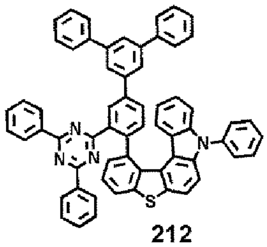
212

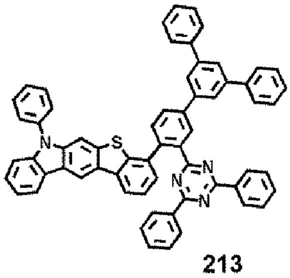
213

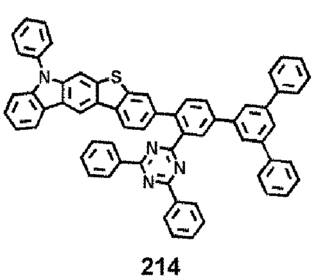
214

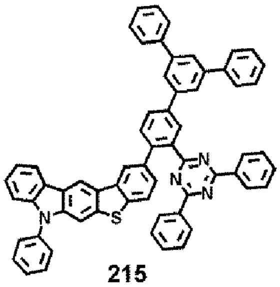
215

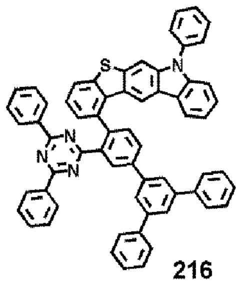
216

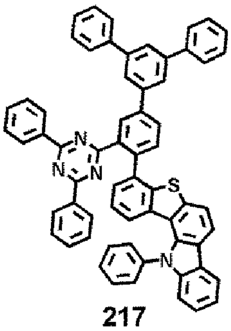
217

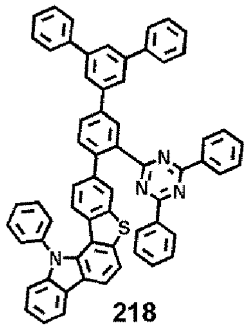
218

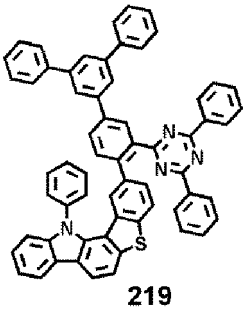
219

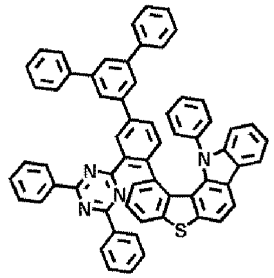
220

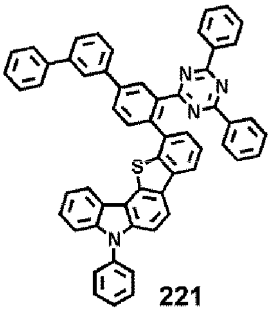
221

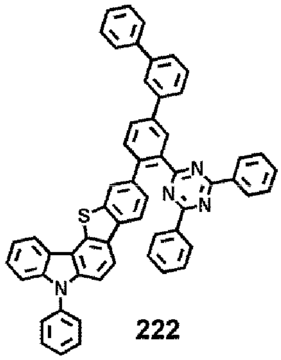
222

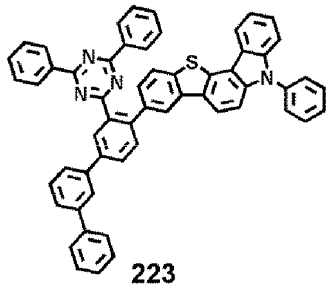
223

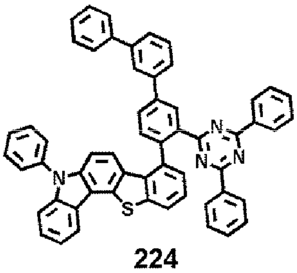
224

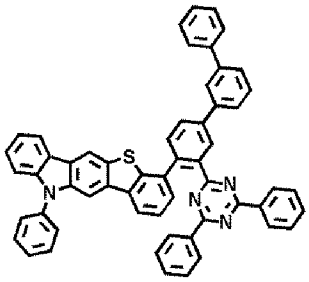
225

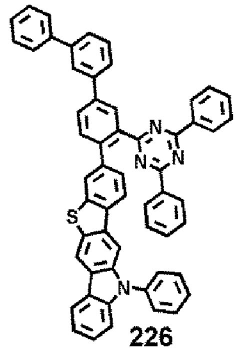
226

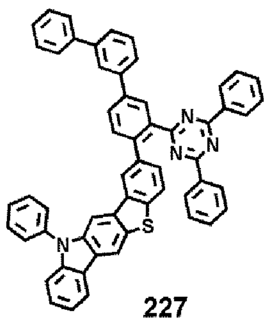
227

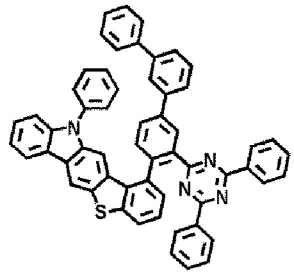
228

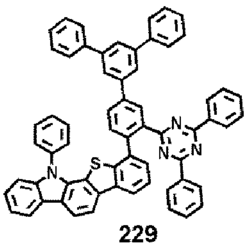
229

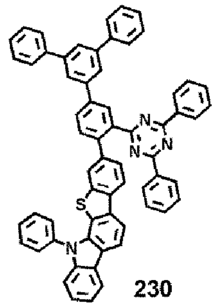
230

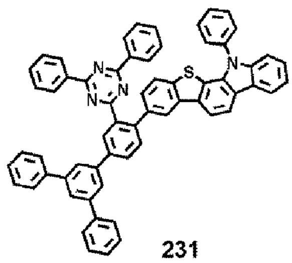
231

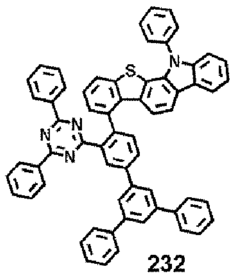
232

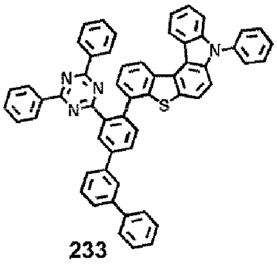
233

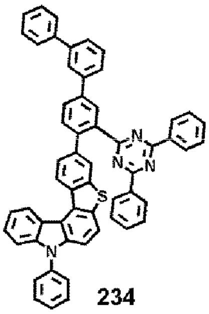
234

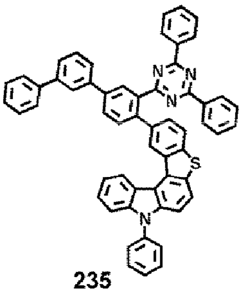
235

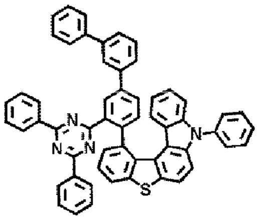
236

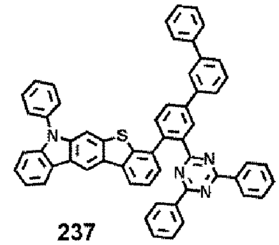
237

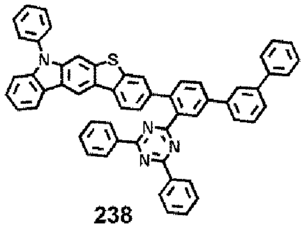
238

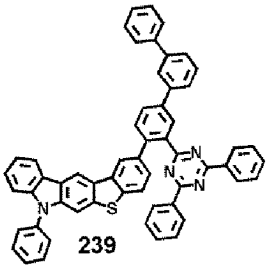
239

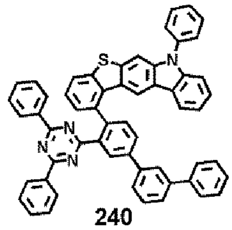
240

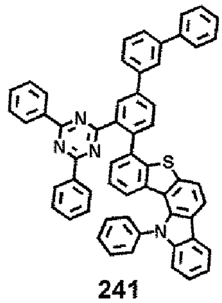
241

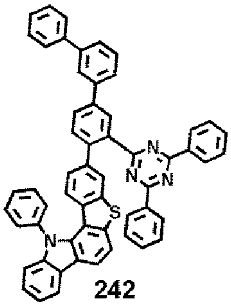
242

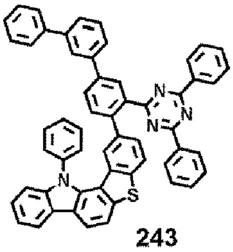
243

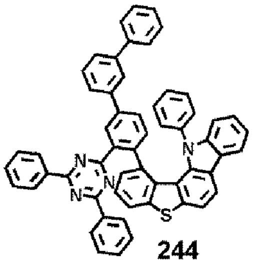
244

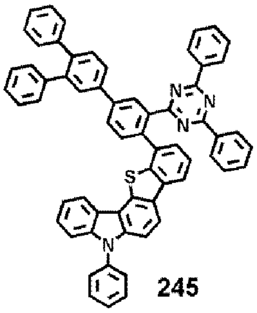
245

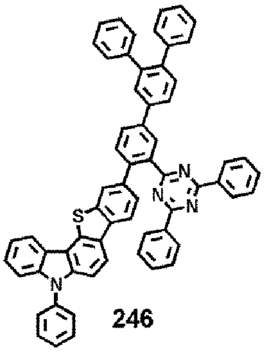
246

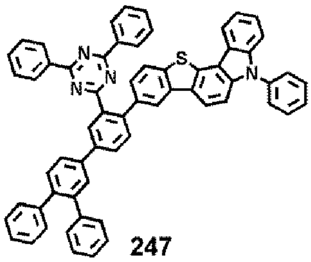
247

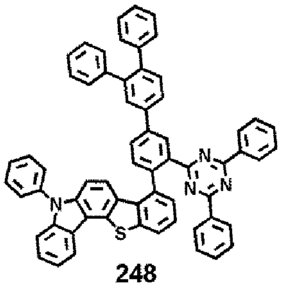
248

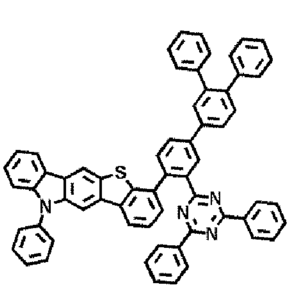
249

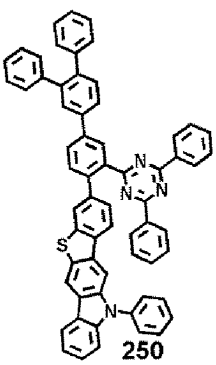
250

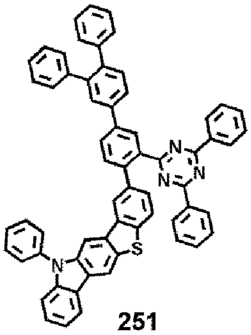
251

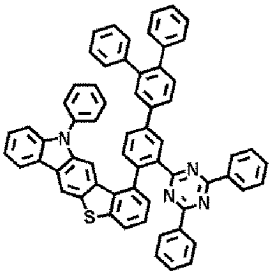
252

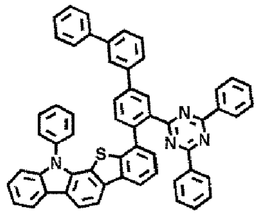
253

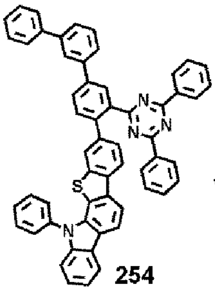
254

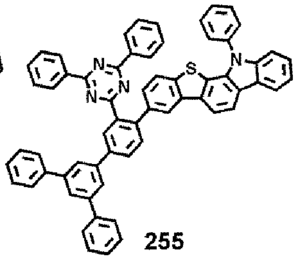
255

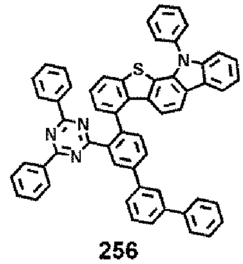
256

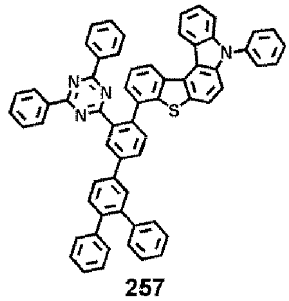
257

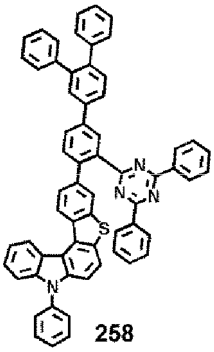
258

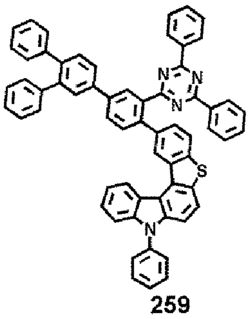
259

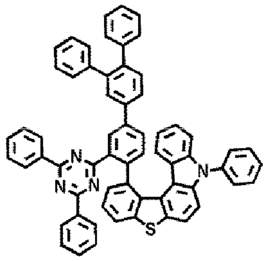
260

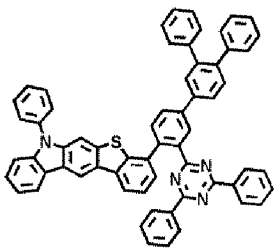
261

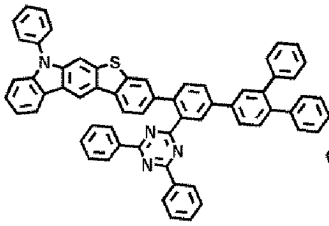
262

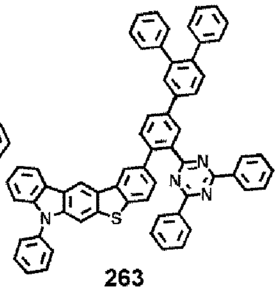
263

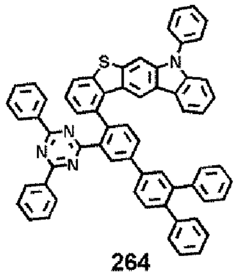
264

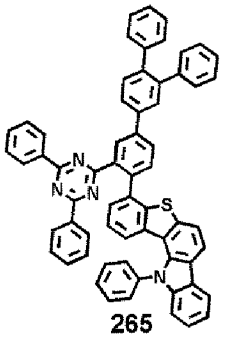
265

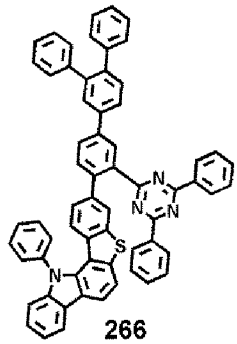
266

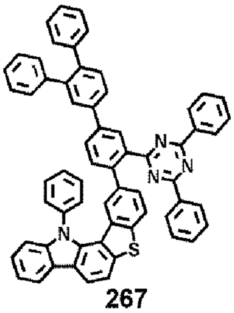
267

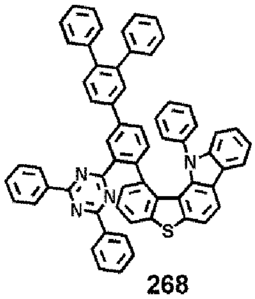
268

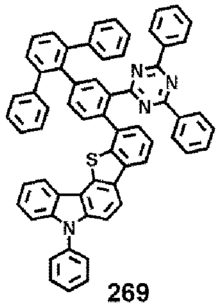
269

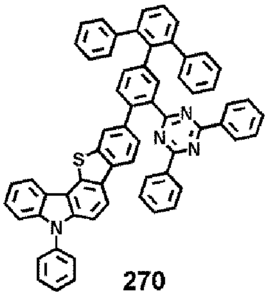
270

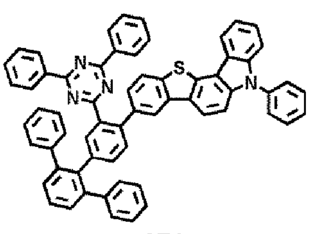
271

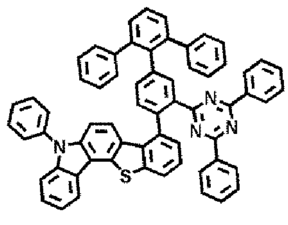
272

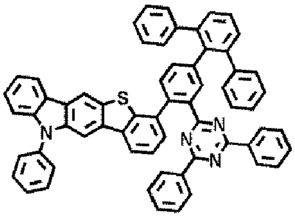
273

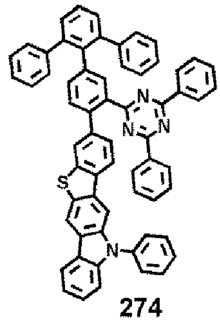
274

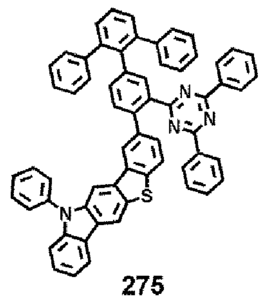
275

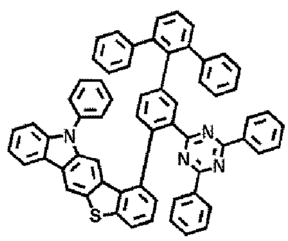
276

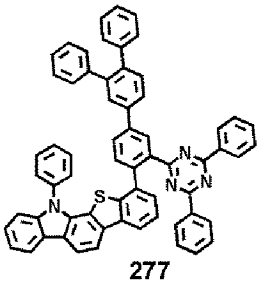
277

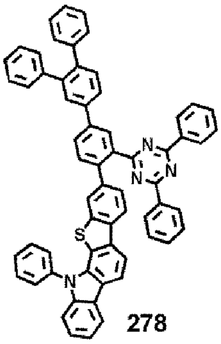
278

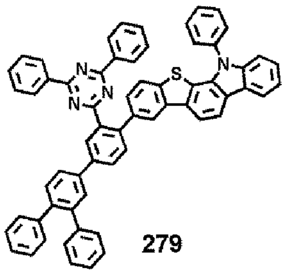
279

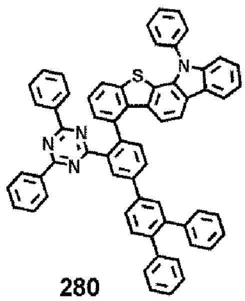
280

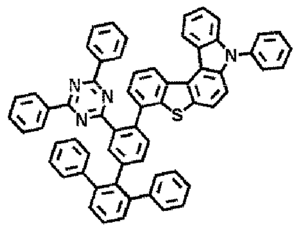
281

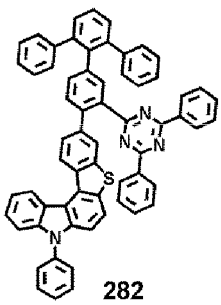
282

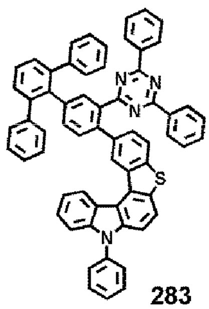
283

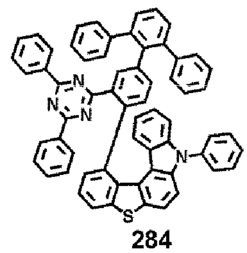
284

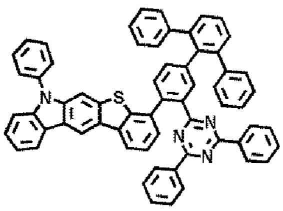
285

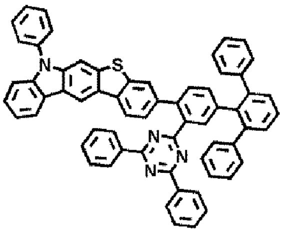
286

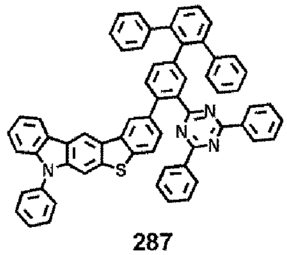
287

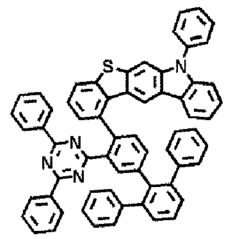
288

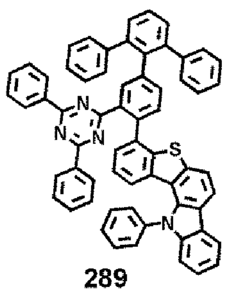
289

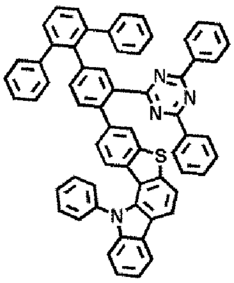
290

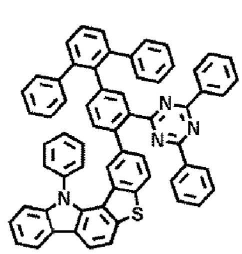
291

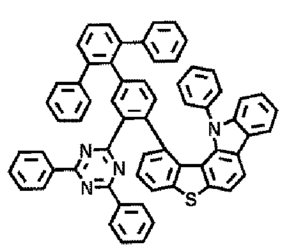
292

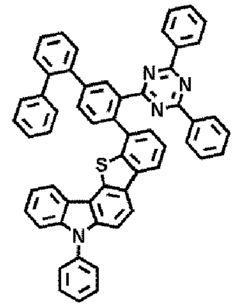
293

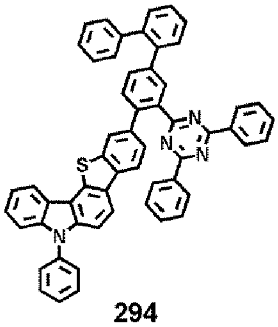
294

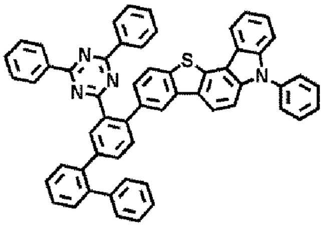
295

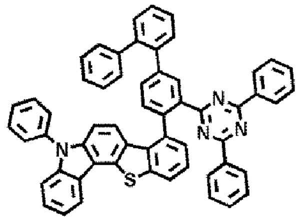
296

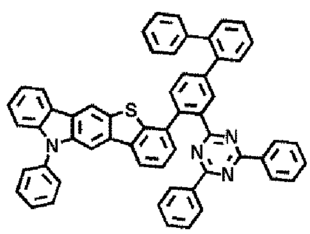
297

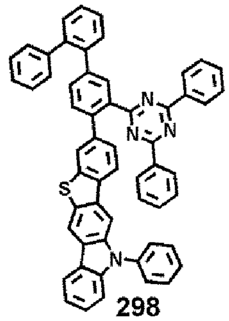
298

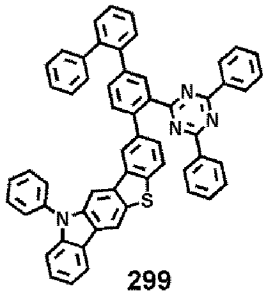
299

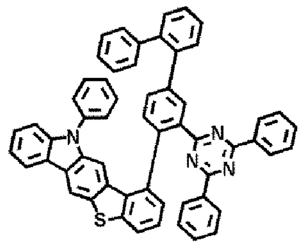
300

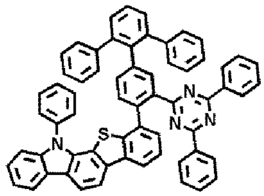
301

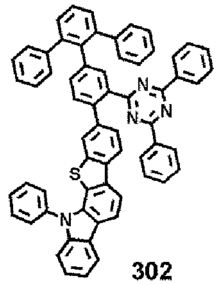
302

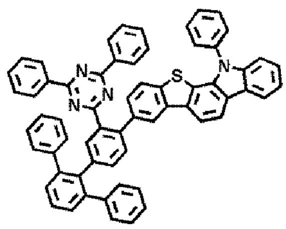
303

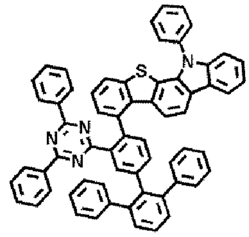
304

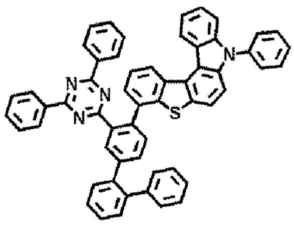
305

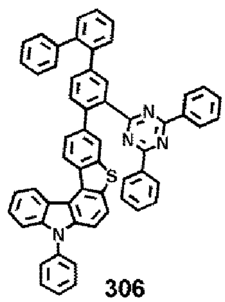
306

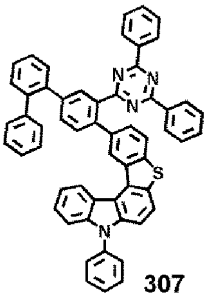
307

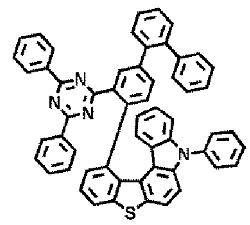
308

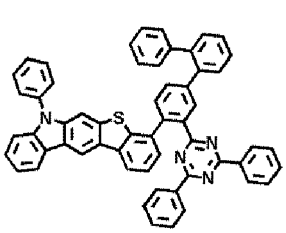
309

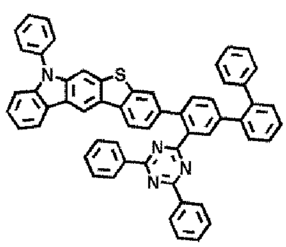
310

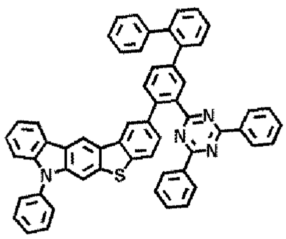
311

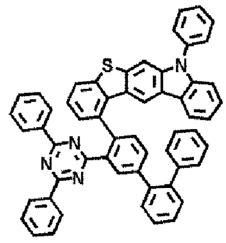
312

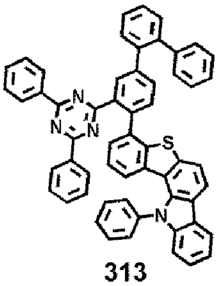
313

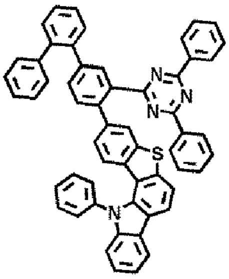
314

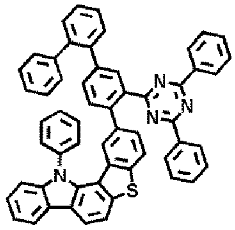
315

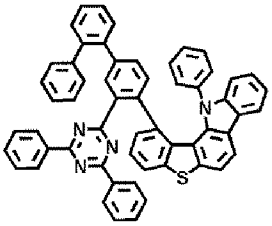
316

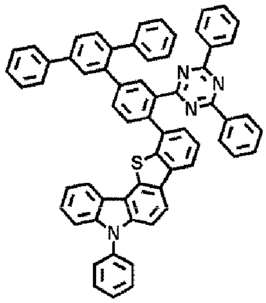
317

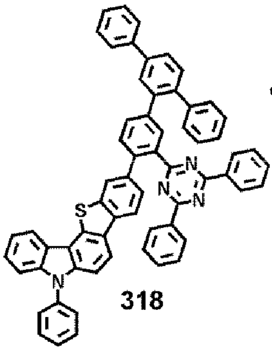
318

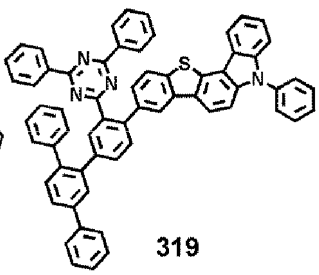
319

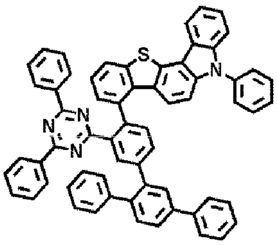
320

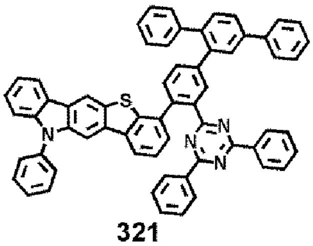
321

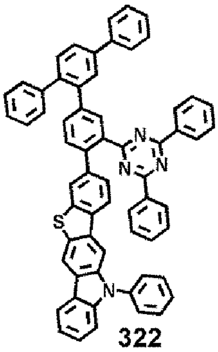
322

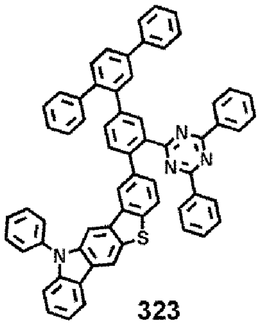
323

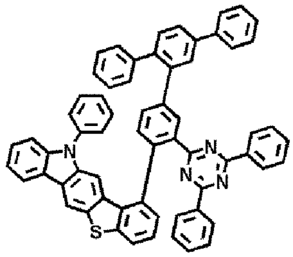
324

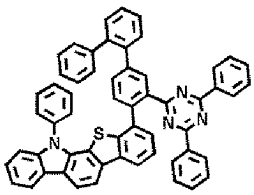
325

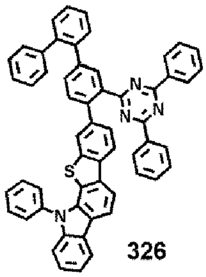
326

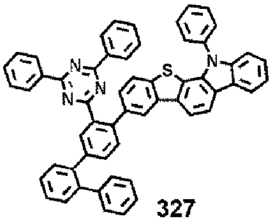
327

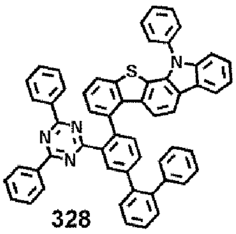
328

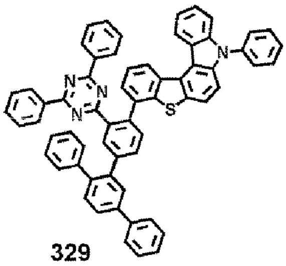
329

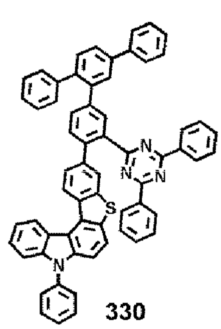
330

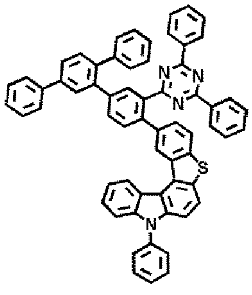
331

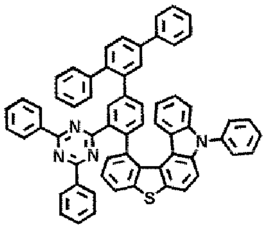
332

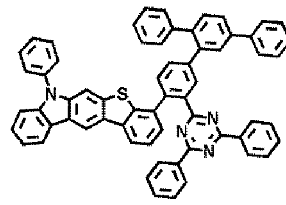
333

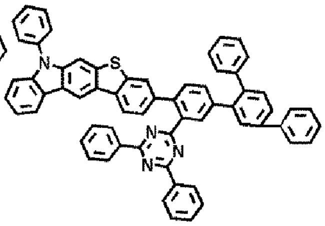
334

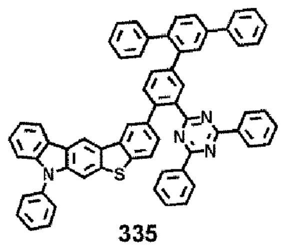
335

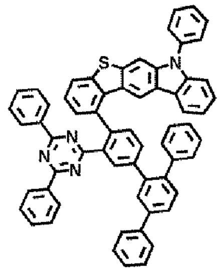
336

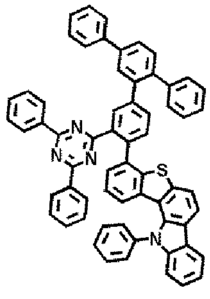
337

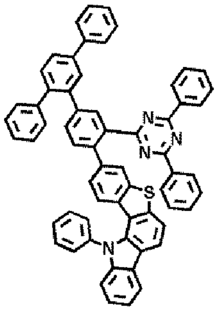
338

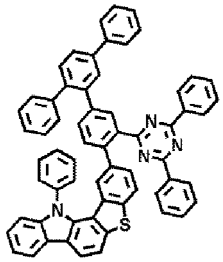
339

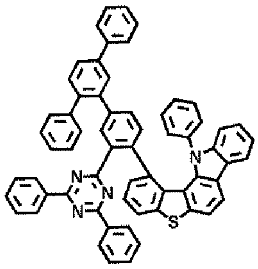
340

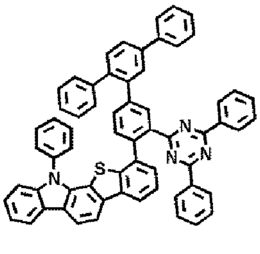
341

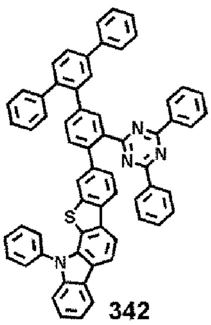
342

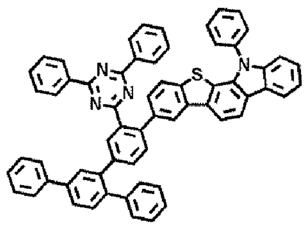
343

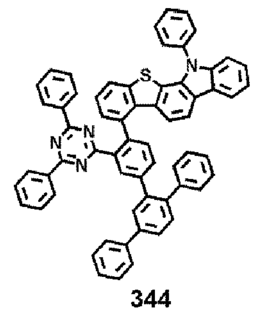
344

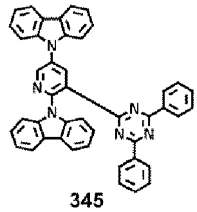
345

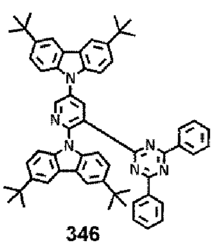
346

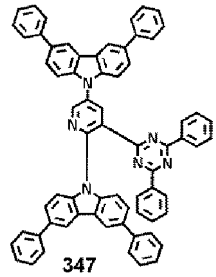
347

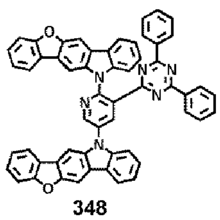
348

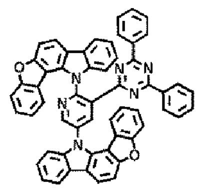
349

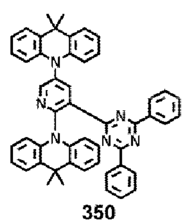
350

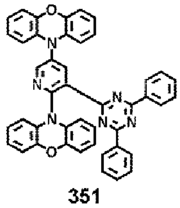
351

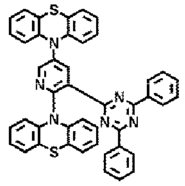
352

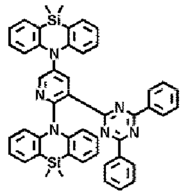
353

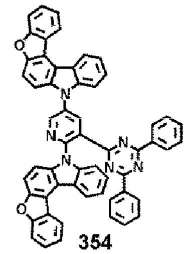
354

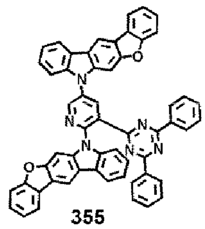
355

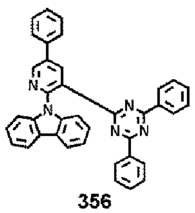
356

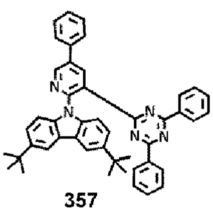
357

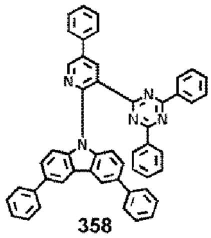
358

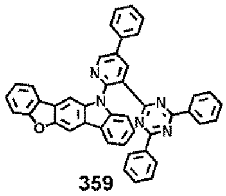
359

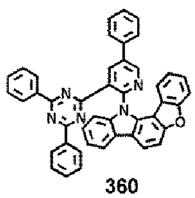
360

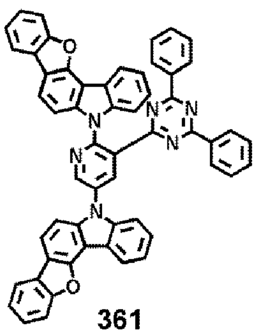
361

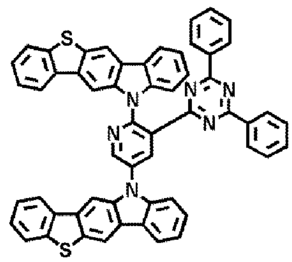
362

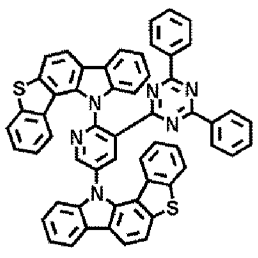
363

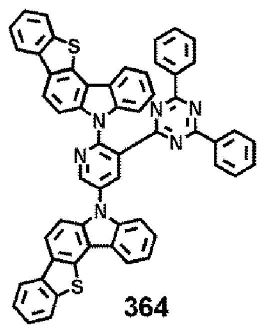
364

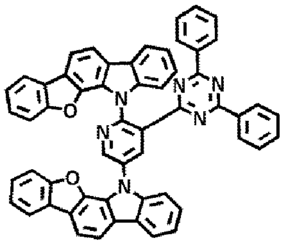
365

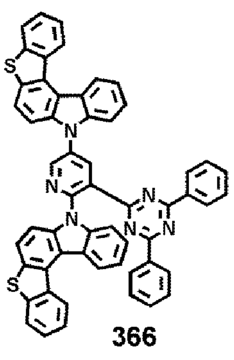
366

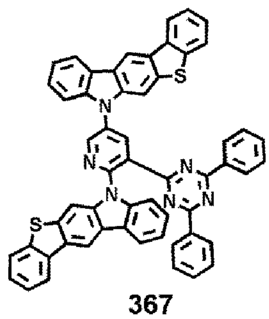
367

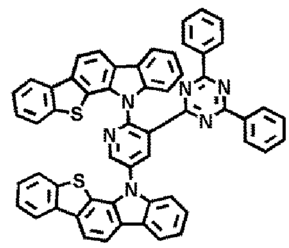
368

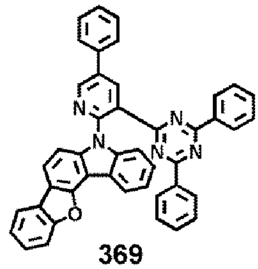
369

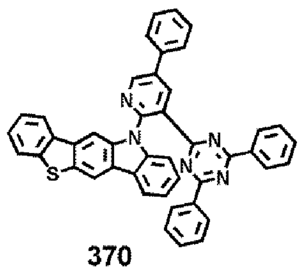
370

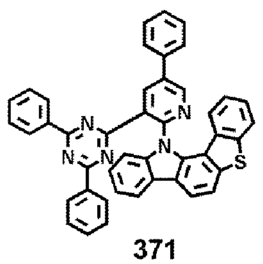
371

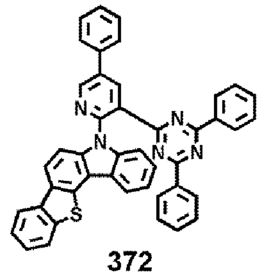
372

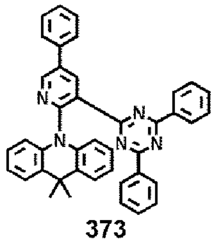
373

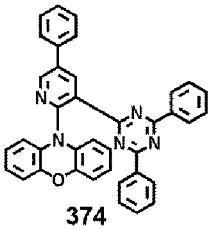
374

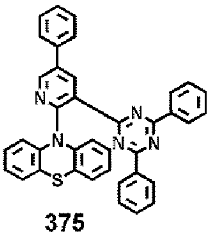
375

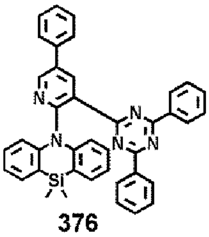
376

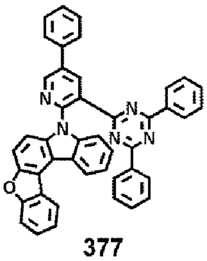
377

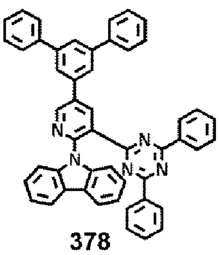
378

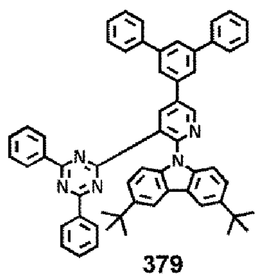
379

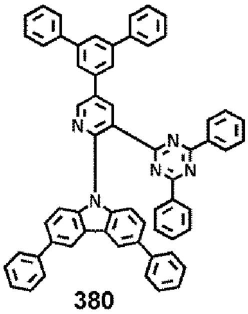
380

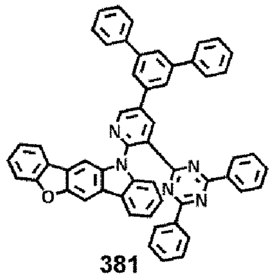
381

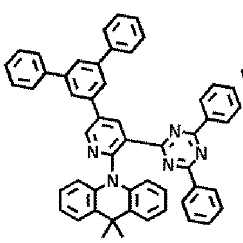
382

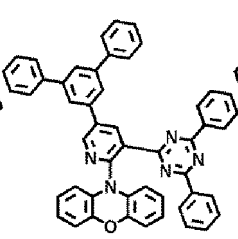
383

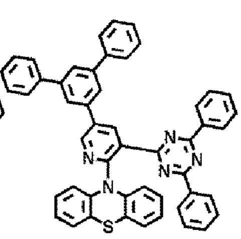
384

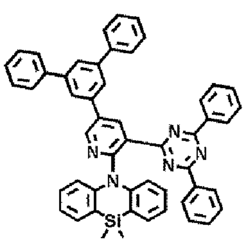
385

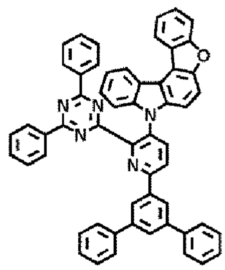
386

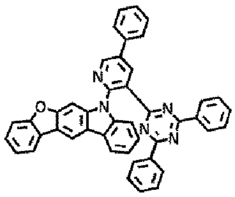
387

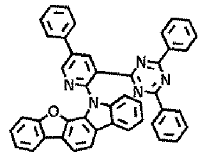
388

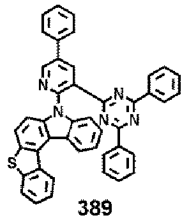
389

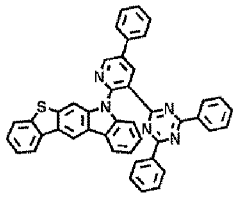
390

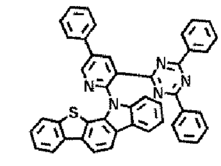
391

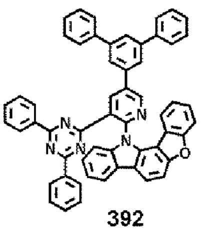
392

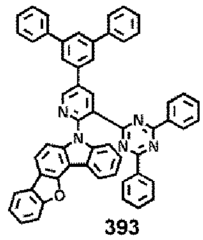
393

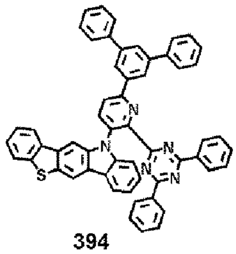
394

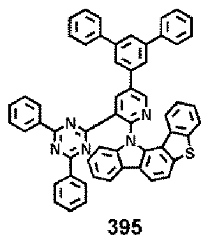
395

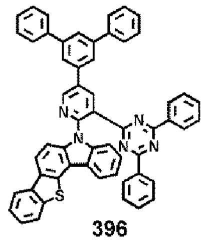
396

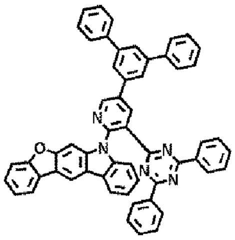
397

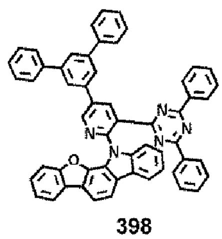
398

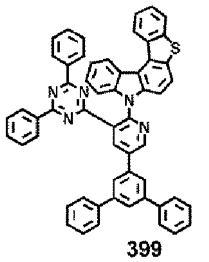
399

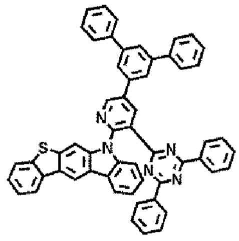
400

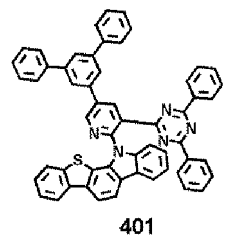
401

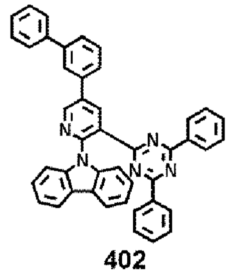
402

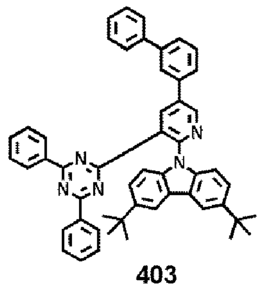
403

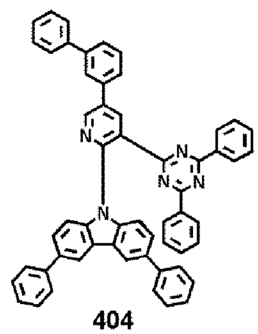
404

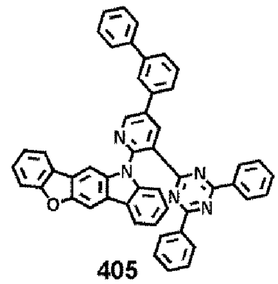
405

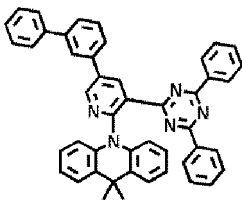
406

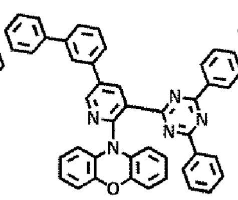
407

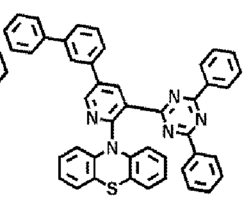
408

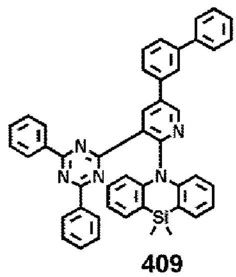
409

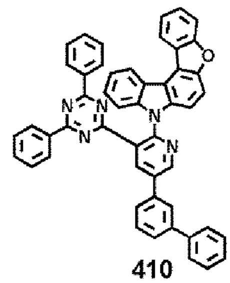
410

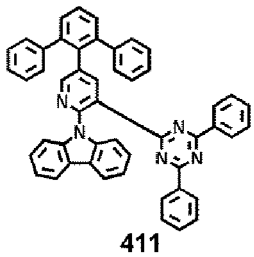
411

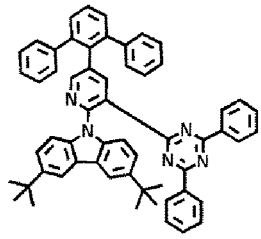
412

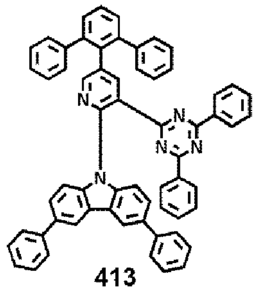
413

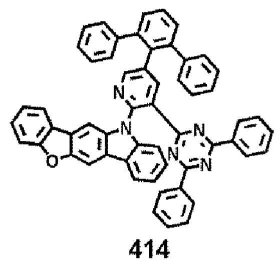
414

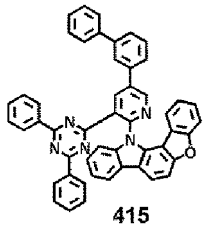
415

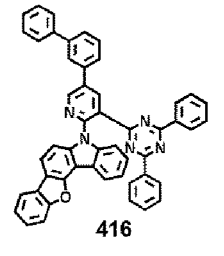
416

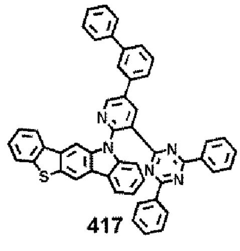
417

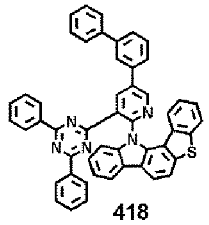
418

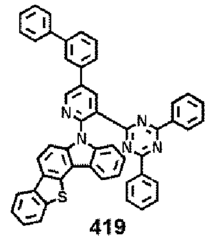
419

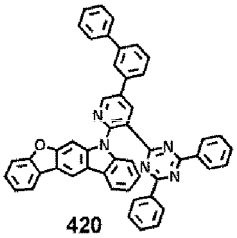
420

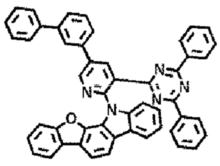
421

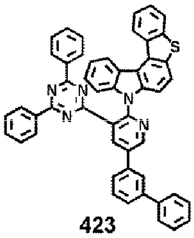
423

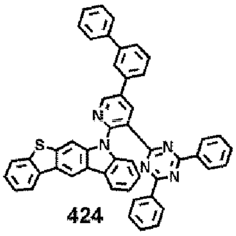
424

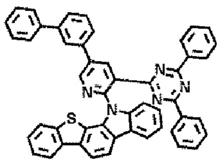
425

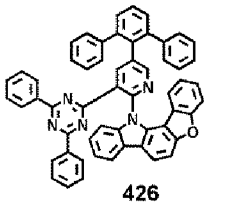
426

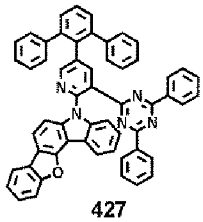
427

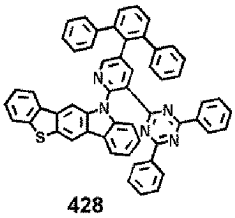
428

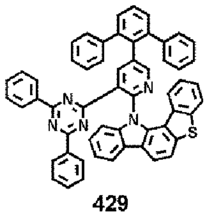
429

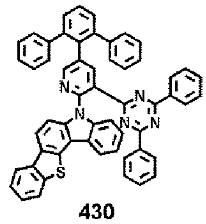
430

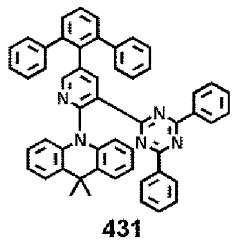
431

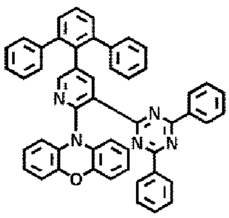
432

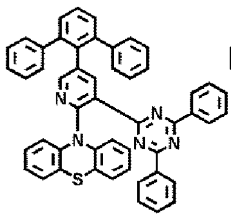
433

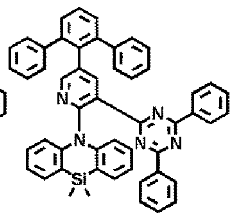
434

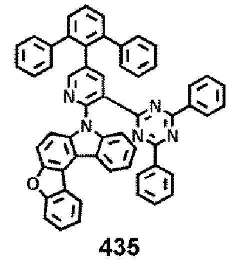
435

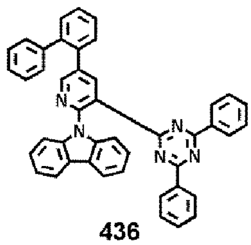
436

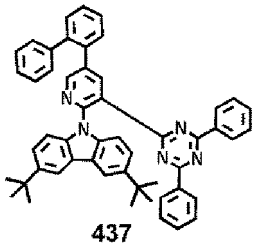
437

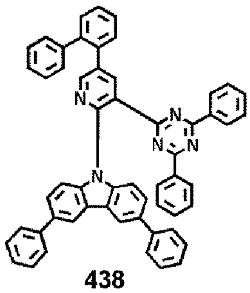
438

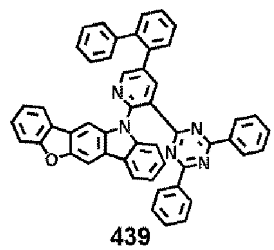
439

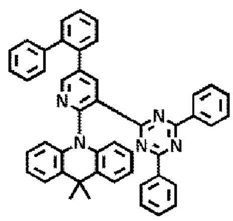
440

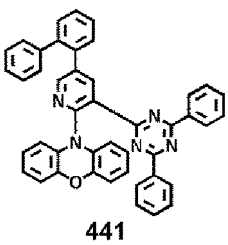
441

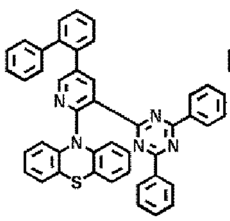
442

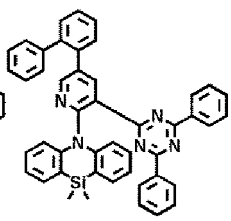
443

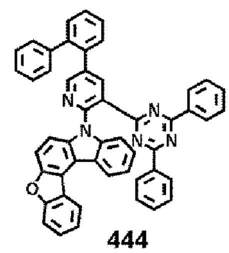
444

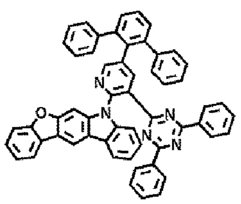
445

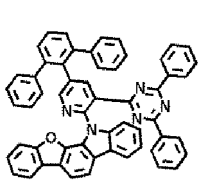
446

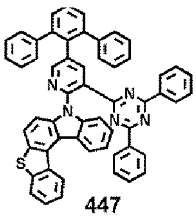
447

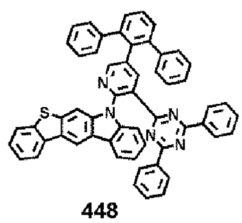
448

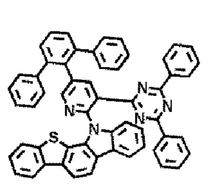
449

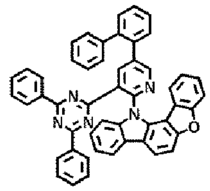
450
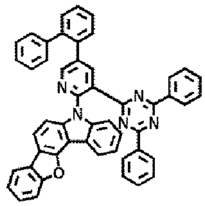
451
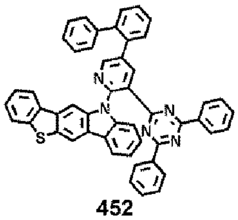
452
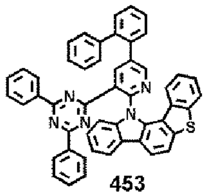
453
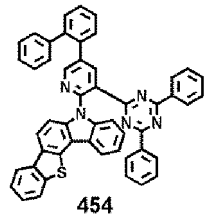
454

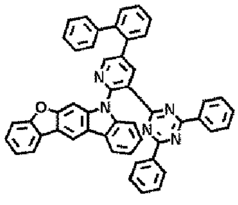
455
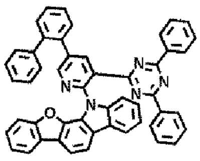
456
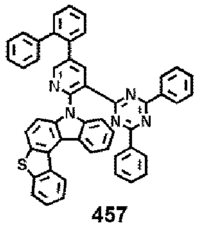
457
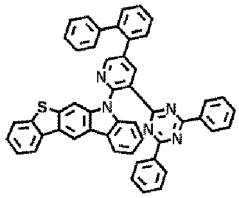
458
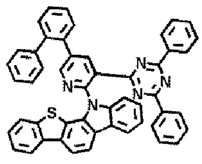
459

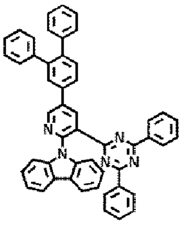
460
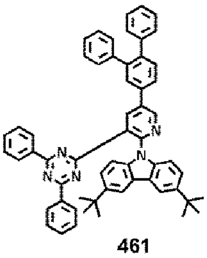
461
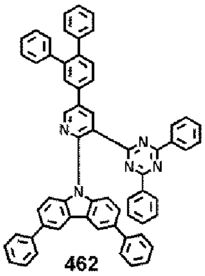
462
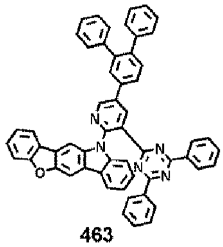
463
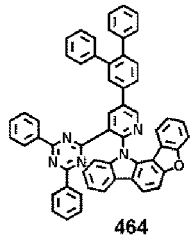
464

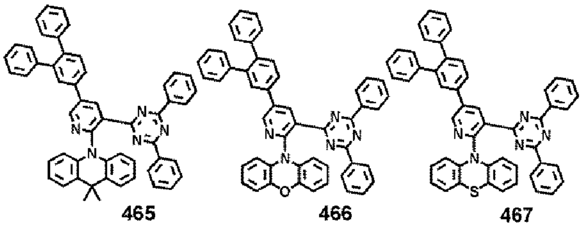
465
466
467
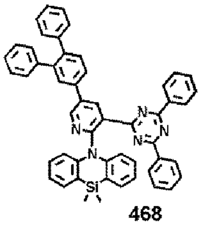
468
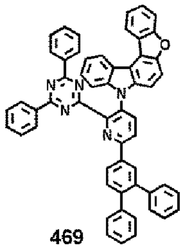
469
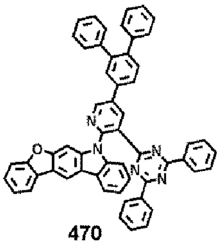
470

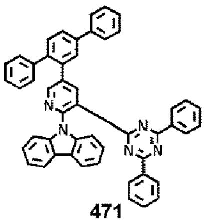
471
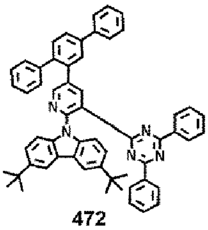
472
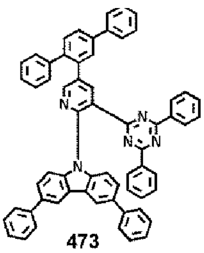
473
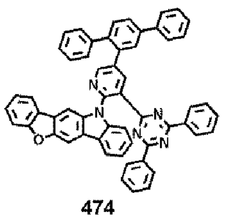
474
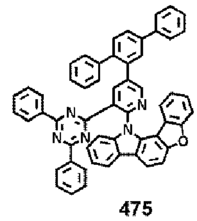
475

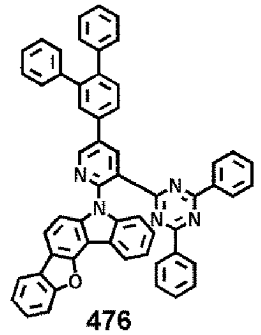
476
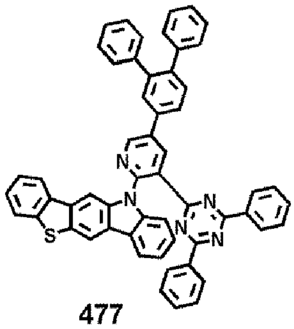
477
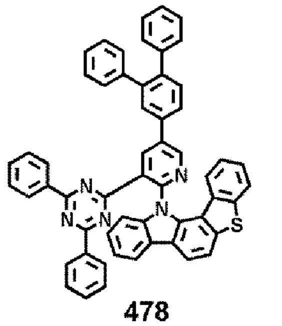
478
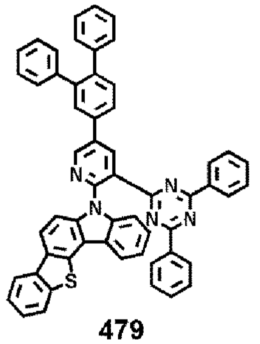
479

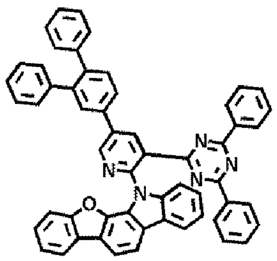
480
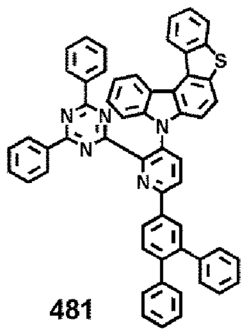
481
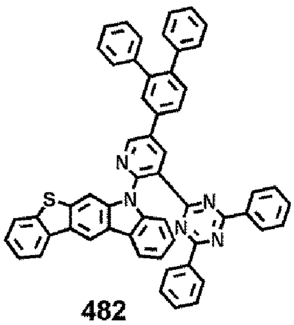
482
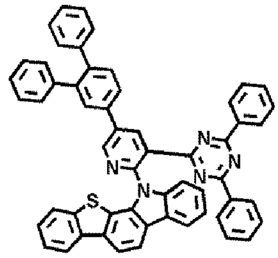
483

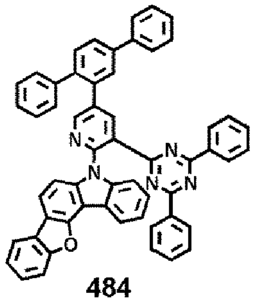
484
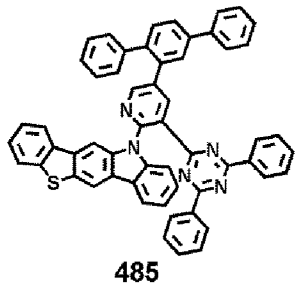
485
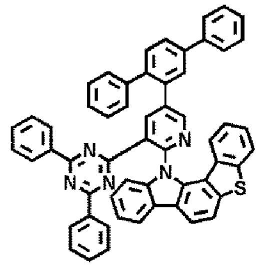
486
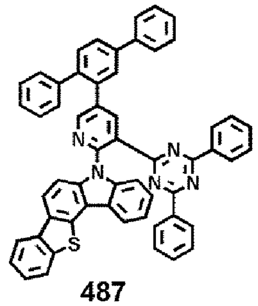
487

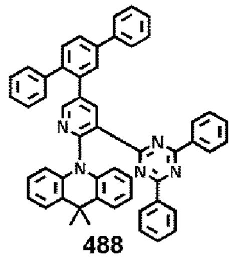
488
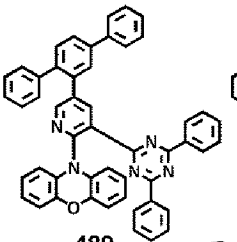
489
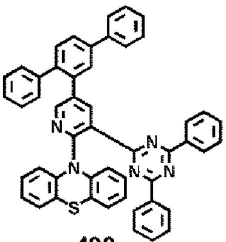
490
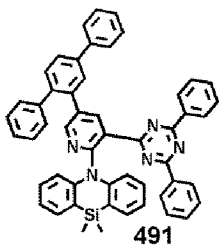
491
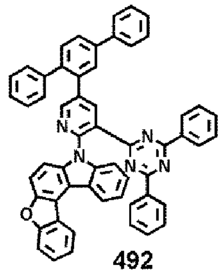
492

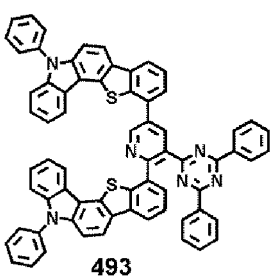
493
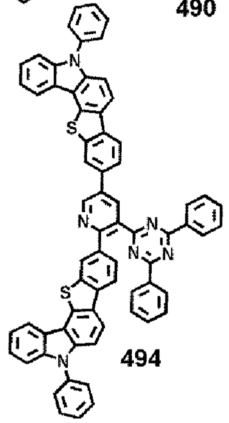
494
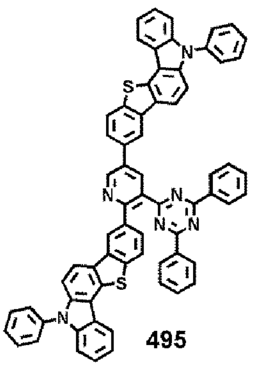
495
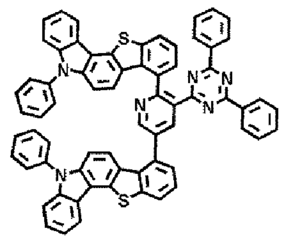
496

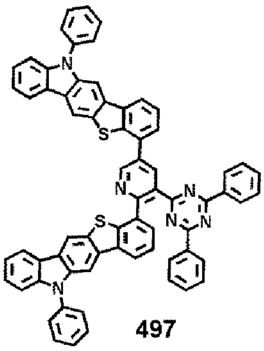
497

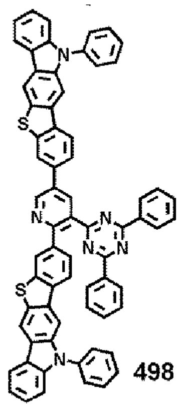
498

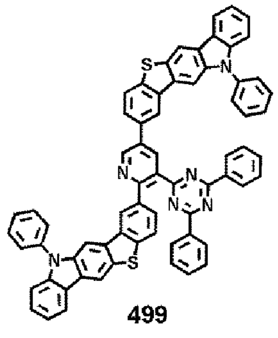
499

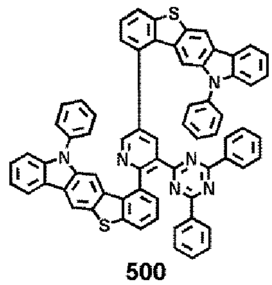
500

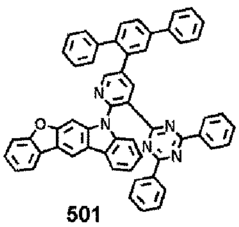
501

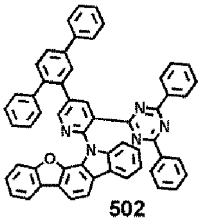
502

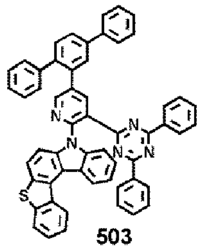
503

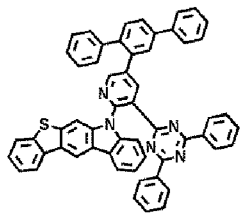
504

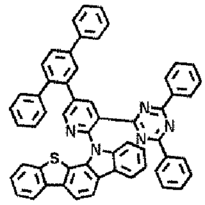
505

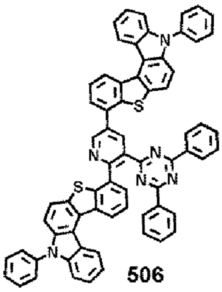
506

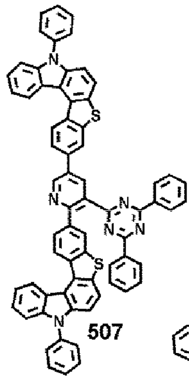
507

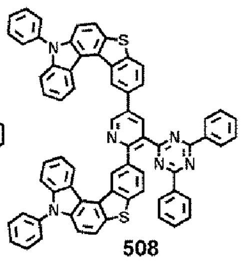
508

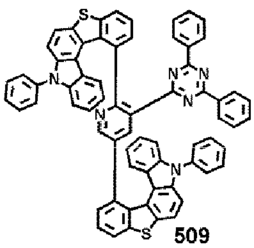
509

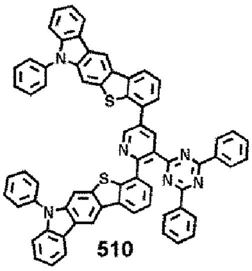
510

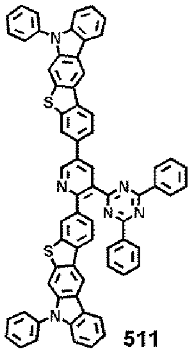
511

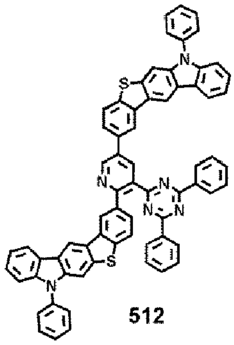
512

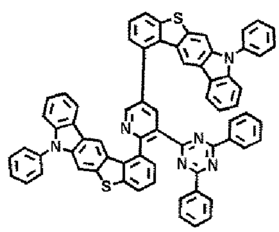
513

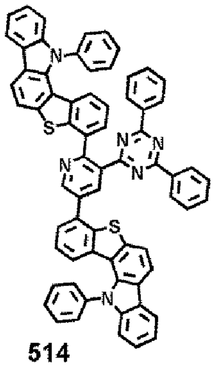
514

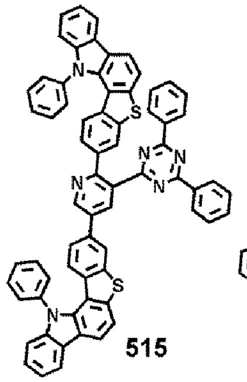
515

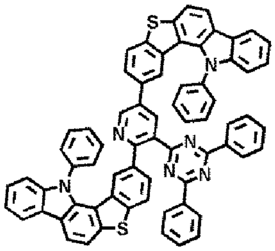
516

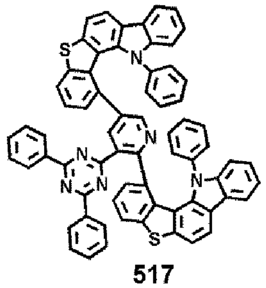
517

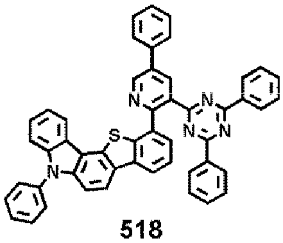
518

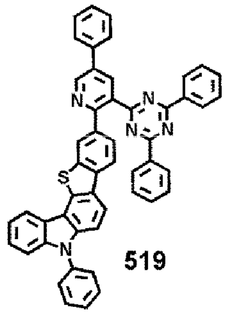
519

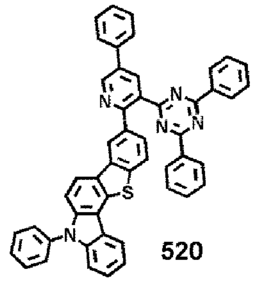
520

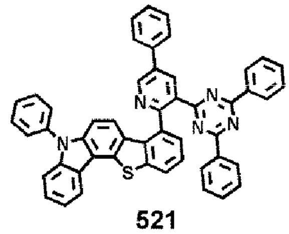
521

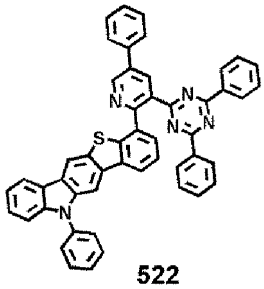
522

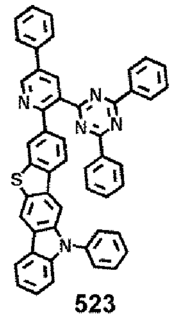
523

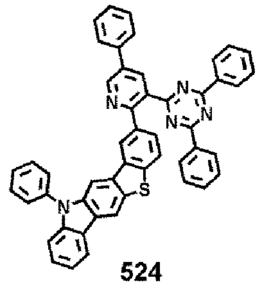
524

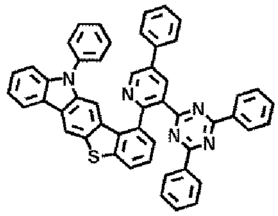
525

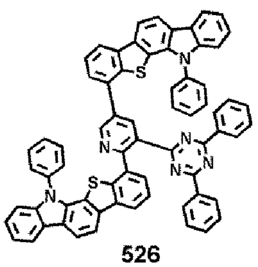
526

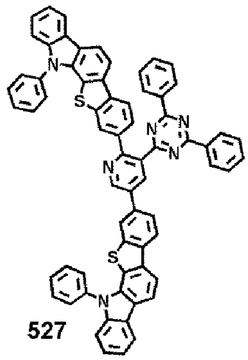
527

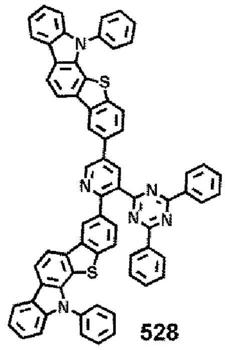
528

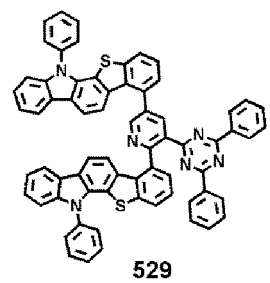
529

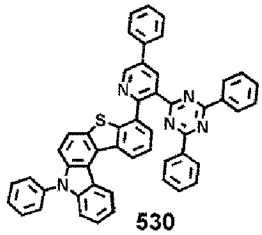
530

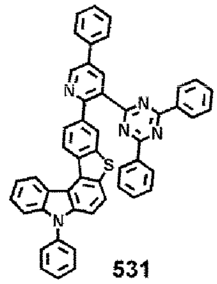
531

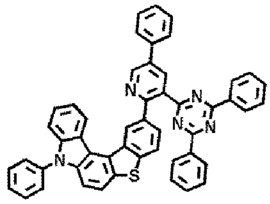
532

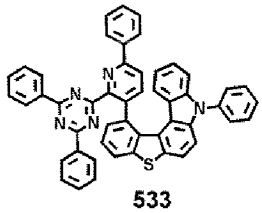
533

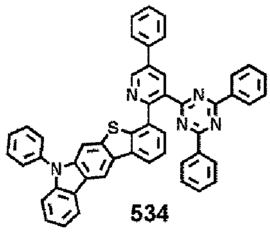
534

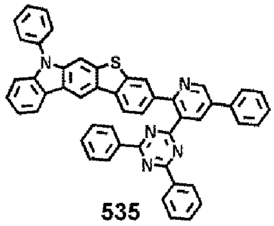
535

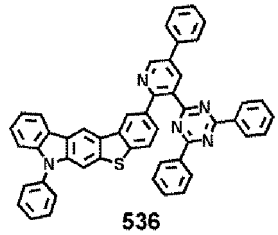
536

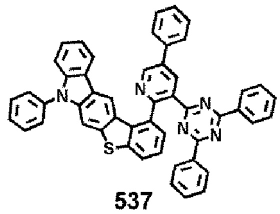
537

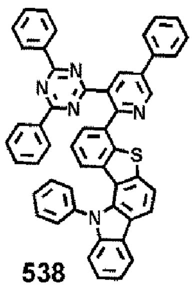
538

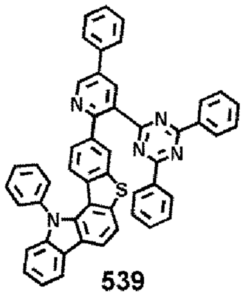
539

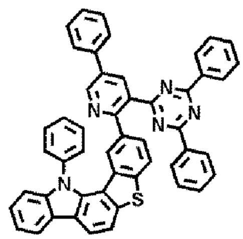
540

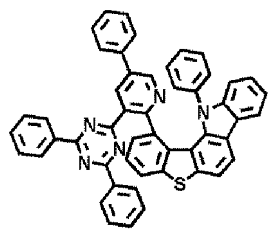
541

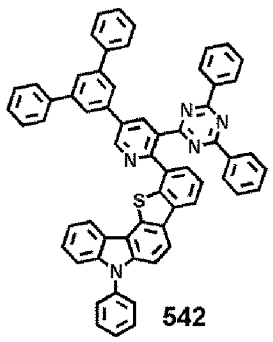
542

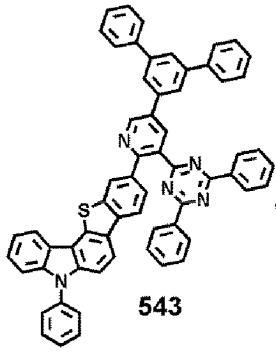
543

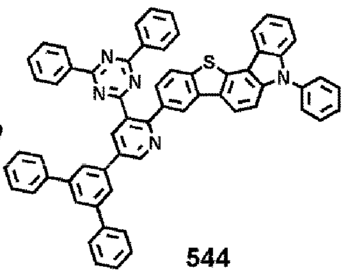
544

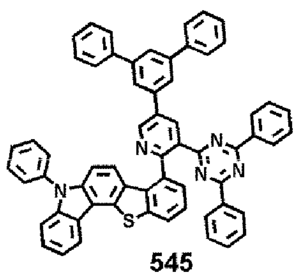
545

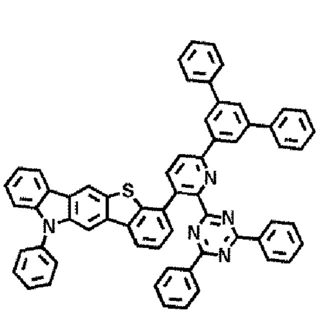
546

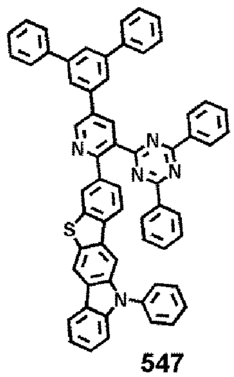
547

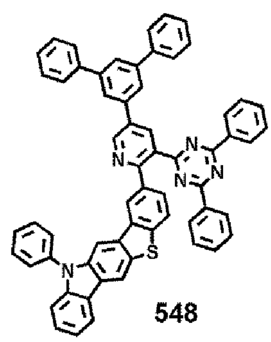
548

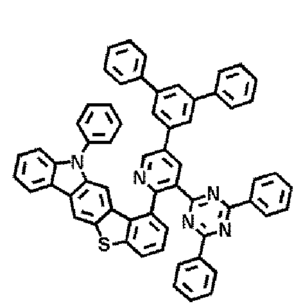
549

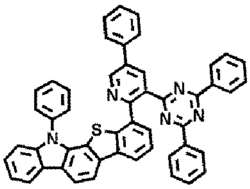
550

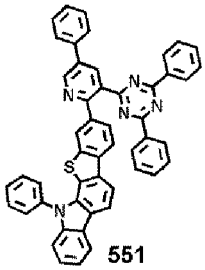
551

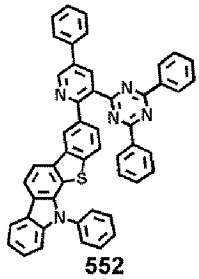
552

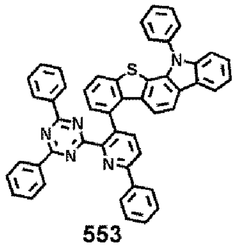
553

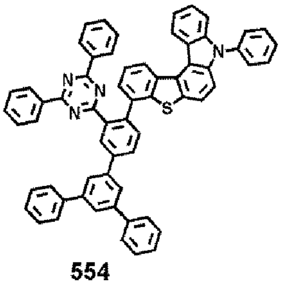
554

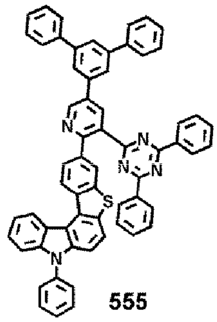
555

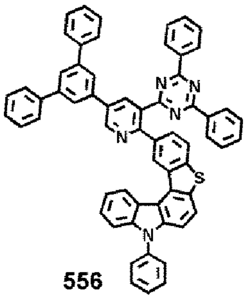
556

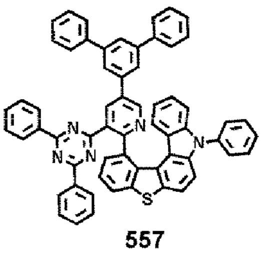
557

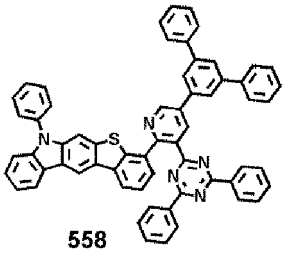
558

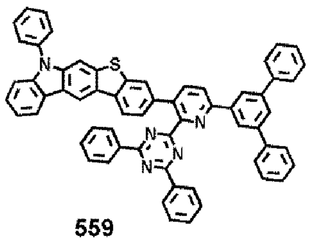
559

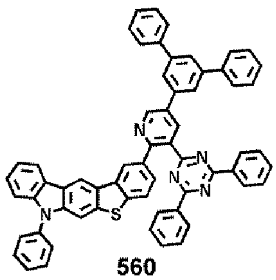
560

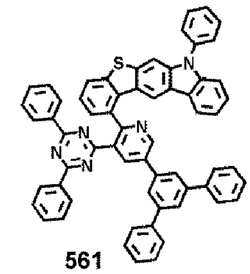
561

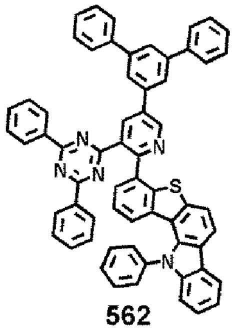
562

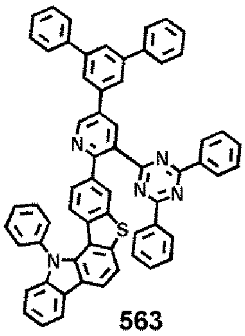
563

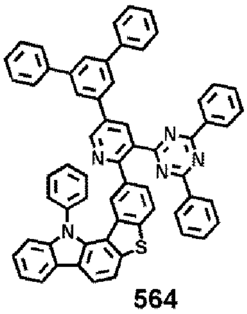
564

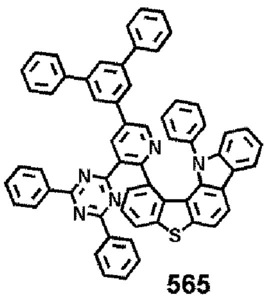
565

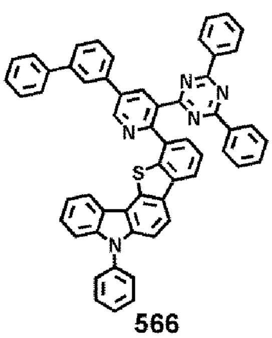
566

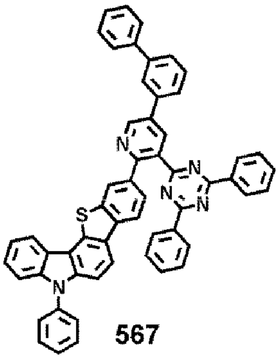
567

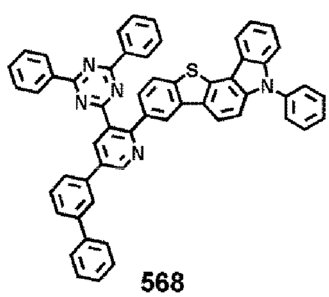
568

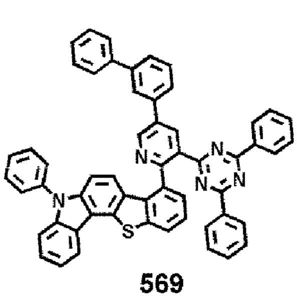
569

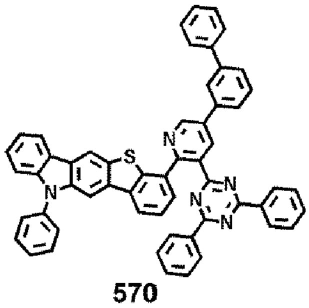
570
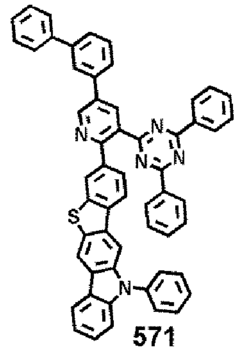
571
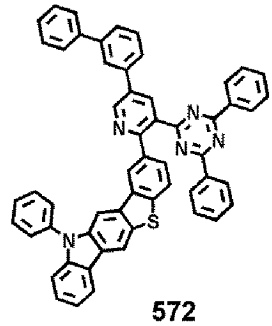
572
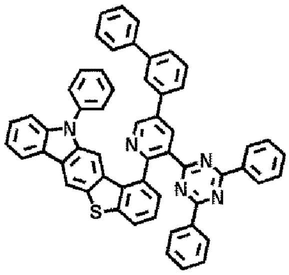
573

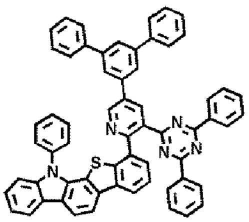
574
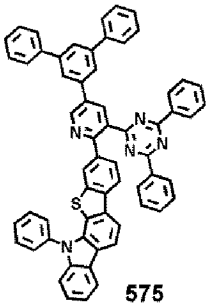
575
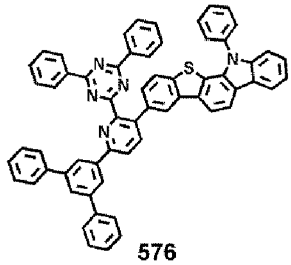
576
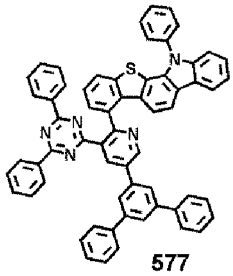
577

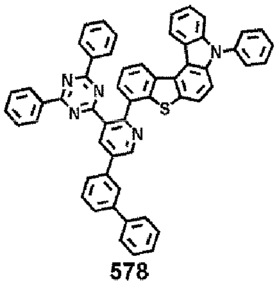
578
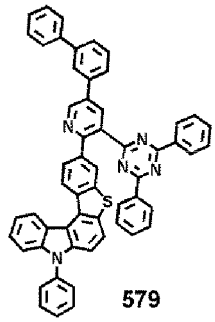
579
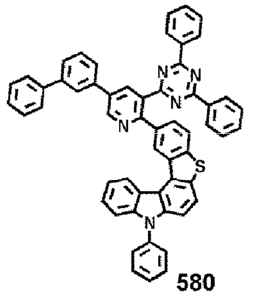
580
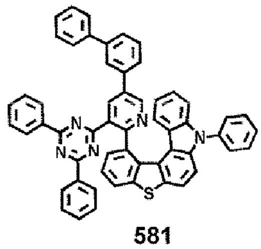
581

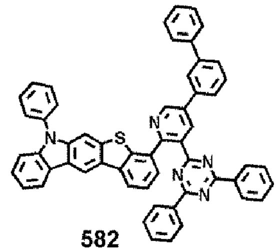
582
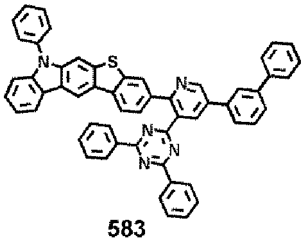
583
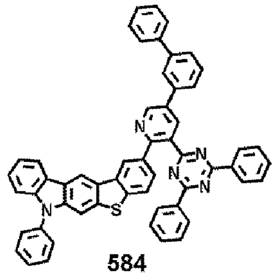
584
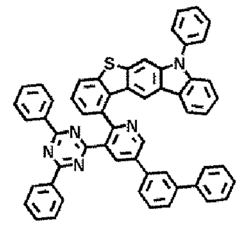
585

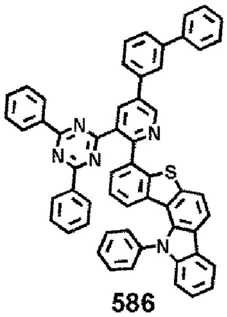
586
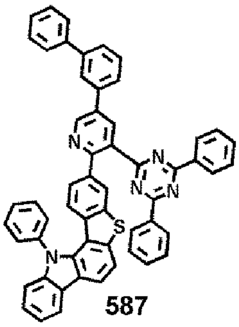
587
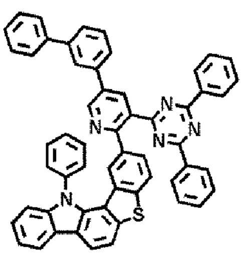
588
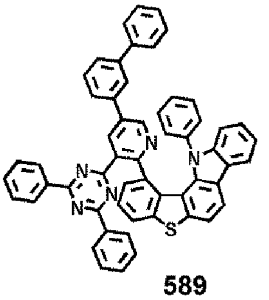
589

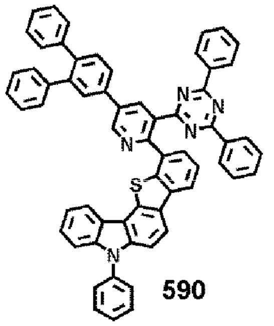
590

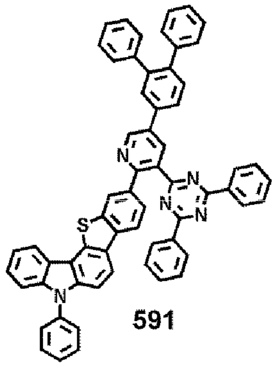
591

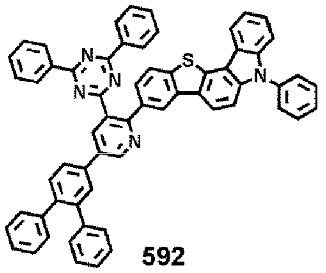
592

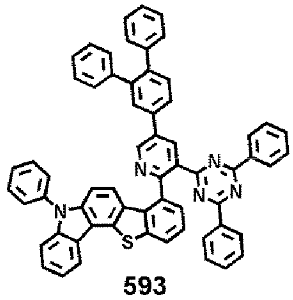
593

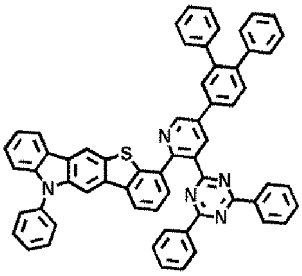
594

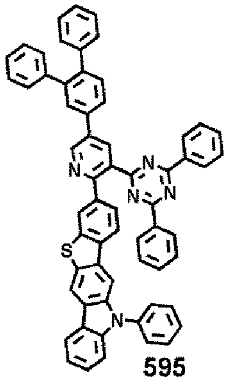
595

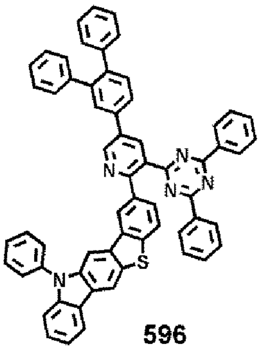
596

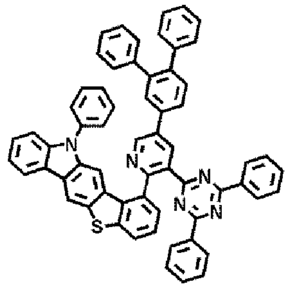
597

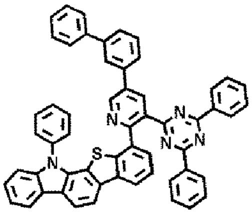
598

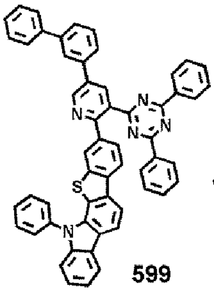
599

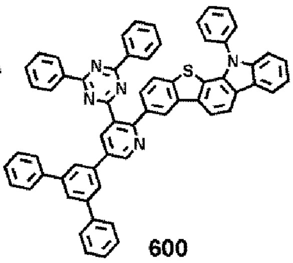
600

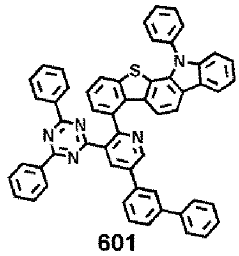
601

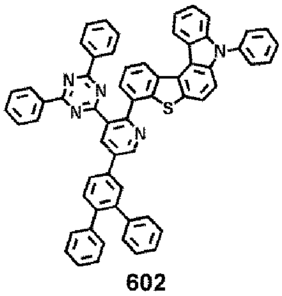
602

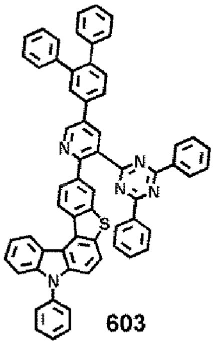
603

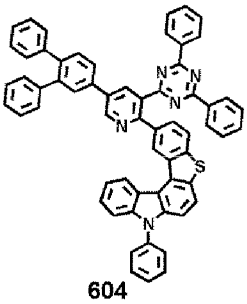
604

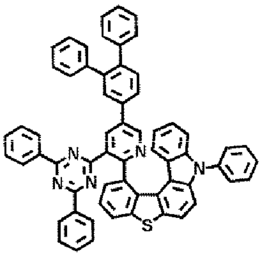
605

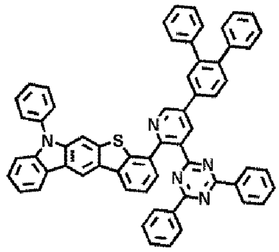
606

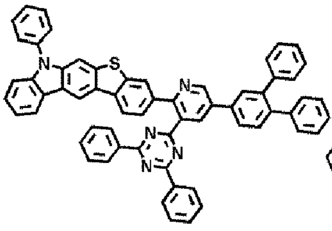
607

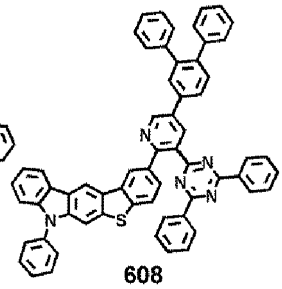
608

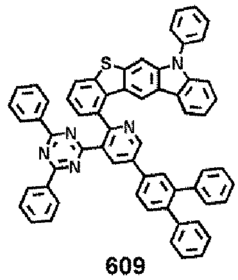
609

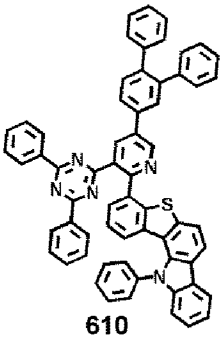
610

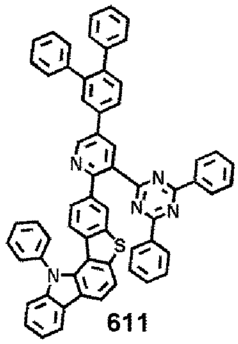
611

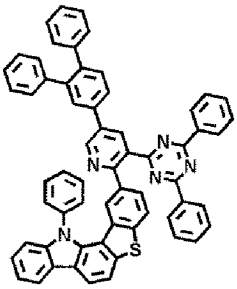
612

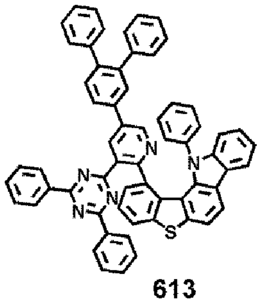
613

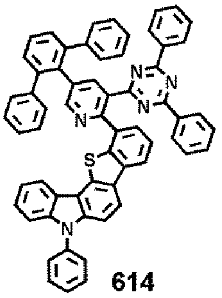
614

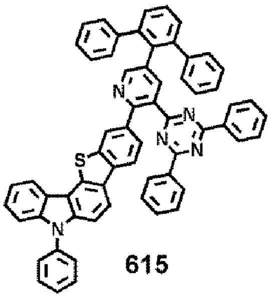
615

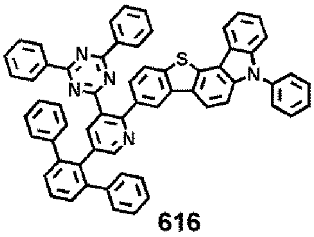
616

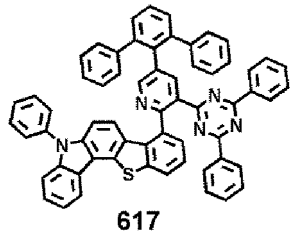
617

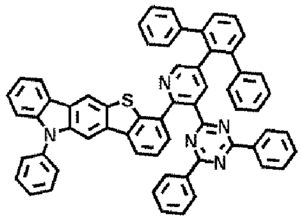
618

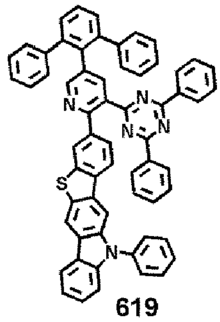
619

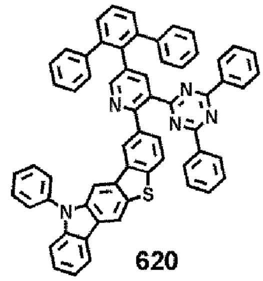
620

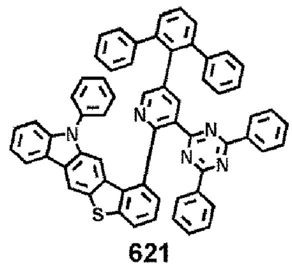
621

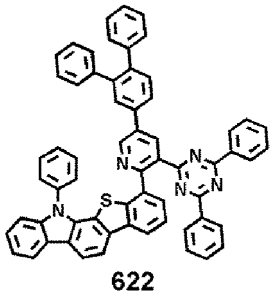
622

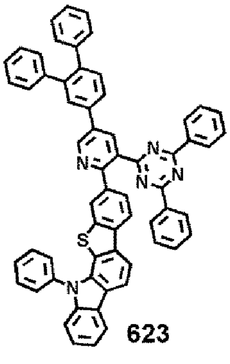
623

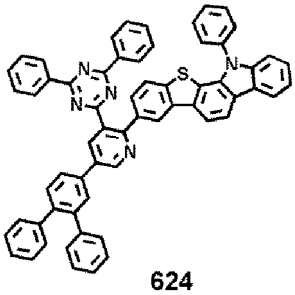
624

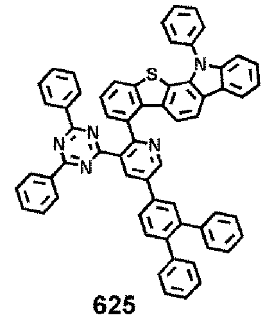
625

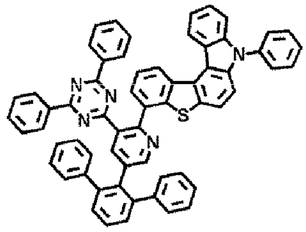
626

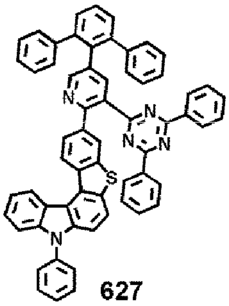
627

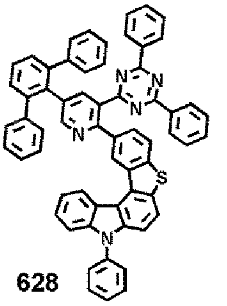
628

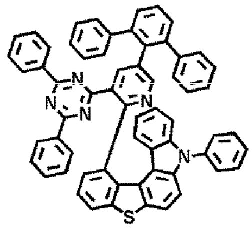
629

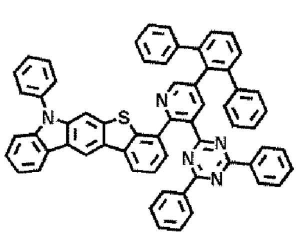
630

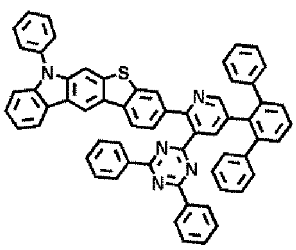
631

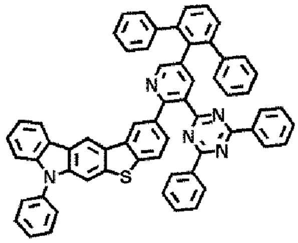
632

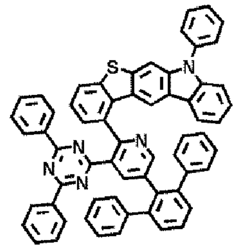
633

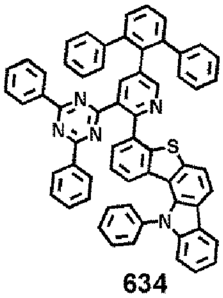
634

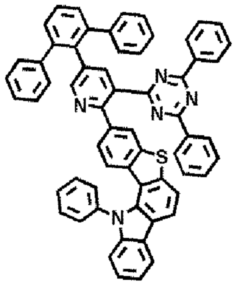
635

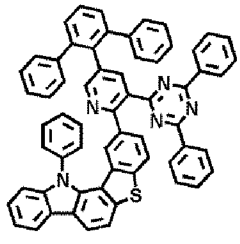
636

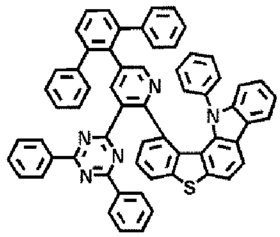
637

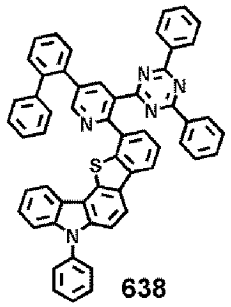
638

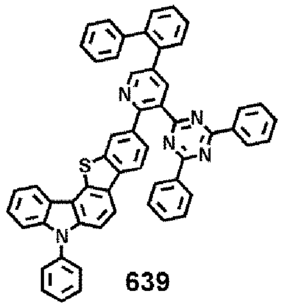
639

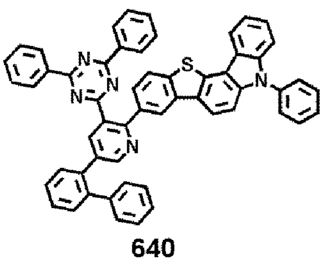
640

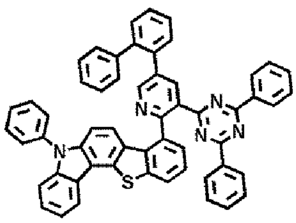
641

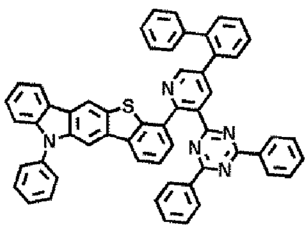
642

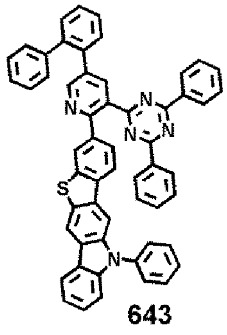
643

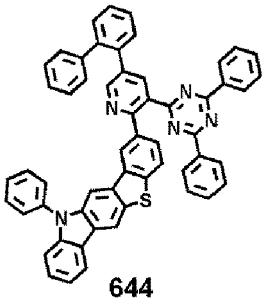
644

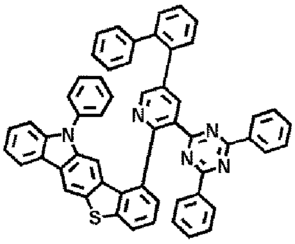
645

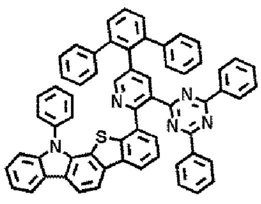
646

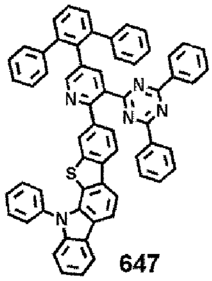
647

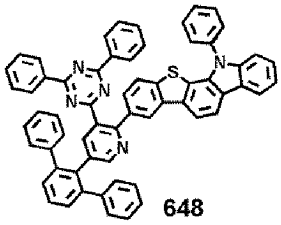
648

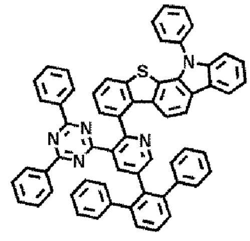
649

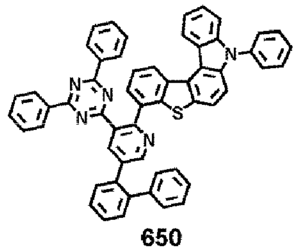
650

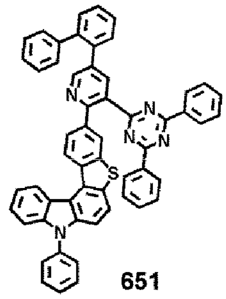
651

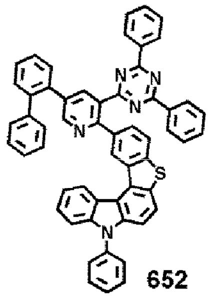
652

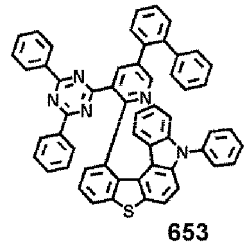
653

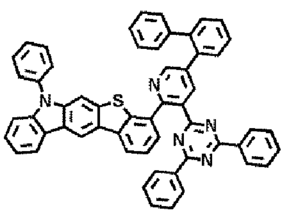
654

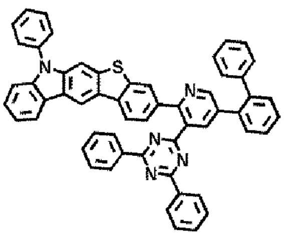
655

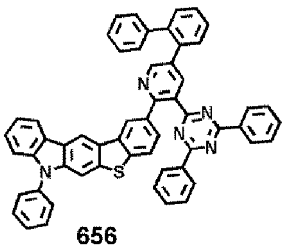
656

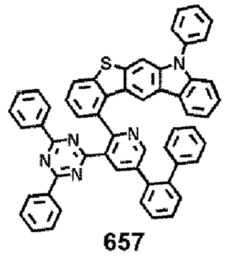
657

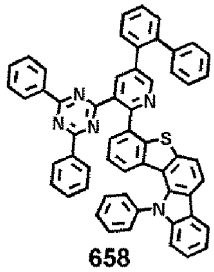
658

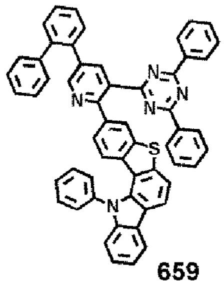
659

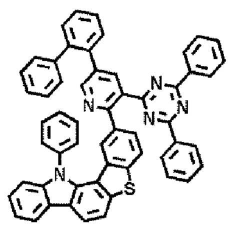
660

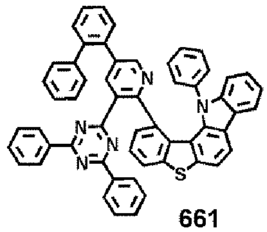
661

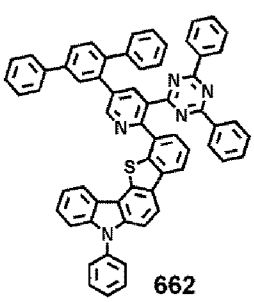
662

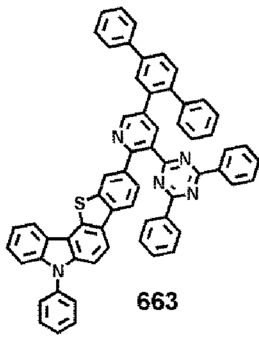
663

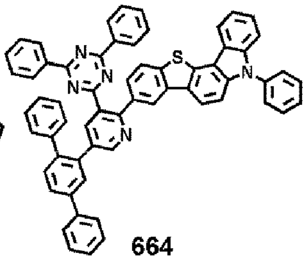
664

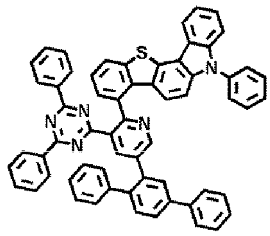
665

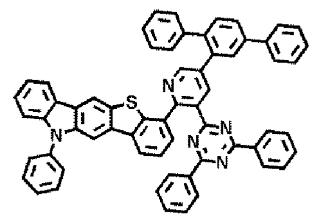
666

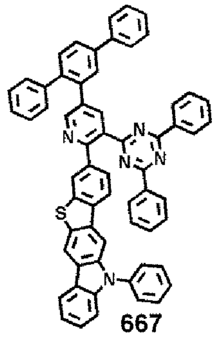
667

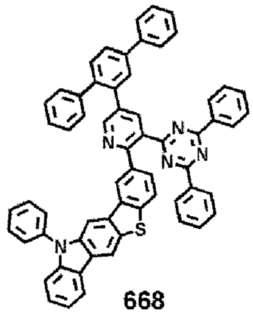
668

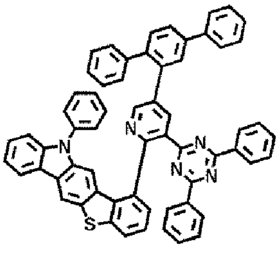
669

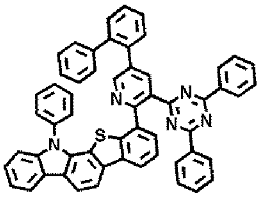
670

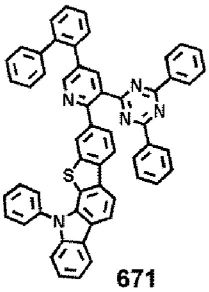
671

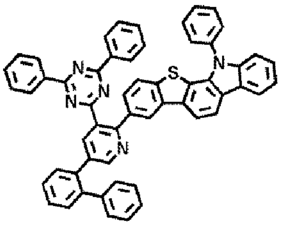
672

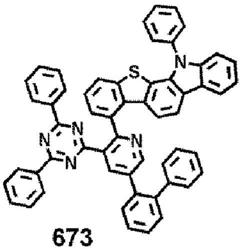
673

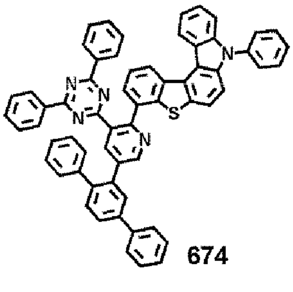
674

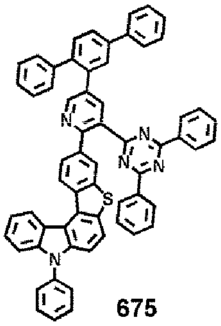
675

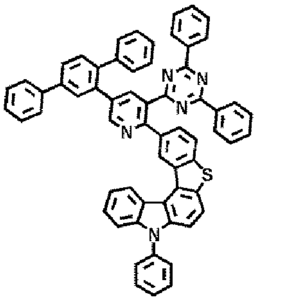
676

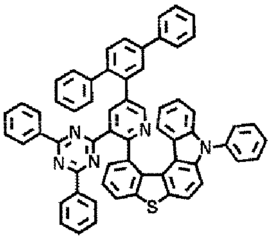
677

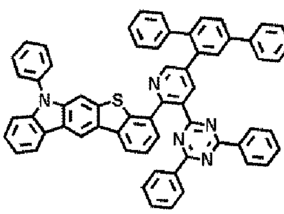
678

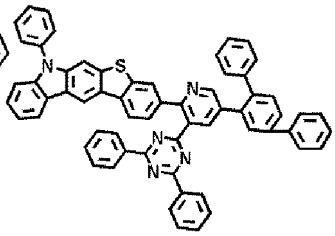
679

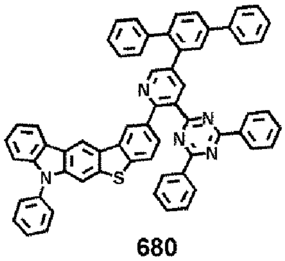
680

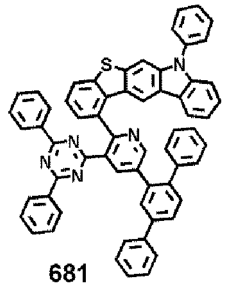
681

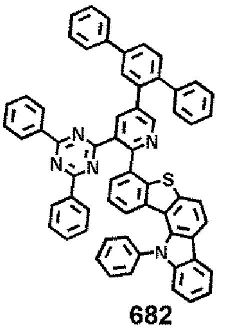
682

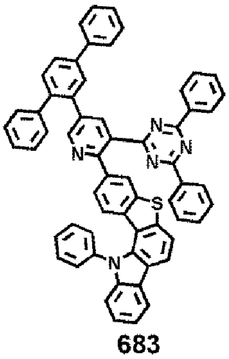
683

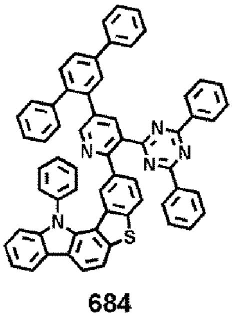
684

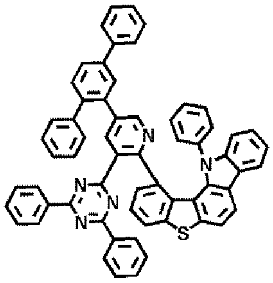
685

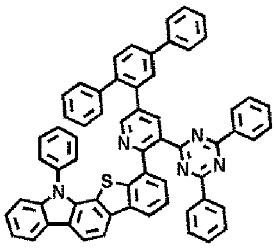

686

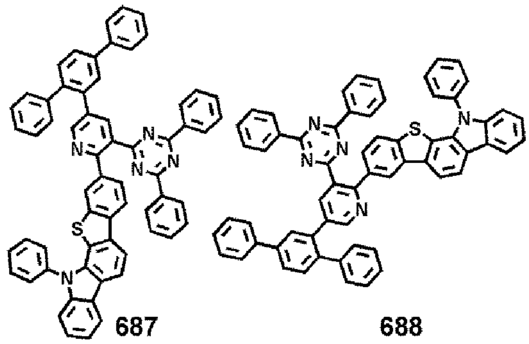

687

688

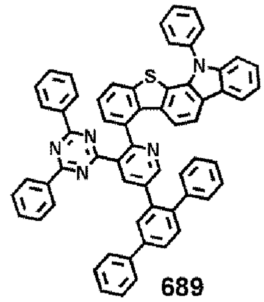

689

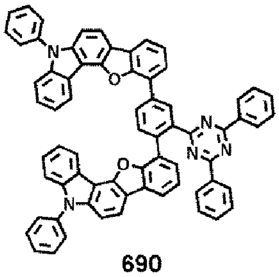

690

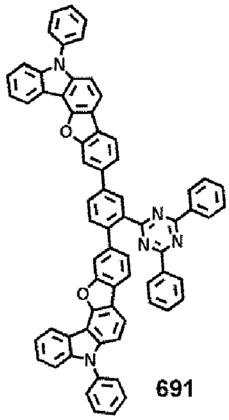

691

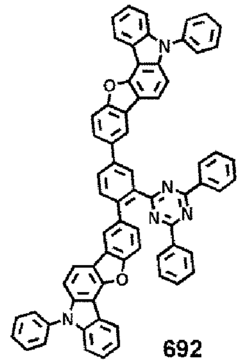

692

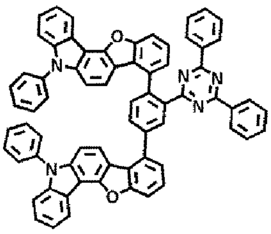

693

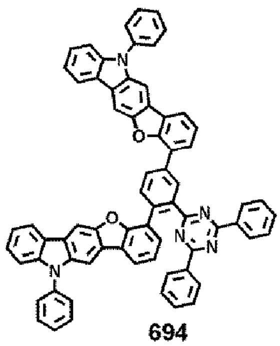

694

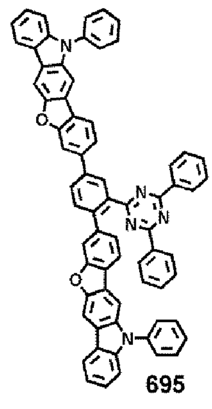

695

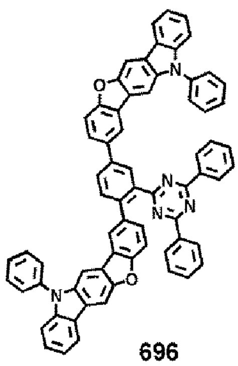

696

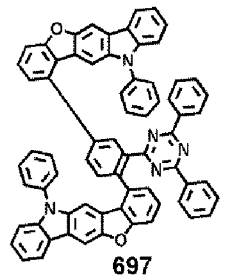

697

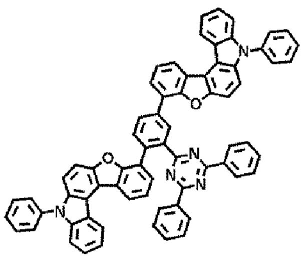

698

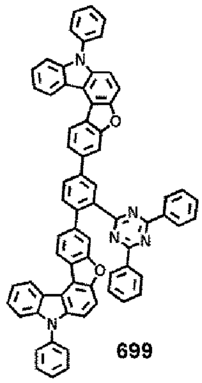

699

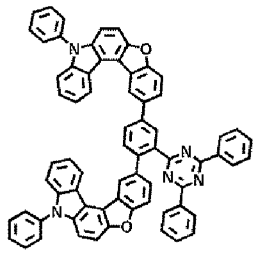

700

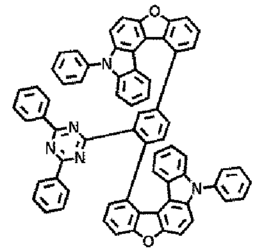

701

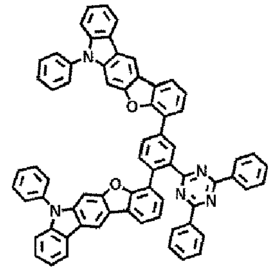

702

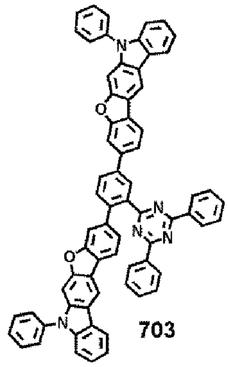

703

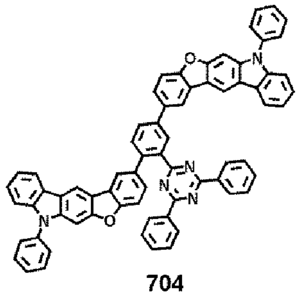

704

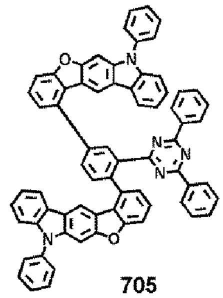

705

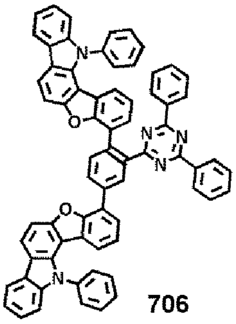

706

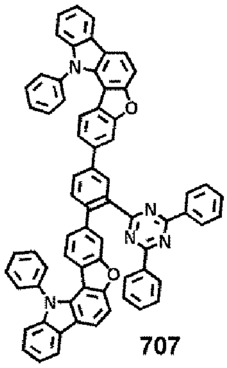

707

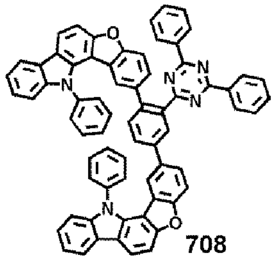

708

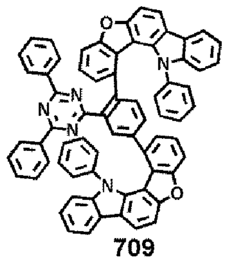

709

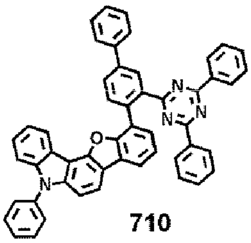

710

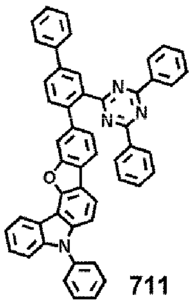

711

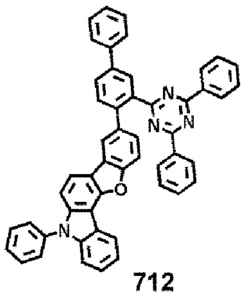

712

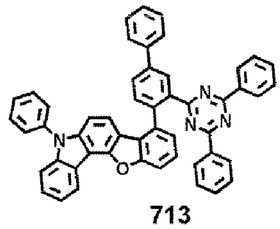

713

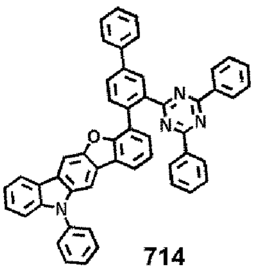

714

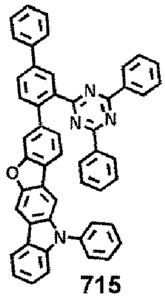

715

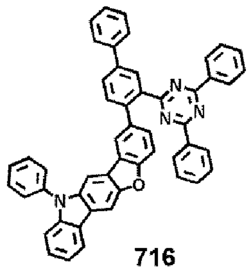

716

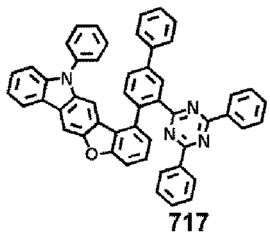

717

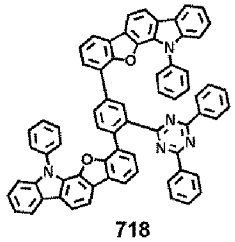
718

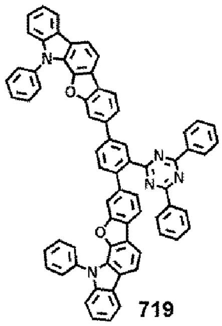
719

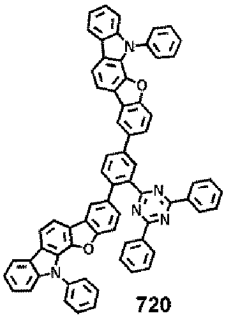
720

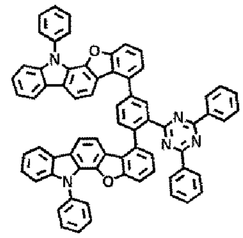
721

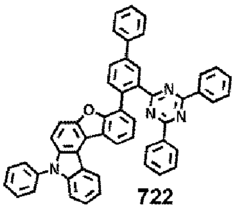
722

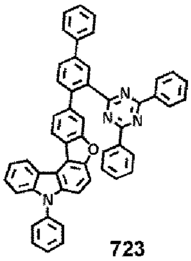
723

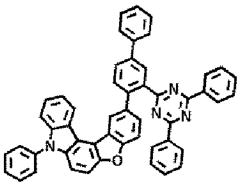
724

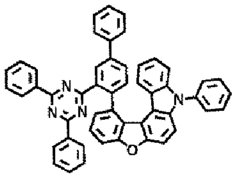
725

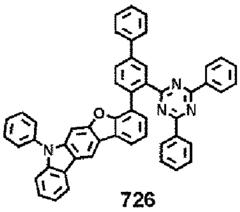
726

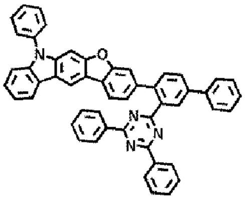
727

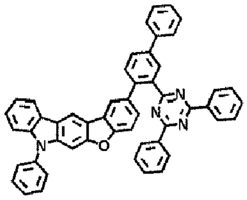
728

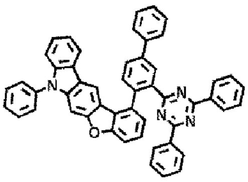
729

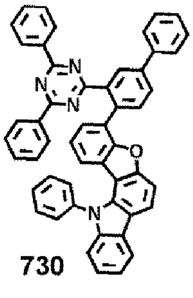
730

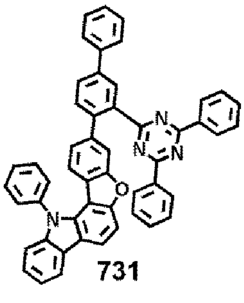
731

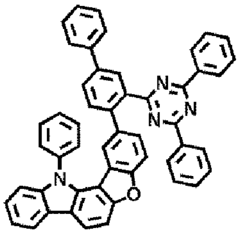
732

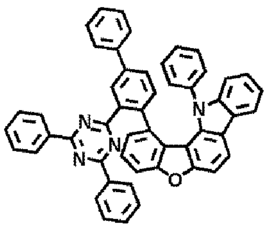
733

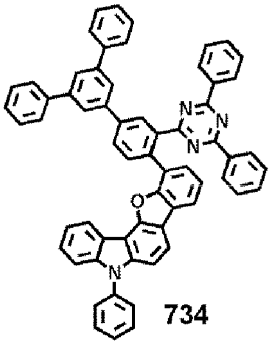
734

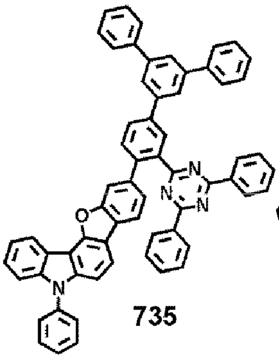
735

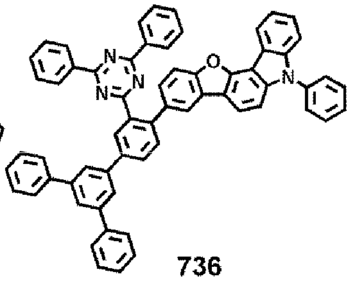
736

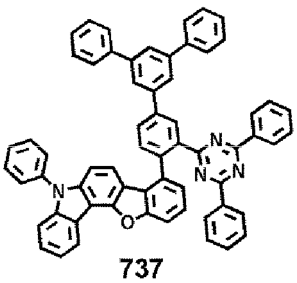
737

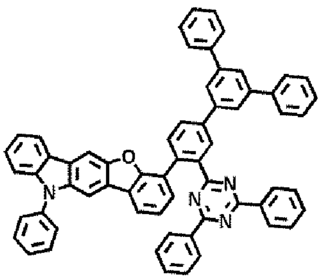

738

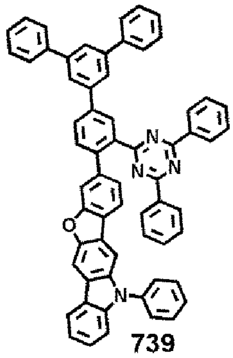

739

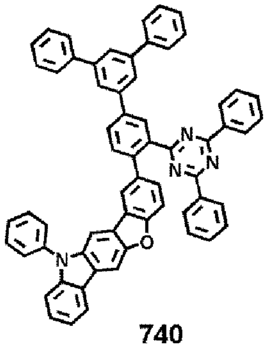

740

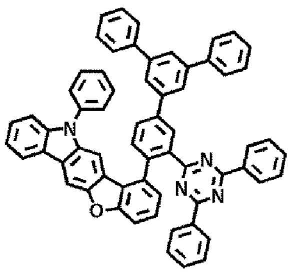

741

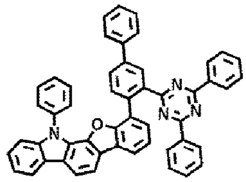

742

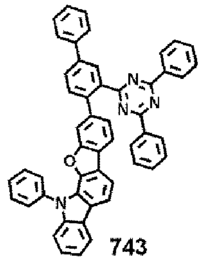

743

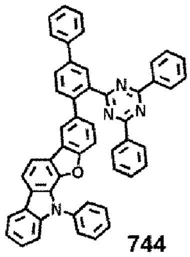

744

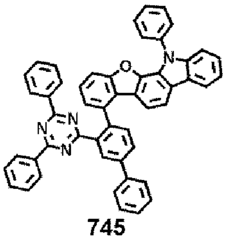

745

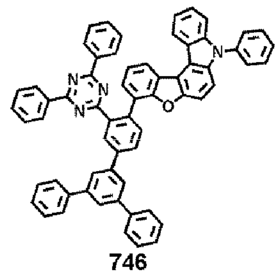

746

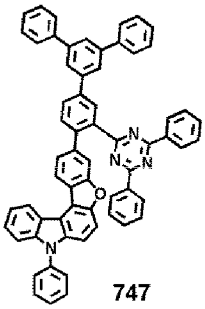

747

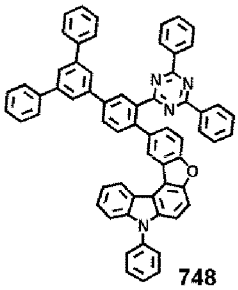

748

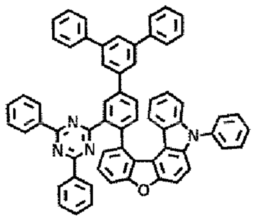

749

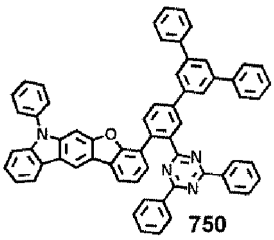

750

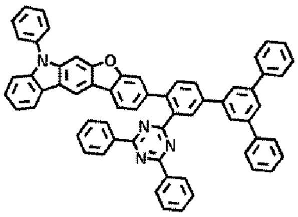

751

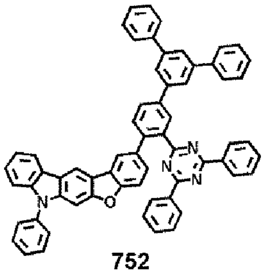

752

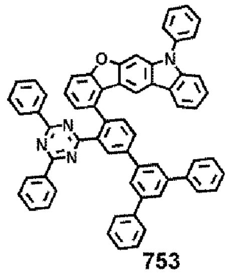

753

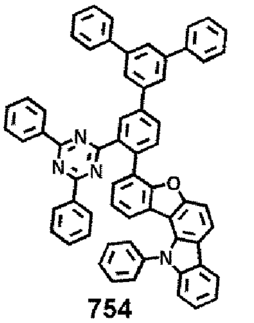

754

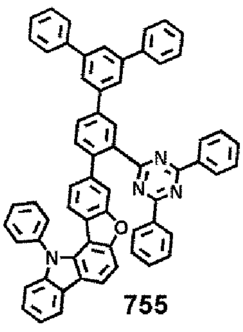

755

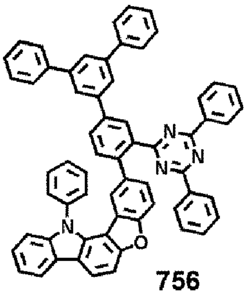

756

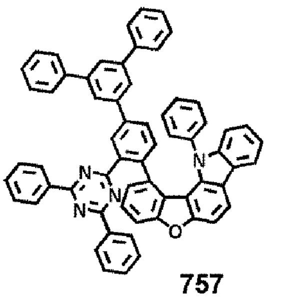

757

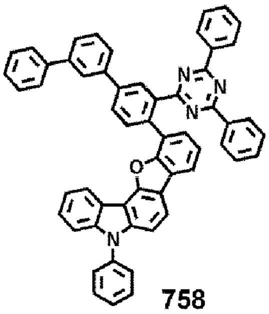
758

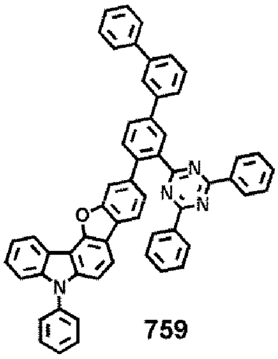
759

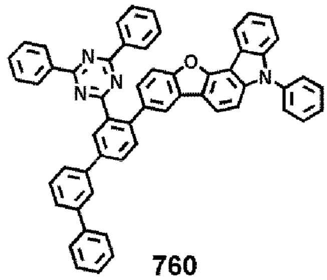
760

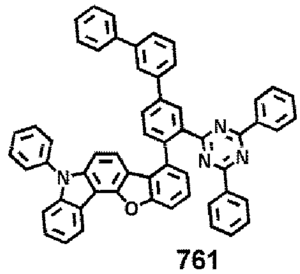
761

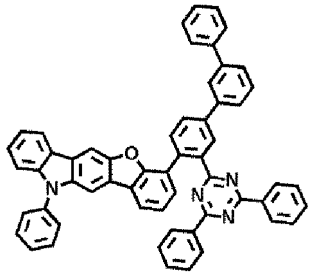
762

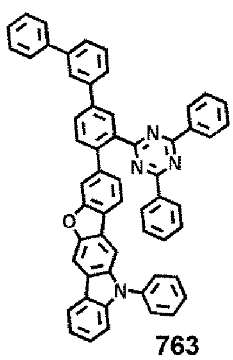
763

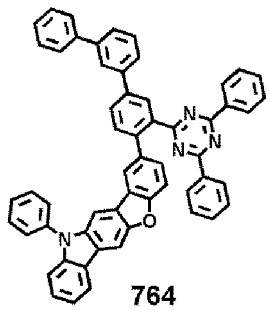
764

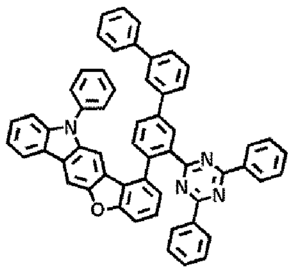
765

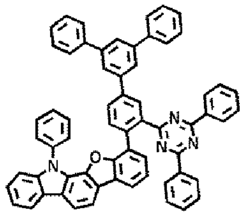
766

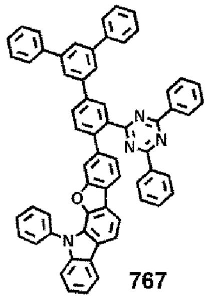
767

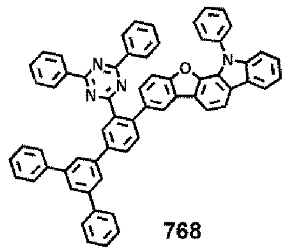
768

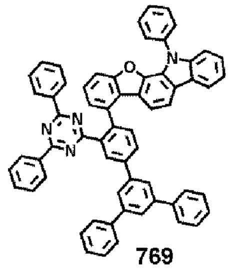
769

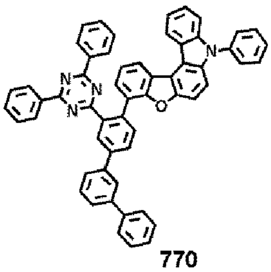
770

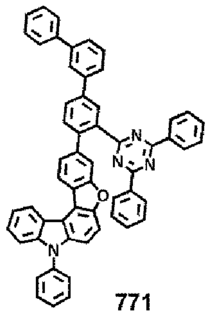
771

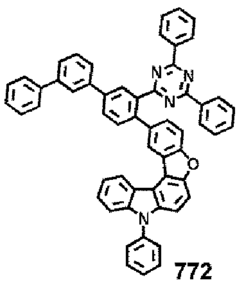
772

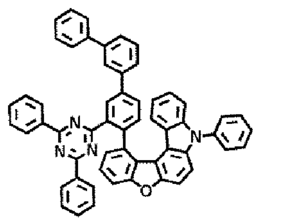
773

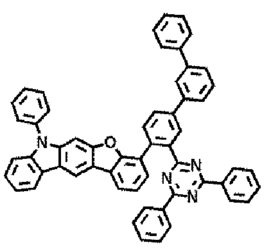
774

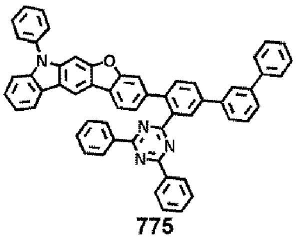
775

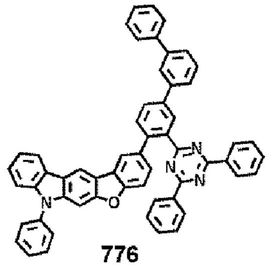
776

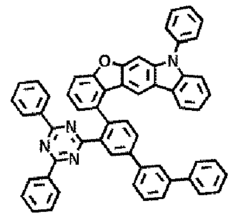
777

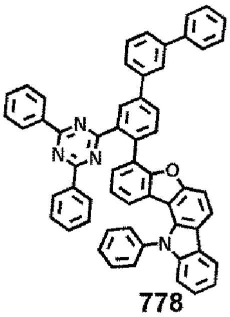

778

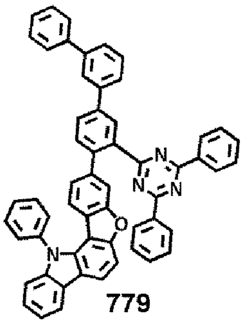

779

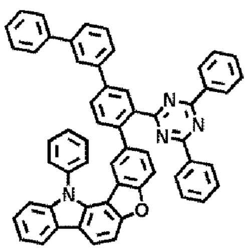

780

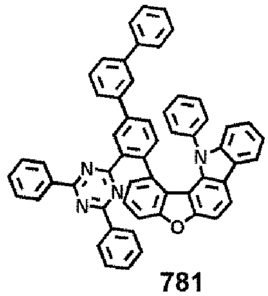

781

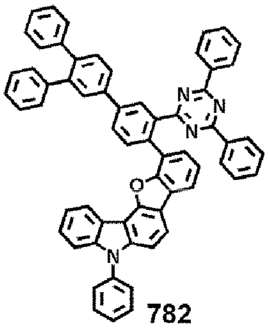

782

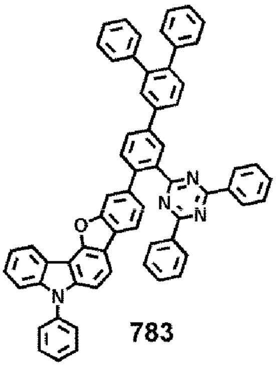

783

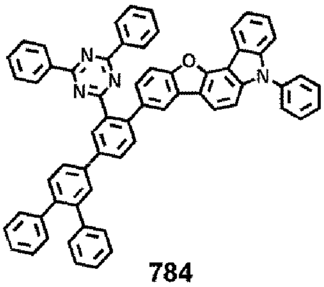

784

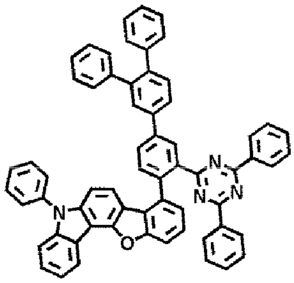

785

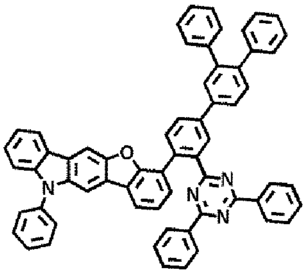

786

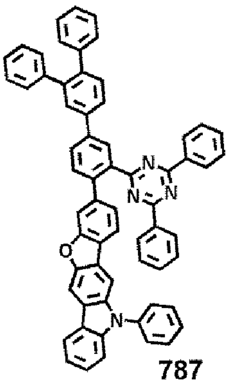

787

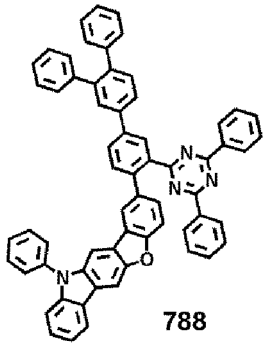

788

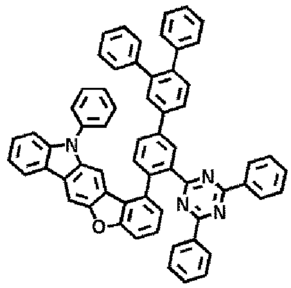

789

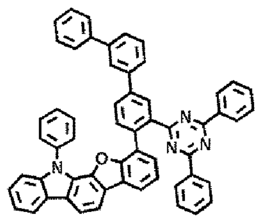

790

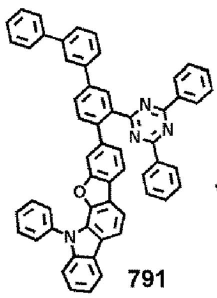

791

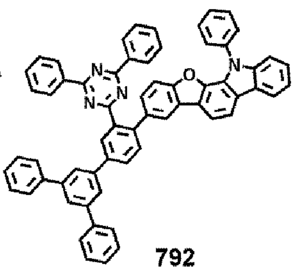

792

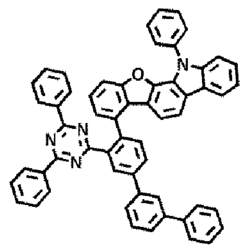

793

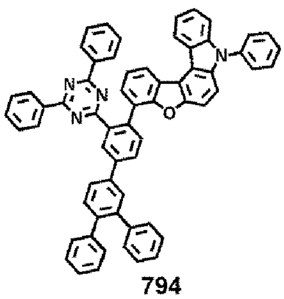
794

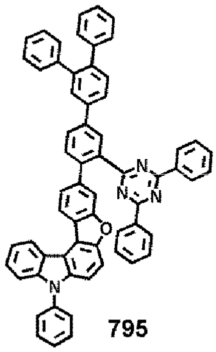
795

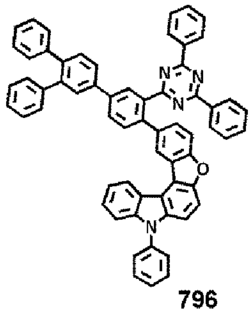
796

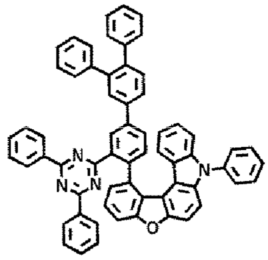
797

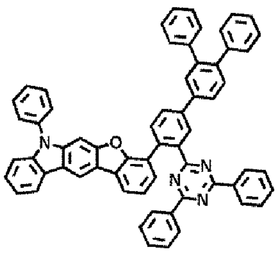
798

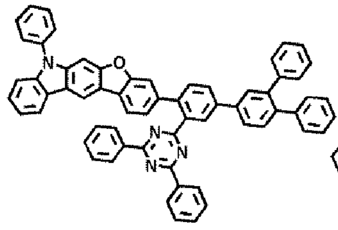
799

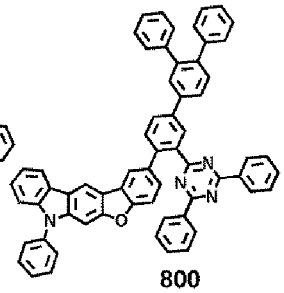
800

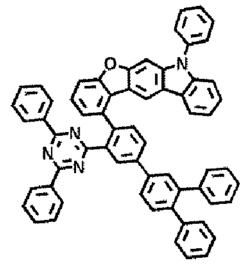
801

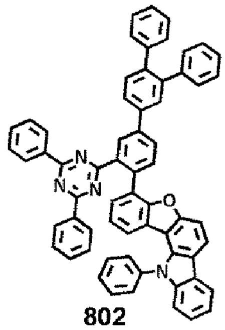
802

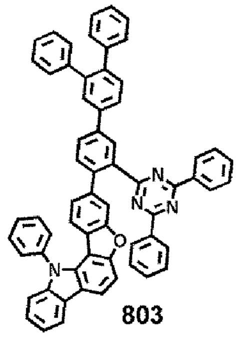
803

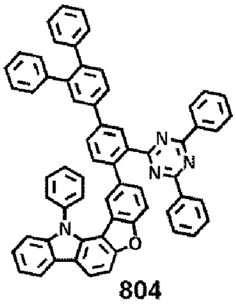
804

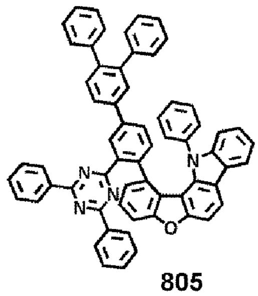
805

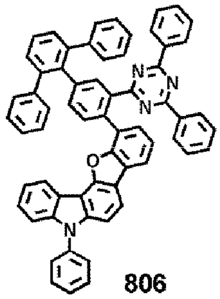
806

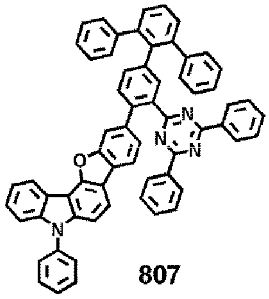
807

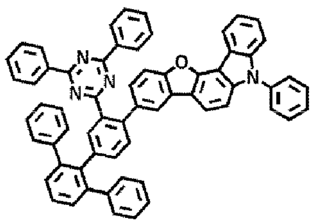
808

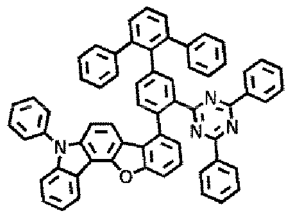
809

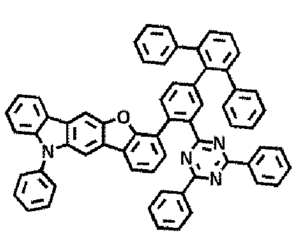
810

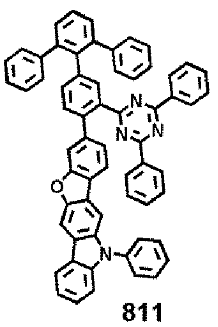
811

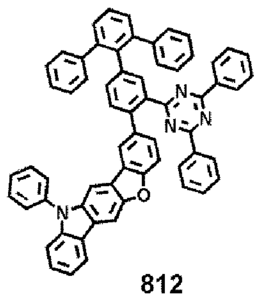
812

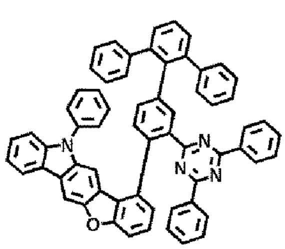
813

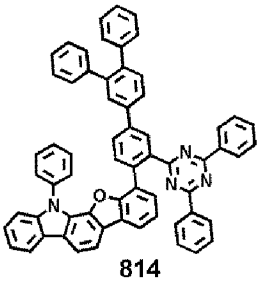
814

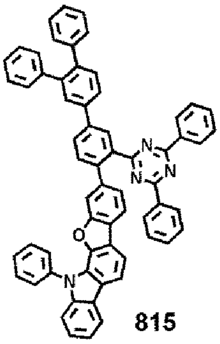
815

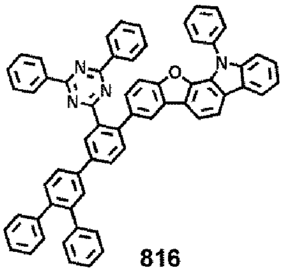
816

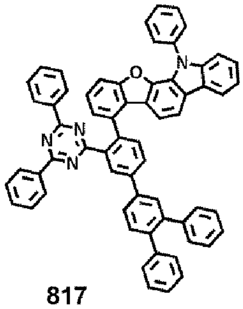
817

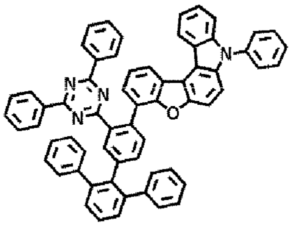
818

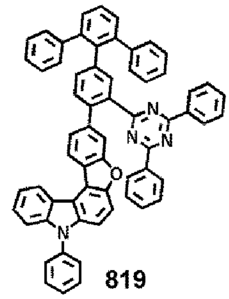
819

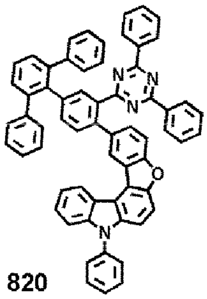
820

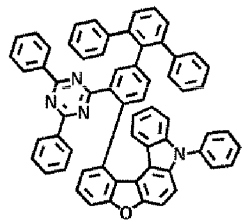
821

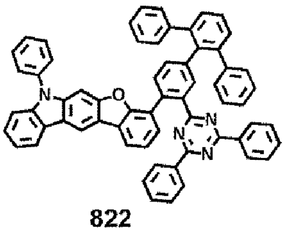
822

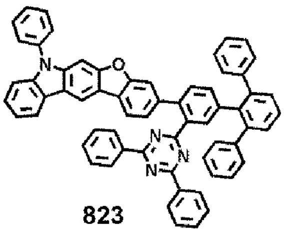
823

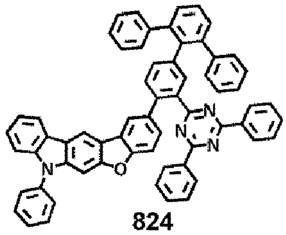
824

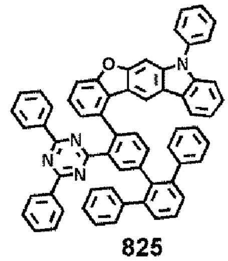
825

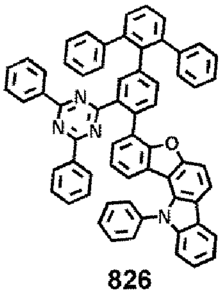
826

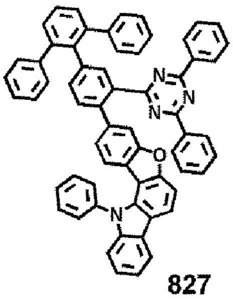
827

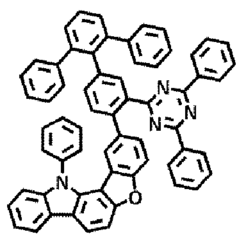
828

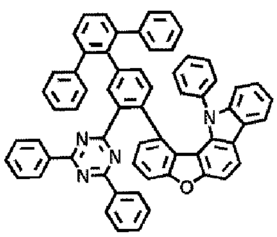
829

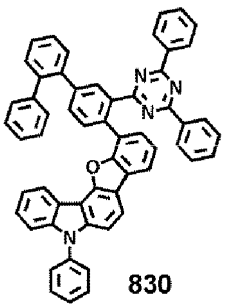
830

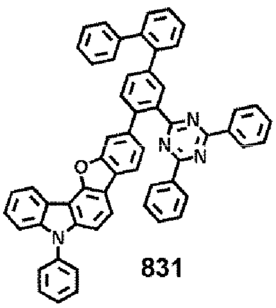
831

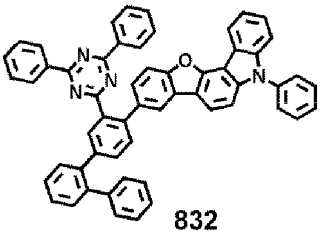
832

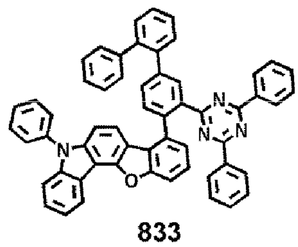
833

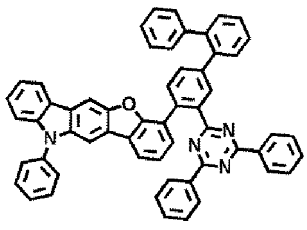
834

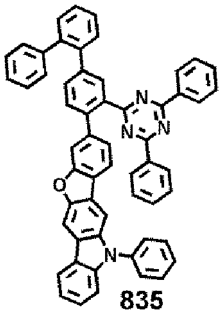
835

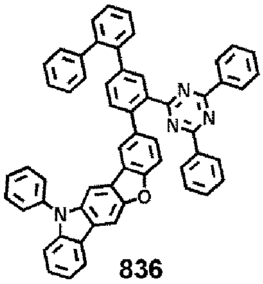
836

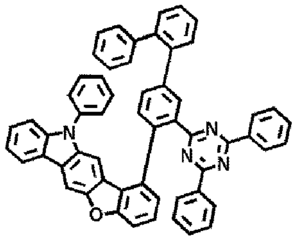
837

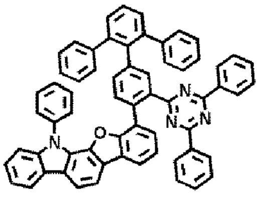
838

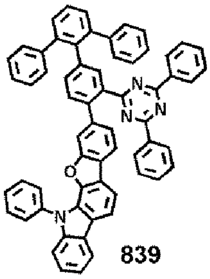
839

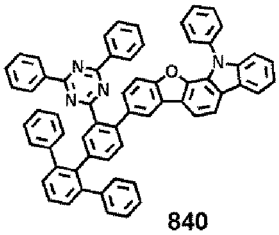
840

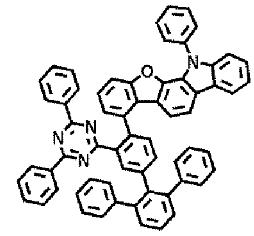
841

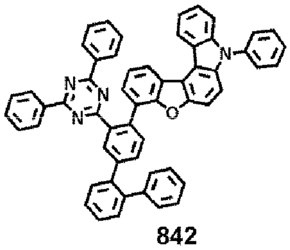
842

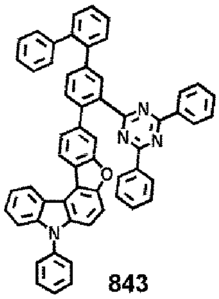
843

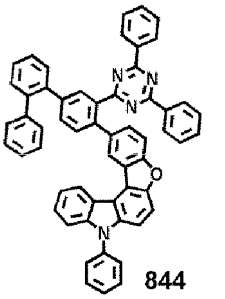
844

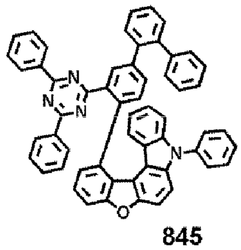
845

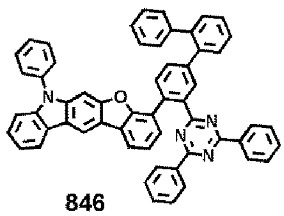
846

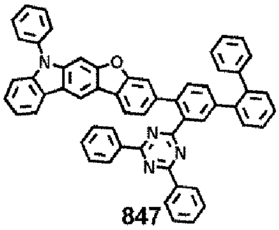
847

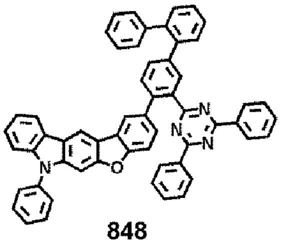
848

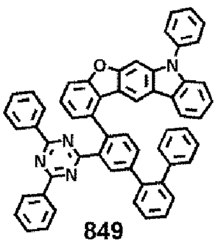
849

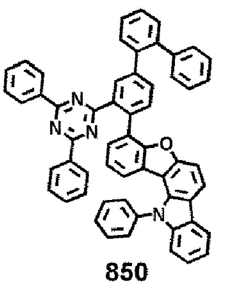
850

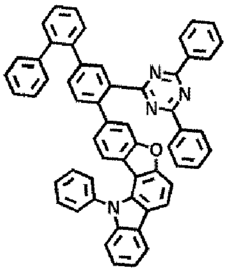
851

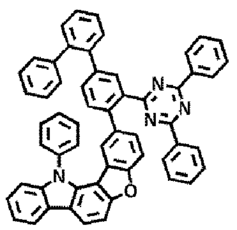
852

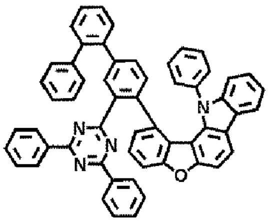
853

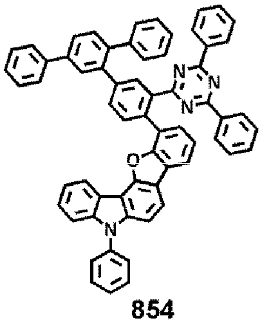

854

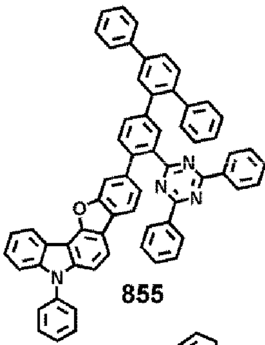

855

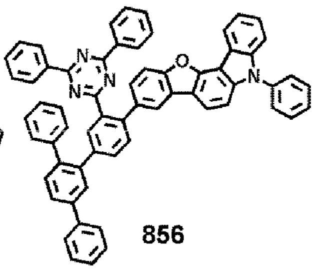

856

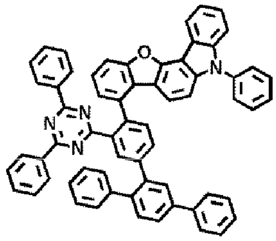

857

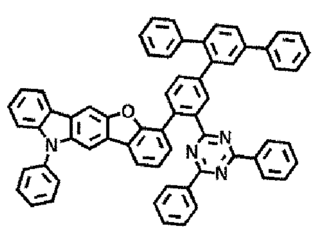

858

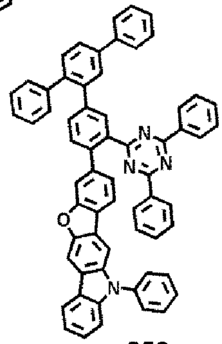

859

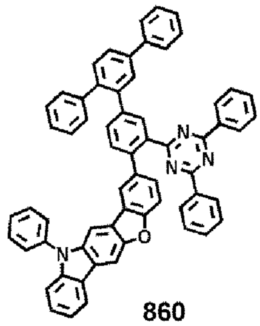

860

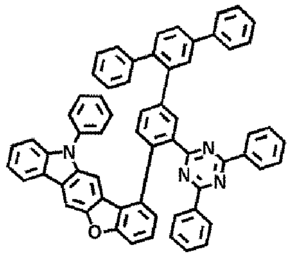

861

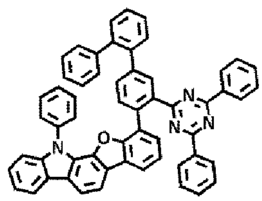

862

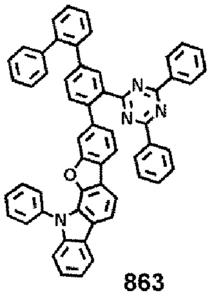

863

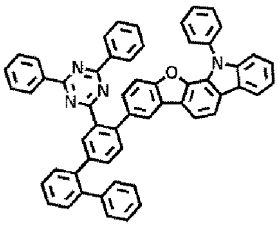

864

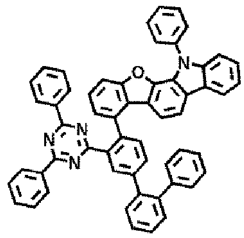

865

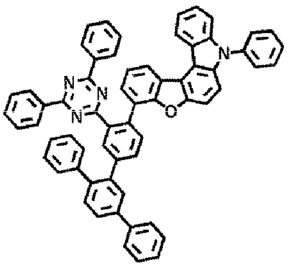

866

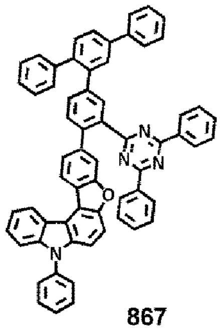

867

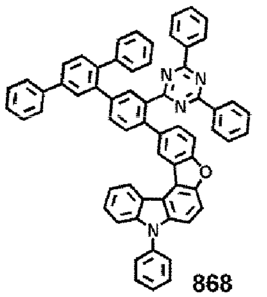

868

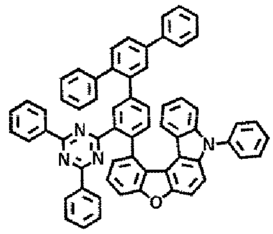

869

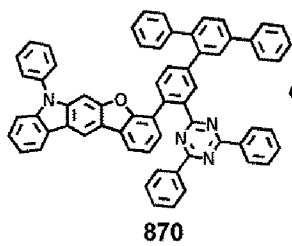

870

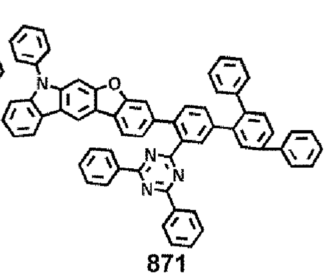

871

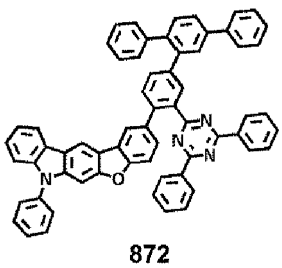

872

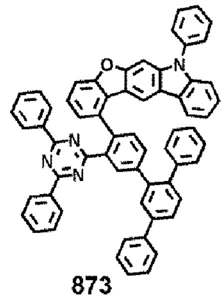

873

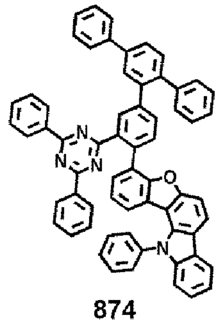

874

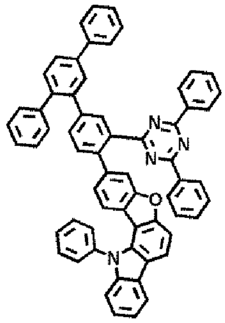

875

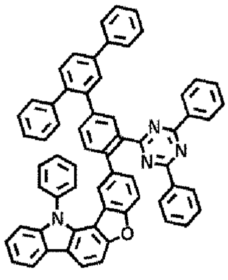

876

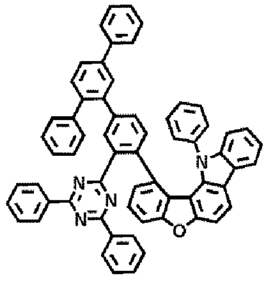

877

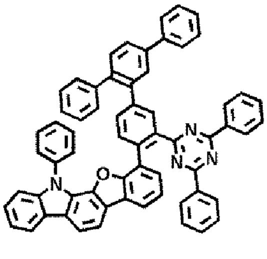

878

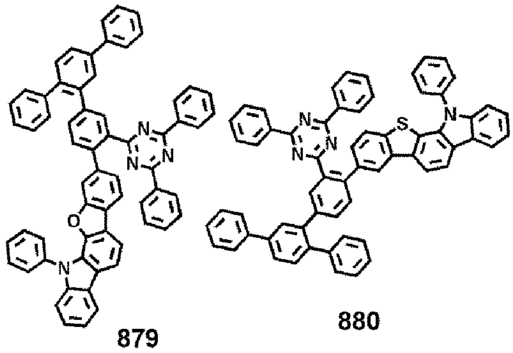

879

880

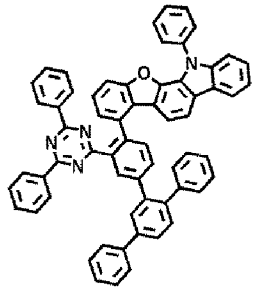

881

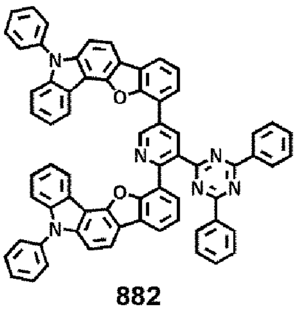

882

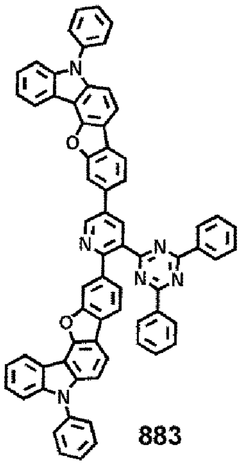

883

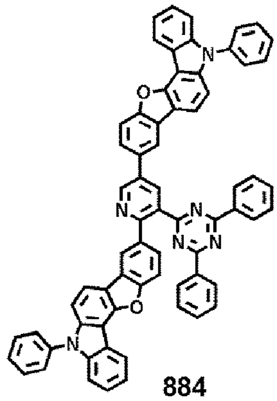

884

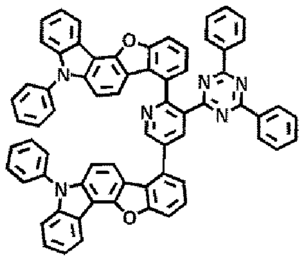

885

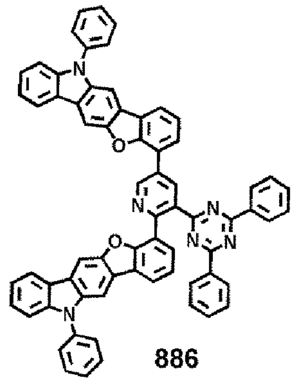

886

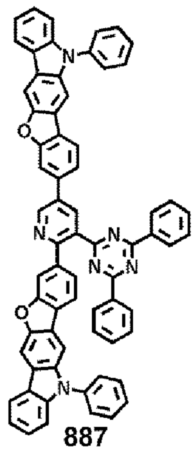

887

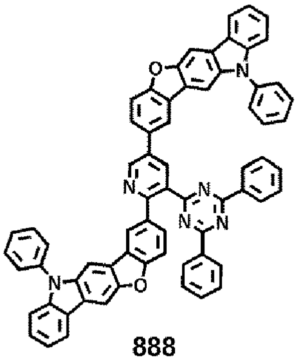

888

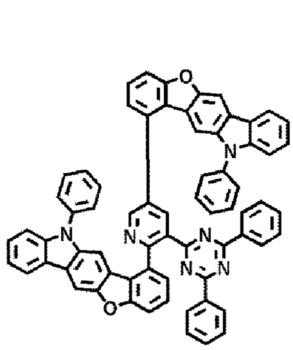

889

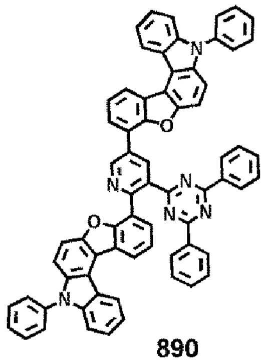
**890**

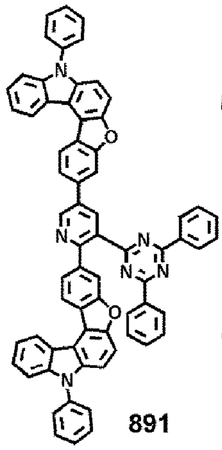
**891**

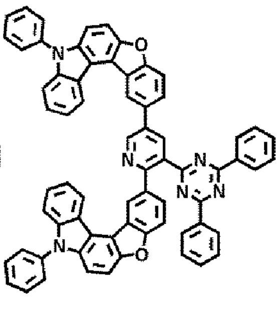
**892**

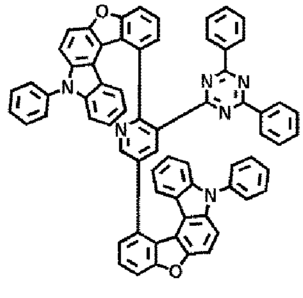
**893**

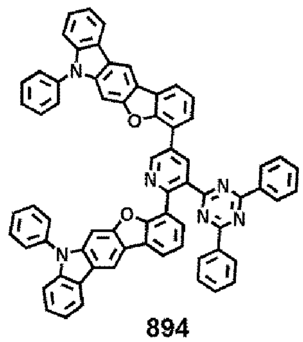
**894**

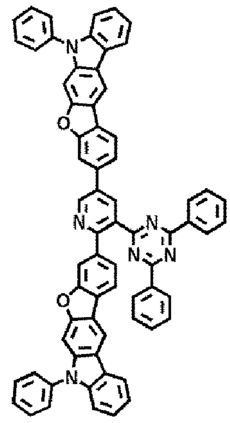
**895**

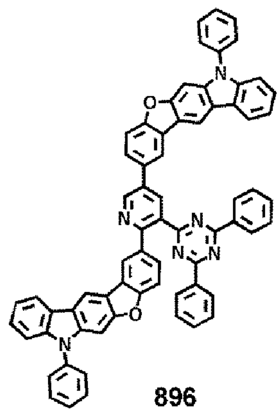
**896**

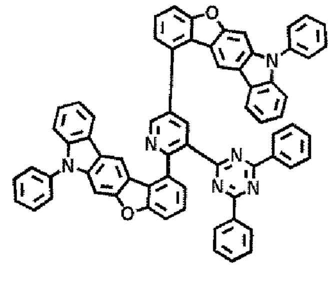
**897**

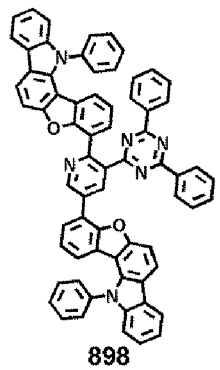
**898**

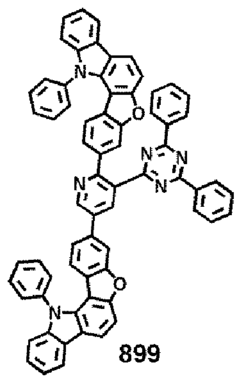
**899**

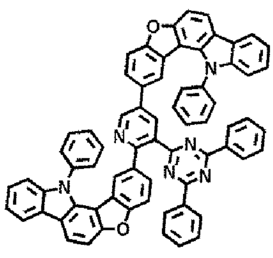
**900**

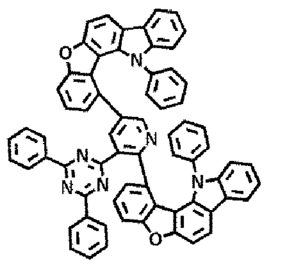
**901**

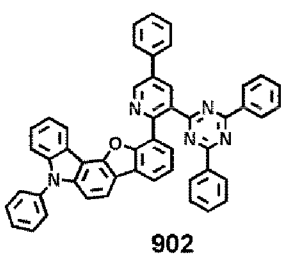
**902**

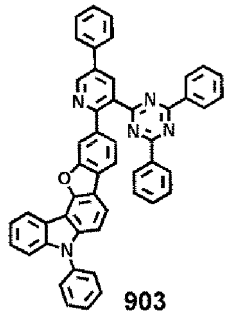
**903**

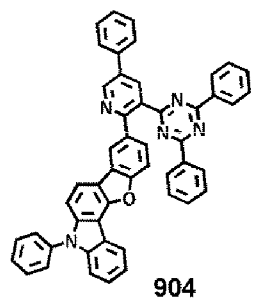
**904**

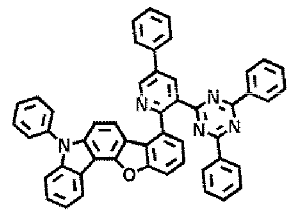
**905**

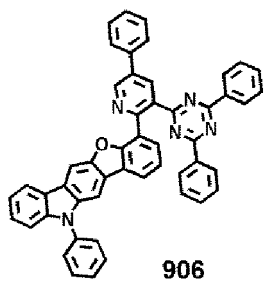

906

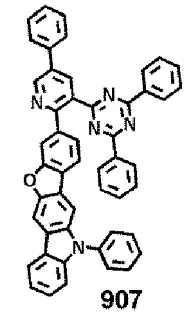

907

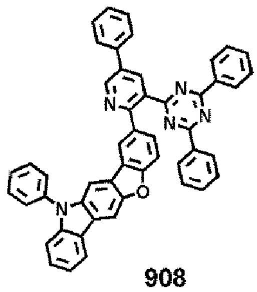

908

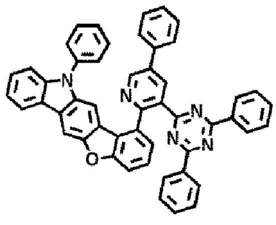

909

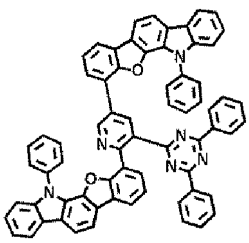

910

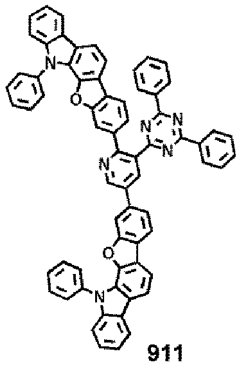

911

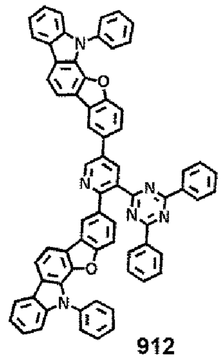

912

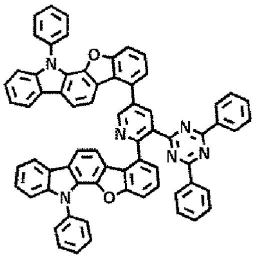

913

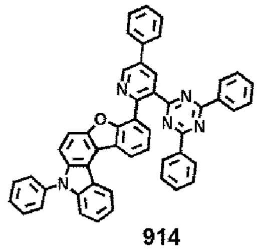

914

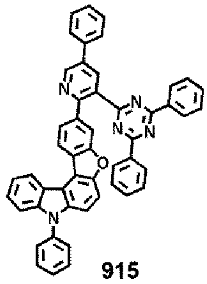

915

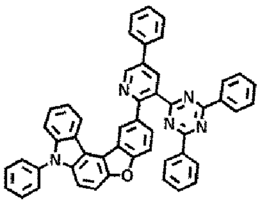

916

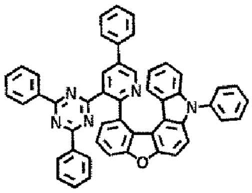

917

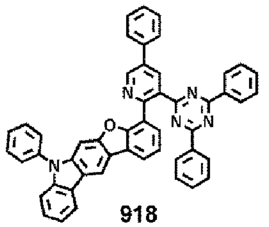

918

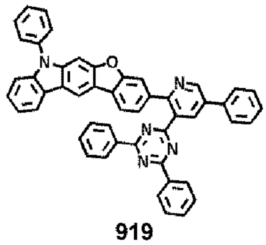

919

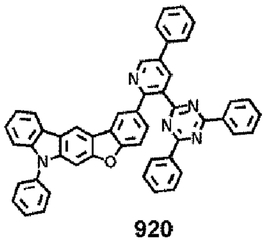

920

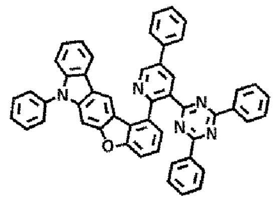

921

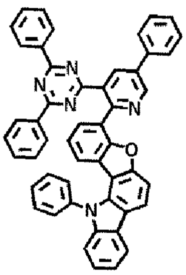

922

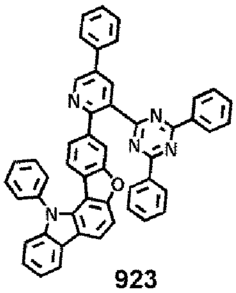

923

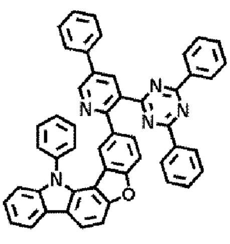

924

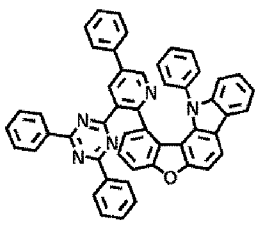

925

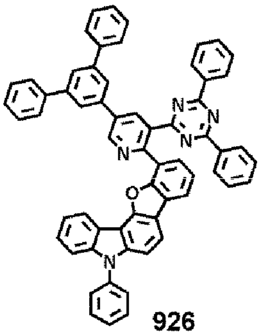
926

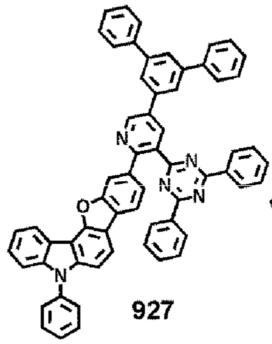
927

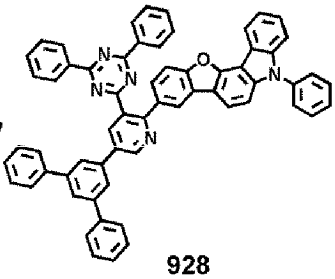
928

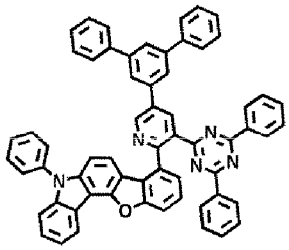
929

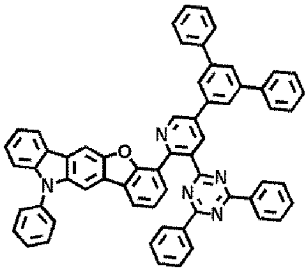
930

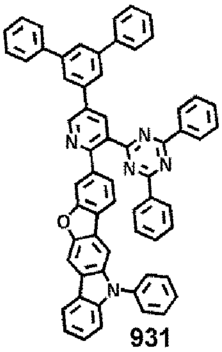
931

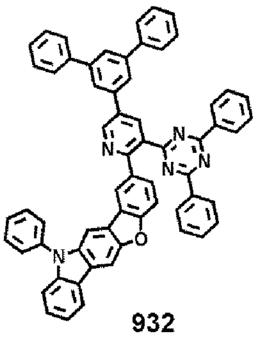
932

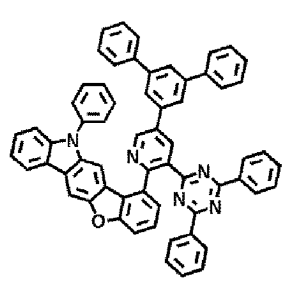
933

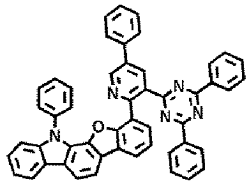
934

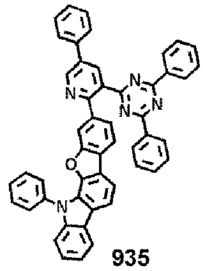
935

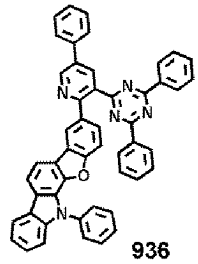
936

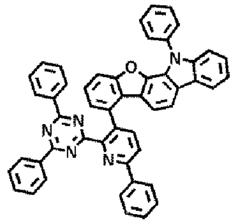
937

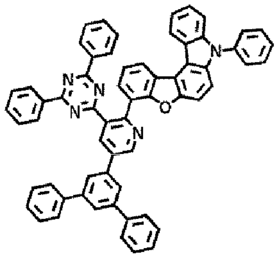
938

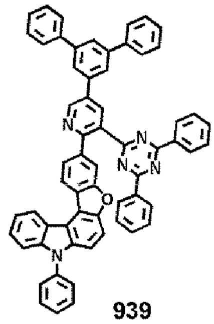
939

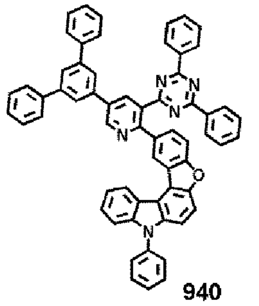
940

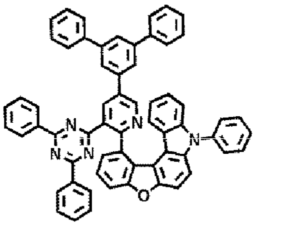
941

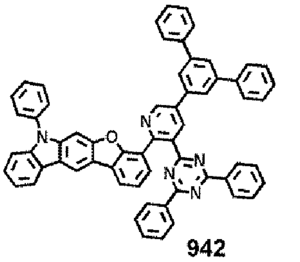
942

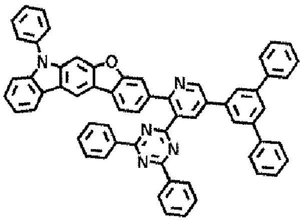
943

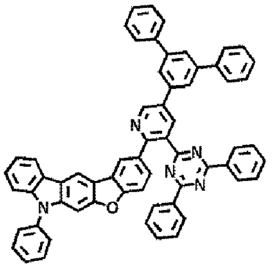
944

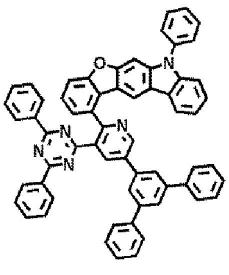
945

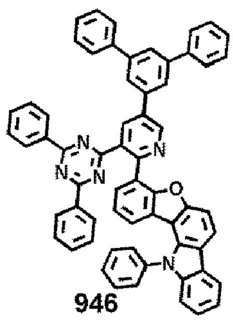

946

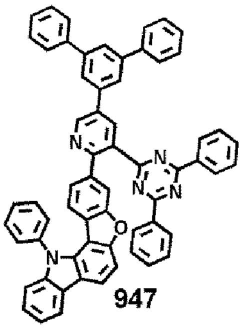

947

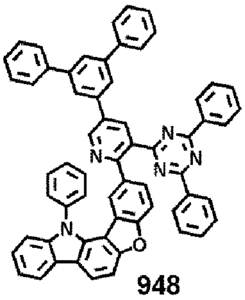

948

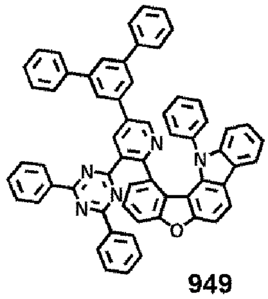

949

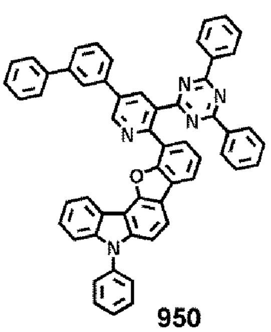

950

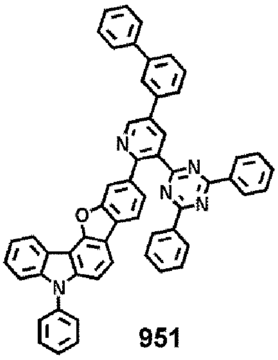

951

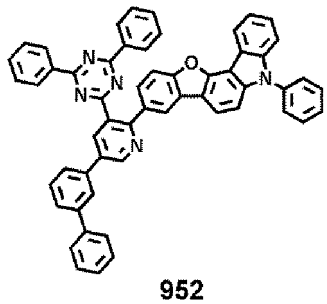

952

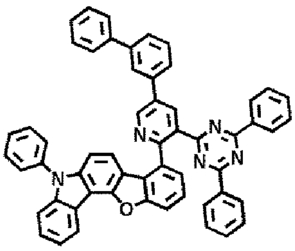

953

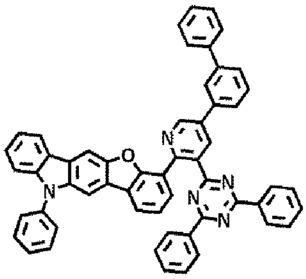

954

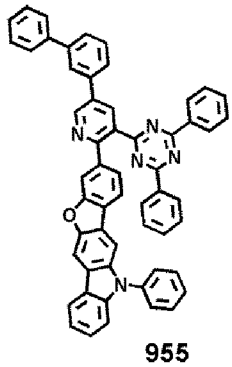

955

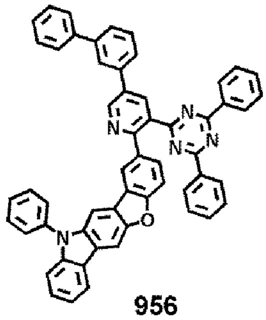

956

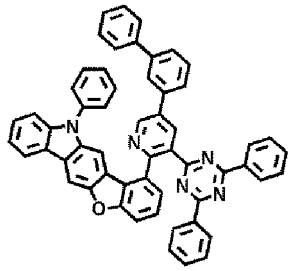

957

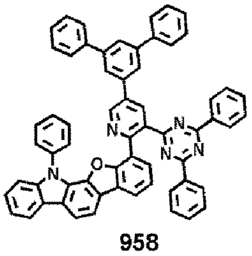

958

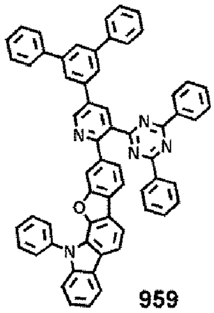

959

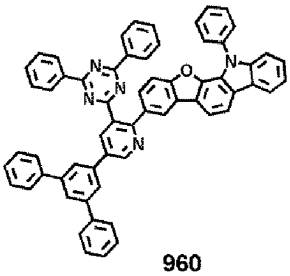

960

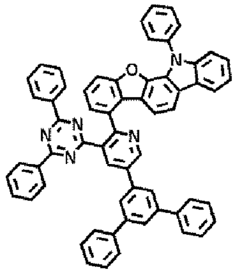

961

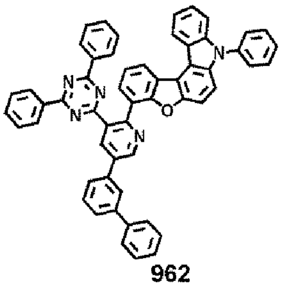

962

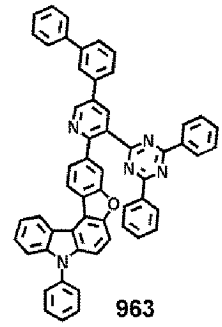

963

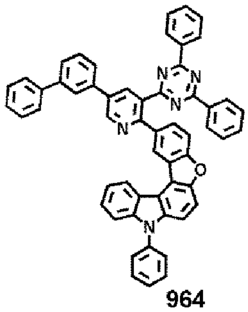

964

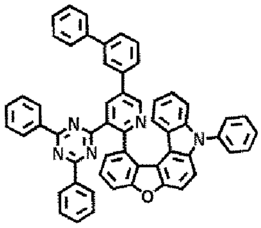

965

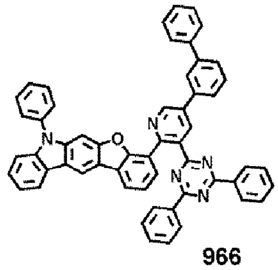

966

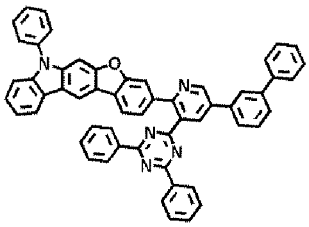

967

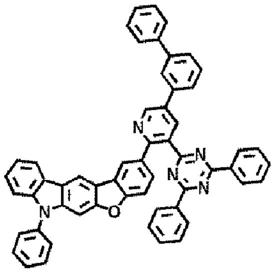

968

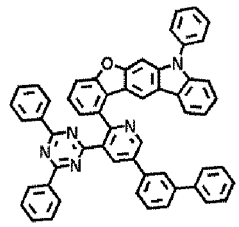

969

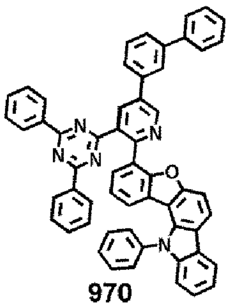

970

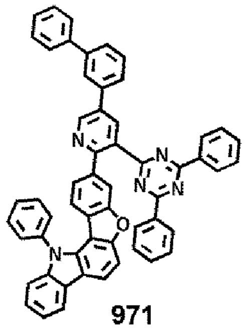

971

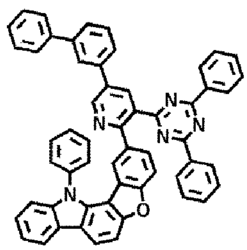

972

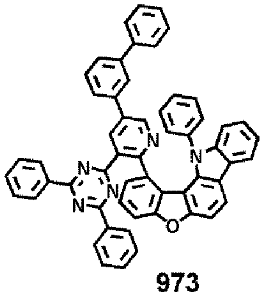

973

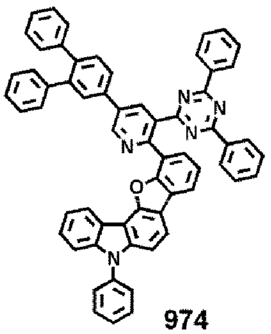

974

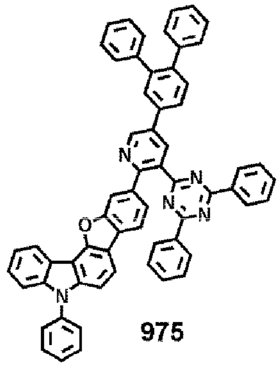

975

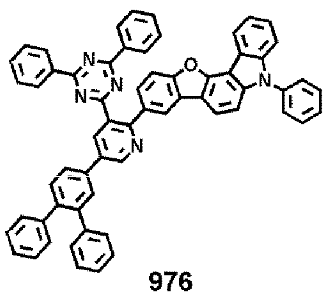

976

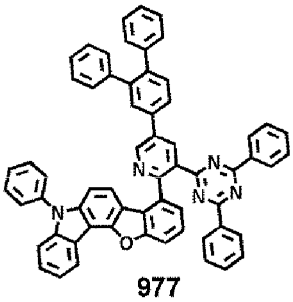

977

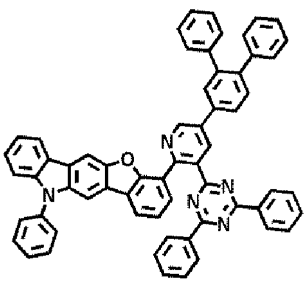

978

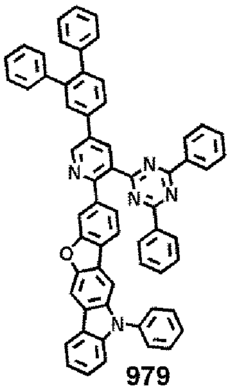

979

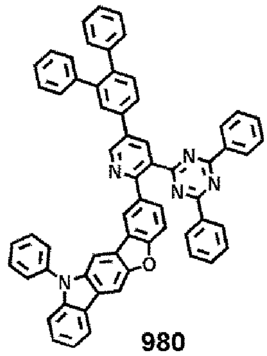

980

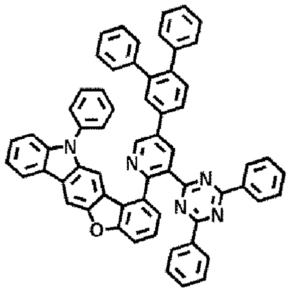

981

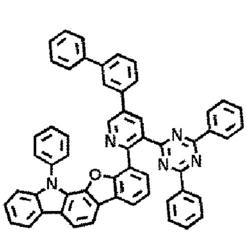

982

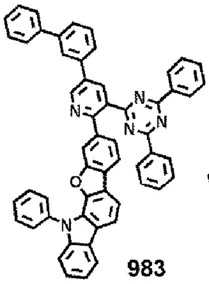

983

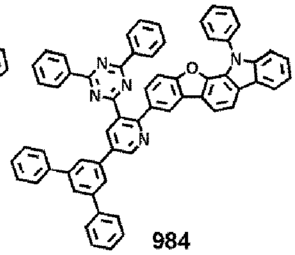

984

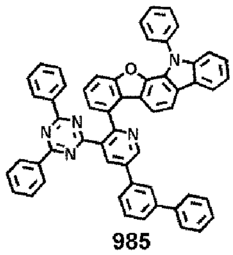

985

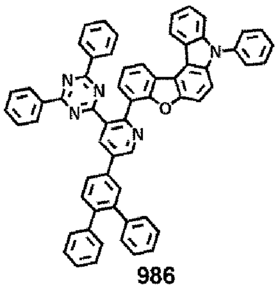

986

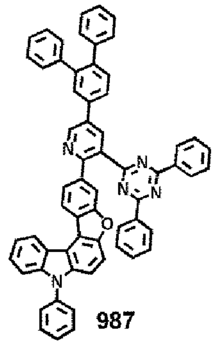

987

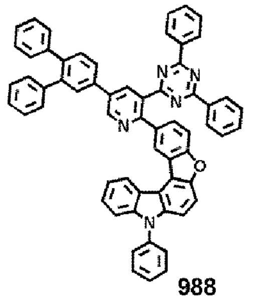

988

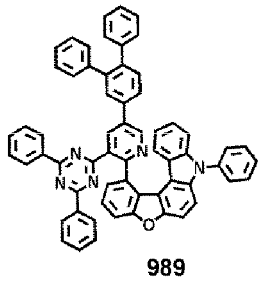

989

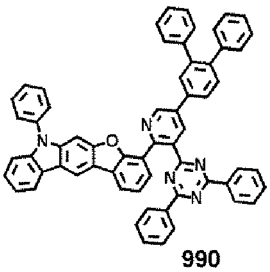

990

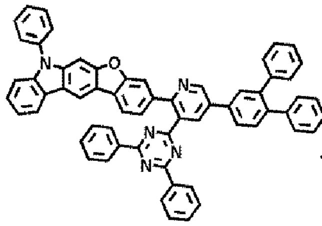

991

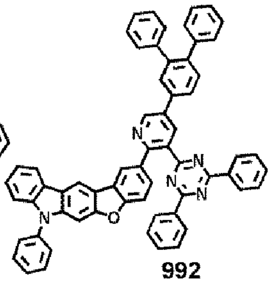

992

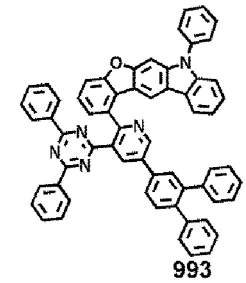

993

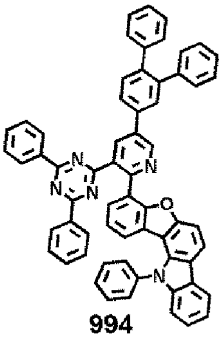

994

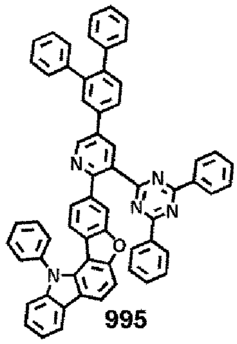

995

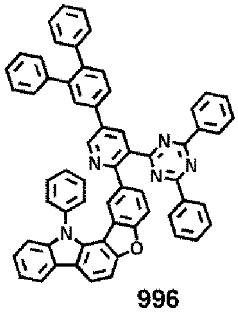

996

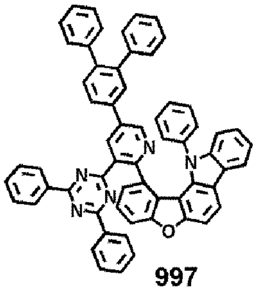

997

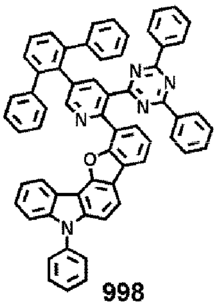

998

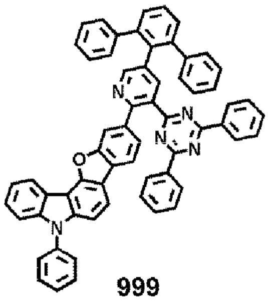

999

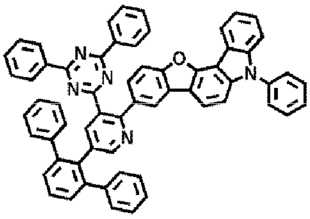

1000

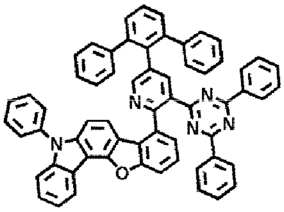

1001

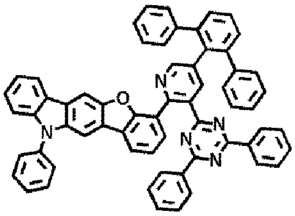

1002

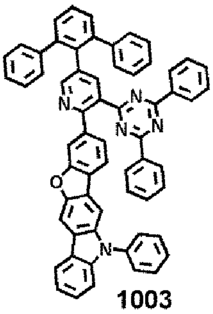

1003

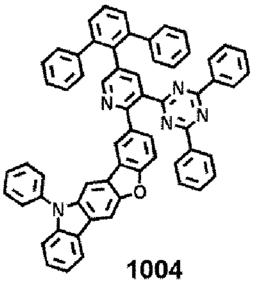

1004

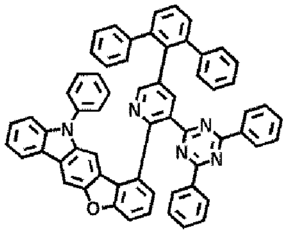

1005

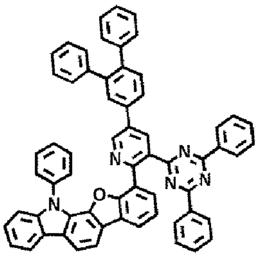

1006

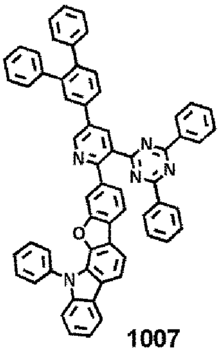

1007

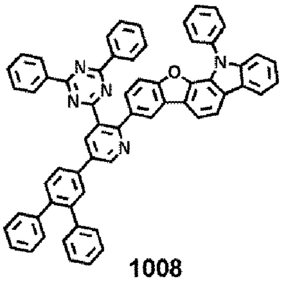

1008

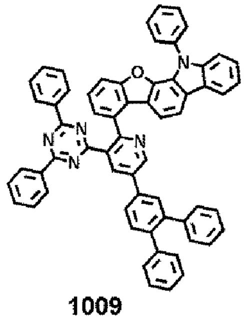

1009

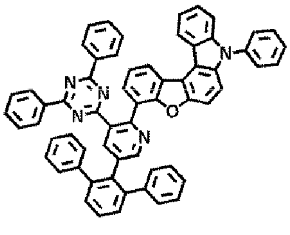

1010

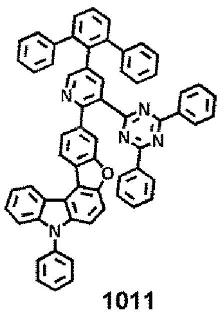

1011

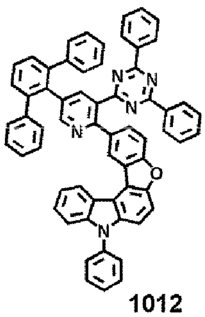

1012

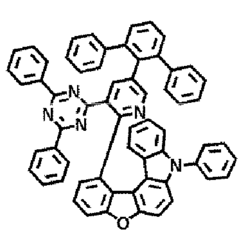

1013

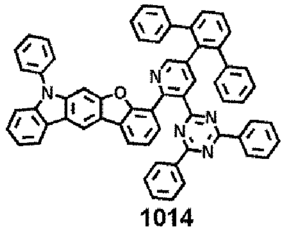
1014

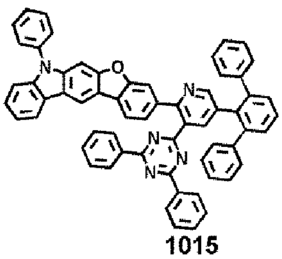
1015

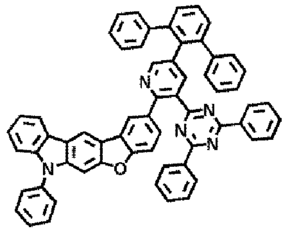
1016

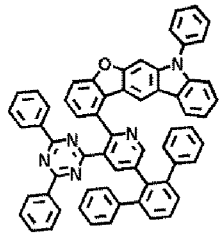
1017

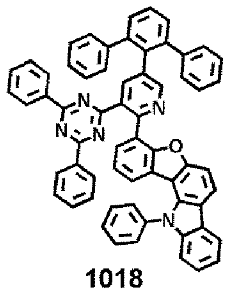
1018

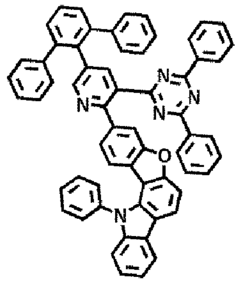
1019

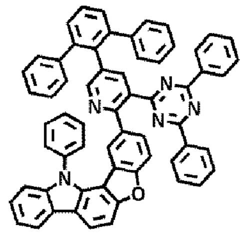
1020

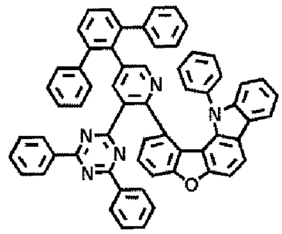
1021

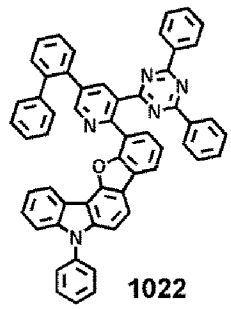
1022

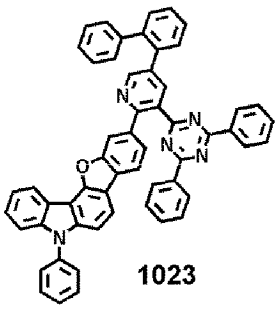
1023

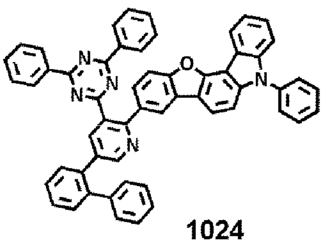
1024

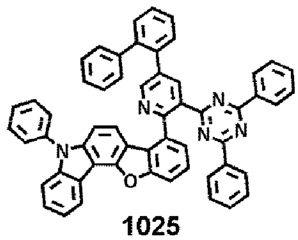
1025

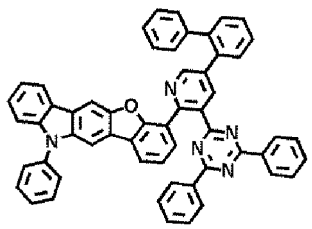
1026

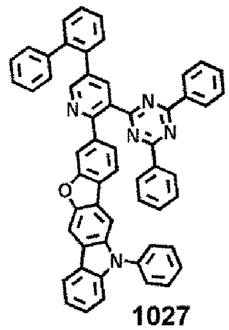
1027

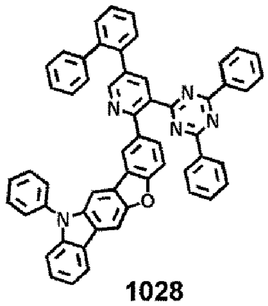
1028

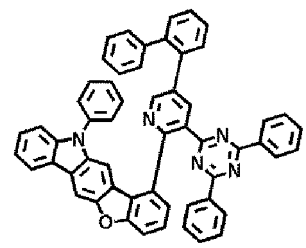
1029

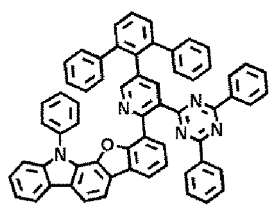
1030

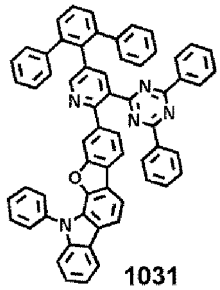
1031

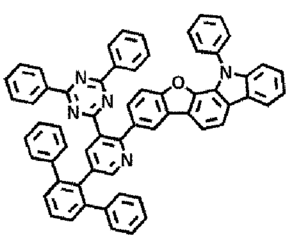
1032

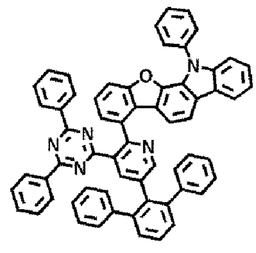
1033

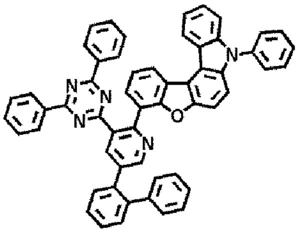
1034

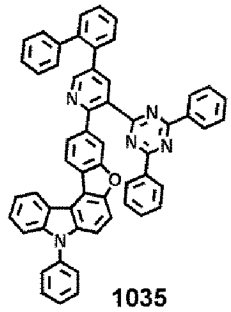
1035

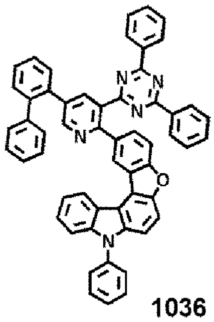
1036

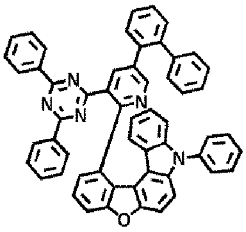
1037

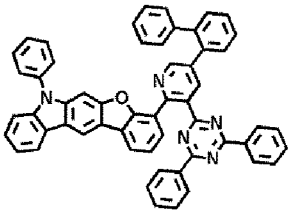
1038

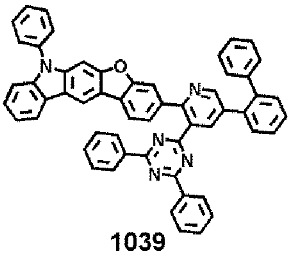
1039

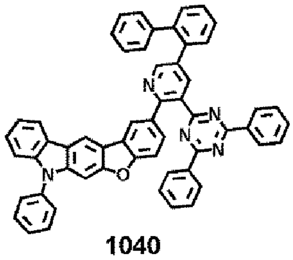
1040

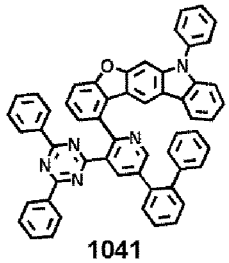
1041

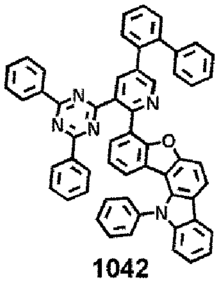
1042

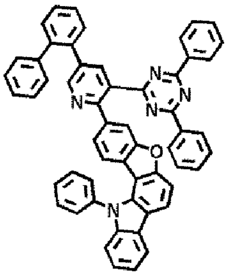
1043

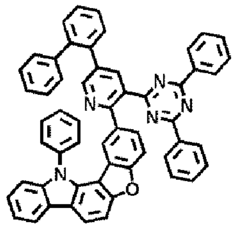
1044

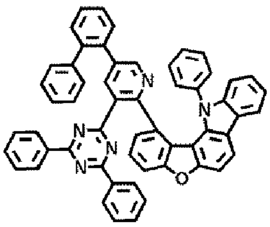
1045

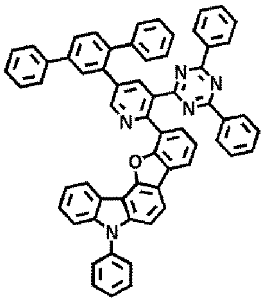
1046

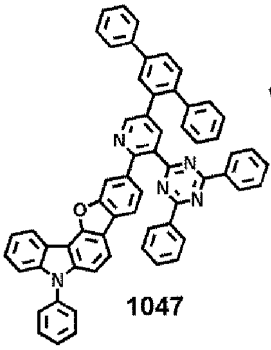
1047

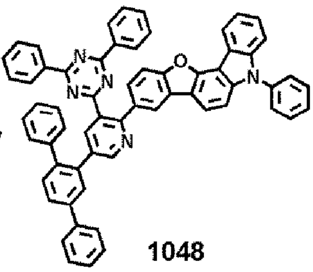
1048

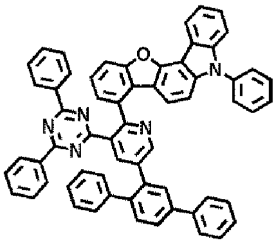
1049

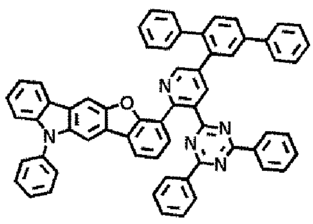
1050

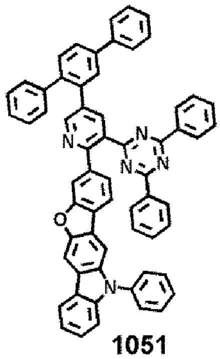
1051

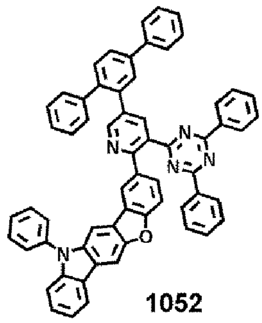
1052

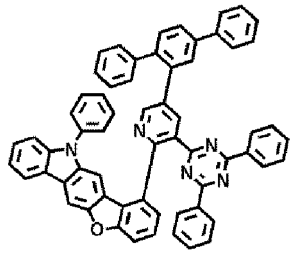
1053

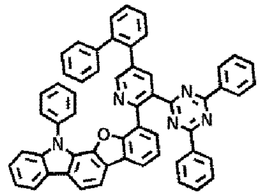
1054

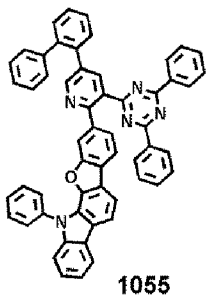
1055

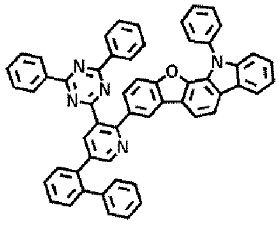
1056

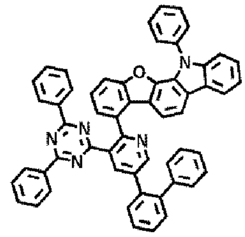
1057

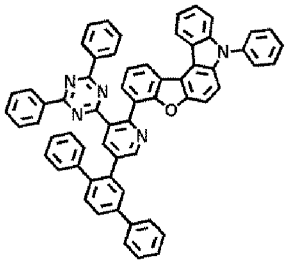
1058

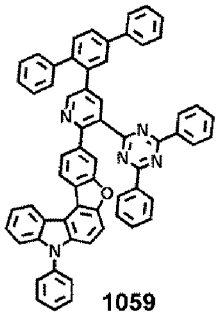
1059

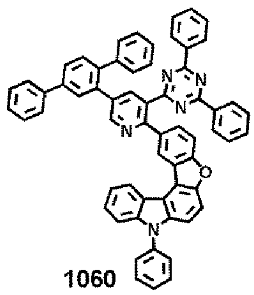
1060

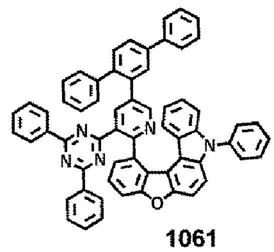
1061

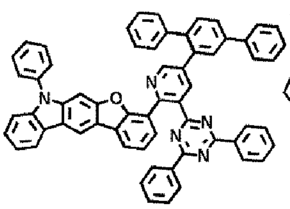
1062

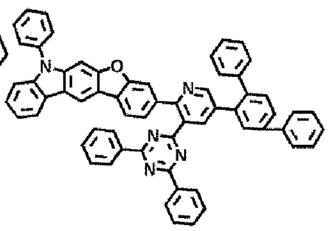
1063

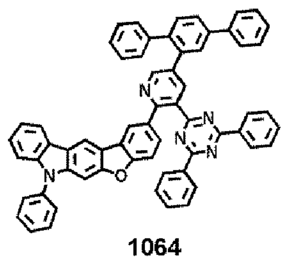
1064

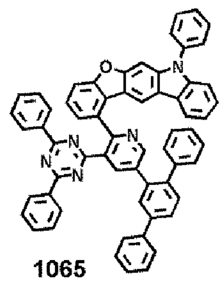
1065

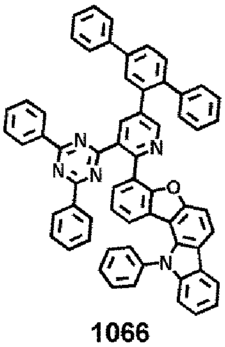

1066

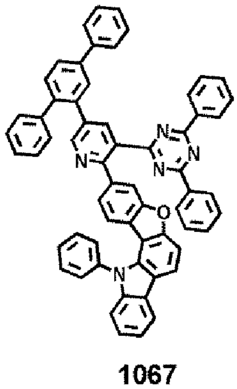

1067

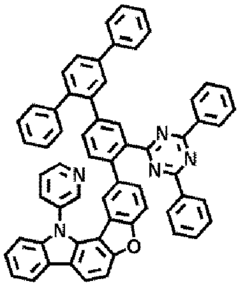

1068

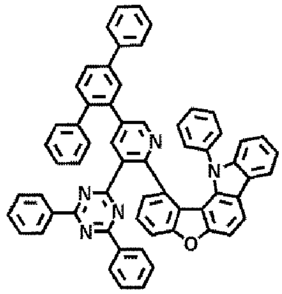

1069

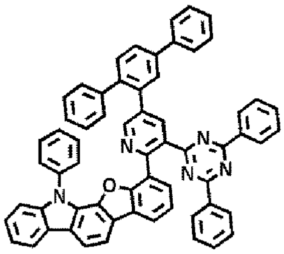

1070

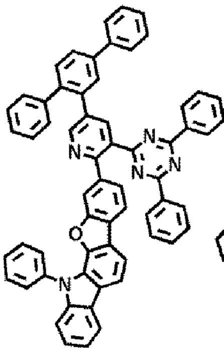

1071

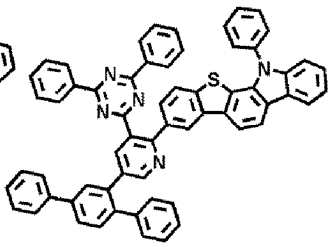

1072

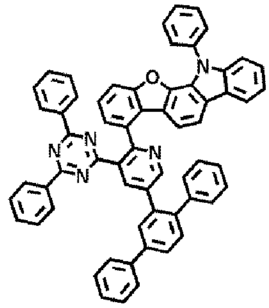

1073

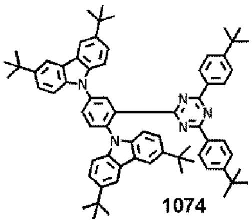

1074

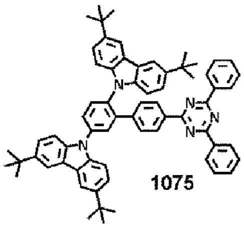

1075

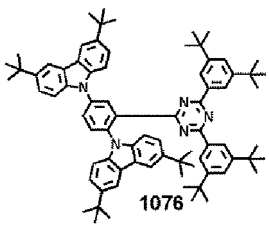

1076

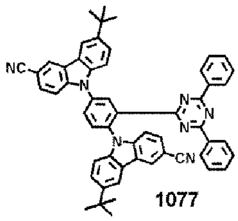

1077

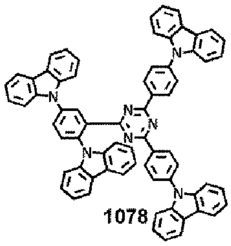

1078

$Ar_1$ and $Ar_2$ in Formula 1 are located at "para" positions to each other as can be confirmed in Formula 1. Therefore, an overlap density of the highest occupied molecular orbital (HOMO)-lowest unoccupied molecular orbital (LUMO) in a molecule of the heterocyclic compound represented by Formula 1 increases, and a value of an oscillator strength (f) increases. As a result, the luminescence efficiency of an electronic device, for example, an organic light-emitting device, which includes the heterocyclic compound, may be improved.

Also, in Formula 1, $X_1$ is N or C($R_1$), $X_2$ is N or C($R_2$), $X_3$ is N or C($R_3$), $R_1$ to $R_3$ are each hydrogen, deuterium, a $C_1$-$C_{20}$ alkyl group, a $C_1$-$C_{20}$ alkyl group substituted with at least one deuterium, a $C_1$-$C_{20}$ alkoxy group, or a $C_1$-$C_{20}$ alkoxy group substituted with at least one deuterium. That is, in Formula 1, $R_1$ to $R_3$ are each a non-cyclic group. Therefore, since the heterocyclic compound represented by Formula 1 may have a relatively low deposition temperature, an electronic device, for example, an organic light-emitting device, which includes the heterocyclic compound, may have excellent lifespan characteristics.

Furthermore, in Formula 1, $X_{11}$ to $X_{13}$ are each N. Therefore, since the heterocyclic compound represented by

Formula 1 has a relatively small decay time (Tau), an electronic device, for example, an organic light-emitting device, which includes the heterocyclic compound, may have excellent lifespan characteristics.

The heterocyclic compound represented by Formula 1 may have a singlet energy level (expressed in electron volts, eV) in a range of about 2.5 eV to about 3.0 eV.

Also, a difference (being an absolute value) between a singlet energy level (eV) of the heterocyclic compound and a triplet energy level (eV) of the heterocyclic compound may be in a range of about 0 eV to about 0.5 eV. Therefore, the heterocyclic compound represented by Formula 1 may emit delayed fluorescence having high efficiency and/or high luminance.

When a difference between a singlet energy level (eV) of the heterocyclic compound represented by Formula 1 and a triplet energy level (eV) of the heterocyclic compound represented by Formula 1 is within this range, up-conversion from a triplet state to a singlet state is effectively performed, and the heterocyclic compound may emit delayed fluorescence having high efficiency.

The singlet energy level and the triplet energy level are evaluated by using a density functional theory (DFT) method (for example, a DFT method of Gaussian program) structurally optimized at a level of B3LYP/6-31G(d,p).

Synthesis methods of the heterocyclic compound represented by Formula 1 may be recognized by those of ordinary skill in the art by referring to Synthesis Examples.

Another embodiment of the present invention provides an organic light-emitting device including: a first electrode; a second electrode; and an organic layer disposed between the first electrode and the second electrode, wherein the organic layer includes an emission layer, and wherein the organic layer includes at least one heterocyclic compound represented by Formula 1.

For example, the emission layer may include the heterocyclic compound represented by Formula 1. The emission layer including the heterocyclic compound may be an emission layer according to a first embodiment or a second embodiment.

First embodiment

The heterocyclic compound represented by Formula 1, which is included in the emission layer, may emit fluorescence (for example, thermally activated delayed fluorescence).

For example, the emission layer may consist of the heterocyclic compound represented by Formula 1.

In an embodiment, the emission layer may include a host and a dopant. The dopant may include the heterocyclic compound represented by Formula 1. An amount of the host may be larger than an amount of the dopant. The host and the dopant may be different from each other.

The heterocyclic compound included in the emission layer may act as an emitter (for example, a thermally activated delayed fluorescence emitter).

The emission layer may not include a phosphorescent dopant. That is, the emission layer may not include a compound capable of emitting light in accordance to a phosphorescence emission mechanism. Therefore, the emission layer may not include a phosphorescence emitter and does not substantially emit phosphorescence. Instead, the emission layer may be a "delayed fluorescence" emission layer that emits delayed fluorescence by transiting a triplet exciton of the heterocyclic compound represented by Formula 1 from a triplet state to a singlet state by reverse intersystem crossing (RISC), followed by transiting to a ground state.

As described above, the "delayed fluorescence" emission layer used herein is distinctly different from a "phosphorescence" emission layer that includes a phosphorescence emitter (for example, a transition metal (for example, an iridium or a platinum) complex) as a dopant and causes an energy transfer from a host to a phosphorescence emitter, without a process of emitting delayed fluorescence by transiting a triplet exciton of the heterocyclic compound represented by Formula 1 from a triplet state to a singlet state by reverse intersystem crossing (RISC), followed by transiting to a ground state.

Second embodiment

The emission layer may include a host and a dopant. The host may include the heterocyclic compound represented by Formula 1. An amount of the host may be larger than an amount of the dopant. The host and the dopant may be different from each other. The heterocyclic compound included in the emission layer may serve to transfer energy to the dopant, instead of being an emitter.

The emission layer (for example, the emission layer according to the first embodiment or the second embodiment) may emit red light, green light, or blue light. For example, the emission layer may emit blue light, but embodiments of the present disclosure are not limited thereto.

A ratio of a delayed fluorescence component emitted from the heterocyclic compound represented by Formula 1 to a total emission component emitted from the emission layer may be 90 % or more, 92 % or more, 94 % or more, 96 % or

more, or 98 % or more, but embodiments of the present disclosure are not limited thereto.

The host in the emission layer in the first embodiment may include at least one selected from a first material and a second material, in addition to the heterocyclic compound represented by Formula 1, and the host in the emission layer in the second embodiment may further include at least one selected from a first material and a second material.

the first material may include at least one π electron-rich cyclic group, and may not include an electron transport moiety,
the second material may include at least one π electron-rich cyclic group and at least one electron transport moiety, and
the electron transport moiety may be selected from a cyano group, a π electron-depleted nitrogen-containing cyclic group, and a group represented by one selected from the following formulae:

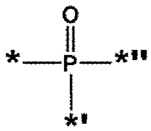 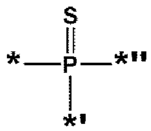 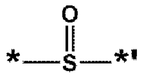 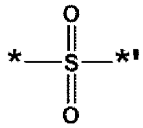 .

In the formulae, *, *', and *" each indicate a binding site to a neighboring atom.

For example, i) the host may consist of at least one compound of the first material, ii) the host may consist of two different compounds of the first material, iii) the host may consist of one compound of the second material, iv) the host may consist of two different compounds of the second material, or v) the host may consist of at least one compound of the first material and at least one compound of the second material. In this manner, the host may be variously modified.

The term "π electron-depleted nitrogen-containing cyclic group" as used herein refers to a cyclic group having at least one *-N=*' moiety. For example, the π electron-depleted nitrogen-containing cyclic group may be an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a benzoisoquinoline group, a phthalazine group, a naphthyridine group, a quinoxaline group, a benzoquinoxaline group, a quinazoline group, a cinnoline group, a phenanthridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, an azaindene group, an azaindole group, an azabenzofuran group, an azabenzothiophene group, an azabenzosilole group, an azafluorene group, an azacarbazole group, an azadibenzofuran group, an azadibenzothiophene group, an azadibenzosilole group, or a cyclic group condensed with any one of the foregoing groups.

The π electron-rich cyclic group may be, for example, a benzene group, a heptalene group, an indene group, a naphthalene group, an azulene group, a heptalene group, an indacene group, acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran group, a benzothiophene group, a benzosilole group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a benzosilolocarbazole group, a triindolobenzene group, an acridine group, or a dihydroacridine group, but embodiments of the present disclosure are not limited thereto.

The first material and the second material may be different from each other.

In an embodiment, the first material and the second material may each independently include at least one carbazole group.

In one or more embodiments, the first material and the second material may each independently include at least two carbazole groups, but embodiments of the present disclosure are not limited thereto.

In one or more embodiments, the second material may include at least one cyano group (for example, one, two, three, or four cyano groups).

In one or more embodiments, the first material may include a cyano group-free benzene group and a cyano group-free carbazole group.

In one or more embodiments, the second material may include at least one cyano group and at least one carbazole ring.

In one or more embodiments, the second material may include at least one of a cyano group-containing benzene

group and a cyano group-containing carbazole group.

In one or more embodiments,

an absolute value of a lowest unoccupied molecular orbital (LUMO) energy level of the first material may be in a range of about 0.90 eV to about 1.20 eV,
an absolute value of a HOMO energy level of the first material may be in a range of about 5.20 eV to about 5.60 eV,
an absolute value of a LUMO energy level of the second material may be in a range of about 1.80 eV to about 2.20 eV,
an absolute value of a HOMO energy level of the second material may be in a range of about 5.40 eV to about 6.00 eV,
but embodiments of the present disclosure are not limited thereto.

When the HOMO and LUMO energy level ranges of the first material and the second material are within these ranges, charge and/or exciton movement and energy flow in the emission layer are smooth, and an organic light-emitting device having high luminescence efficiency and long lifespan may be implemented.

In one or more embodiments, the first material may include at least one selected from a compound represented by Formula H-1(1), a compound represented by Formula H-1(2), and a compound represented by Formula H-1(3):

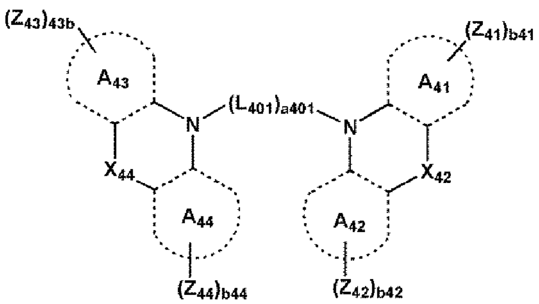

H-1(1)

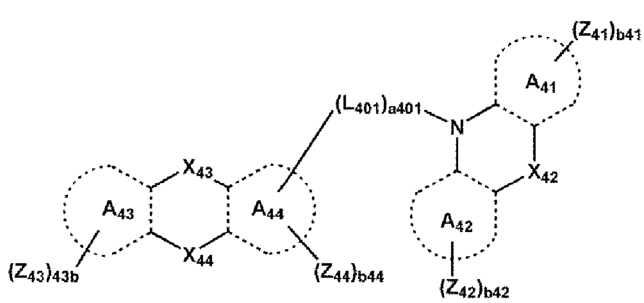

H-1(2)

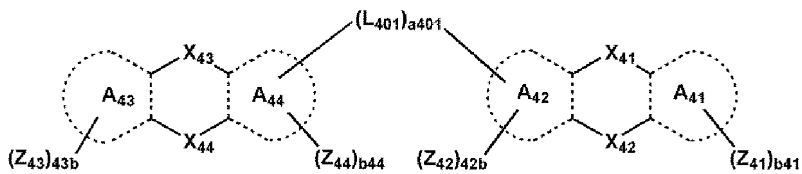

H-1(3)

In Formulae H-1(1) to H-1(3), ring $A_{41}$ to ring $A_{44}$ may each independently be a benzene group, a naphthalene group, an indene group, an indole group, a benzofuran group, a benzothiophene group, a benzosilole group, a fluorene group, a carbazole group, a dibenzofuran group, a dibenzothiophene group, or a dibenzosilole group.

For example, ring $A_{41}$ to ring $A_{44}$ may each independently be a benzene group, a fluorene group, a carbazole group, a dibenzofuran group, a dibenzothiophene group, or a dibenzosilole group, wherein at least one of ring $A_{41}$ and ring $A_{42}$ may be a benzene group, and at least one of ring $A_{43}$ and ring $A_{44}$ may be a benzene group.

In Formulae H-1(1) to H-1(3),

$X_{41}$ may be N-[$(L_{411})_{c411}$-$Z_{411}$], C($Z_{415}$)($Z_{416}$), O, or S,
$X_{42}$ may be a single bond, N-[$(L_{412})_{c412}$-$Z_{412}$], C($Z_{417}$)($Z_{418}$), O, or S,
$X_{43}$ may be N-[$(L_{413})_{c413}$-$Z_{413}$], C($Z_{419}$)($Z_{420}$), O, or S, and
$X_{44}$ may be a single bond, N-[$(L_{414})_{c414}$-$Z_{414}$], C($Z_{421}$)($Z_{422}$), O, or S.

$L_{401}$ and $L_{411}$ to $L_{414}$ may each independently be selected from:

a single bond; and
a $\pi$ electron-rich cyclic group unsubstituted or substituted with at least one $R_{10d}$ (for example, a benzene group, a heptalene group, an indene group, a naphthalene group, an azulene group, a heptalene group, an indacene group, acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene

group, a pentaphene group, a rubicene group, a coronene group, an ovalene group, a pyrrole group, an isoindole group, an indole group, a furan group, a thiophene group, a benzofuran group, a benzothiophene group, a benzosilole group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a benzosilolocarbazole group, a triindolobenzene group, an acridine group, or a dihydroacridine group, each unsubstituted or substituted with at least one $R_{10d}$).

Each of a401 and c411 to c414 indicates the number of each of $L_{401}$ and $L_{411}$ to $L_{414}$, respectively, and may independently be an integer from 1 to 10 (for example, an integer from 1 to 5). When a401 is two or more, two or more of groups $L_{401}$ may be identical to or different from each other, when c411 is two or more, two or more of groups $L_{411}$ may be identical to or different from each other, when c412 is two or more, two or more of groups $L_{412}$ may be identical to or different from each other, when c413 is two or more, two or more of groups $L_{413}$ may be identical to or different from each other, and when c414 is two or more, two or more of groups $L_{414}$ may be identical to or different from each other.

$Z_{41}$ to $Z_{44}$ and $Z_{411}$ to $Z_{422}$ may each independently be selected from:

hydrogen, deuterium, a $C_1$-$C_{20}$ alkyl group, and a $C_1$-$C_{20}$ alkoxy group; and
a $\pi$ electron-rich cyclic group unsubstituted or substituted with at least one $R_{10d}$ (for example, a phenyl group, a biphenyl group, a terphenyl group, a tetraphenyl group, a naphthyl group, a fluorenyl group, a spiro-bifluorenyl group, a benzofluorenyl group, a dibenzofluorenyl group, a phenanthrenyl group, an anthracenyl group, a triphenylenyl group, a pyrenyl group, a chrysenyl group, a pyrrolyl group, an isoindolyl group, an indolyl group, a furanyl group, a thiophenyl group, a benzofuranyl group, a benzothiophenyl group, a benzosilolyl group, a benzocarbazolyl group, a dibenzocarbazolyl group, a dibenzofuranyl group, a dibenzothiophenyl group, a carbazolyl group, a dibenzosilolyl group, an indenocarbazolyl group, an indolocarbazolyl group, a benzofurocarbazolyl group, a benzothienocarbazolyl group, a benzosilolocarbazolyl group, an acridinyl group, or a dihydroacridinyl group, each unsubstituted or substituted with at least one $R_{10d}$).

Each of b41 to b44 indicates the number of each of $Z_{41}$ to $Z_{44}$, respectively, and may independently be 1, 2, 3, or 4.

$R_{10d}$ may be defined the same as $R_{10a}$, but $R_{10d}$ is not a cyano group.

In an embodiment, in Formulae H-1(1) to H-1(3),
$L_{401}$ and $L_{411}$ to $L_{414}$ may each independently be selected from:

a single bond; and
a benzene group, a fluorene group, a dibenzofuran group, a dibenzothiophene group, a carbazole group, a dibenzosilole group, an indenocarbazole group, an indolocarbazole group, a benzofurocarbazole group, a benzothienocarbazole group, a benzosilolocarbazole group, an acridine group, or a dihydroacridine group, each unsubstituted or substituted with at least one $R_{10d}$,
$Z_{41}$ to $Z_{44}$ and $Z_{411}$ to $Z_{422}$ may each independently be selected from:

hydrogen, deuterium, a $C_1$-$C_{10}$ alkyl group, and a $C_1$-$C_{10}$ alkoxy group; and
a phenyl group, a biphenyl group, a terphenyl group, a tetraphenyl group, a fluorenyl group, a dibenzocarbazolyl group, a dibenzofuranyl group, a dibenzothiophenyl group, a carbazolyl group, a dibenzosilolyl group, an indenocarbazolyl group, an indolocarbazolyl group, a benzofurocarbazolyl group, a benzothienocarbazolyl group, a benzosilolocarbazolyl group, an acridinyl group, or a dihydroacridinyl group, each unsubstituted or substituted with at least one $R_{10d}$,
but embodiments of the present disclosure are not limited thereto.

In an embodiment, the first material may include at least one compound selected from Compounds H1 to H32, but embodiments of the present disclosure are not limited thereto:

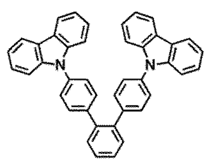
H1

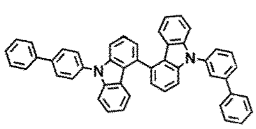
H2

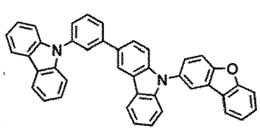
H3

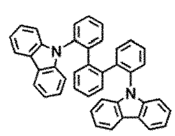
H4

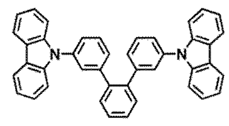
H5

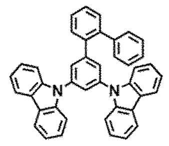
H6

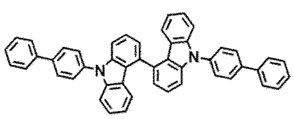
H7

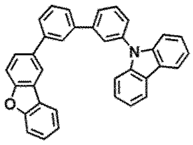
H8

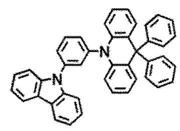
H9

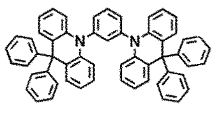
H10

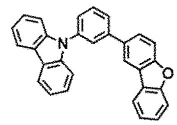
H11

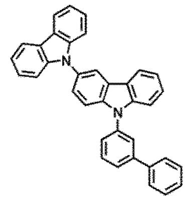
H12

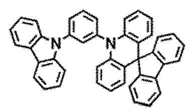
H13

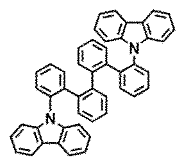
H14

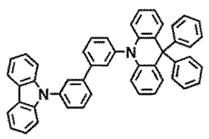
H15

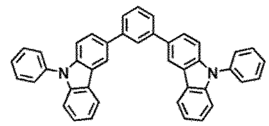
H16

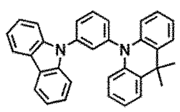
H17

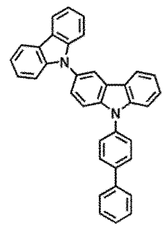
H18

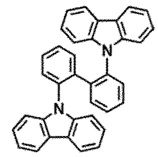
H19

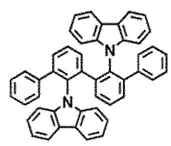
H20

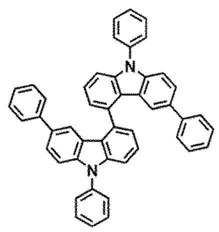
H21

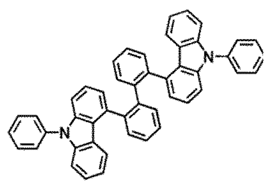
H22

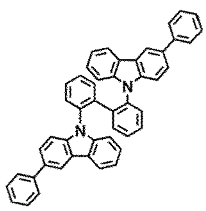
H23

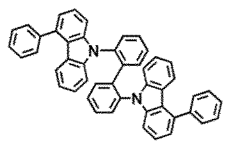
H24

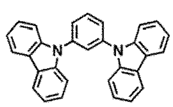
H25

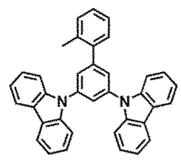
H26

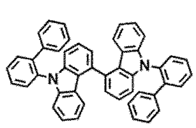
H27

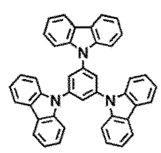
H28

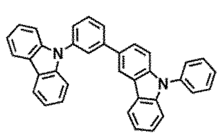
H29

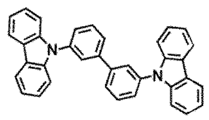
H30

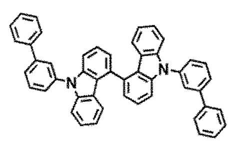
H31

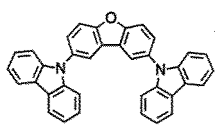
H32

In an embodiment, the first material may not be an amine-based compound.

In one or more embodiments, the first material may not be 1,3-bis(9-carbazolyl)benzene (mCP), tris(4-carbazoyl-9-ylphenyl)amine (TCTA), 4,4'-bis(N-carbazolyl)-1,1'-biphenyl (CBP), 3,3-bis(carbazo-9-yl)biphenyl (mCBP), N,N'-di(1-naphthyl)-N,N'-diphenyl-(1,1'-biphenyl)-4,4'-diamine (NPB), 4,4',4"-tris[phenyl(m-tolyl)amino]triphenylamine (m-MTDATA), or N,N'-bis(3-methylphenyl)-N,N'-diphenylbenzidine (TPD).

In one or more embodiments, the second material may include a compound represented by Formula E-1:

Formula E-1 $[Ar_{301}]_{xb11}$-$[(L_{301})_{xb1}$-$R_{301}]_{xb21}$.

In Formula E-1,

$Ar_{301}$ may be selected from a substituted or unsubstituted $C_5$-$C_{60}$ carbocyclic group and a substituted or unsubstituted $C_1$-$C_{60}$ heterocyclic group,
xb11 may be 1, 2, or 3,
$L_{301}$ may be selected from a single bond, a group represented by one selected from the following formulae, a substituted or unsubstituted $C_5$-$C_{60}$ carbocyclic group, and a substituted or unsubstituted $C_1$-$C_{60}$ heterocyclic group, wherein *, *', and *" in the following formulae each indicate a binding site to a neighboring atom,

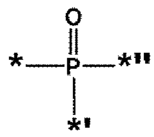
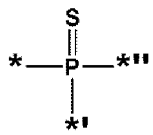
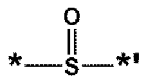
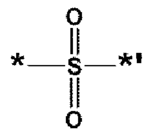
,

xb1 may be an integer from 1 to 5,
$R_{301}$ may be selected from hydrogen, deuterium, -F, -Cl, -Br, -I, a hydroxyl group, a cyano group, a nitro group, an amidino group, a hydrazino group, a hydrazono group, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_7$-$C_{60}$ arylalkyl group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted $C_2$-$C_{60}$ heteroarylalkyl group, a substituted or unsubstituted monovalent non-aromatic condensed polycyclic group, a substituted or unsubstituted monovalent non-aromatic condensed heteropolycyclic group, $-Si(Q_{301})(Q_{302})(Q_{303})$, $-N(Q_{301})(Q_{302})$, $-B(Q_{301})(Q_{302})$, $-C(=O)(Q_{301})$, $-S(=O)_2(Q_{301})$, $-S(=O)(Q_{301})$, $-P(=O)(Q_{301})(Q_{302})$, and $-P(=S)(Q_{301})(Q_{302})$,
xb21 may be an integer from 1 to 5, and
$Q_{301}$ to $Q_{303}$ may each independently be selected from a $C_1$-$C_{10}$ alkyl group, a $C_1$-$C_{10}$ alkoxy group, a phenyl group, a biphenyl group, a terphenyl group, and a naphthyl group, and satisfies one of Condition 1 to Condition 3:

Condition 1
Each of at least one selected from $Ar_{301}$, $L_{301}$, and $R_{301}$ in Formula E-1 may independently include a $\pi$ electron-depleted nitrogen-containing cyclic group
Condition 2 At least one $L_{301}$ in Formula E-1 may include a group represented by one selected from the following formulae:

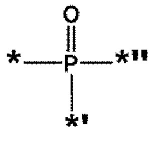
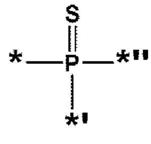
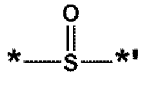
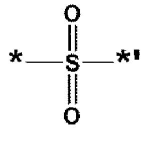

Condition 3 At least one $R_{301}$ in Formula E-1 may be selected from a cyano group, $-S(=O)_2(Q_{301})$, $-S(=O)(Q_{301})$, $-P(=O)(Q_{301})(Q_{302})$, and $-P(=S)(Q_{301})(Q_{302})$.

In one or more embodiments, the second material may include at least one selected from a compound

represented by Formula E-1(1), a compound represented by Formula E-1(2), and a compound represented by Formula E-1(3):

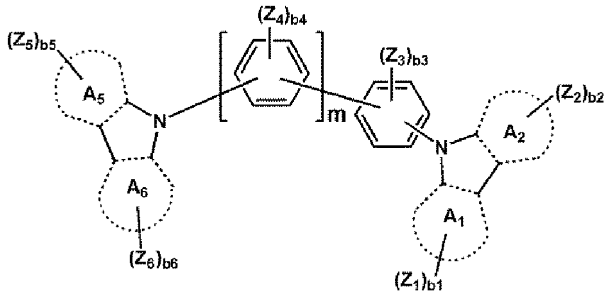

E-1(1)

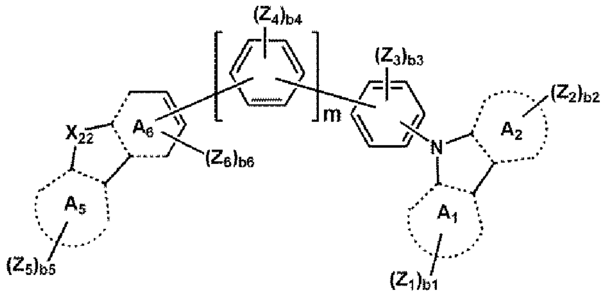

E-1(2)

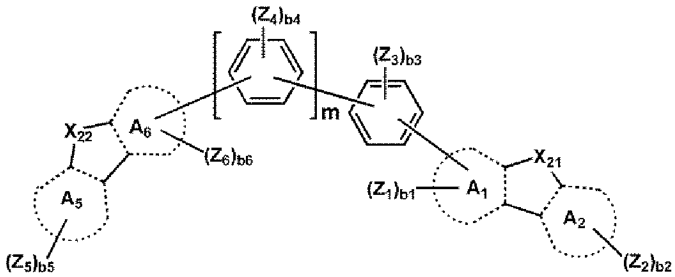

E-1(3)

.

In Formulae E-1(1) to E-1(3),
ring $A_1$, ring $A_2$, ring $A_5$, and ring $A_6$ may each independently be selected from a benzene group, a naphthalene group, an anthracene group, a phenanthrene group, a triphenylene group, a pyrene group, a chrysene group, a cyclopentadiene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, and a phenanthroline group.

For example, ring $A_1$, ring $A_2$, ring $A_5$, and ring $A_6$ may each independently be selected from a benzene group, a carbazole group, a fluorene group, a dibenzothiophene group, and a dibenzofuran group.

In Formulae E-1(1) to E-1(3), $Z_1$ to $Z_6$ may each independently be selected from:

hydrogen, deuterium, and a cyano group; or
a $C_1$-$C_{20}$ alkyl group, a phenyl group, a biphenyl group, a terphenyl group, a dibenzofuranyl group, and a dibenzothiophenyl group, each unsubstituted or substituted with at least one selected from deuterium, a cyano group, a $C_1$-$C_{20}$ alkyl group, a phenyl group, and a biphenyl group.

For example, in Formulae E-1(1) to E-1(3), $Z_1$ to $Z_6$ may each independently be selected from:

hydrogen, deuterium, and a cyano group; or
a $C_3$-$C_{10}$ alkyl group, a phenyl group, a biphenyl group, a terphenyl group, a dibenzofuranyl group, and a dibenzothiophenyl group, each unsubstituted or substituted with at least one selected from deuterium, a cyano group, a $C_3$-$C_{10}$ alkyl group, a phenyl group, and a biphenyl group.

In an embodiment, in Formulae E-1(1) to E-1(3), $Z_1$ to $Z_6$ may each independently be selected from:

hydrogen, deuterium, and a cyano group; or
an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, a biphenyl group, and a terphenyl group, each unsubstituted or substituted with at least one selected from deuterium, a cyano group, an n-propyl group, an iso-propyl group, an n-butyl group, an iso-butyl group, a sec-butyl group, a tert-butyl group, an n-pentyl group, an iso-pentyl group, a sec-pentyl group, a tert-pentyl group, a phenyl group, and a biphenyl group.

In Formulae E-1(1) to E-1(3), b1 to b6 respectively indicate the number of groups $Z_1$ to groups $Z_6$, and may each independently be 1, 2, or 3. When b1 to b6 are two or more, two or more groups $Z_1$ to groups $Z_6$ may be identical to or different from each other.
In an embodiment, in Formulae E-1(1) to E-1(3), at least one selected from groups $Z_1$ in the number of b1, groups $Z_2$ in the number of b2, groups $Z_3$ in the number of b3, groups $Z_4$ in the number of b4, groups $Z_5$ in the number of b5, and groups $R_6$ in the number of b6 may be a cyano group.
For example, the number of cyano groups included in the compound represented by Formula E-1(1), the number of cyano groups included in the compound represented by Formula E-1(2), and the number of cyano groups included in the compound represented by Formula E-1(3) may each independently be 1, 2, or 3, but embodiments of the present disclosure are not limited thereto.
In an embodiment, in Formulae E-1(1) to E-1(3),

at least one selected from groups $Z_1$ in the number of b1 and groups $Z_2$ in the number of b2 may be a cyano group,
at least one selected from groups $Z_3$ in the number of b3 and groups $Z_4$ in the number of b4 may be a cyano group,
at least one selected from groups $Z_5$ in the number of b5 and groups $Z_6$ in the number of b6 may be a cyano group,
at least one selected from groups $Z_1$ in the number of b1 and groups $Z_2$ in the number of b2 may be a cyano group, and at least one selected from groups $Z_3$ in the number of b3 and groups $Z_4$ in the number of b4 may be a cyano group,
at least one selected from groups $Z_1$ in the number of b1 and groups $Z_2$ in the number of b2 may be a cyano group, and at least one selected from groups $Z_5$ in the number of b5 and groups $Z_6$ in the number of b6 may be a cyano group,
at least one selected from groups $Z_3$ in the number of b3 and groups $Z_4$ in the number of b4 may be a cyano group, and at least one selected from groups $Z_5$ in the number of b5 and groups $Z_6$ in the number of b6 may be a cyano group, or
at least one selected from groups $Z_1$ in the number of b1 and groups $Z_2$ in the number of b2 may be a cyano group, at least one selected from groups $Z_3$ in the number of b3 and groups $Z_4$ in the number of b4 may be a cyano group, and at least one selected from groups $Z_5$ in the number of b5 and groups $Z_6$ in the number of b6 may be a cyano group.

In Formulae E-1(1) to E-1(3), $X_{21}$ and $X_{22}$ may each independently be O or S, and m may be 0 or 1.
In an embodiment, a group represented by

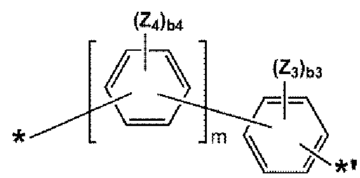

in Formulae E-1(1) to E-1(3) may be one selected from groups represented by Formulae PO1 to PO25, PM1 to PM25, PP1 to PP18, MO1 to MO37, MM1 to MM37, MP1 to MP25, OO1 to OO37, OM1 to OM37, OP1 to OP25, O1 to 016, M1 to M16, and P1 to P9:

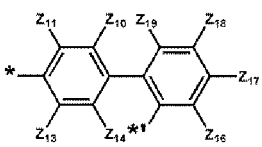
PO1

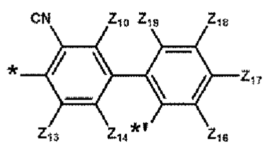
PO2

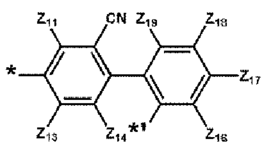
PO3

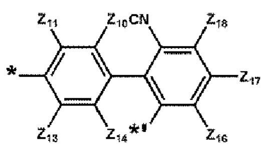
PO4

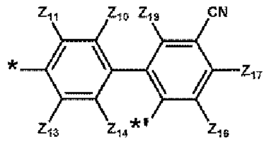
PO5

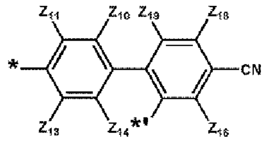
PO6

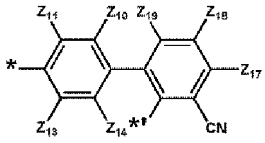
PO7

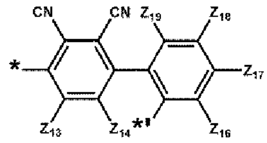
PO8

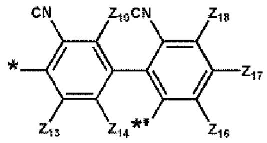
PO9

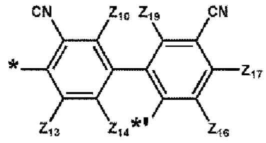
PO10

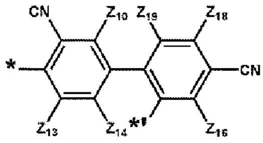
PO11

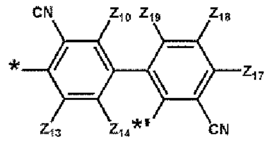
PO12

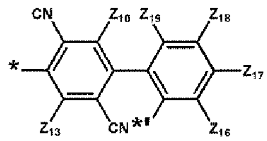
PO13

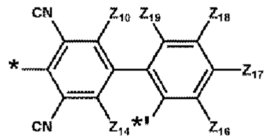
PO14

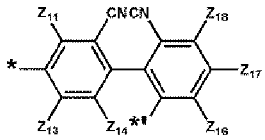
PO15

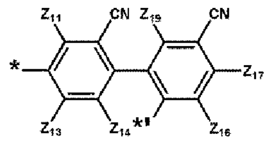
PO16

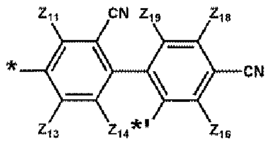
PO17

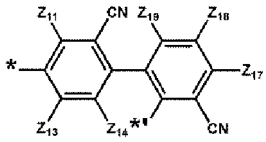
PO18

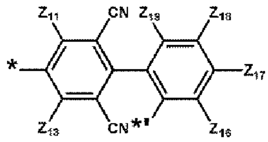
PO19

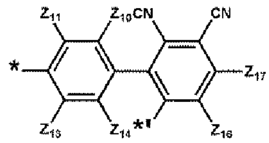
PO20

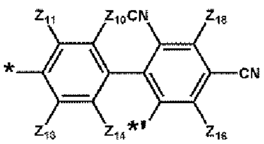
PO21

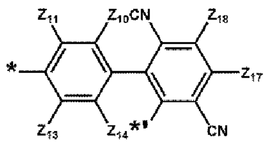
PO22

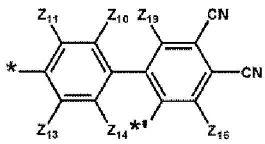
PO23

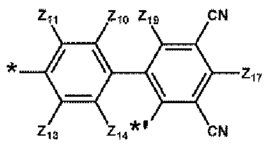
PO24

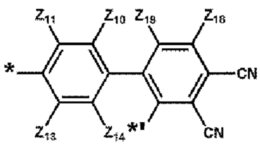
PO25

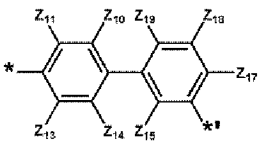
PM1

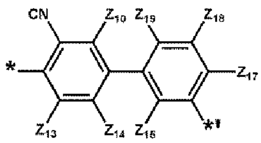
PM2

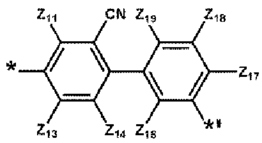
PM3

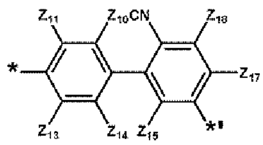
PM4

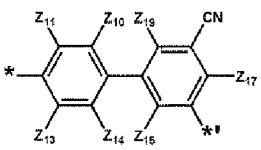
PM5

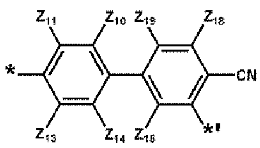
PM6

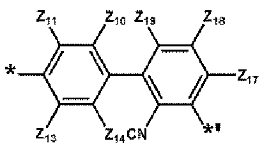
PM7

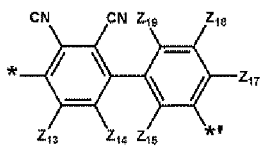
PM8

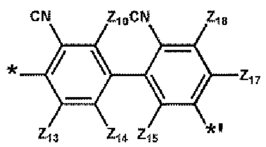
PM9

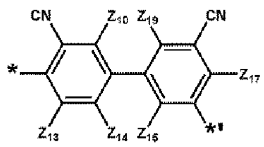
PM10

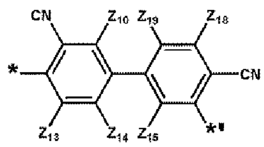
PM11

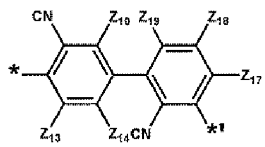
PM12

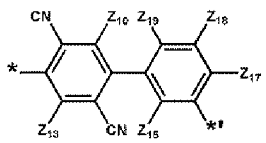
PM13

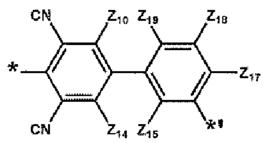
PM14

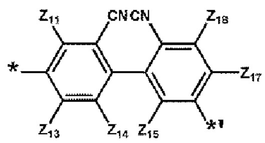
PM15

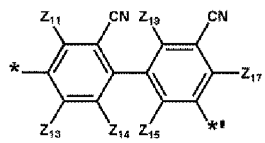
PM16

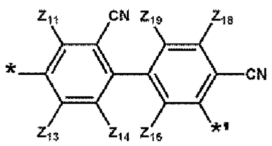
PM17

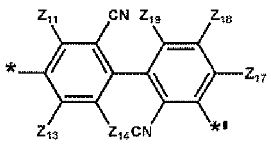
PM18

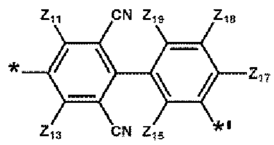
PM19

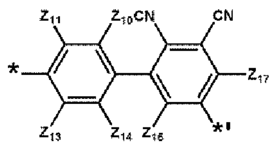
PM20

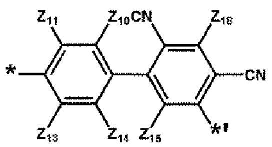
PM21

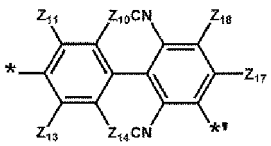
PM22

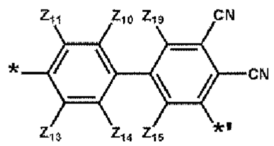
PM23

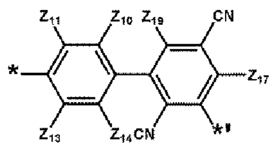
PM24

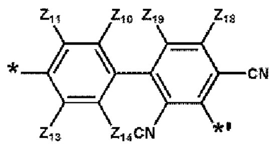
PM25

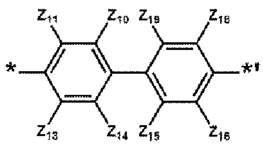
PP1

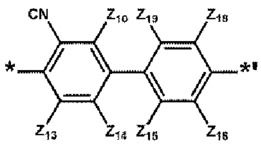
PP2

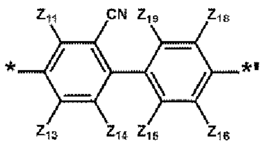
PP3

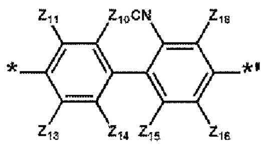
PP4

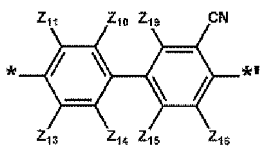
PP5

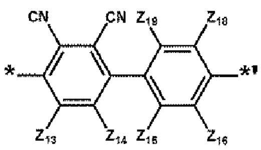
PP6

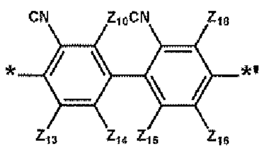
PP7

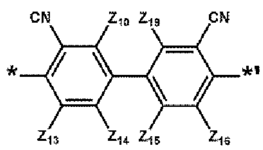
PP8

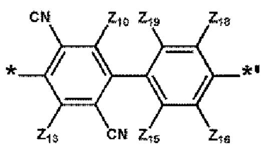
PP9

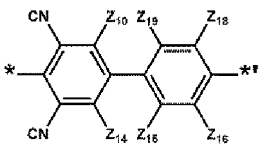
PP10

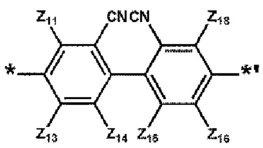
PP11

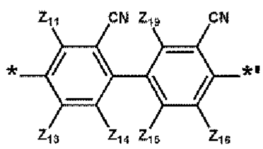
PP12

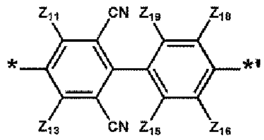
PP13

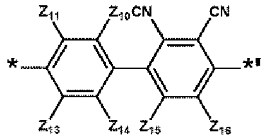
PP14

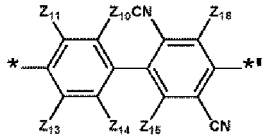
PP15

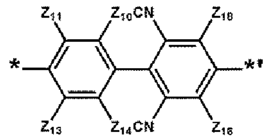
PP16

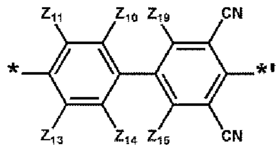
PP17

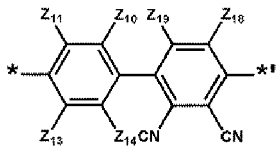
PP18

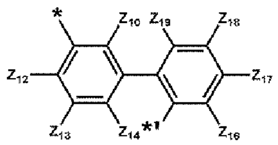
MO1

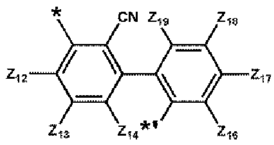
MO2

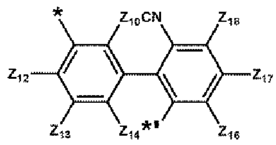
MO3

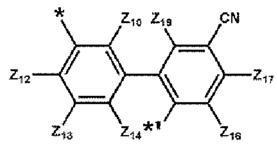
MO4

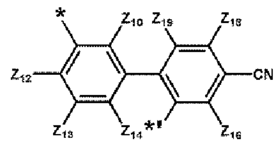
MO5

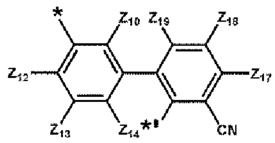
MO6

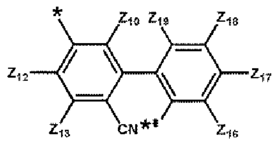
MO7

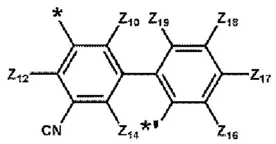
MO8

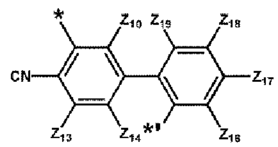
MO9

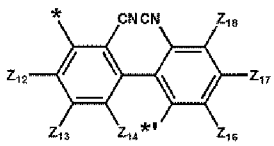
MO10

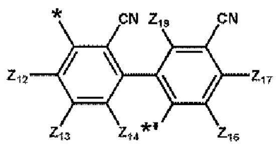
MO11

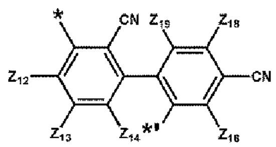
MO12

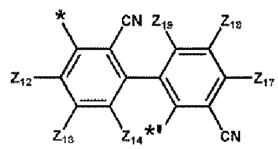
MO13

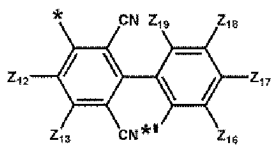
MO14

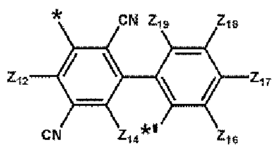
MO15

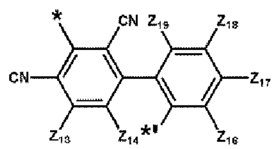
MO16

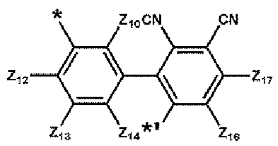
MO17

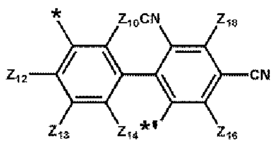
MO18

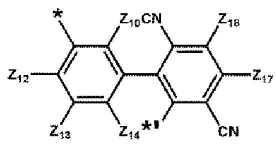
MO19

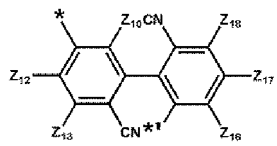
MO20

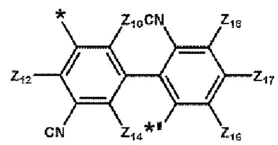
MO21

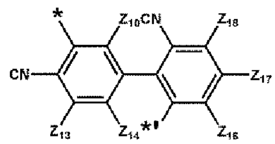
MO22

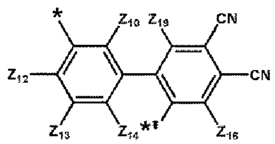
MO23

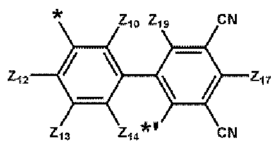
MO24

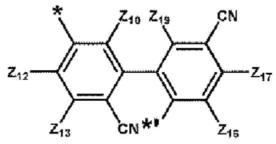
MO25

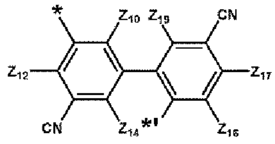
MO26

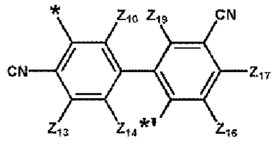
MO27

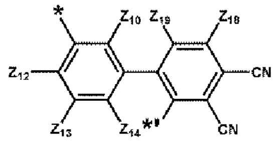
MO28

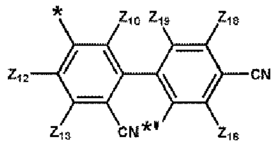
MO29

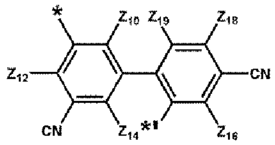
MO30

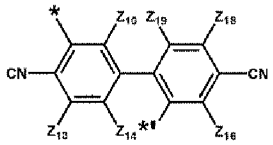
MO31

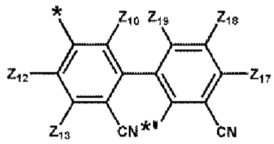
MO32

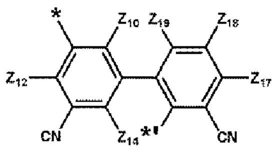
MO33

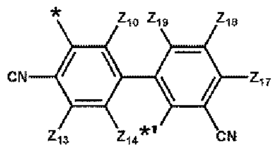
MO34

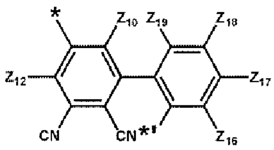
MO35

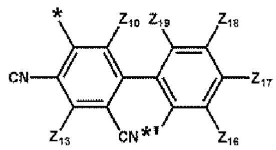
MO36

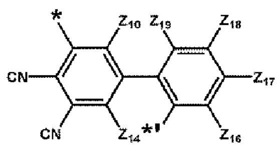
MO37

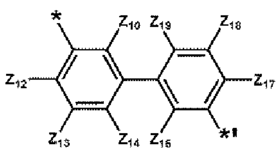
MM1

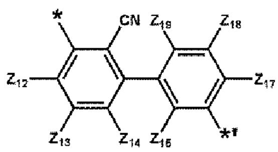
MM2

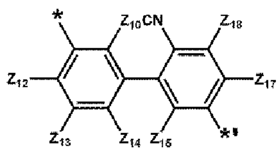
MM3

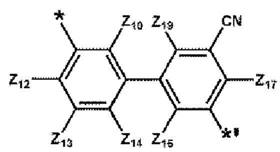
MM4

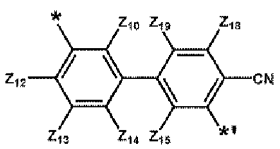
MM5

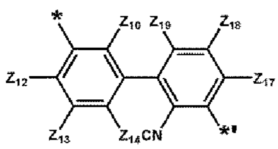
MM6

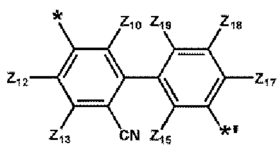
MM7

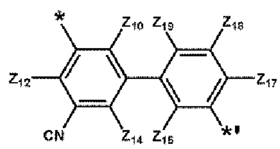
MM8

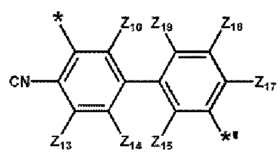
MM9

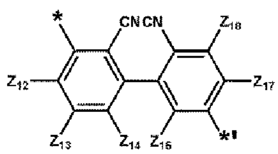
MM10

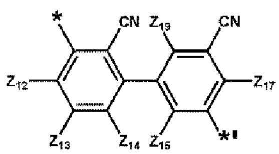
MM11

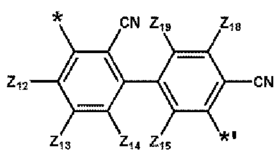
MM12

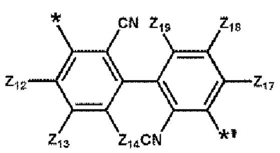
MM13

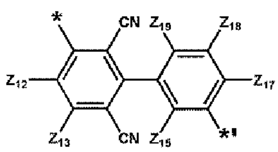
MM14

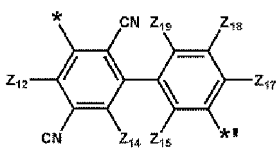
MM15

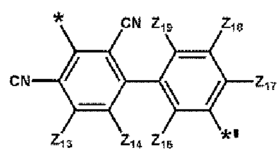
MM16

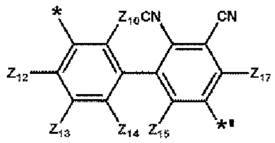
MM17

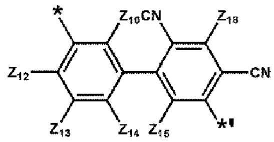
MM18

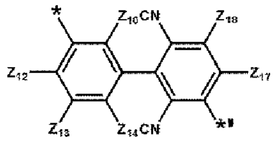
MM19

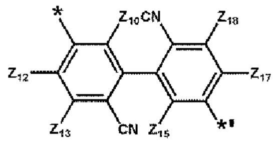
MM20

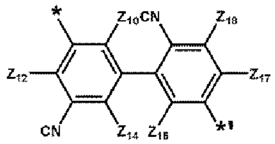
MM21

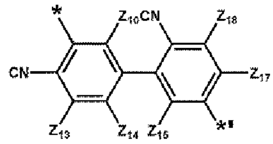
MM22

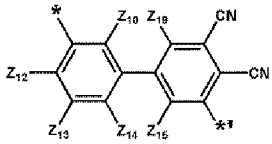
MM23

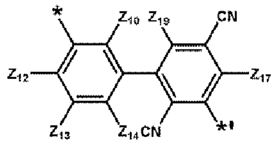
MM24

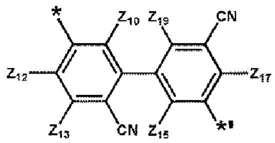
MM25

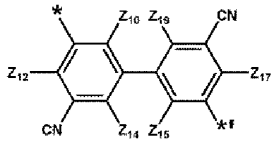
MM26

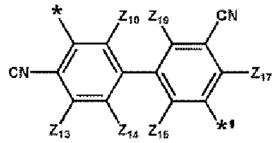
MM27

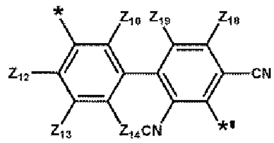
MM28

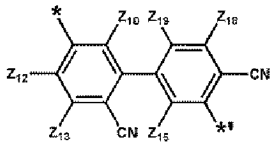
MM29

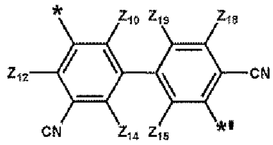
MM30

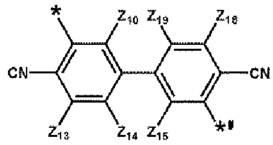
MM31

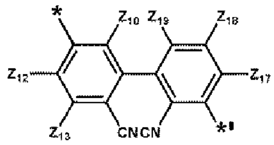
MM32

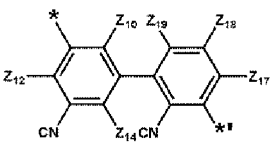
MM33

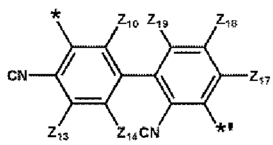
MM34

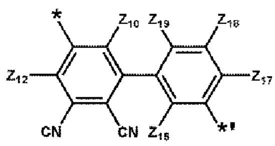
MM35

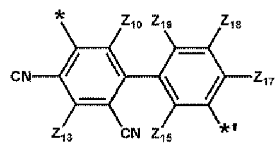
MM36

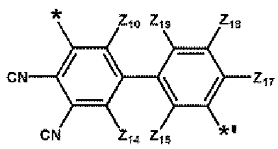
MM37

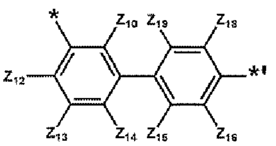
MP1

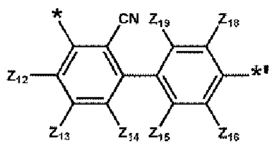
MP2

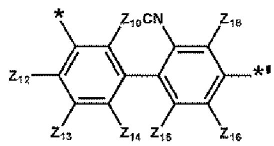
MP3

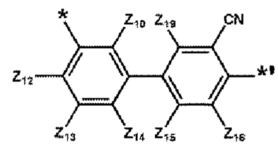
MP4

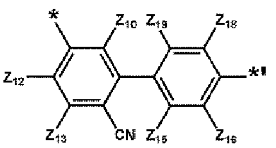
MP5

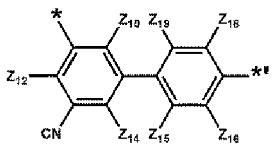
MP6

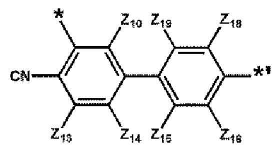
MP7

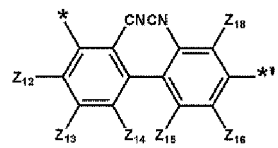
MP8

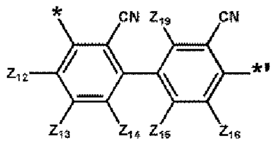
MP9

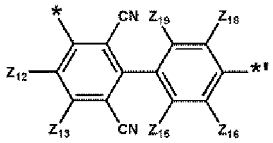
MP10

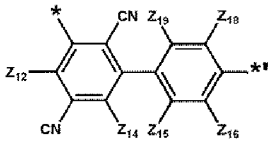
MP11

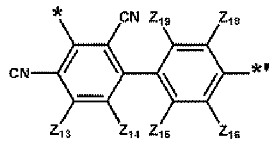
MP12

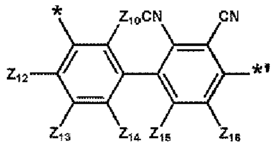
MP13

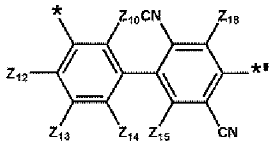
MP14

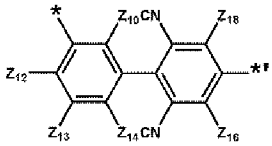
MP15

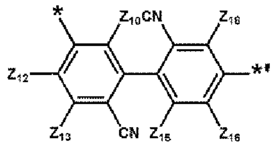
MP16

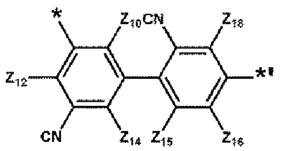
MP17

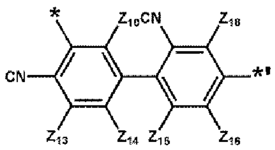
MP18

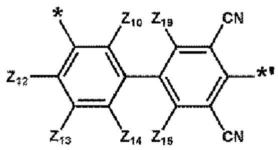
MP19

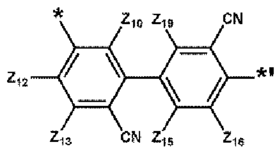
MP20

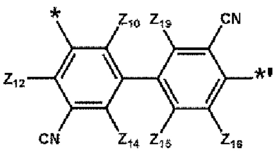
MP21

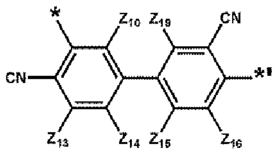
MP22

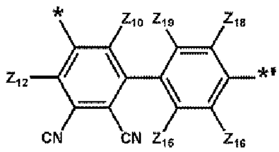
MP23

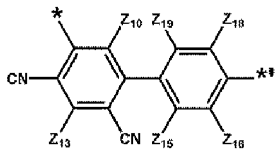
MP24

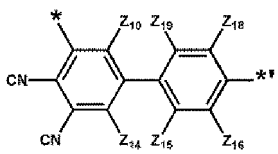
MP25

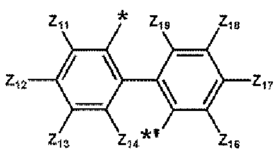
OO1

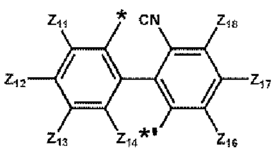
OO2

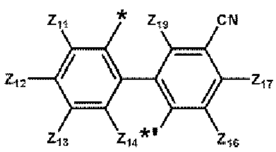
OO3

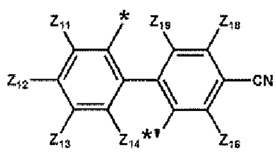
OO4

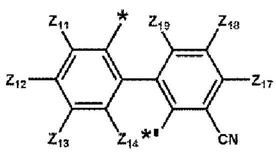
OO5

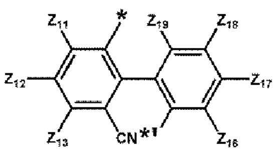
OO6

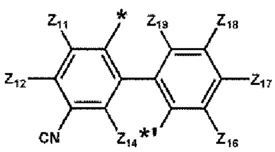
OO7

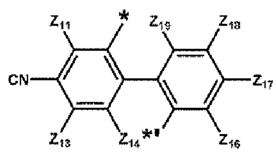
OO8

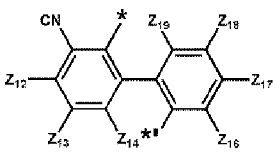
OO9

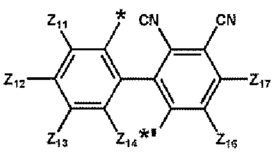
OO10

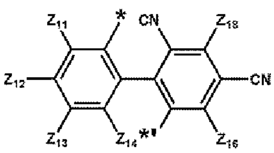
OO11

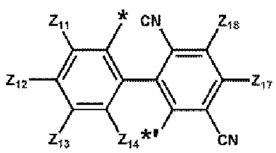
OO12

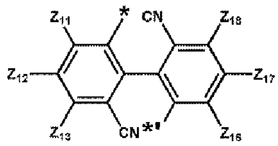

OO13

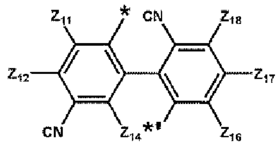

OO14

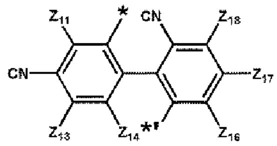

OO15

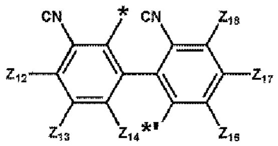

OO16

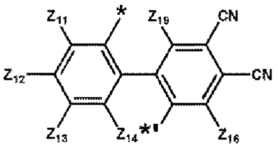

OO17

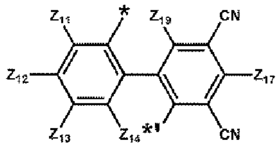

OO18

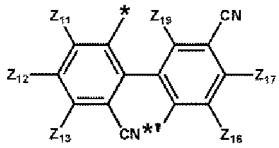

OO19

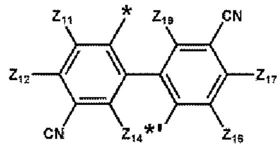

OO20

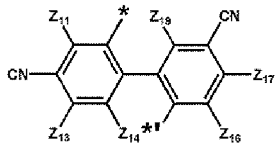

OO21

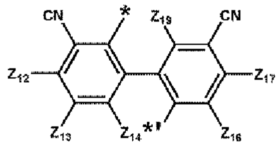

OO22

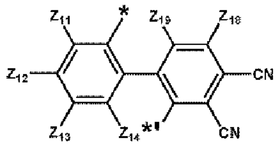

OO23

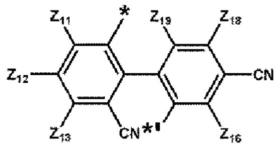

OO24

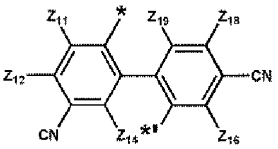

OO25

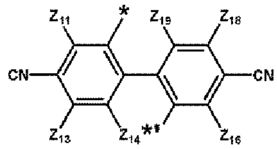

OO26

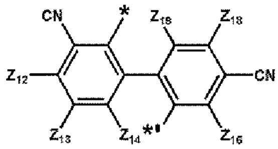

OO27

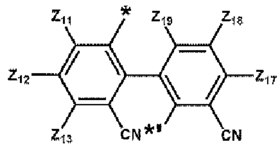

OO28

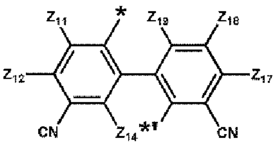

OO29

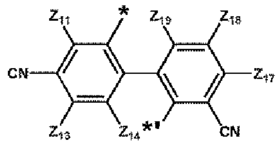

OO30

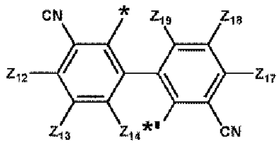

OO31

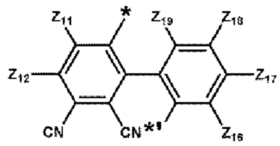

OO32

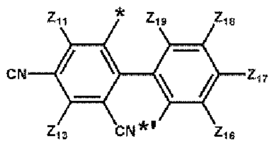

OO33

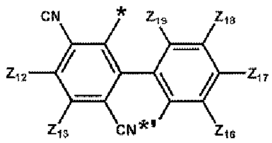

OO34

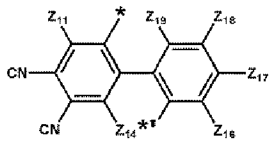

OO35

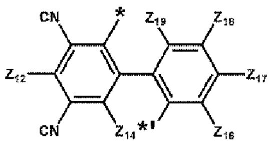

OO36

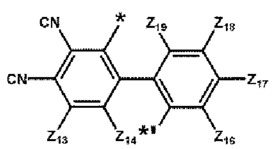

OO37

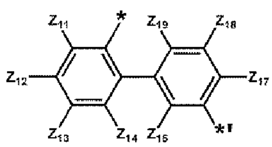

OM1

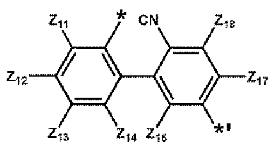

OM2

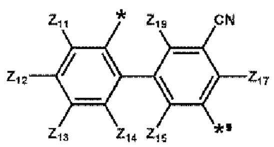

OM3

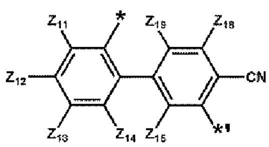

OM4

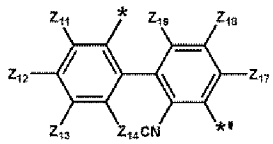
OM5

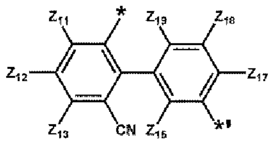
OM6

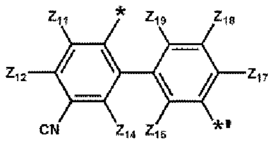
OM7

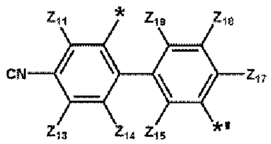
OM8

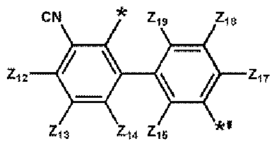
OM9

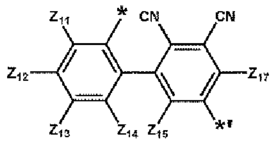
OM10

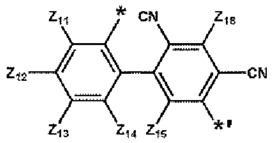
OM11

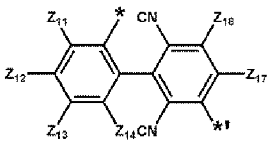
OM12

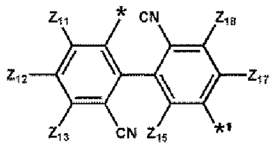
OM13

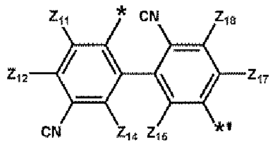
OM14

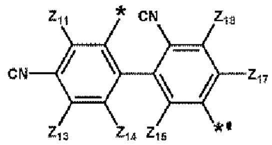
OM15

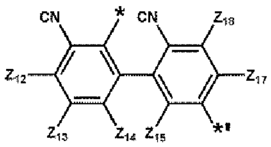
OM16

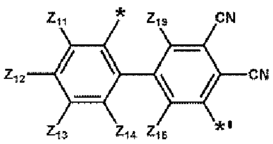
OM17

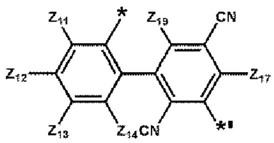
OM18

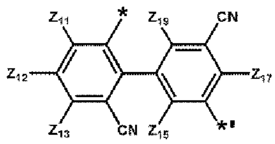
OM19

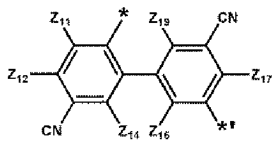
OM20

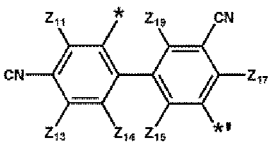
OM21

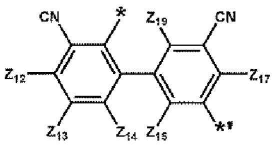
OM22

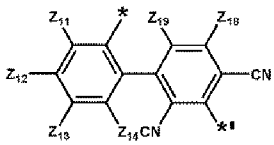
OM23

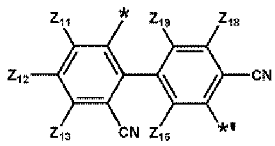
OM24

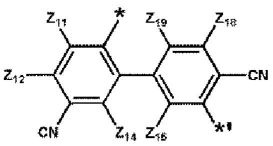
OM25

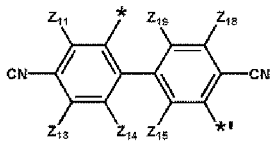
OM26

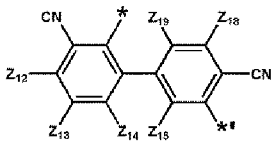
OM27

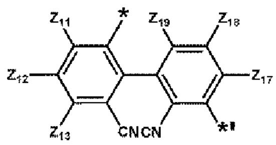
OM28

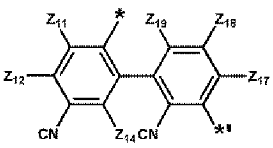
OM29

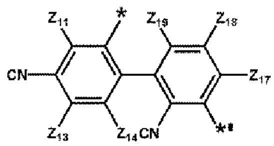
OM30

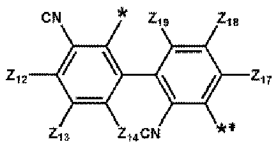
OM31

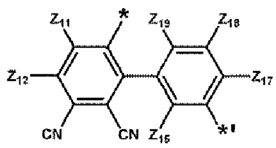
OM32

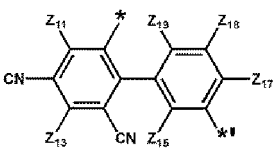
OM33

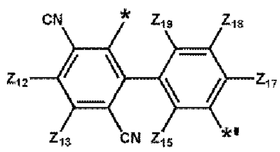
OM34

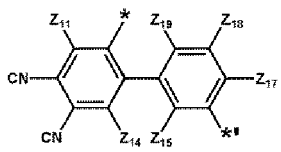
OM35

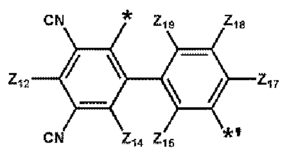
OM36

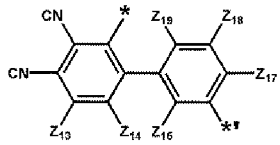

OM37

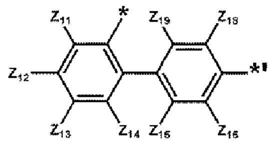

OP1

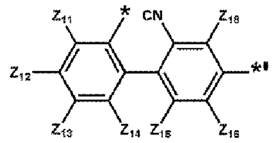

OP2

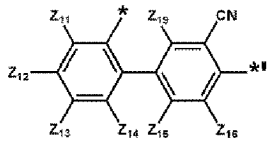

OP3

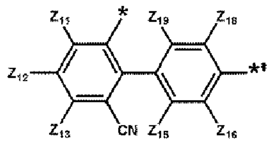

OP4

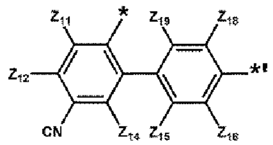

OP5

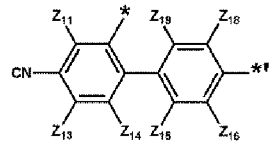

OP6

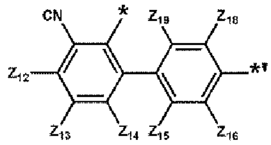

OP7

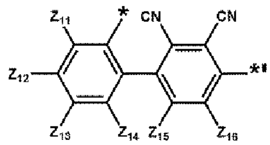

OP8

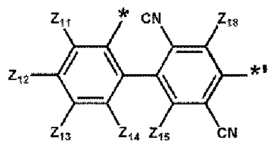

OP9

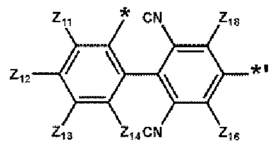

OP10

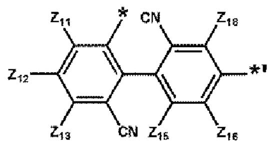

OP11

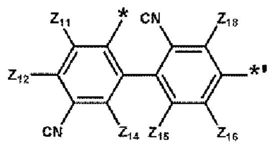

OP12

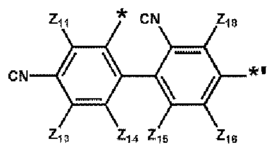

OP13

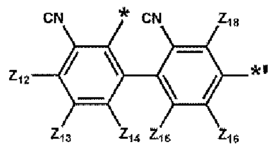

OP14

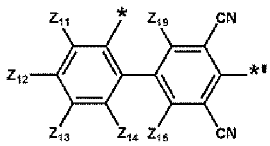

OP15

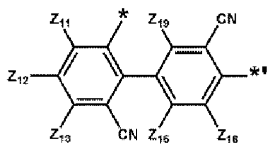

OP16

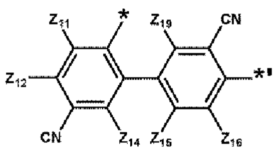

OP17

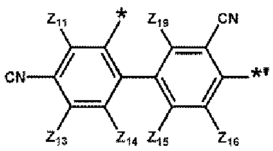

OP18

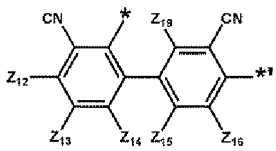

OP19

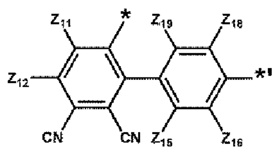

OP20

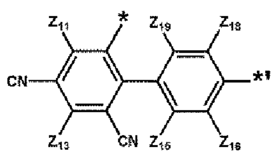

OP21

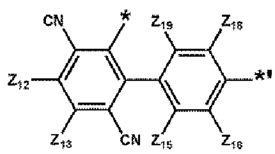

OP22

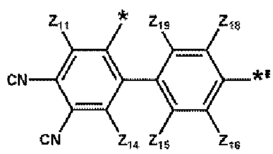

OP23

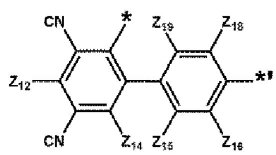

OP24

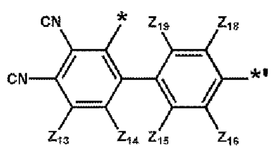

OP25

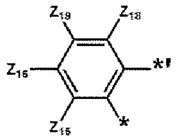
O1
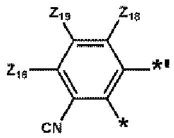
O2
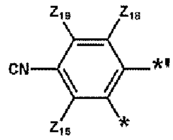
O3
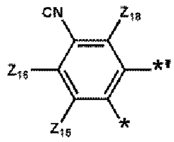
O4
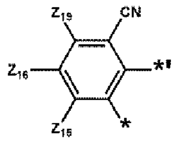
O5
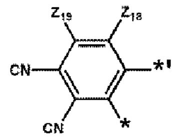
O6

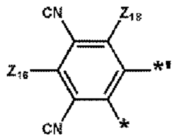
O7
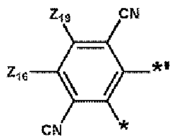
O8
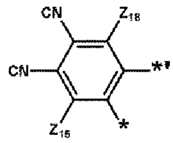
O9
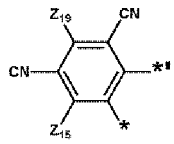
O11
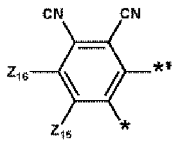
O11
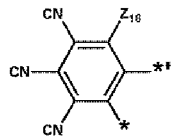
O12

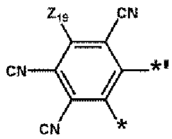
O13
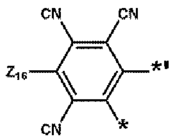
O14
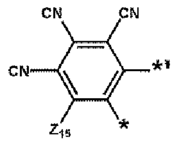
O15
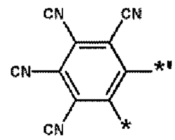
O16

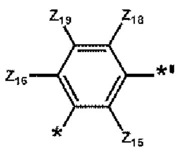
M1
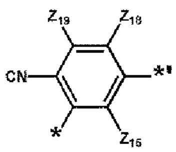
M2
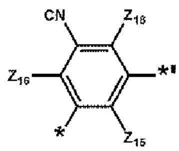
M3
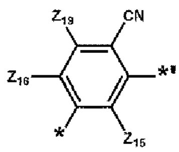
M4
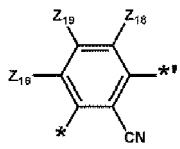
M5
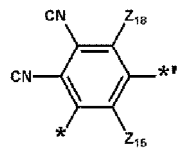
M6

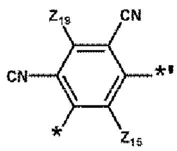
M7
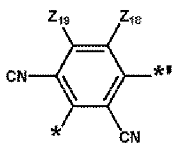
M8
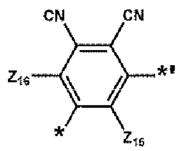
M9
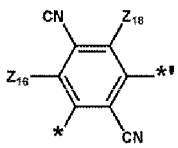
M10
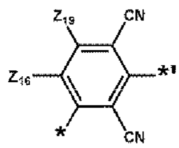
M11
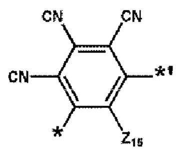
M12

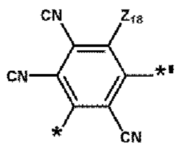
M13
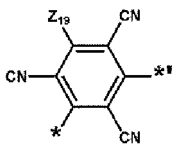
M14
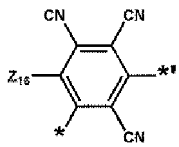
M15
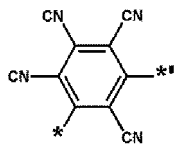
M16

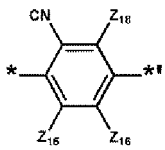
P1
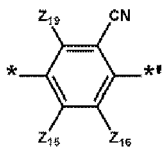
P2
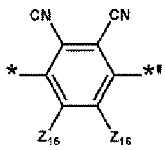
P3
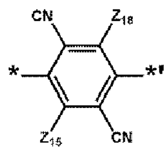
P4
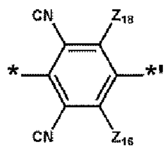
P5
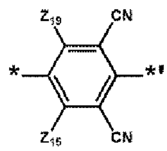
P6

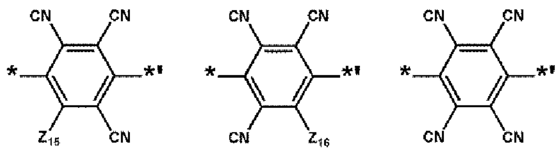
P7
P8
P9

In Formulae PO1 to PO25, PM1 to PM25, PP1 to PP18, MO1 to MO37, MM1 to MM37, MP1 to MP25, OO1 to OO37, OM1 to OM37, OP1 to OP25, O1 to 016, M1 to M16, and P1 to P9, $Z_{10}$ to $Z_{19}$ may each independently be the same as described in connection with $Z_3$ and $Z_4$, and * and *' each indicate a binding site to a neighboring atom.

In an embodiment, in Formulae PO1 to PO25, PM1 to PM25, PP1 to PP18, MO1 to MO37, MM1 to MM37, MP1 to MP25, OO1 to OO37, OM1 to OM37, OP1 to OP25, O1 to 016, M1 to M16 and P1 to P9, $Z_{10}$ to $Z_{19}$ may not be a cyano group.

In one or more embodiments, in Formulae PO1 to PO25, PM1 to PM25, PP1 to PP18, MO1 to MO37, MM1 to MM37, MP1 to MP25, OO1 to OO37, OM1 to OM37, OP1 to OP25, O1 to 016, M1 to M16, and P1 to P9, $Z_{10}$ to $Z_{19}$ may each independently be selected from:

hydrogen, deuterium, or a cyano group; or
a $C_1$-$C_{20}$ alkyl group, a phenyl group, a biphenyl group, a terphenyl group, a dibenzofuranyl group, or a dibenzothiophenyl group, each unsubstituted or substituted with at least one selected from deuterium, a cyano group, a $C_1$-$C_{20}$ alkyl group, a phenyl group, and a biphenyl group.

In an embodiment, the second material may include at least one compound selected from Compounds E1 to E8, but embodiments of the present disclosure are not limited thereto:

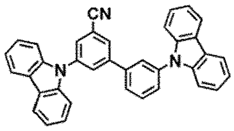
E1

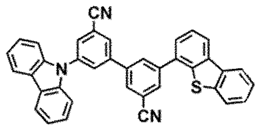
E2

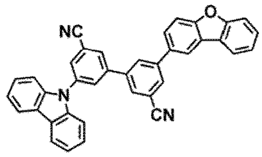
E3

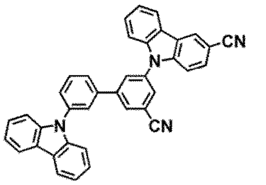
E4

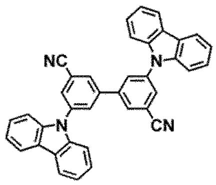
E5

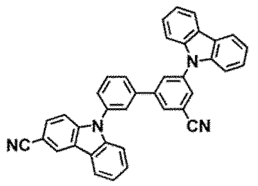
E6

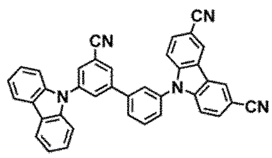
E7

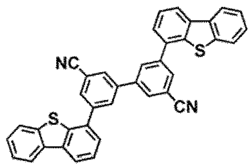
E8

A difference between a triplet energy level (eV) of the host and a triplet energy level (eV) of the heterocyclic compound represented by Formula 1 may be in a range of about 0.2 eV to about 0.5 eV. While not wishing to be bound by theory, it is understood that when the difference between the triplet energy level (eV) of the host and the triplet energy level (eV) of the heterocyclic compound represented by Formula 1 is within this range, it is possible to prevent energy of the triplet exciton generated in the heterocyclic compound represented by Formula 1 from leaking toward the host in the emission layer, thereby implementing efficient light emission. An activated excitation energy level of the host is suppressed, thereby implementing long lifespan driving of the organic light-emitting device.

The triplet energy level is evaluated by using a DFT method (for example, a DFT method of Gaussian program) structurally optimized at a level of B3LYP/6-31G(d,p).

In the first embodiment, an amount of the heterocyclic compound represented by Formula 1 in the emission layer may be in a range of about 0.01 parts by weight to about 30 parts by weight based on 100 pars by weight of the host, but embodiments of the present disclosure are not limited thereto. While not wishing to be bound by theory, it is understood that when the amount of the heterocyclic compound represented by Formula 1 is within this range, a high-quality organic light-emitting device may be implemented without concentration quenching.

The FIGURE is a schematic view of an organic light-emitting device 10 according to an embodiment. Hereinafter, the structure of an organic light-emitting device according to an embodiment and a method of manufacturing an organic light-emitting device according to an embodiment will be described in connection with the FIGURE. The organic light-emitting device 10 includes a first electrode 11, an organic layer 15, and a second electrode 19, which are sequentially stacked.

A substrate may be additionally disposed under the first electrode 11 or above the second electrode 19. For use as the substrate, any substrate that is used in general organic light-emitting devices may be used, and the substrate may be a glass substrate or a transparent plastic substrate, each having excellent mechanical strength, thermal stability, transparency, surface smoothness, ease of handling, and water resistance.

The first electrode 11 may be formed by depositing or sputtering a material for forming the first electrode 11 on the substrate. The first electrode 11 may be an anode. The material for forming the first electrode 11 may be selected from materials with a high work function to facilitate hole injection. The first electrode 11 may be a reflective electrode, a semi-transmissive electrode, or a transmissive electrode. The material for forming the first electrode may be, for example, indium tin oxide (ITO), indium zinc oxide (IZO), tin oxide ($SnO_2$), and zinc oxide (ZnO). In one or more embodiments, magnesium (Mg), aluminum (Al), aluminum-lithium (Al-Li), calcium (Ca), magnesium-indium (Mg-In), or magnesium-silver (Mg-Ag) may be used as the material for forming the first electrode.

The first electrode 11 may have a single-layered structure or a multi-layered structure including two or more layers. For example, the first electrode 11 may have a three-layered structure of ITO/Ag/ITO, but the structure of the first electrode 110 is not limited thereto.

The organic layer 15 may be disposed on the first electrode 11.

The organic layer 15 may include a hole transport region, an emission layer, and an electron transport region.

The hole transport region may be disposed between the first electrode 11 and the emission layer.

The hole transport region may include at least one selected from a hole injection layer, a hole transport layer, an electron blocking layer, and a buffer layer.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

When the hole transport region includes a hole injection layer, the hole injection layer may be disposed on the first electrode 11 by using one or more suitable methods selected from vacuum deposition, spin coating, casting, or Langmuir-Blodgett (LB) deposition.

When the hole injection layer is formed by vacuum deposition, the deposition conditions may vary according to a compound that is used to form the hole injection layer, and the structure and thermal characteristics of the hole injection layer. For example, the deposition conditions may include a deposition temperature of about 100 °C to about 500 °C, a vacuum pressure of about $10^{-8}$ torr to about $10^{-3}$ torr, and a deposition rate of about 0.01 Angstroms per second (Å/sec) to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 revolutions per minute (rpm) to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include at least one selected from m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzene sulfonic acid (PANI/DBSA), poly(3,4-ethylenedioxythiophene)/poly(4-styrene sulfonate) (PEDOT/PSS), polyaniline/camphor sulfonic acid (PANI/CSA), polyaniline/poly(4-styrene sulfonate) (PANI/PSS), a compound represented by Formula 201 below, and a compound represented by Formula 202:

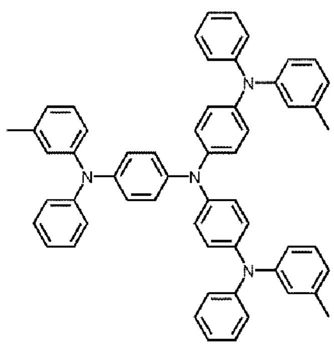
m-MTDATA

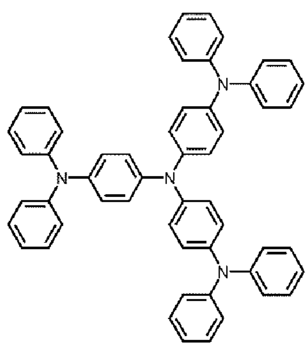
TDATA

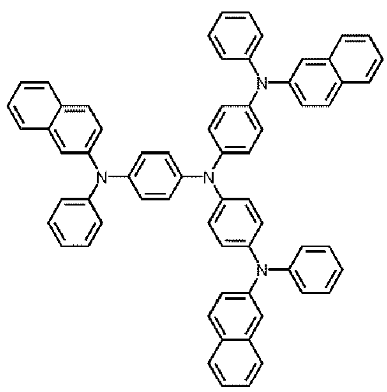
2-TNATA

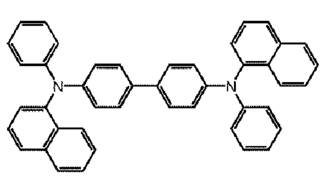

NPB

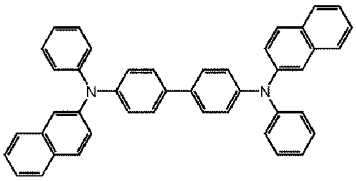

β-NPB

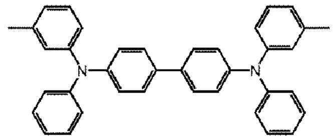

TPD

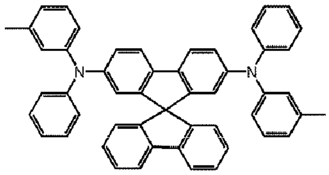

Spiro-TPD

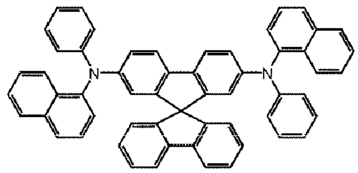

Spiro-NPB

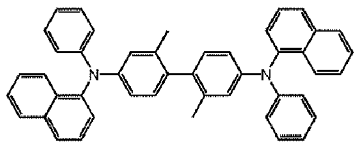

methylated NPB

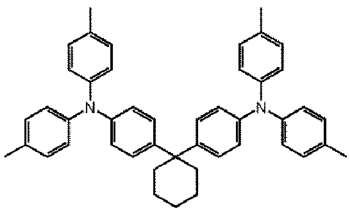

TAPC

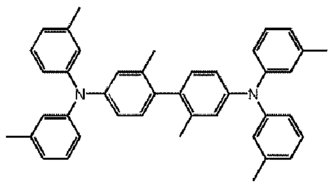

HMTPD

Formula 201

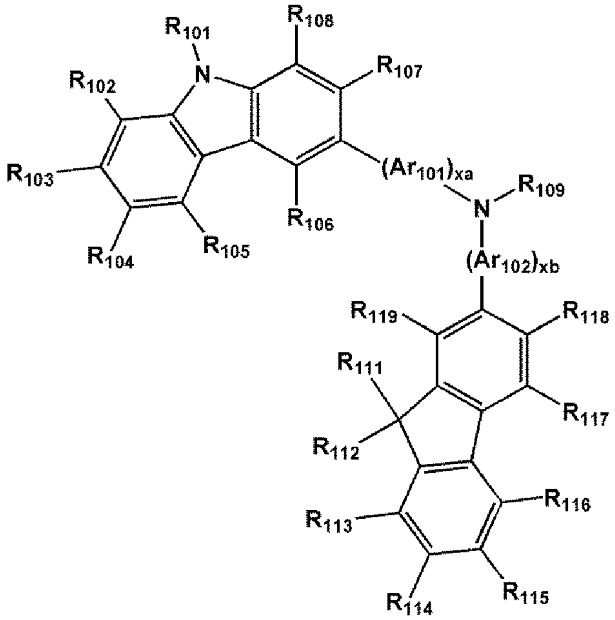

Formula 202

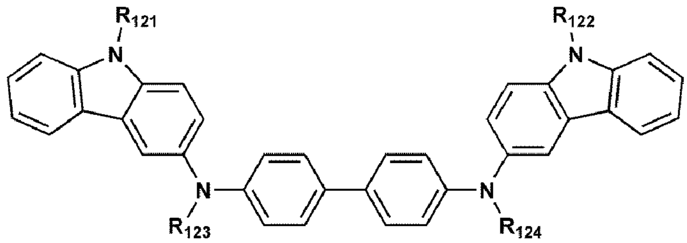

$Ar_{101}$ and $Ar_{102}$ in Formula 201 may each independently be selected from:

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, and a pentacenylene group; and

a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, and a pentacenylene group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, and a monovalent non-aromatic condensed heteropolycyclic group.

In Formula 201, xa and xb may each independently be an integer from 0 to 5, or may be 0, 1, or 2. For example, xa may be 1 and xb may be 0, but embodiments of the present disclosure are not limited thereto.

$R_{101}$ to $R_{108}$, $R_{111}$ to $R_{119}$, and $R_{121}$ to $R_{124}$ in Formulae 201 and 202 may each independently be selected from:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group (for example, a methyl group, an ethyl group, a propyl group, a butyl group, a pentyl group, a hexyl group, and so on), and a $C_1$-$C_{10}$ alkoxy group (for example, a methoxy group, an ethoxy group, a propoxy group, a butoxy group, a pentoxy group, and so on);

a $C_1$-$C_{10}$ alkyl group or a $C_1$-$C_{10}$ alkoxy group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof;

a phenyl group, a naphthyl group, an anthracenyl group, a fluorenyl group, and a pyrenyl group; and

a phenyl group, a naphthyl group, an anthracenyl group, a fluorenyl group, and a pyrenyl group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, or a $C_1$-$C_{10}$ alkoxy group,

but embodiments of the present disclosure are not limited thereto.

$R_{109}$ in Formula 201 may be selected from:

a phenyl group, a naphthyl group, an anthracenyl group, and a pyridinyl group; and

a phenyl group, a naphthyl group, an anthracenyl group, and a pyridinyl group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a

naphthyl group, an anthracenyl group, and a pyridinyl group.

In an embodiment, the compound represented by Formula 201 may be represented by Formula 201A, but embodiments of the present disclosure are not limited thereto:

Formula 201A

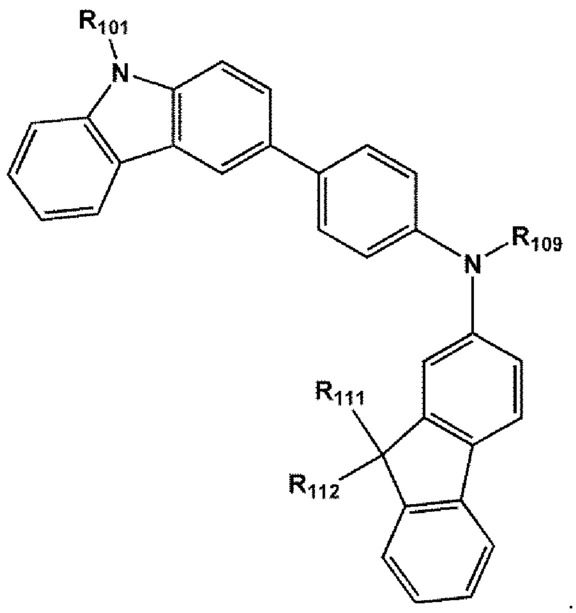

$R_{101}$, $R_{111}$, $R_{112}$, and $R_{109}$ in Formula 201A are the same as described above.

For example, the compound represented by Formula 201, and the compound represented by Formula 202 may include Compounds HT1 to HT20, but embodiments of the present disclosure are not limited thereto:

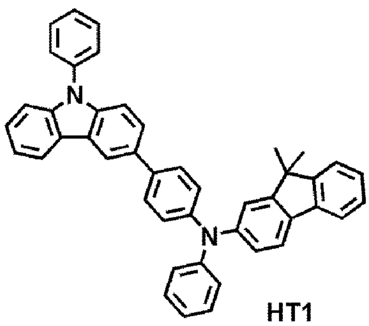

HT1

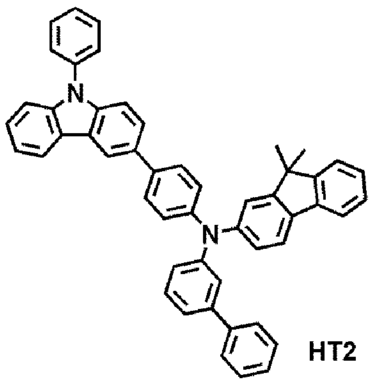

HT2

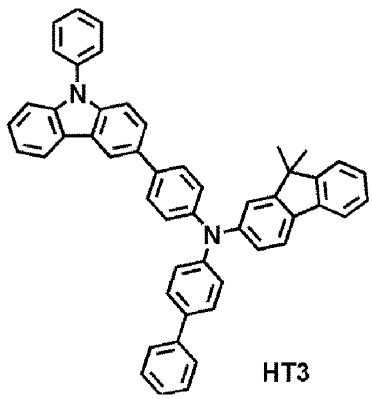

HT3

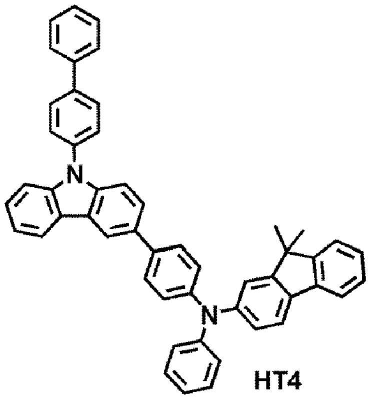
HT4

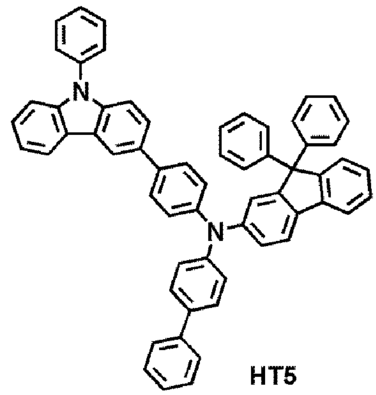
HT5

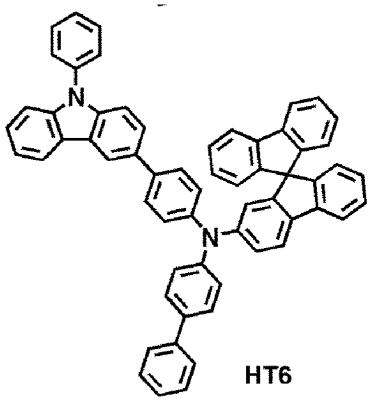
HT6

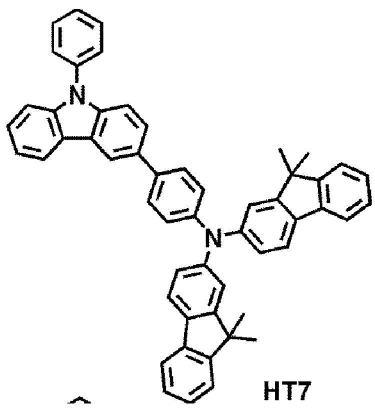
HT7

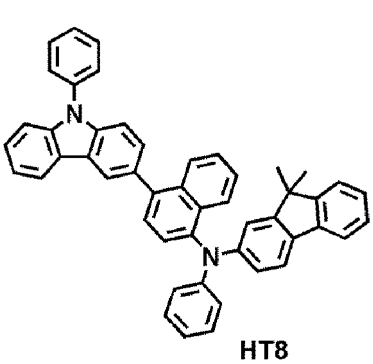
HT8

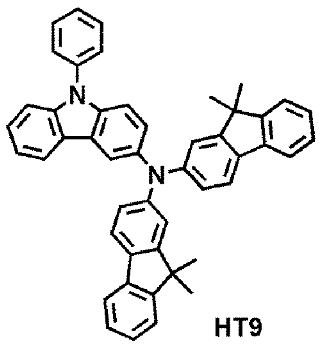
HT9

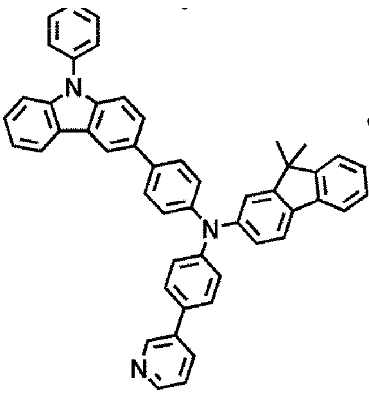
HT10

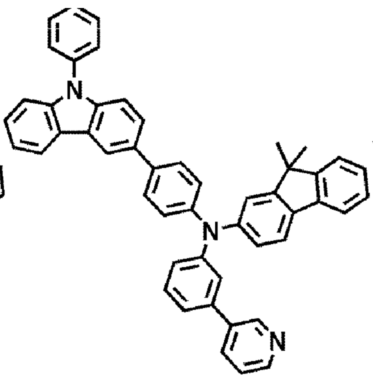
HT11

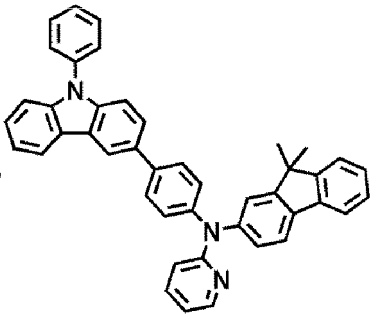
HT12

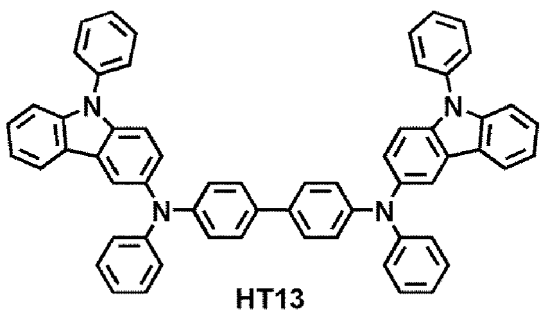
HT13

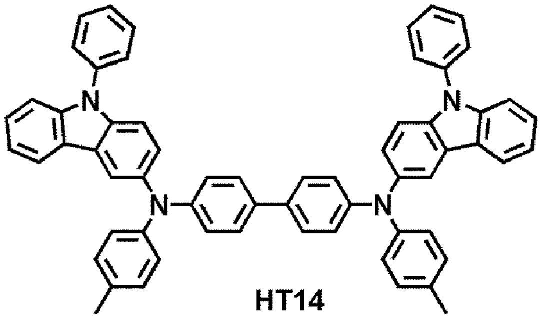
HT14

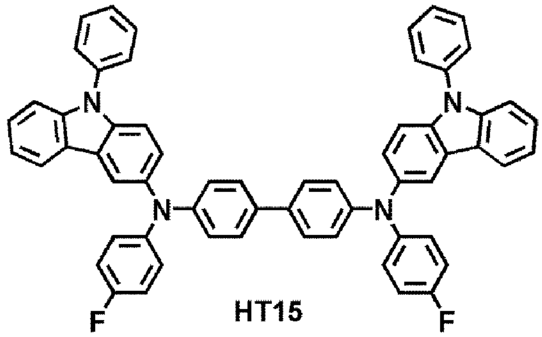

HT15

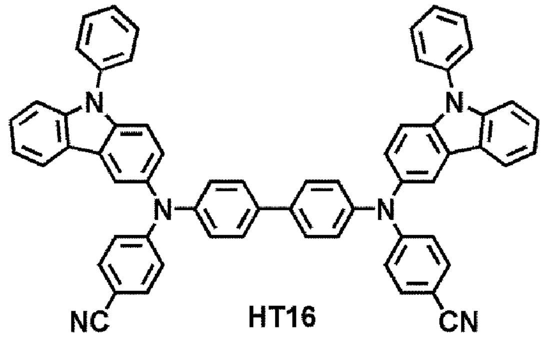

HT16

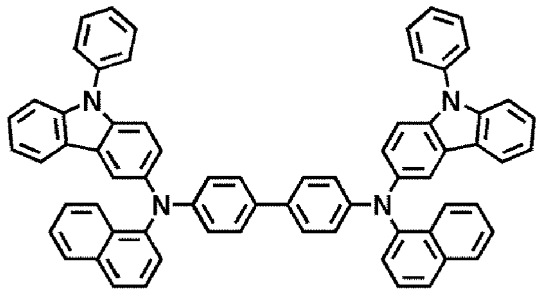

HT17

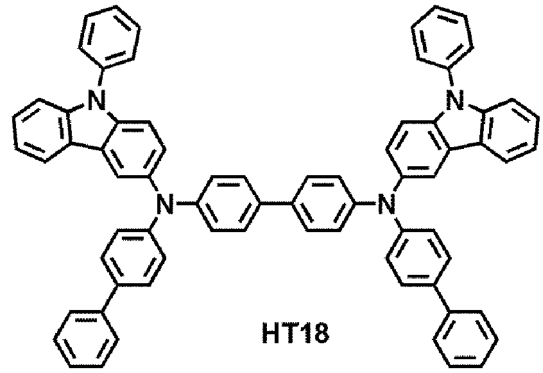

HT18

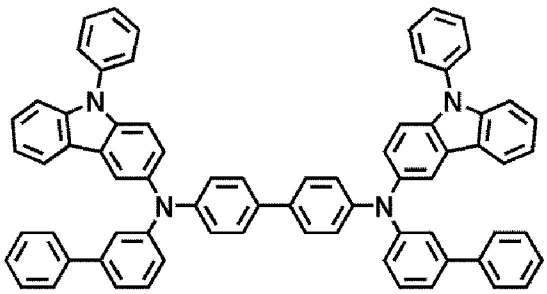

HT19

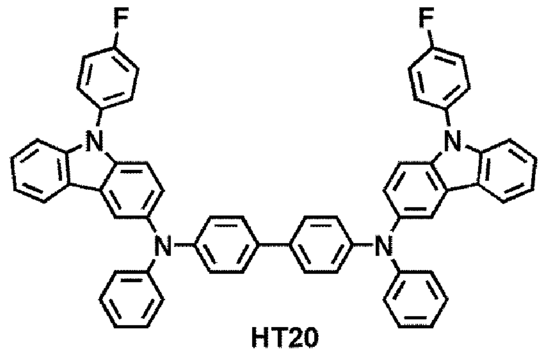

HT20

A thickness of the hole transport region may be in a range of about 100 Angstroms (Å) to about 10,000 Å, for example, about 100 Å to about 1,000 Å. When the hole transport region includes a hole injection layer and a hole transport layer, the thickness of the hole injection layer may be in a range of about 100 Å to about 10,000 Å, and for example, about 100 Å to about 1,000 Å, and the thickness of the hole transport layer may be in a range of about 50 Å to about 2,000 Å, and for example, about 100 Å to about 1,500 Å. While not wishing to be bound by theory, it is understood that when the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one selected from a quinone derivative, a metal oxide, and a cyano group-containing compound, but embodiments of the present disclosure are not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenum oxide; and a cyano group-containing compound, such as Compound HT-D1 or Compound HT-D2 below, but are not limited thereto.

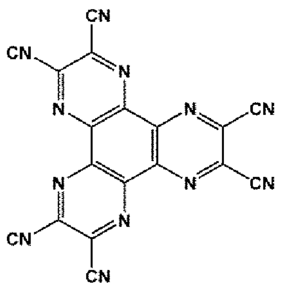

HT-D1

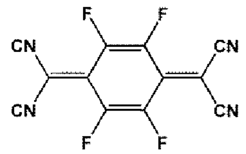

F4-TCNQ

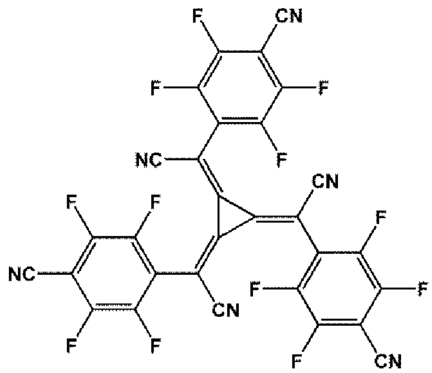

HT-D2

The hole transport region may include a buffer layer.

Also, the buffer layer may compensate for an optical resonance distance according to a wavelength of light emitted from the emission layer, and thus, efficiency of a formed organic light-emitting device may be improved.

The hole transport region may further include an electron blocking layer. The electron blocking layer may include, for example, mCP, but embodiments of the present disclosure are not limited thereto:

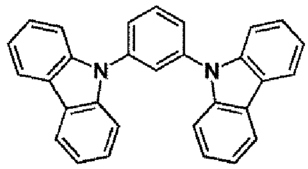

mCP .

Then, an emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a compound that is used to form the emission layer.

When the organic light-emitting device is a full-color organic light-emitting device, the emission layer may be patterned into a red emission layer, a green emission layer, and a blue emission layer. In one or more embodiments, due to a stacked structure including a red emission layer, a green emission layer, and/or a blue emission layer, the emission layer may emit white light.

The emission layer may include a host and a thermally activated delayed fluorescent dopant, and the host and the fluorescent dopant may be the same as described above.

A thickness of the emission layer may be in a range of about 100 Å to about 1,000 Å, for example, about 200 Å to about 600 Å. While not wishing to be bound by theory, it is understood that when the thickness of the emission layer is within this range, excellent light-emission characteristics may be obtained without a substantial increase in driving voltage.

Then, an electron transport region may be disposed on the emission layer.

The electron transport region may include at least one selected from a hole blocking layer, an electron transport layer, and an electron injection layer.

For example, the electron transport region may have a hole blocking layer/electron transport layer/electron injection layer structure or an electron transport layer/electron injection layer structure, but the structure of the electron transport region is not limited thereto. The electron transport layer may have a single-layered structure or a multi-layered

structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, at least one of BCP and BPhen, but may also include other materials:

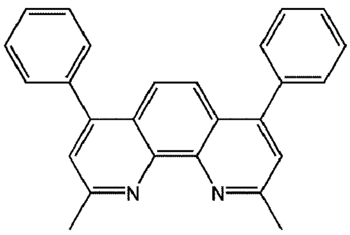

BCP

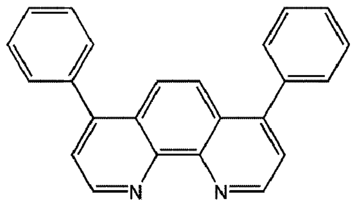

Bphen

.

A thickness of the hole blocking layer may be in a range of about 20 Å to about 1,000 Å, for example, about 30 Å to about 300 Å. While not wishing to be bound by theory, it is understood that when the thickness of the hole blocking layer is within these ranges, the hole blocking layer may have excellent hole blocking characteristics without a substantial increase in driving voltage.

The electron transport layer may include at least one selected from BCP, BPhen, $Alq_3$, BAlq, TAZ, and NTAZ:

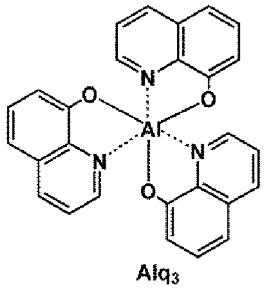

$Alq_3$

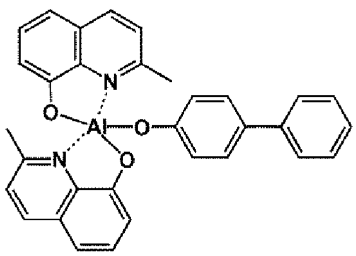

BAlq

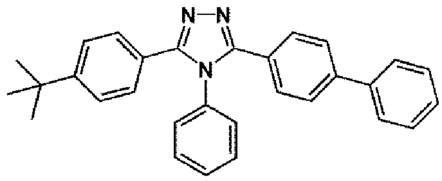

TAZ

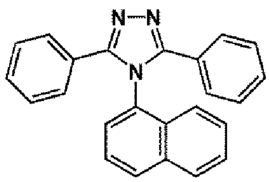

NTAZ

In one or more embodiments, the electron transport layer may include at least one of Compounds ET1 to ET25, but are not limited thereto:

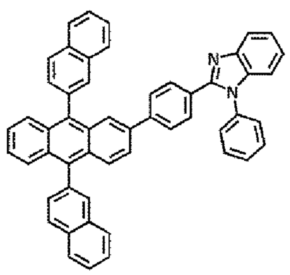

ET1

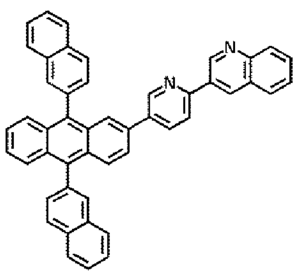

ET2

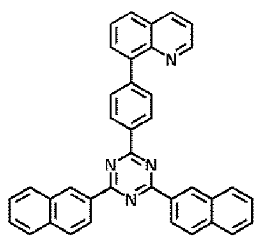

ET3

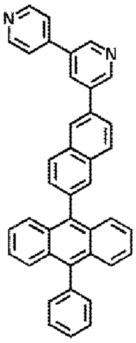

ET4

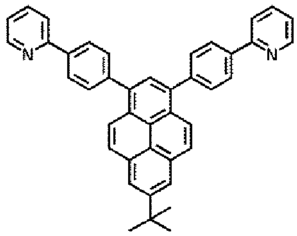

ET5

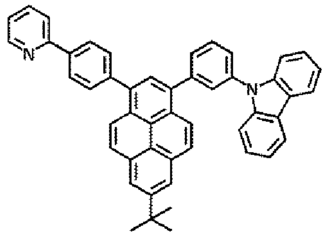

ET6

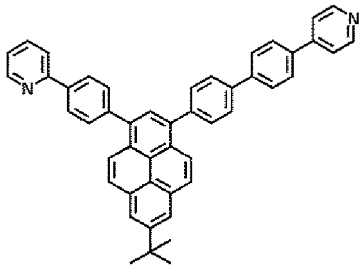

ET7

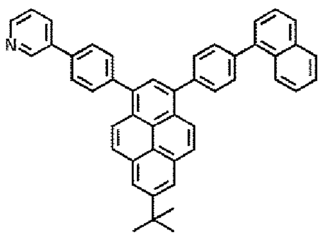

ET8

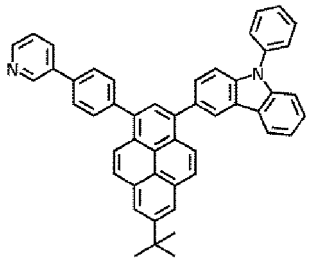

ET9

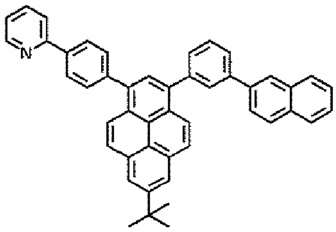

ET10

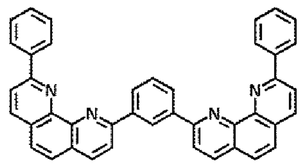

ET11

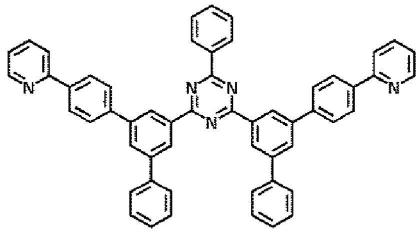

ET12

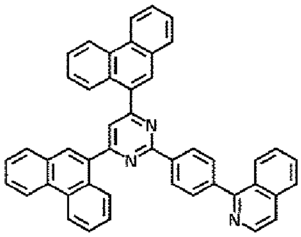

ET13

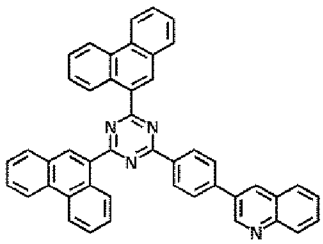

ET14

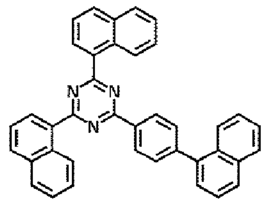

ET15

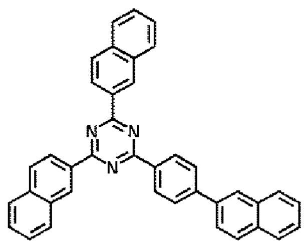

ET16

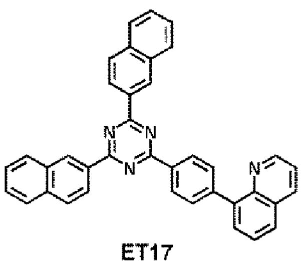

ET17

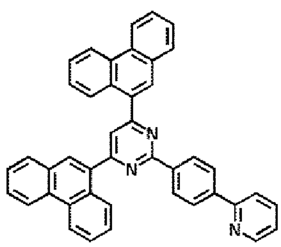

ET18

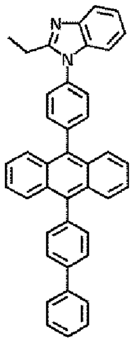

ET19

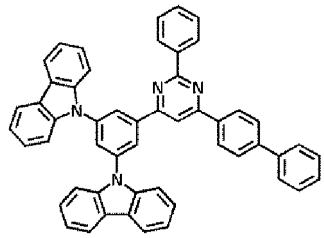

ET20

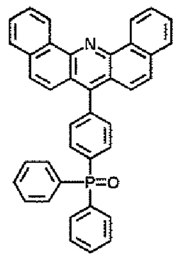

ET21

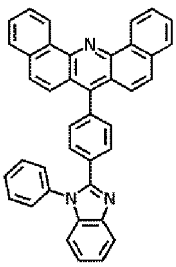

ET22

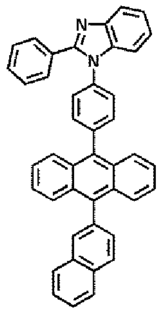

ET23

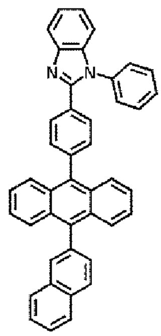

ET24

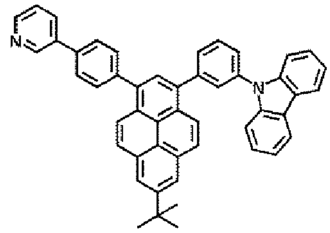

ET25

A thickness of the electron transport layer may be in a range of about 100 Å to about 2,000 Å, for example, about 150 Å to about 1,500 Å. While not wishing to be bound by theory, it is understood that when the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium 8-hydroxyquinolate, LiQ) or ET-D2.

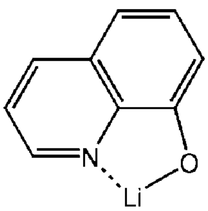

ET-D1

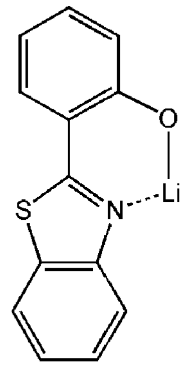

ET-D2

The electron transport region may include an electron injection layer (EIL) that promotes flow of electrons from the second electrode 19 thereinto.

The electron injection layer may include at least one selected from LiF, NaCl, CsF, $Li_2O$, and BaO.

A thickness of the electron injection layer may be in a range of about 1 Å to about 100 Å, for example, about 3 Å to about 90 Å. While not wishing to be bound by theory, it is understood that when the thickness of the electron injection layer is within the range described above, the electron injection layer may have satisfactory electron injection characteristics without a substantial increase in driving voltage.

The second electrode 19 may be formed on the organic layer 150. The second electrode 19 may be a cathode. A material for forming the second electrode 19 may be selected from metal, an alloy, an electrically conductive compound,

and a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as a material for forming the second electrode 19. In one or more embodiments, to manufacture a top-emission type light-emitting device, a transmissive electrode formed using ITO or IZO may be used as the second electrode 19.

Hereinbefore, the organic light-emitting device has been described with reference to FIG. 1, but embodiments of the present disclosure are not limited thereto.

The term "$C_1$-$C_{60}$ alkyl group" as used herein refers to a linear or branched saturated aliphatic hydrocarbon monovalent group having 1 to 60 carbon atoms, and non-limiting examples thereof include a methyl group, an ethyl group, a propyl group, an iso-butyl group, a sec-butyl group, a tert-butyl group, a pentyl group, an iso-amyl group, and a hexyl group. The term "$C_1$-$C_{60}$ alkylene group" as used herein refers to a divalent group having the same structure as the $C_1$-$C_{60}$ alkyl group.

The term "$C_1$-$C_{60}$ alkoxy group" as used herein refers to a monovalent group represented by $-OA_{101}$ (wherein $A_{101}$ is the $C_1$-$C_{60}$ alkyl group), and examples thereof include a methoxy group, an ethoxy group, and an iso-propyloxy group.

The term "$C_2$-$C_{60}$ alkenyl group" as used herein refers to a hydrocarbon group having at least one carbon-carbon double bond in the middle or at the terminus of the $C_2$-$C_{60}$ alkyl group, and examples thereof include an ethenyl group, a propenyl group, and a butenyl group. The term "$C_2$-$C_{60}$ alkenylene group" as used herein refers to a divalent group having the same structure as the $C_2$-$C_{60}$ alkenyl group.

The term "$C_2$-$C_{60}$ alkynyl group" as used herein refers to a hydrocarbon group having at least one carbon-carbon triple bond in the middle or at the terminus of the $C_2$-$C_{60}$ alkyl group, and examples thereof include an ethynyl group, and a propynyl group. The term "$C_2$-$C_{60}$ alkynylene group" as used herein refers to a divalent group having the same structure as the $C_2$-$C_{60}$ alkynyl group.

The term "$C_3$-$C_{10}$ cycloalkyl group" as used herein refers to a monovalent saturated hydrocarbon monocyclic group having 3 to 10 carbon atoms, and non-limiting examples thereof include a cyclopropyl group, a cyclobutyl group, a cyclopentyl group, a cyclohexyl group, and a cycloheptyl group. The term "$C_3$-$C_{10}$ cycloalkylene group" as used herein refers to a divalent group having the same structure as the $C_3$-$C_{10}$ cycloalkyl group.

The term "$C_2$-$C_{10}$ heterocycloalkyl group" as used herein refers to a monovalent saturated monocyclic group having at least one heteroatom selected from N, O, P, Si and S as a ring-forming atom and 2 to 10 carbon atoms, and non-limiting examples thereof include a tetrahydrofuranyl group, and a tetrahydrothiophenyl group. The term "$C_2$-$C_{10}$ heterocycloalkylene group" as used herein refers to a divalent group having the same structure as the $C_2$-$C_{10}$ heterocycloalkyl group.

The term "$C_3$-$C_{10}$ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "$C_3$-$C_{10}$ cycloalkenylene group" as used herein refers to a divalent group having the same structure as the $C_3$-$C_{10}$ cycloalkenyl group.

The term "$C_2$-$C_{10}$ heterocycloalkenyl group" as used herein refers to a monovalent monocyclic group that has at least one heteroatom selected from **N, O, P,** Si, and S as a ring-forming atom, 2 to 10 carbon atoms, and at least one double bond in its ring. Non-limiting examples of the $C_2$-$C_{10}$ heterocycloalkenyl group include a 2,3-dihydrofuranyl group, and a 2,3-dihydrothiophenyl group. The term "$C_2$-$C_{10}$ heterocycloalkenylene group" as used herein refers to a divalent group having the same structure as the $C_2$-$C_{10}$ heterocycloalkenyl group.

The term "$C_6$-$C_{60}$ aryl group" as used herein refers to a monovalent group having a carbocyclic aromatic system having 6 to 60 carbon atoms, and the term "$C_6$-$C_{60}$ arylene group" as used herein refers to a divalent group having a carbocyclic aromatic system having 6 to 60 carbon atoms. Non-limiting examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each include two or more rings, the rings may be fused to each other.

The term "$C_2$-$C_{60}$ heteroaryl group" as used herein refers to a monovalent group having a heterocyclic aromatic system that has at least one heteroatom selected from **N, O, P,** Si, and S as a ring-forming atom, and 2 to 60 carbon atoms. The term "$C_2$-$C_{60}$ heteroarylene group," as used herein refers to a divalent group having a heterocyclic aromatic system that has at least one heteroatom selected from **N, O, P,** Si, and S as a ring-forming atom, and 2 to 60 carbon atoms. Non-limiting examples of the $C_2$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_2$-$C_{60}$ heteroaryl group and the $C_2$-$C_{60}$ heteroarylene group each include two or more rings, the rings may be fused to each other.

The term "$C_6$-$C_{60}$ aryloxy group" as used herein refers to $-OA_{102}$ (wherein $A_{102}$ is the $C_6$-$C_{60}$ aryl group), a $C_6$-$C_{60}$ arylthio group as used herein indicates $-SA_{103}$ (wherein $A_{103}$ is the $C_6$-$C_{60}$ aryl group), and the term "$C_7$-$C_{60}$ arylalkyl group" as used herein indicates $-A_{104}A_{105}$ (wherein $A_{105}$ is the $C_6$-$C_{59}$ aryl group and $A_{104}$ is the $C_1$-$C_{53}$ alkylene group).

The term "$C_1$-$C_{60}$ heteroaryloxy group" as used herein refers to $-OA_{106}$ (wherein $A_{106}$ is the $C_2$-$C_{60}$ heteroaryl group), the term "$C_1$-$C_{60}$ heteroarylthio group" as used herein indicates $-SA_{107}$ (wherein $A_{107}$ is the $C_1$-$C_{60}$ heteroaryl

group), and the term "$C_2$-$C_{60}$ heteroarylalkyl group" as used herein refers to -$A_{108}A_{109}$ ($A_{109}$ is a $C_1$-$C_{59}$ heteroaryl group, and $A_{108}$ is a $C_1$-$C_{59}$ alkylene group).

The term "monovalent non-aromatic condensed polycyclic group" as used herein refers to a monovalent group having two or more rings condensed to each other, only carbon atoms (for example, the number of carbon atoms may be in a range of 8 to 60) as a ring-forming atom, and no aromaticity in its entire molecular structure. Non-limiting examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group having two or more rings condensed to each other, a heteroatom selected from **N,** O, P, Si, and S, other than carbon atoms (for example, the number of carbon atoms may be in a range of 2 to 60), as a ring-forming atom, and no aromaticity in its entire molecular structure. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

At least one substituent of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_7$-$C_{60}$ arylalkyl group, the substituted $C_1$-$C_{60}$ heteroaryl group, the substituted $C_1$-$C_{60}$ heteroaryloxy group, the substituted $C_1$-$C_{60}$ heteroarylthio group, the substituted $C_2$-$C_{60}$ heteroarylalkyl group, the substituted monovalent non-aromatic condensed polycyclic group, and the substituted monovalent non-aromatic condensed heteropolycyclic group may be selected from:

deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, and a $C_1$-$C_{60}$ alkoxy group;
a $C_1$-$C_{60}$ alkyl group, a $C_2$-$C_{60}$ alkenyl group, a $C_2$-$C_{60}$ alkynyl group, and a $C_1$-$C_{60}$ alkoxy group, each substituted with at least one selected from deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, -N($Q_{11}$)($Q_{12}$), -Si($Q_{13}$)($Q_{14}$)($Q_{15}$), -B($Q_{16}$)($Q_{17}$), and -P(=O)($Q_{18}$)($Q_{19}$);
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, and a monovalent non-aromatic condensed heteropolycyclic group;
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, and a monovalent non-aromatic condensed heteropolycyclic group, each substituted with at least one selected from deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, -N($Q_{21}$)($Q_{22}$), - Si($Q_{23}$)($Q_{24}$)($Q_{25}$), -B($Q_{26}$)($Q_{27}$) and -P(=O)($Q_{28}$)($Q_{29}$); and
-N($Q_{31}$)($Q_{32}$), -Si($Q_{33}$)($Q_{34}$)($Q_{35}$), -B($Q_{36}$)($Q_{37}$) and -P(=O)($Q_{38}$)($Q_{39}$), and
$Q_{101}$ to $Q_{103}$, $Q_{111}$ to $Q_{113}$, $Q_{121}$ to $Q_{123}$, $Q_{11}$ to $Q_{19}$, $Q_{21}$ to $Q_{29}$, and $Q_{31}$ to $Q_{39}$ may each independently be selected from hydrogen, deuterium, -F, -Cl, - Br, -I, a hydroxyl group, a cyano group, a nitro group, an amino group, an amidino group, a hydrazine group, a hydrazone group, a carboxylic acid group or a salt thereof, a sulfonic acid group or a salt

thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a biphenyl group, a terphenyl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, and a monovalent non-aromatic condensed heteropolycyclic group.

For example, $Q_{101}$ to $Q_{103}$, $Q_{111}$ to $Q_{113}$, $Q_{121}$ to $Q_{123}$, $Q_{11}$ to $Q_{19}$, $Q_{21}$ to $Q_{29}$, and $Q_{31}$ to $Q_{39}$ may each independently be hydrogen, deuterium, a $C_1$-$C_{20}$ alkyl group, a $C_1$-$C_{20}$ alkoxy group, a phenyl group, a naphthyl group, a fluorenyl group, a carbazolyl group, a dibenzofuranyl group, a dibenzothiophenyl group, a triphenylenyl group, a biphenyl group, a terphenyl group, or a tetraphenyl group, but embodiments of the present disclosure are not limited thereto.

The term "room temperature" as used herein refers to about 25 °C.

The terms "biphenyl group", "terphenyl group", and "tetraphenyl group" as used herein each refer to a monovalent group in which two, three, or four benzene groups are linked to each other via a single bond, respectively.

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that an amount of A used was identical to an amount of B used, in terms of a molar equivalent.

EXAMPLES

Synthesis Example 1: Synthesis of Compound 2

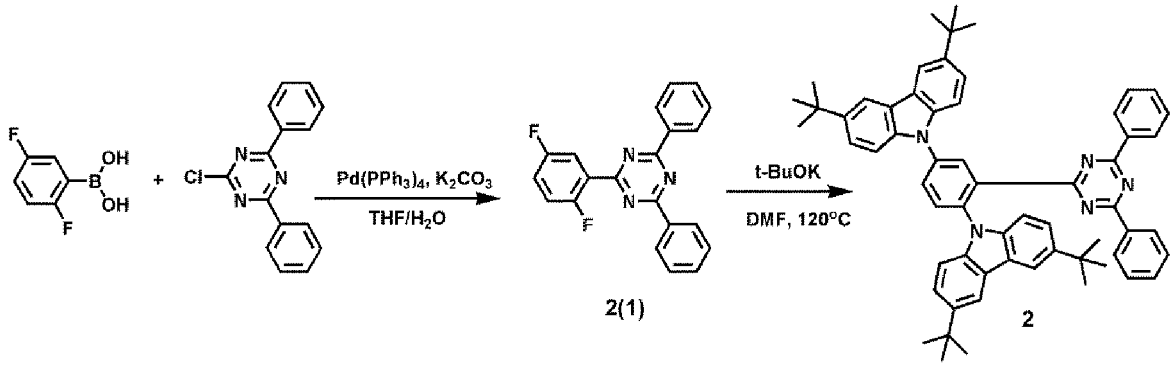


Synthesis of Intermediate 2(1)

2-chloro-4,6-diphenyl-1,3,5-triazine (3.0 grams (g), 11.21 millimoles, mmol), (2,5-difluorophenyl)boronic acid (2.12 g, 13.45 mmol), palladium tetrakis(triphenylphosphine) ($Pd(PPh_3)_4$) (0.65 g, 0.56 mmol), and potassium carbonate ($K_2CO_3$) (4.65 g, 33.62 mmol) were added to a mixture of 20 milliliters (mL) of tetrahydrofuran and 20 mL of distilled water, and the reaction mixture was heated under reflux. After the reaction was completed, the reaction product was cooled to room temperature, and methanol was added thereto. The reaction solution was filtered through silica gel. An organic layer obtained therefrom was concentrated and precipitated by adding methanol thereto to synthesize Intermediate 2(1) (white solid, 3.23 g, yield of 83 %).

Synthesis of Compound 2

Intermediate 2(1) (5.18 g, 15 mmol), 3,6-di-tert-butyl-9H-carbazole (12.57 g, 45 mmol), and potassium-tert-butoxide (t-BuOK) (4.21 g, 37.5 mmol) were added to 30 mL of N,N-dimethylformamide and stirred at a temperature of 120 °C for 12 hours. After the reaction was completed, the reaction product was cooled to room temperature, and methanol was added thereto. The reaction solution was filtered through silica gel. The organic layer obtained therefrom was concentrated, redissolved in toluene, filtered through silica gel, and then concentrated. The resultant obtained therefrom was recrystallized by using toluene to synthesize Compound 2 (yellow solid, 9.39 g, yield of 48 %).

LC-MS (Calcd.: 864.19 g/mol, Found: 864.27 g/mol (M+1)).

Synthesis Example 2 : Synthesis of Compound 1074

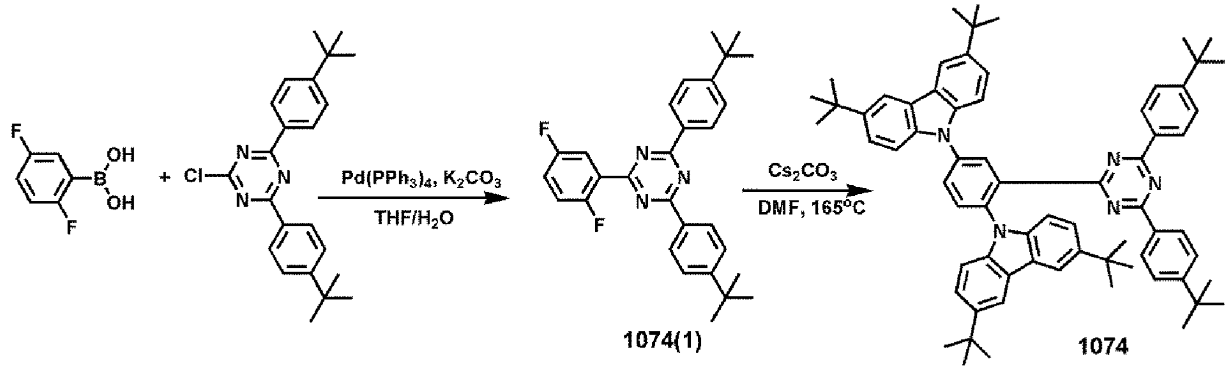


Synthesis of Intermediate 1074(1)

2,4-bis(4-(tert-butyl)phenyl)-6-chloro-1,3,5-triazine (5.0 g, 13.16 mmol), (2,5-difluorophenyl)boronic acid (2.49 g, 15.79 mmol), palladium tetrakis(triphenylphosphine ($Pd(PPh_3)_4$) (0.76 g, 0.66 mmol), and potassium carbonate ($K_2CO_3$) (5.46 g, 39.48 mmol) were added to a mixture of 22 mL of tetrahydrofuran and 22 mL of distilled water, and the reaction mixture was heated under reflux. After the reaction was completed, the reaction product was cooled to room temperature, and the organic layer was extracted therefrom by using dichloromethane and water and filtered through silica gel. The obtained organic layer was concentrated and precipitated by adding methanol thereto to synthesize Intermediate 1074(1) (white solid, 5.0 g, yield of 83 %).

Synthesis of Compound 1074

Intermediate 1074(1) (2.62 g, 5.73 mmol), 3,6-di-tert-butyl-9H-carbazole (4.0 g, 14.31 mmol), and cesium carbonate ($Cs_2CO_3$) (9.33 g, 28.63 mmol) were added to 30 mL of N,N-dimethylformamide, and the reaction mixture was stirred at a temperature of 165 °C for 12 hours. After the reaction was completed, the reaction product was cooled to room temperature, and methanol was added thereto. The reaction solution was filtered through silica gel. The organic layer obtained therefrom was concentrated, redissolved in toluene, filtered through silica gel, and then concentrated. The resultant obtained therefrom was recrystallized by using dichloromethane and methanol to synthesize Compound 1074 (yellow solid, 4.34 g, yield of 78 %).

LC-MS (Calcd.: 976.41 g/mol, Found: 976.5 g/mol (M+1)).

Reference Synthesis Example 3 : Synthesis of Compound 1075

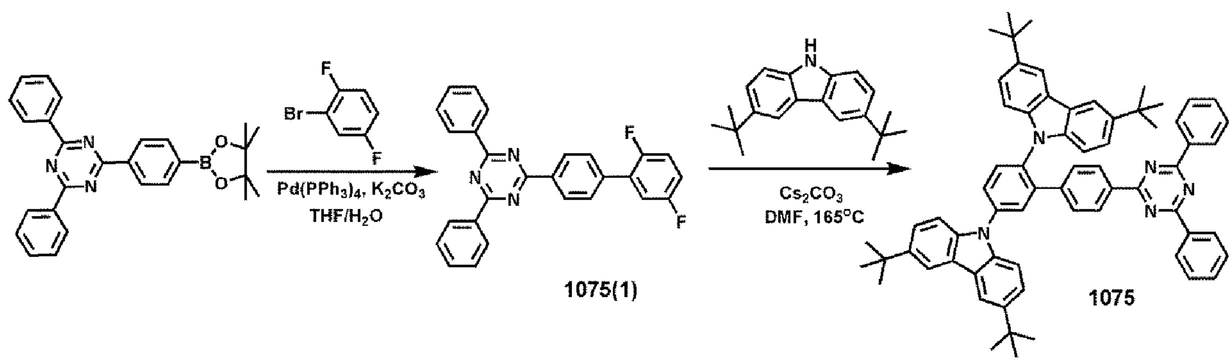


Synthesis of Intermediate 1075(1)

2,4-diphenyl-6-(4-(4,4,5,5-tetramethyl-1,3,2-dioxaborolan-2-yl)phenyl)-1,3,5-triazine (8.12 g, 18.65 mmol), 2-bromo-1,4-difluorobenzene (3.0 g, 15.54 mmol), palladium tetrakis(triphenylphosphine) ($Pd(PPh_3)_4$) (0.89 g, 0.78 mmol), and potassium carbonate ($K_2CO_3$) (6.44 g, 46.63 mmol) were added to a mixture of 20 mL of tetrahydrofuran and 25 mL of distilled water, and the reaction mixture was heated under reflux. After the reaction was completed, the reaction product was cooled to room temperature, and the organic layer was extracted therefrom by using dichloromethane and water and filtered through silica gel. The obtained organic layer was concentrated and recrystallized by using a mixture of dichloromethane and ethyl acetate to synthesize Intermediate 1075(1) (white solid, 4.8 g, yield of 73 %).

Synthesis of Compound 1075

Intermediate 1075(1) (1.81 g, 4.29 mmol), 3,6-di-tert-butyl-9H-carbazole (3.0 g, 10.74 mmol), and cesium carbonate ($Cs_2CO_3$) (7.0 g, 21.47 mmol) were added to 43 mL of N,N-dimethylformamide, and the reaction mixture was stirred at a temperature of 165 °C for 12 hours. After the reaction was completed, the reaction product was cooled to room temperature, and methanol was added thereto. The reaction solution was filtered through silica gel. The organic layer obtained therefrom was concentrated, redissolved in toluene, filtered through silica gel, and then concentrated. The resultant obtained therefrom was recrystallized by using a mixture of dichloromethane and hexane to synthesize Compound 1075 (yellow solid, 2.83 g, yield of 70 %).

LC-MS (Calcd.: 940.29 g/mol, Found: 976.39 g/mol (M+1)).

Synthesis Example 4 : Synthesis of Compound 1076

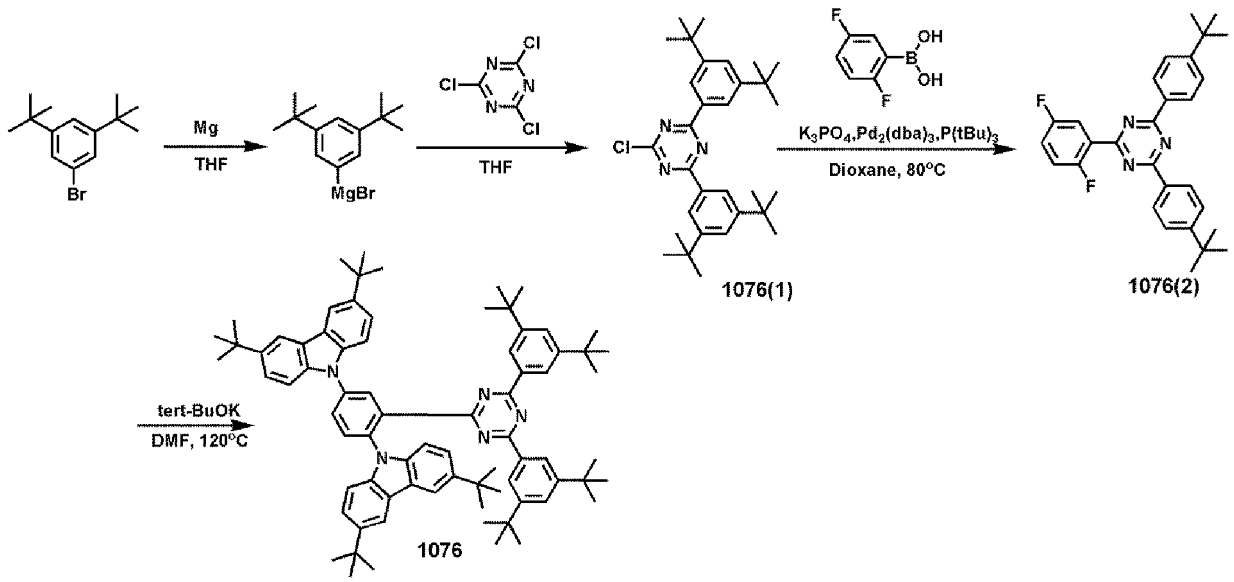


1076(1)

1076(2)

1076

Synthesis of Intermediate 1076(1)

Magnesium (7.76 g, 319.2 mmol) was added to a flask in a nitrogen atmosphere, and 1-bromo-3,5-di-tert-butylbenzene (90.03 g, 334.4 mmol) was dissolved in 300 mL of tetrahydrofuran, slowly added to the flask containing magnesium, and the reaction mixture was stirred at a temperature of 70 °C for 2 hours. After the reaction was completed, the reaction product was cooled to room temperature to obtain (3,5-di-tert-butylphenyl)magnesium bromide Grignard reagent. The Grignard reagent was directly used in a subsequent reaction without any purification. The Grignard reagent was slowly added dropwise to a reaction container in which 2,4,6-trichloro-1,3,5-triazine was dissolved in 300 mL of tetrahydrofuran at a temperature of -40 °C for 20 minutes, and the reaction mixture was stirred at the same temperature for 30 minutes. Then, the reaction container was heated to room temperature and stirred for 16 hours, and was further heated to a temperature of 60 °C and additionally stirred for 4 hours. After the reaction was completed, the reaction product was cooled to room temperature, and the organic layer extracted therefrom by using 1 L of dichloromethane, hydrochloric acid (1 normal (N), 500 mL), and distilled water was concentrated and then recrystallized by using dichloromethane and acetonitrile to synthesize Intermediate 1076(1) (white solid, 53.56 g, yield of 72 %).

Synthesis of Intermediate 1076(2)

Intermediate 1076(1) (7.38 g, 15 mmol), (2,5-difluorophenyl)boronic acid (3.55 g, 22.5 mmol), potassium phosphate tribasic ($K_3PO_4$) (4.78 g, 22.5 mmol), tris(dibenzylideneacetone)dipalladium ($Pd_2(dba_3)$) (275 mg, 0.3 mmol), and tri-tert-butylphosphine ($P(tBu)_3$) (242 mg, 12 mmol) were added to 30 mL of dioxane, and the reaction mixture was heated under reflux. After the reaction was completed, the reaction product was cooled to room temperature, the organic layer was extracted therefrom by using toluene and water, filtered through silica gel, concentrated, and then precipitated by using methanol to synthesize Intermediate 1076(2) (white solid, 6.17 g, yield of 72 %).

Synthesis of Compound 1076

Compound 1076 (6.8 g, yield of 58 %) was synthesized in the same manner as Compound 2 of Synthesis Example 1, except that Intermediate 1076(2) was used instead of Intermediate 2(1).

LC-MS (Calcd.: 1088.63 g/mol, Found: 1088.73 g/mol (M+1)).

Reference Synthesis Example 5 : Synthesis of Compound 1

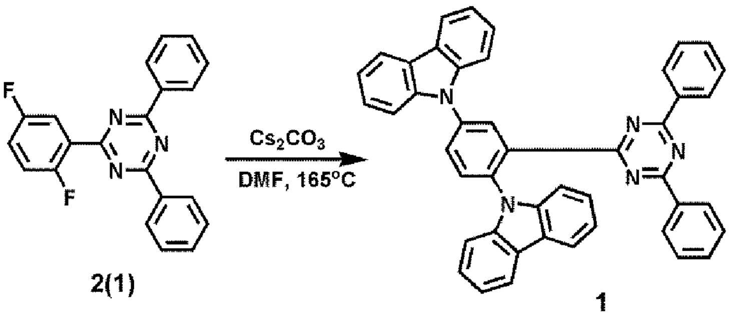


2(1)

1

Compound 1 (2.4 g, yield of 63 %) was synthesized in the same manner as Compound 1074 of Synthesis Example 2, except that carbazole (9H-carbazole) was used instead of 3,6-di-tert-butyl-9H-carbazole, and Intermediate 2(1) was used instead of Intermediate 1074(1).

LC-MS (Calcd.: 716.26 g/mol, Found: 717.26 g/mol (M+1)).

Synthesis Example 6 : Synthesis of Compound 1077

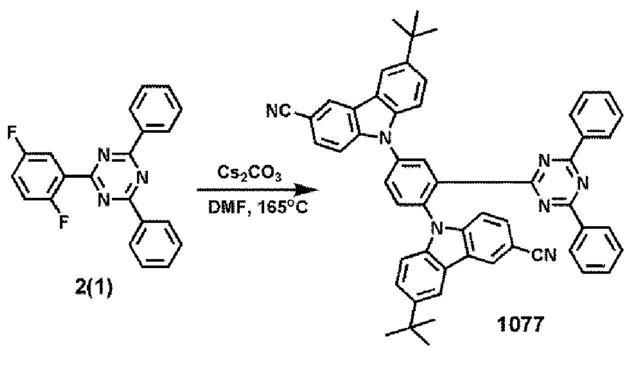


2(1)

1077

Compound 1077 (2.4 g, yield of 50 %) was synthesized in the same manner as in Synthesis Example 5, except that 6-(tert-butyl)-9H-carbazole-3-carbonitrile was used instead of carbazole.

LC-MS (Calcd.: 802.00 g/mol, Found: 802.10 g/mol (M+1)).

Synthesis Example 7 : Synthesis of Compound 14

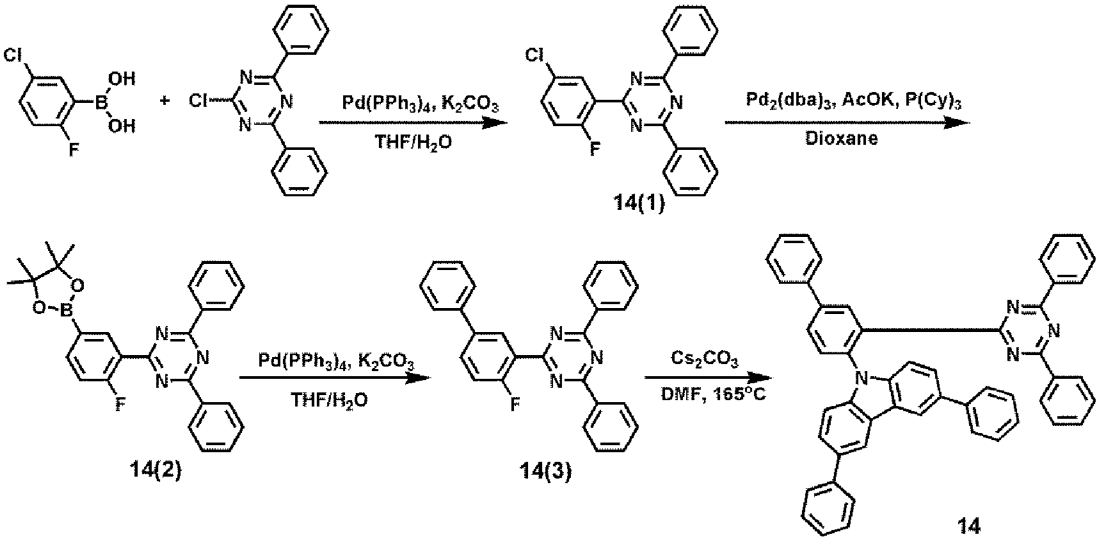


Synthesis of Intermediate 14(1)

Intermediate 14(1) (15.30 g, yield of 87 %) was synthesized in the same manner as Intermediate 2(1) of Synthesis Example 1, except that (5-chloro-2-fluorophenyl)boronic acid was used instead of (2,5-difluorophenyl)boronic acid.

LC-MS (Calcd.: 361.80 g/mol, Found: 361.90 g/mol (M+1)).

Synthesis of Intermediate 14(2)

Intermediate 14(1) (28.1 g, 77.67 mmol), bis(pinacolato)diboron (39.45 g, 155.33 mmol), potassium acetate (AcOK) (22.87 g, 233.00 mmol), tris(dibenzylideneacetone)dipalladium ($Pd_2(dba)_3$) (7.11 g, 7.77 mmol), and tricyclohexylphosphine ($P(Cy)_3$) (2.178 g, 7.77 mmol) were added to a reaction container, dissolved in 155 mL of dioxane, and the reaction mixture was stirred at a temperature of 120 °C. After the reaction was completed, the reaction product was cooled to room temperature, and the organic layer was extracted therefrom by using ethyl acetate and water, filtered through silica gel, and then concentrated. The solid compound (Intermediate 14(2)) (32.40 g, yield of 92 %) obtained therefrom was directly used in a subsequent reaction without any purification.

Synthesis of Intermediate 14(3)

Intermediate 14(3) (6 g, yield of 83 %) was synthesized in the same manner as Intermediate 2(1) of Synthesis Example 1, except that bromobenzene was used instead of 2-chloro-4,6-diphenyl-1,3,5-triazine.

LC-MS (Calcd.: 403.46 g/mol, Found: 400.56 g/mol (M+1)).

Synthesis of Compound 14

Compound 14 (0.66 g, yield of 30 %) was synthesized in the same manner as Compound 1074 of Synthesis Example 2, except that 3,6-diphenyl-9H-carbazole was used instead of 3,6-di-tert-butyl-9H-carbazole, and Intermediate 14(3) was used instead of Intermediate 1074(1).

LC-MS (Calcd.: 702.86 g/mol, Found: 702.96 g/mol (M+1)).

Synthesis Example 8 : Synthesis of Compound 13

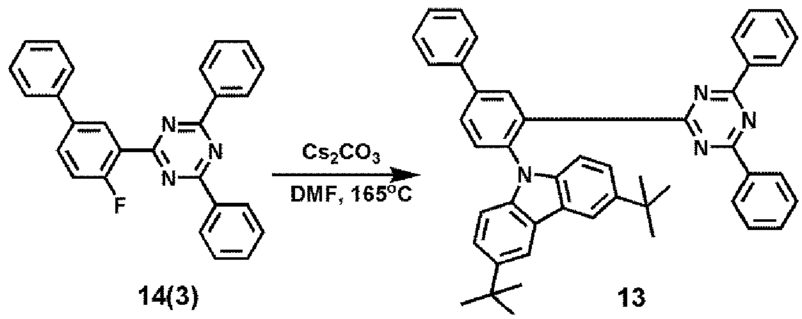


14(3) 13

Compound 13 (0.70 g, yield of 33 %) was synthesized in the same manner as Compound 14 of Synthesis Example 7, except that 3,6-di-tert-butyl-9H-carbazole was used instead of 3,6-diphenyl-9H-carbazole.
LC-MS (Calcd.: 662.88 g/mol, Found: 662.98 g/mol (M+1)).

Synthesis Example 9 : Synthesis of Compound 71

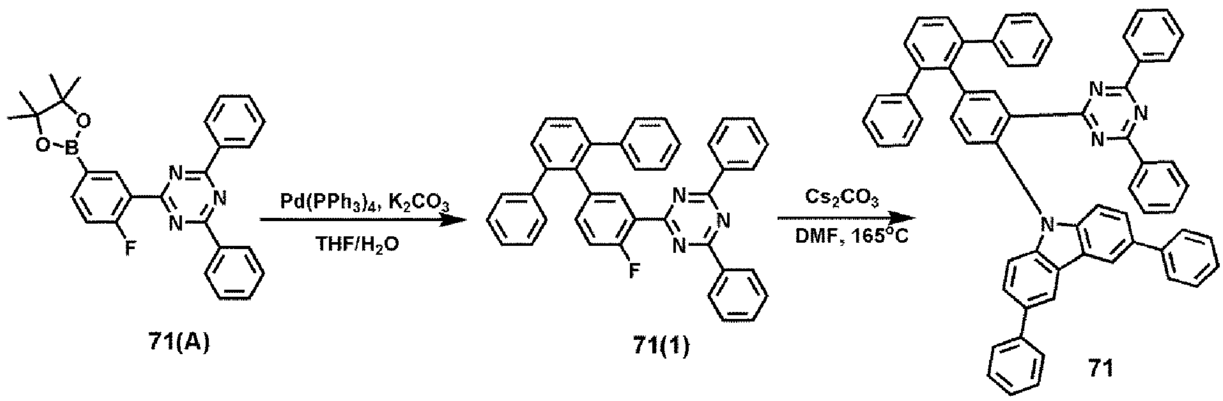


71(A) 71(1) 71

Synthesis of Intermediate 71(1)

Compound 71(1) (6 g, yield of 83 %) was synthesized in the same manner as Intermediate 2(1) of Synthesis Example 1, except that 2'-bromo-1,1':3',1"-terphenyl was used instead of 2-chloro-4,6-diphenyl-1,3,5-triazine, and a starting material 71(A) was used instead of (2,5-difluorophenyl)boronic acid.
LC-MS (Calcd.: 555.66 g/mol, Found: 555.76 g/mol (M+1)).

Synthesis of Compound 71

Compound 71 (4.7 g, yield of 70 %) was synthesized in the same manner as Compound 14 of Synthesis Example 7, except that Intermediate 71(1) was used instead of Intermediate 14(3).
LC-MS (Calcd.: 855.03 g/mol, Found: 855.13 g/mol (M+1)).

Synthesis Example 10 : Synthesis of Compound 70

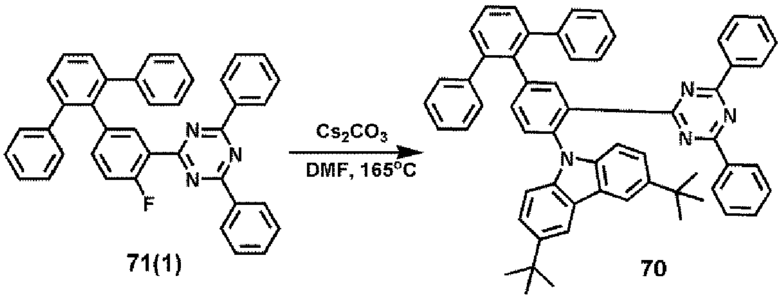


71(1) 70

Compound 70 (3.8 g, yield of 72 %) was synthesized in the same manner as Compound 71 of Synthesis Example 9, except that 3,6-di-tert-butyl-9H-carbazole was used instead of 3,6-diphenyl-9H-carbazole.
LC-MS (Calcd.: 815.06 g/mol, Found: 815.16 g/mol (M+1)).

Synthesis Example 11 : Synthesis of Compound 37

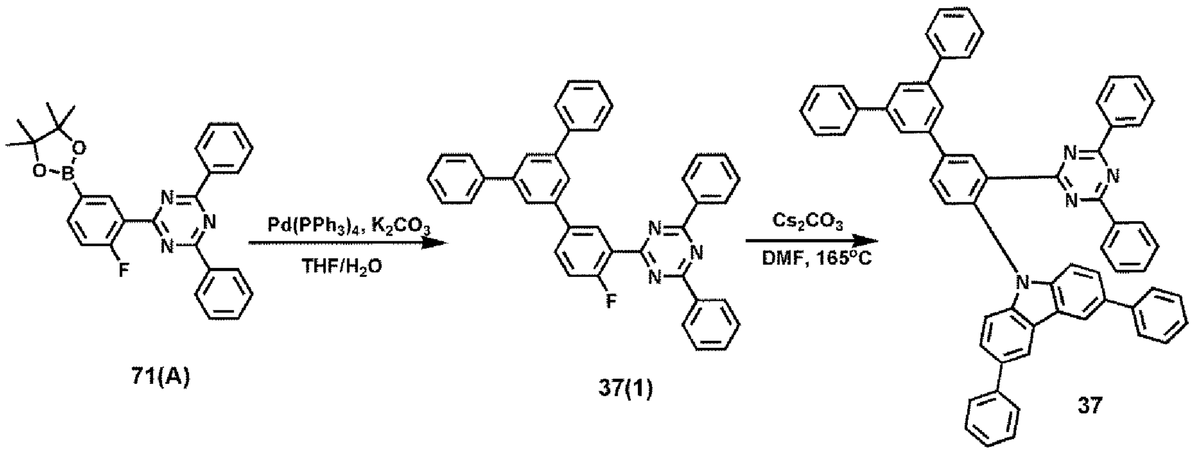


71(A) 37(1) 37

Synthesis of Intermediate 37(1)

Intermediate 37(1) (6 g, yield of 83 %) was synthesized in the same manner as Intermediate 2(1) of Synthesis Example 1, except that 5'-bromo-1,1':3',1"-terphenyl was used instead of 2-chloro-4,6-diphenyl-1,3,5-triazine, and a starting material 71(A) was used instead of (2,5-difluorophenyl)boronic acid.
LC-MS (Calcd.: 555.66 g/mol, Found: 555.76 g/mol (M+1)).

Synthesis of Compound 37

Compound 37 (4.9 g, yield of 82 %) was synthesized in the same manner as Compound 14 of Synthesis Example 7, except that Intermediate 37(1) was used instead of Intermediate 14(3).
LC-MS (Calcd.: 855.03 g/mol, Found: 855.13 g/mol (M+1)).

Synthesis Example 12 : Synthesis of Compound 46

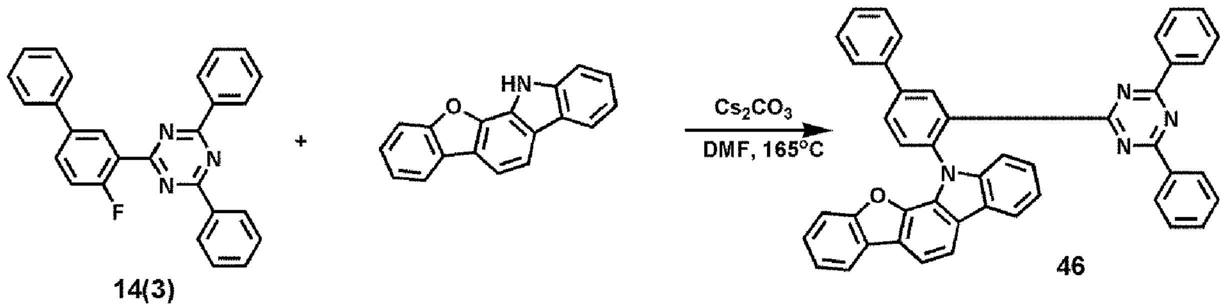


14(3)

46

Compound 46 (0.7 g, yield of 34 %) was synthesized in the same manner as Compound 14 of Synthesis Example 7, except that 12H-benzofuro[2,3-a]carbazole was used instead of 3,6-diphenyl-9H-carbazole.
LC-MS (Calcd.: 640.75 g/mol, Found: 640.85 g/mol (M+1)).

Synthesis Example 13 : Synthesis of Compound 21

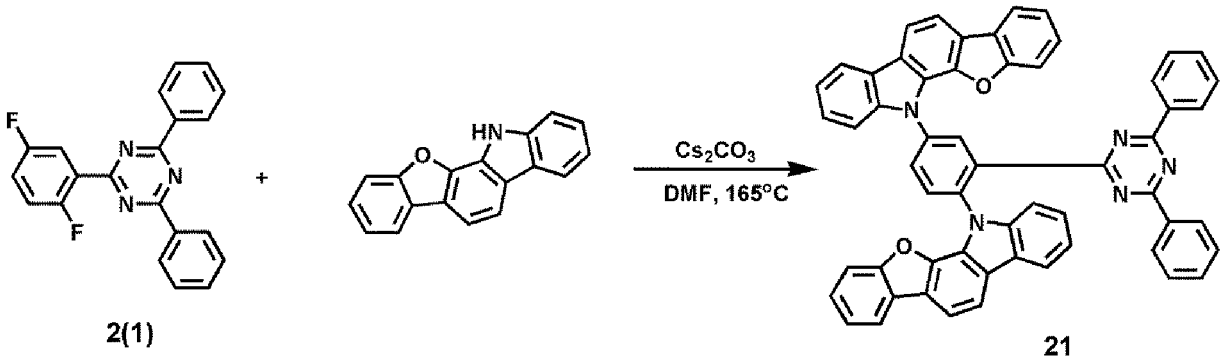


2(1)

21

Compound 21 (0.7 g, yield of 10 %) was synthesized in the same manner as in Synthesis Example 5, except that 12H-benzofuro[2,3-a]carbazole was used instead of carbazole.
LC-MS (Calcd.: 819.92 g/mol, Found: 820.02 g/mol (M+1)).

Synthesis Example 14 : Synthesis of Compound 346

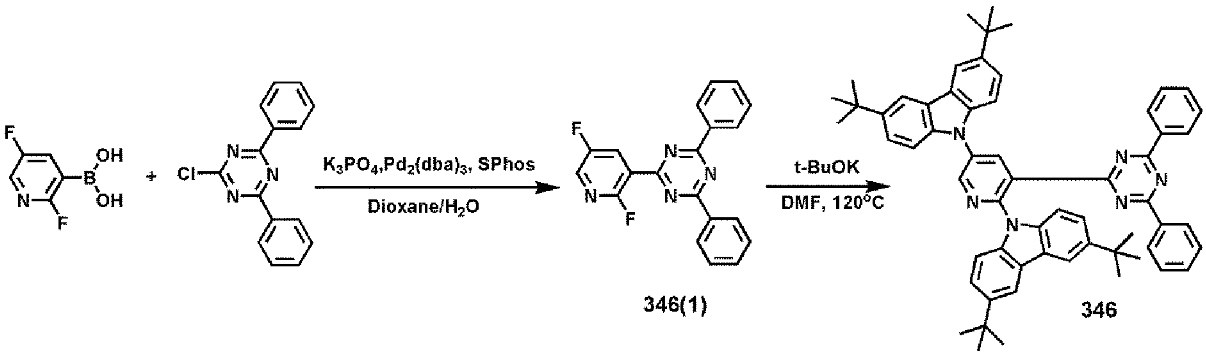


346(1)

346

Synthesis of Intermediate 346(1)

4-chloro-2,6-diphenyltriazine (10 g, 37.35 mmol), (2,5-difluoropyridin-3-yl)boronic acid (7.122 g, 44.82 mmol), potassium phosphate tribasic ($K_3PO_4$) (15.86 g, 74.70 mmol), tris(dibenzylideneacetone)dipalladium ($Pd_2(dba_3)$) (0.68 g, 0.75 mmol), and a phosphine ligand (SPhos) (1.533 g, 3.74 mmol) were added to a mixture of 60 mL of dioxane and 60 mL of distilled water, and the reaction mixture was heated under reflux. After the reaction was completed, the reaction product

was cooled to room temperature, and the organic layer was extracted therefrom by using toluene and water and filtered through silica gel. The obtained organic layer was concentrated and precipitated by using methanol to synthesize Intermediate 346(1) (white solid, 2.32 g, yield of 18 %).

LC-MS (Calcd.: 346.34 g/mol, Found: 346.3 g/mol (M+1)).

Synthesis of Compound 346

Compound 346 (3.2 g, yield of 55 %) was synthesized in the same manner as Compound 1074 of Synthesis Example 2, except that Intermediate 346(1) was used instead of Intermediate 1074(1).

LC-MS (Calcd.: 865.18 g/mol, Found: 866.2 g/mol (M+1)).

Synthesis Example 15 : Synthesis of Compound 25

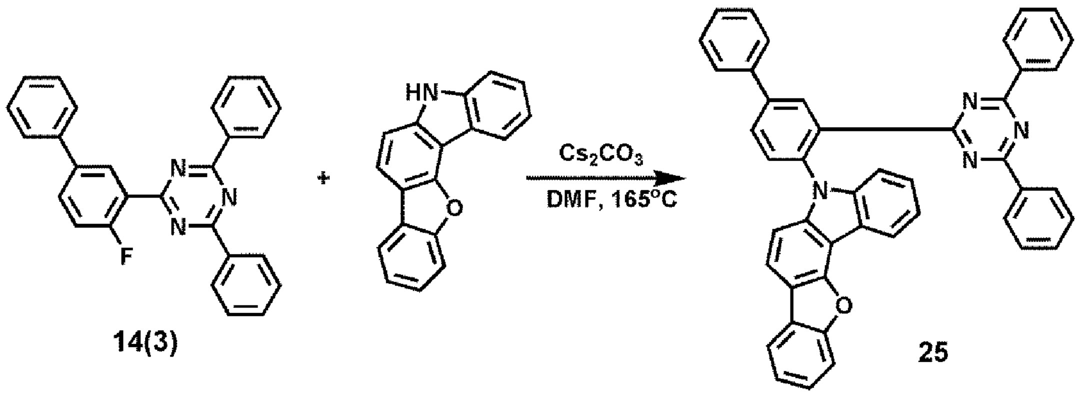


14(3)

25

Compound 25 (2.9 g, yield of 34 %) was synthesized in the same manner as Compound 14 of Synthesis Example 7, except that 5H-benzofuro[3,2-c]carbazole was used instead of 3,6-diphenyl-9H-carbazole.

LC-MS (Calcd.: 640.75 g/mol, Found: 640.85 g/mol (M+1)).

Reference Synthesis Example 16 : Synthesis of Compound 1078

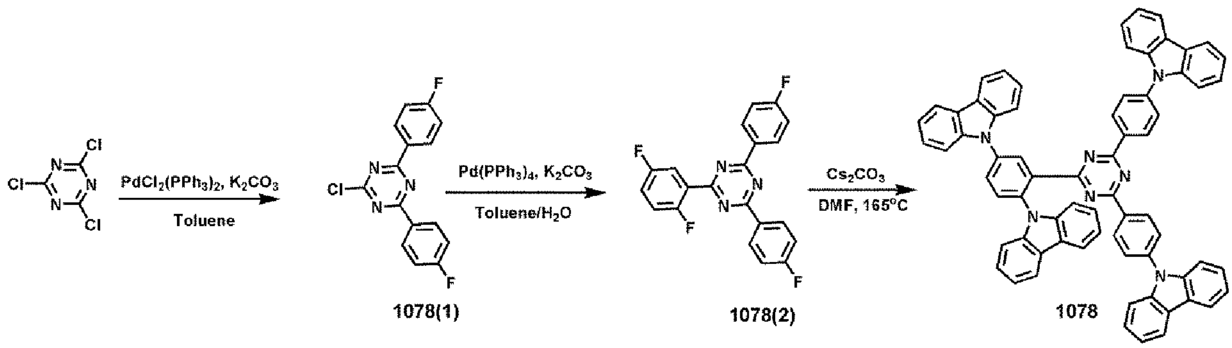


1078(1)

1078(2)

1078

Synthesis of Intermediate 1078(1)

2,4,6-trichloro-1,3,5-triazine (6.0 g, 32.54 mmol), (4-fluorophenyl)boronic acid (9.56 g, 68.33 mmol), potassium carbonate ($K_2CO_3$) (17.98 g, 130.15 mmol), and bis(triphenylphosphine)palladium(II), dichloride $PdCl_2(PPh_3)_4)$ (1.14 g, 1.63 mmol) were added to 70 mL of toluene, and the reaction mixture was heated under reflux at a temperature of 60 °C. After the reaction was completed, the reaction product was cooled to room temperature, and the organic layer was extracted by using toluene and water and filtered through silica gel. The obtained organic layer was concentrated and precipitated by using methanol to synthesize Intermediate 1078(1) (white solid, 4.01 g, yield of 40 %).

LC-MS (Calcd.: 303.54 g/mol, Found: 303.64 g/mol (M+1)).

Synthesis of Intermediate 1078(2)

Intermediate 1078(1) (i.e., 2-chloro-4,6-bis(4-fluorophenyl)-1,3,5-triazine) (3.8 g, 12.51 mmol), (2,5-difluorophenyl)boronic acid (2.37 g, 15.01 mmol), palladium tetrakis(triphenylphosphine) ($Pd(PPh_3)_4$) (0.72 g, 0.63 mmol), and potassium carbonate ($K_2CO_3$) (3.46 g, 25.02 mmol) were added to a mixture of 20 mL of toluene and 7 mL of distilled water, and the reaction mixture was heated under reflux at a temperature of 100 °C. After the reaction was completed, the reaction product was cooled to room temperature, and the organic layer was extracted therefrom by using toluene and water and filtered through silica gel. The obtained organic layer was concentrated and precipitated by using methanol to synthesize Intermediate 1078(2) (white solid, 4.2 g, yield of 88 %).

Synthesis of Compound 1078

Compound 1078 (2.6 g, yield of 27 %) was synthesized in the same manner as Compound 1 ofSynthesis Example 5, except that Intermediate 1078(2) was used instead of Intermediate 2(1).
LC-MS (Calcd.: 970.15 g/mol, Found: 970.25 g/mol (M+1)).

Evaluation Example 1: Evaluation of HOMO, LUMO, $T_1$, and $S_1$ energy levels

HOMO, LUMO, $T_1$, and $S_1$ energy levels of Compounds shown in Table 2 were measured by using methods described in Table 1, and results thereof are shown in Table 2.

Table 1

| | |
|---|---|
| HOMO energy level evaluation method | A voltage-current (V-A) graph of each Compound was obtained by using a cyclic voltammetry (CV) (electrolyte: 0.1 molar (M) $Bu_4NPF_6$ / solvent: $CH_2Cl_2$ / electrode: 3-electrode system (work electrode: glassy carbon, reference electrode: Ag/AgCl, auxiliary electrode: Pt wire)), and a HOMO energy level of each Compound was calculated from onset reduction potential of the graph. |
| LUMO energy level evaluation method | Each Compound was diluted at a concentration of $1x10^{-5}$ M in toluene, a UV absorption spectrum was measured at room temperature by using a Shimadzu UV-350 spectrometer, and a LUMO energy level thereof was calculated by using a HOMO energy level and an optical band gap ($E_g$) from the edge of the absorption spectrum. |
| $T_1$ energy level evaluation method | A mixture of toluene and each Compound (each Compound was dissolved in 3 mL of toluene so as to have a concentration of $1x10^{-4}$ M) was loaded into a quartz cell, and then, the resultant quartz cell was loaded into liquid nitrogen (77 Kelvin, K). A photoluminescence (PL) spectrum thereof was measured by using a photoluminescence measurement device, the obtained spectrum was compared with a PL spectrum measured at room temperature, and the peaks observed only at low temperature were analyzed to calculate an T1 energy level. |
| $S_1$ energy level evaluation method | A PL spectrum of a mixture of toluene and each Compound (diluted at a concentration of $1x10^{-4}$ M) was measured at room temperature by using a photoluminescence measurement device, and observed peaks were analyzed to calculate an onset S1 energy level. |

Table 2

| Compound No. | HOMO (eV) | LUMO (eV) | $T_1$ energy level (eV) | $S_1$ energy level (eV) | $\Delta E_{ST}$ (eV) |
|---|---|---|---|---|---|
| 2 | -5.153 | -1.886 | 2.58 | 2.685 | 0.105 |
| 1075 (reference) | -5.205 | -1.933 | 2.767 | 2.833 | 0.066 |
| 1076 | -5.09 | -1.728 | 2.69 | 2.811 | 0.121 |
| 1074 | -5.121 | -1.768 | 2.661 | 2.764 | 0.104 |
| 1077 | -5.803 | -2.274 | 2.815 | 2.925 | 0.111 |
| 1 (reference) | -5.354 | -1.942 | 2.705 | 2.806 | 0.102 |
| 13 | -5.134 | -1.807 | 2.632 | 2.725 | 0.093 |
| 70 | -5.108 | -1.756 | 2.668 | 2.75 | 0.082 |

(continued)

| Compound No. | HOMO (eV) | LUMO (eV) | $T_1$ energy level (eV) | $S_1$ energy level (eV) | $\Delta E_{ST}$ (eV) |
|---|---|---|---|---|---|
| 14 | -5.155 | -1.863 | 2.655 | 2.72 | 0.064 |
| 71 | -5.13 | -1.802 | 2.704 | 2.76 | 0.056 |
| 21 | -5.328 | -1.859 | 2.764 | 2.873 | 0.109 |
| 37 | -5.154 | -1.865 | 2.651 | 2.716 | 0.065 |
| 46 | -5.276 | -1.811 | 2.75 | 2.854 | 0.104 |
| 346 | -5.23 | -1.952 | 2.626 | 2.734 | 0.108 |
| 25 | -5.183 | -1.856 | 2.692 | 2.759 | 0.067 |
| 1078 (reference) | -5.423 | -2.109 | 2.589 | 2.748 | 0.158 |

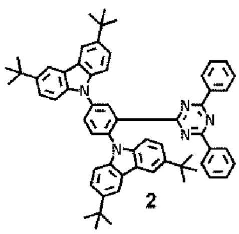
2

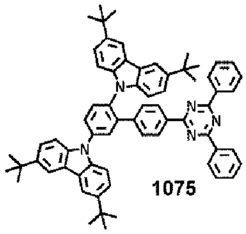
1075

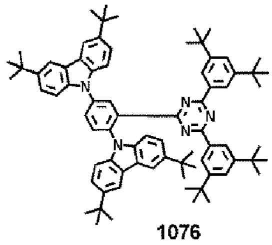
1076

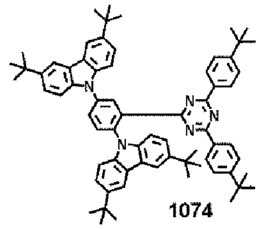
1074

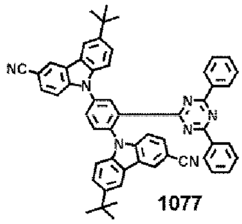
1077

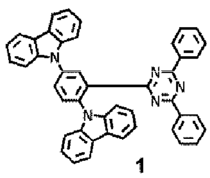
1

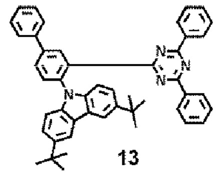
13

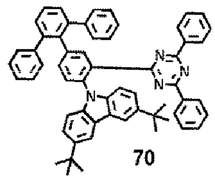
70

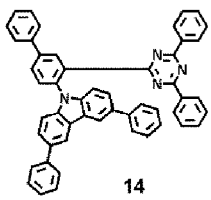
14

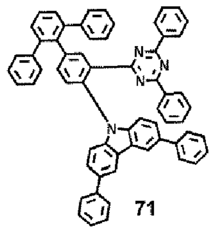
71

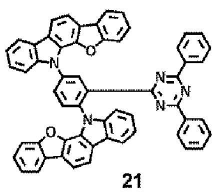
21

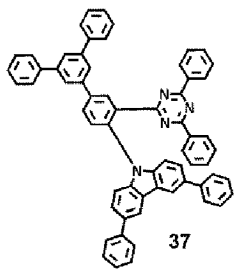
37

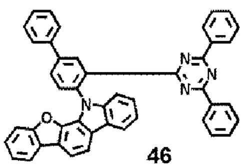
46

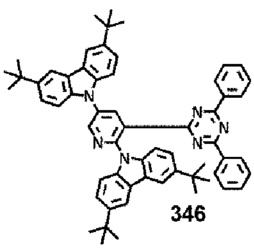
346

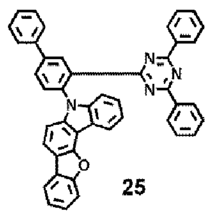
25

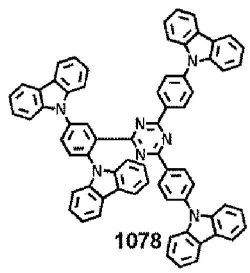
1078

Referring to Table 2, it is confirmed that Compounds shown in Table 2 have excellent electric characteristics.

Evaluation Example 2: Evaluation of full width at half maximum (FWHM)

PL spectra of Compounds shown in Table 4 were measured by using methods described in Table 3, and FWHM of

each Compound was evaluated. The results thereof are shown in Table 4.

Table 3

| | |
|---|---|
| PL spectrum measurement method | Each Compound was diluted at a concentration of $1x10^{-4}$ M in toluene, and PL spectrum was measured by using F7000 Spectrofluorometer equipped with a Spxenon (Xenon) lamp (available from Hitachi) (@ 298 K). |

Table 4

| Compound No. | FWHM (nm) |
|---|---|
| 2 | 65 |
| 1075 (reference) | 61 |
| 1076 | 60 |
| 1074 | 65 |
| 1077 | 61 |
| 1 (reference) | 62 |
| 13 | 75 |
| 70 | 65 |
| 14 | 72 |
| 71 | 64 |
| 21 | 66 |
| 37 | 65 |
| 46 | 61 |
| 346 | 64 |
| 25 | 69 |
| 1078 (reference) | 64 |

Referring to Table 4, it is confirmed that Compounds shown in Table 4 have excellent luminescence characteristics.

Evaluation Example 3 : Evaluation of photoluminescence quantum yield (PLQY) and decay time

(1) Preparation of thin film

A quartz substrate washed with chloroform and pure water was prepared, and Compound 2 and Compound E4 were co-deposited at a weight ratio of 5:5 at a vacuum degree of $10^{-7}$ torr to manufacture a thin film having a thickness of 50 nanometers (nm).

(2) Evaluation of PLQY

A PLQY of the thin film was evaluated by using Hamamatsu a Photonics absolute PL quantum yield measurement system including a xenon light source, a monochromator, a photonic multichannel analyzer, and an integrating sphere and employing a PLQY measurement software (Hamamatsu Photonics, Ltd., Shizuoka, Japan), and a PLQY of Compound 2 in film was evaluated.

(3) Evaluation of decay time

A PL spectrum of the thin film was evaluated at room temperature by using a PicoQuant TRPL measurement system FluoTime 300 and a PicoQuant pumping source PLS340 (excitation wavelength = 340 nm, spectral width = 20 nm), a wavelength of a main peak of the spectrum was determined, PLS340 repeatedly measured the number of photons

emitted from the thin film at the wavelength of the main peak due to a photon pulse (pulse width = 500 picoseconds, ps) applied to the thin film according to time based on time-correlated single photon counting (TCSPC), thereby obtaining a sufficiently fittable TRPL curve. $T_{decay}$(Ex) (decay time) of Compound 2 of the thin film was obtained by fitting two or more exponential decay function to the result obtained therefrom. The function used for fitting is expressed by Equation 1, and the greatest value of $T_{decay}$ obtained from each exponential decay function used for fitting was taken as $T_{decay}$(Ex) and shown in Table 5. The remaining values of $T_{decay}$ may be used to determine a lifetime of a decay of a general fluorescence. At this time, a baseline or background signal curve was obtained by repeating the same measurement once more for the same measurement time as the measurement time for obtaining the TRPL curve in a dark state (a state in which a pumping signal applied to the predetermined film was blocked), and the baseline or background signal curve was fitted and used as a baseline.

Equation 1

$$f(t) = \sum_{i=1}^{n} A_i \, exp\left(-t/T_{decay,i}\right)$$

(4) Table 5

The procedures of the above (1) to (3) were repeated with respect to the remaining Compounds of Table 5, and results thereof are shown in Table 5.

Table 5

| Compound No. | PLQY | $T_{decay}$(EX) ($\mu$s) (decay time) |
|---|---|---|
| 2 | 0.57 | 12.63 |
| 1075 (reference) | 0.41 | 32.679 |
| 1076 | 0.7 | 44.3 |
| 1074 | 0.63 | 25.08 |
| 1077 | 0.38 | 41.5 |
| 1 (reference) | 0.45 | 58.6 |
| 13 | 0.51 | 60.1 |
| 70 | 0.47 | 10.3 |
| 14 | 0.66 | 24.2 |
| 71 | 0.52 | 10.6 |
| 21 | 0.34 | 56 |
| 37 | 0.6 | 19 |
| 46 | 0.3 | 86.2 |
| 346 | 0.44 | 20.3 |
| 25 | 0.52 | 20.9 |
| 1078 (reference) | 0.44 | 61.1 |

Referring to Table 5, it is confirmed that Compounds shown in Table 5 have excellent PLQY (in film) and decay time characteristics.

Example 1

A glass substrate, on which a 1,500 Å-thick ITO electrode (first electrode, anode) was formed, was sonicated with distilled water. After the washing with distilled water was completed, ultrasonic wave washing was performed by using a solvent such as iso-propyl alcohol, acetone, and methanol, and the glass substrate was dried and then transferred to a plasma cleaner. The glass substrate was cleaned for 5 minutes by using oxygen plasma and provided to a vacuum

deposition apparatus.

Compound HT3 and Compound HT-D2 were co-deposited on the ITO electrode of the glass substrate to form a hole injection layer having a thickness of 100 Å, Compound HT3 was deposited on the hole injection layer to form a hole transport layer having a thickness of 1,350 Å, and mCP was deposited on the hole transport layer to form an electron blocking layer having a thickness of 100 Å, thereby forming a hole transport region.

A host and a dopant were co-deposited on the hole transport region at a weight ratio of 85:15 to form an emission layer having a thickness of 300 Å. Compound E4 was used as the host, and Compound 2 was used as the dopant.

Compound BCP was vacuum-deposited on the emission layer to form a hole blocking layer having a thickness of 100 Å, Compound ET3 and LiQ were vacuum-deposited on the hole blocking layer to form an electron transport layer having a thickness of 300 Å, LiQ was deposited on the electron transport layer to form an electron injection layer having a thickness of 10 Å, and Al was deposited on the electron injection layer to form a second electrode (cathode) having a thickness of 1,000 Å, thereby completing the manufacture of an organic light-emitting device.

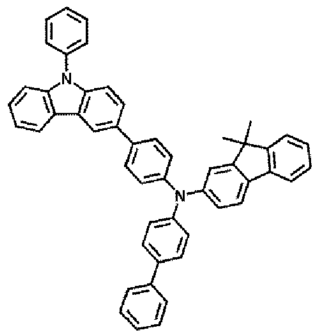
HT3

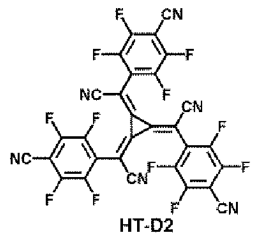
HT-D2

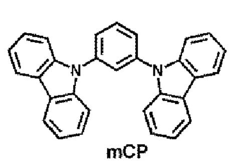
mCP

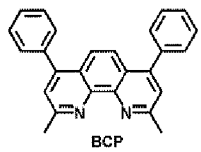
BCP

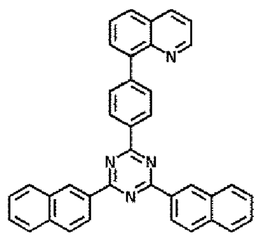
ET3

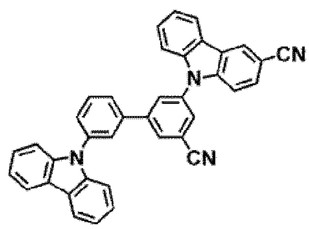
E4

Examples 2 to 8 and Comparative Examples A-1 to D

Organic light-emitting devices were manufactured in the same manner as in Example 1, except that the dopant in the emission layer was changed as shown in Table 6.

Evaluation Example 4 : Evaluation of device data

The driving voltage, luminescence efficiency, and lifespan ($T_{95}$) of each of Examples 1 to 8 and Comparative Examples A-1 to D were measured by using a current-voltage meter (Keithley 2400) and a luminance meter (Minolta Cs-1000A), and the results thereof are shown in Table 6. The lifespan ($T_{95}$) data (at 500 candelas per square meter, cd/$m^2$) in Table 6 indicates an amount of time (hours, hr) that lapsed when luminance was 95 % of initial luminance (100 %). The luminescence efficiency and the lifespan are relative values with respect to the luminescence efficiency and the lifespan of

Example 2.

Table 6

| | Dopan t No. | Driving voltage (V) | Luminescence efficiency (relative value, %) | Lifespan ($T_{95}$) (relative value, %) |
|---|---|---|---|---|
| Example 1 | 2 | 4.26 | 129 | 1152 |
| Example 2 | 1076 | 4.503 | 100 | 100 |
| Example 3 | 1074 | 4.249 | 121 | 352 |
| Example 4 | 13 | 4.312 | 127 | 102 |
| Example 5 | 14 | 4.212 | 142 | 1354 |
| Example 6 | 37 | 4.259 | 141 | 1287 |
| Example 7 | 346 | 4.461 | 147 | 585 |
| Example 8 | 25 | 3.89 | 134 | 354 |
| Reference Example 9 | 1078 | 4.561 | 113 | 94 |
| Comparative Example A-1 | A-1 | 5.267 | 18 | 1 |
| Comparative Example A-2 | A-2 | 5.976 | 13 | 7 |
| Comparative Example A-3 | A-3 | 4.193 | 70 | 30 |
| Comparative Example A-4 | A-4 | 4.663 | 81 | 54 |
| Comparative Example A-5 | A-5 | 4.574 | 111 | 61 |
| Comparative Example B-1 | B-1 | 5.2 | 38 | 1 |
| Comparative Example B-2 | B-2 | 5.063 | 33 | 4 |
| Comparative Example B-3 | B-3 | 4.219 | 106 | 41 |
| Comparative Example B-4 | B-4 | 4.12 | 81 | 65 |
| Comparative Example B-5 | B-5 | 4.149 | 97 | 31 |
| Comparative Example B-6 | B-6 | 4.595 | 70 | 9 |
| Comparative Example C-1 | C-1 | 8.2 | 8 | 0 |
| Comparative Example C-2 | C-2 | 10.2 | 8 | 0 |
| Comparative Example C-3 | C-3 | 7.5 | 22 | 0 |
| Comparative Example D | D | 6.1 | 87 | 20 |

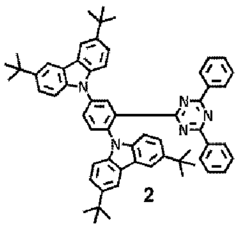
2

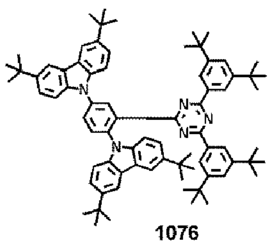
1076

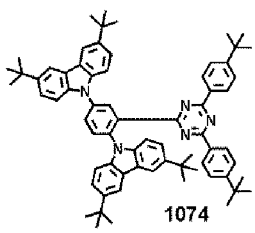
1074

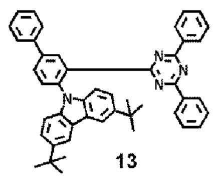
13

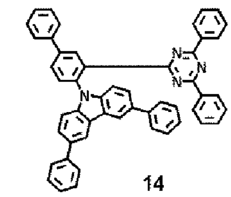
14

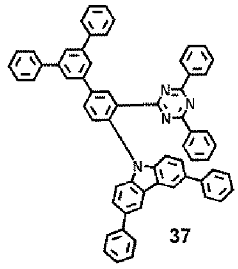
37

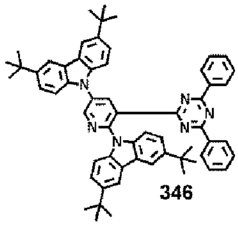
346

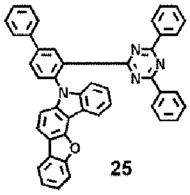
25

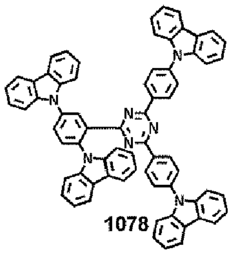
1078

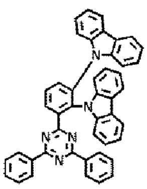
A-1

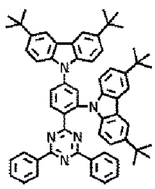
A-2

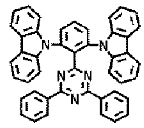
A-3

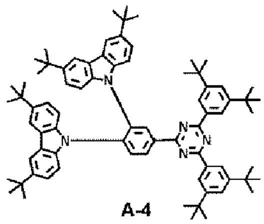
A-4

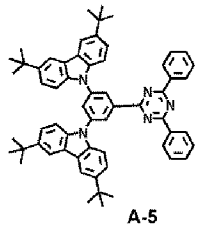
A-5

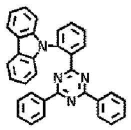
B-1

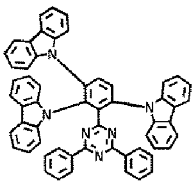
B-2

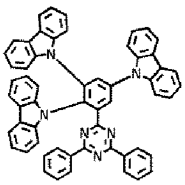
B-3

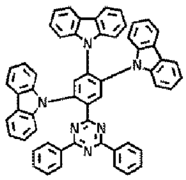
B-4

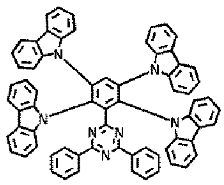
B-5

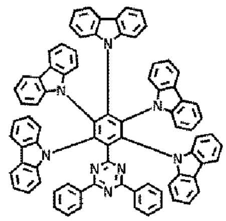
B-6

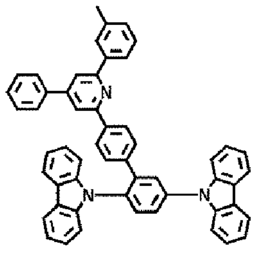
C-1

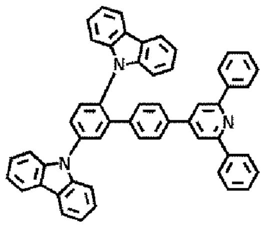
C-2

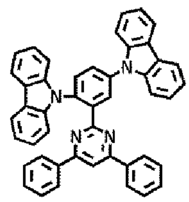
C-3

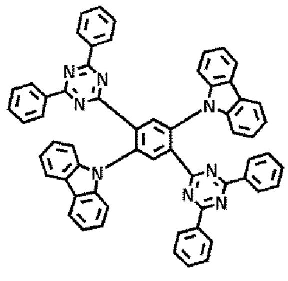
D

Referring to Table 6, it is confirmed that the organic light-emitting devices of Examples 1 to 8 have excellent driving voltage, luminescence efficiency and lifespan "at the same time", as compared with those of the organic light-emitting devices of Comparative Examples A-1 to A-5, B-1 to B-6, C-1 to C-3, and D.

Since the heterocyclic compound has excellent electric characteristics and thermal stability, the organic light-emitting device including the heterocyclic compound may have low driving voltage, low driving voltage, high luminescence efficiency, and long lifespan characteristics.

## Claims

1. A heterocyclic compound represented by Formula 1:

Formula 1

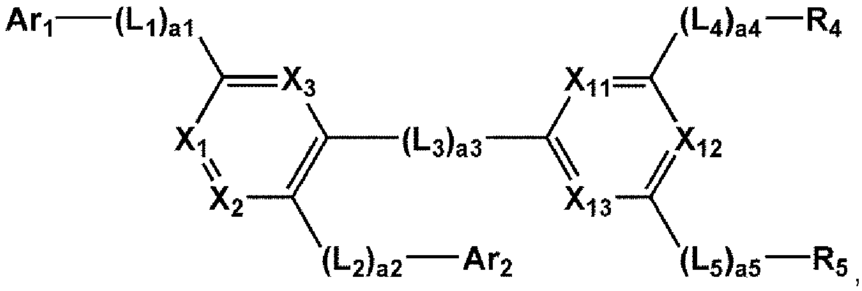

wherein, in Formula 1,

$X_1$ is N or $C(R_1)$, $X_2$ is N or $C(R_2)$, and $X_3$ is N or $C(R_3)$, wherein i) one selected from $X_1$ to $X_3$ is N, and the others thereof are not N; or ii) each of $X_1$ to $X_3$ is not N,
$R_1$ to $R_3$ are each independently hydrogen, deuterium, a $C_1$-$C_{20}$ alkyl group, a $C_1$-$C_{20}$ alkyl group substituted with at least one deuterium, a $C_1$-$C_{20}$ alkoxy group, or a $C_1$-$C_{20}$ alkoxy group substituted with at least one deuterium,
$L_1$ and $L_2$ are a single bond,
$X_{11}$ to $X_{13}$ are each independently N,
$Ar_1$ and $Ar_2$ are each independently a group represented by one selected from Formulae D'-1 to D'-7 and D002 to D005, D015 to D0027, D0029 to D0032 and D0034 to D279,
a group represented by *-$(L_3)_{a3}$-*' is a single bond,
a group represented by

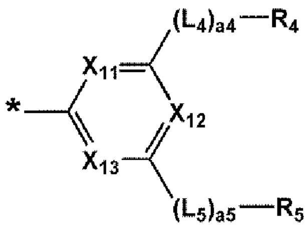

is a group represented by one selected from Formulae A1 to A38,
* in a group represented by

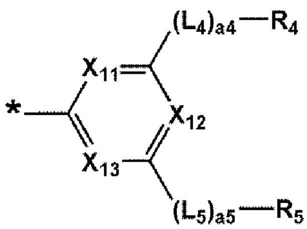

indicates a binding site to a neighboring atom, and
in a group represented by *-$(L_3)_{a3}$-*', * indicates a binding site to a 6-membered ring on the left side in Formula 1, and *' indicates a binding site to a 6-membered ring on the right side in Formula 1:

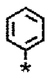 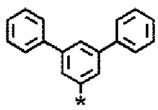 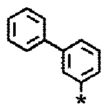 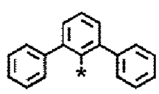 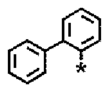 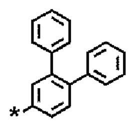 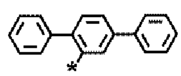

D'-1 D'-2 D'-3 D'-4 D'-5 D'-6 D'-7

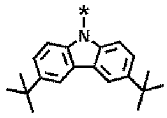 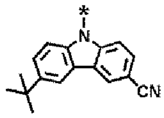 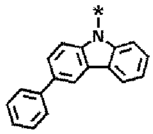 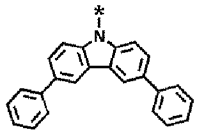

D002 D003 D004 D005

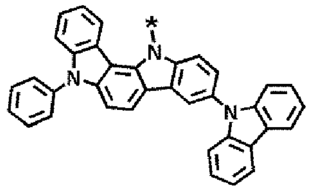 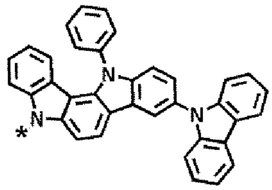 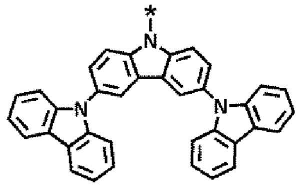

D015 D016 D017

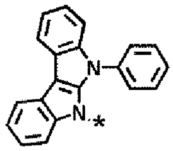 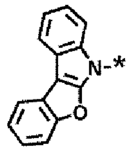 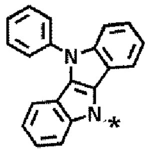 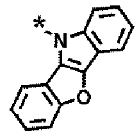 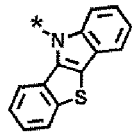 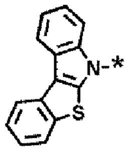

D018 D019 D020 D021 D022 D023

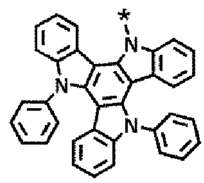 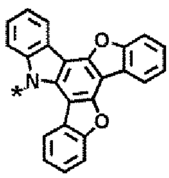 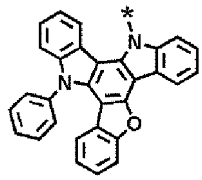 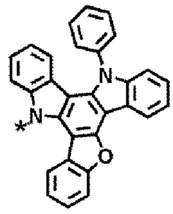

D024 D025 D026 D027

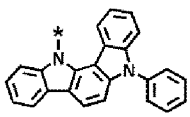 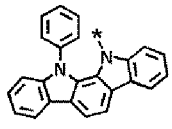 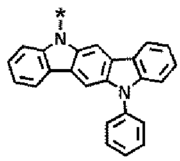 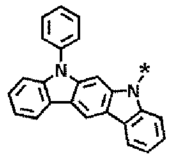

D029 D030 D031 D032

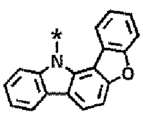 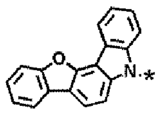 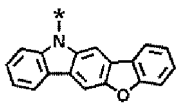 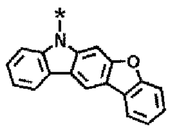 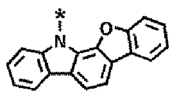 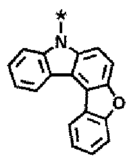

D034 D035 D036 D037 D038 D039

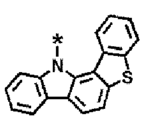 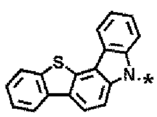 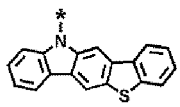 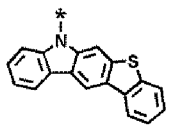 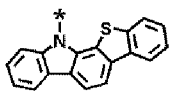 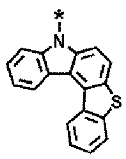

D040 D041 D042 D043 D044 D045

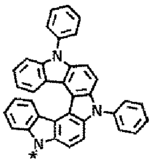
D046
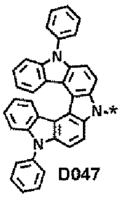
D047
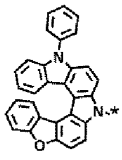
D048
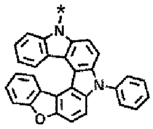
D049
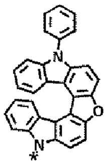
D050
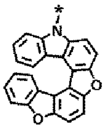
D051
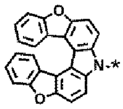
D052

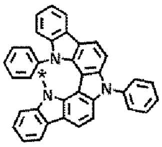
D053
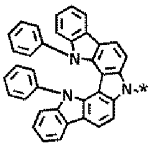
D054
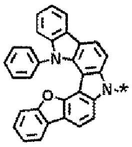
D055
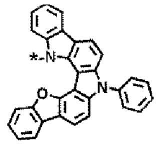
D056
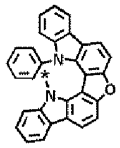
D057
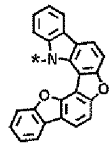
D058
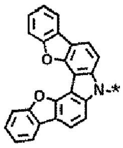
D059

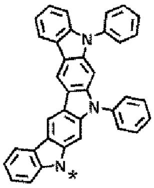
D060
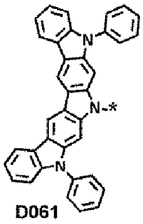
D061
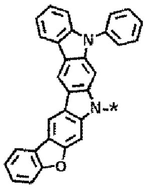
D062
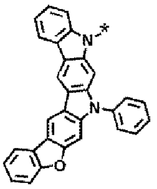
D063
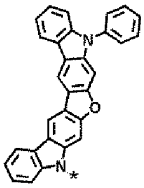
D064
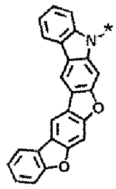
D065
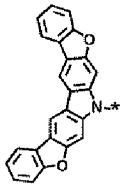
D066

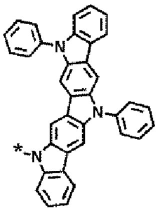
D067
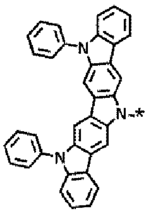
D068
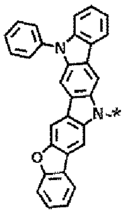
D069
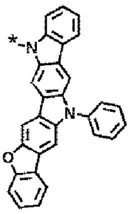
D070
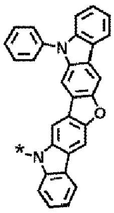
D071
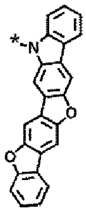
D072
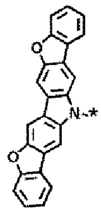
D073

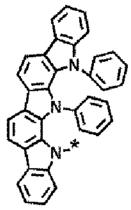
D074
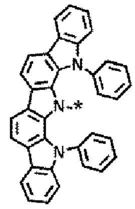
D075
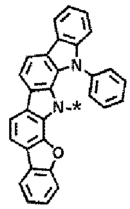
D076
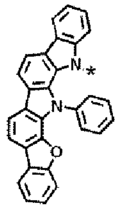
D077
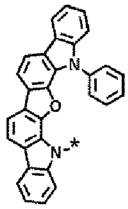
D078
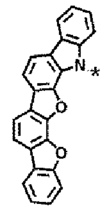
D079
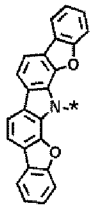
D080

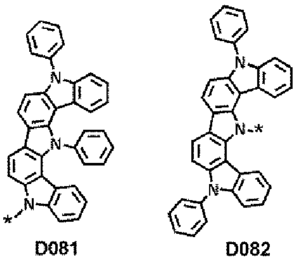
D081
D082
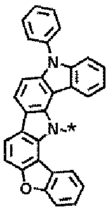
D083
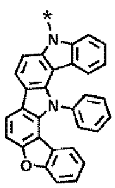
D084
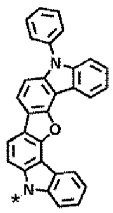
D085
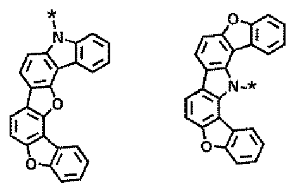
D086
D087

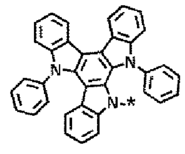
D088
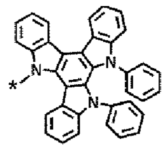
D089
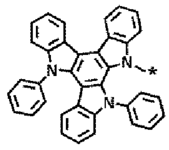
D090
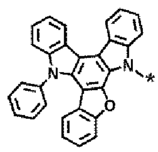
D091
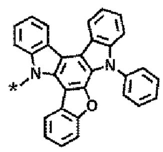
D092
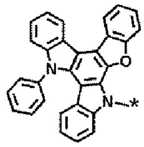
D093

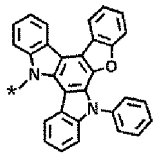
D094
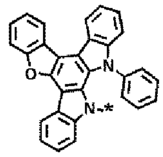
D095
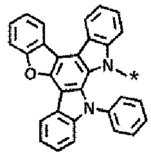
D096
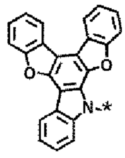
D097
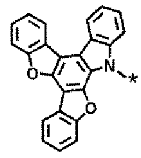
D098
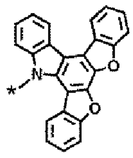
D099

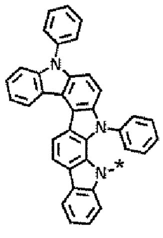
D100
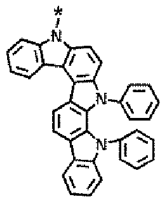
D101
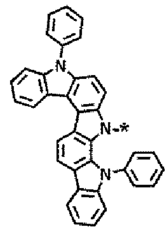
D102
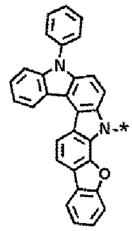
D103
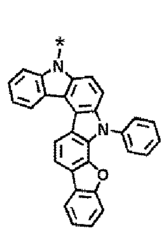
D104
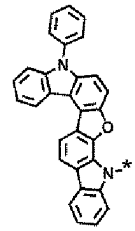
D105

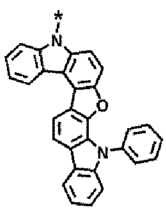
D106
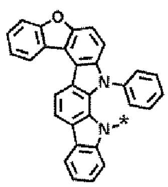
D107
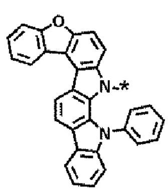
D108
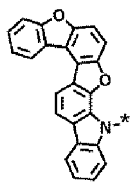
D109
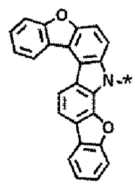
D110
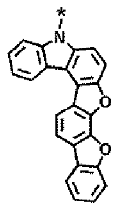
D111

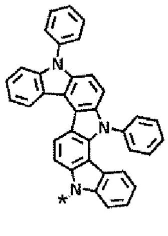
D112
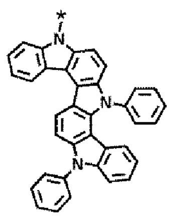
D113
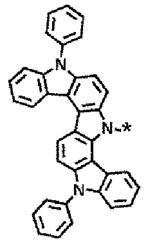
D114
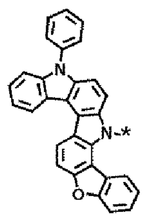
D115
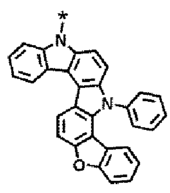
D116
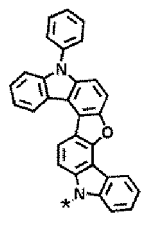
D117

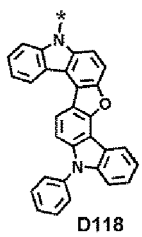
D118
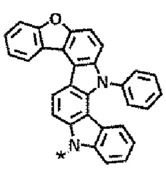
D119
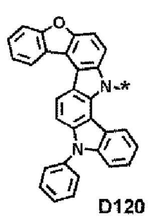
D120
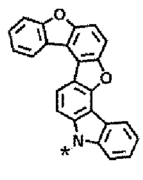
D121
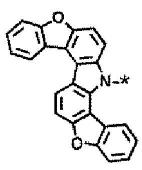
D122
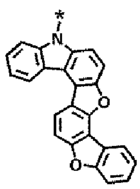
D123

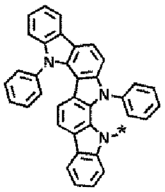
D124
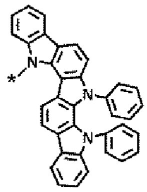
D125
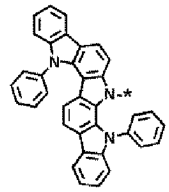
D126
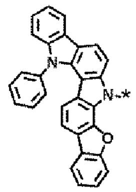
D127
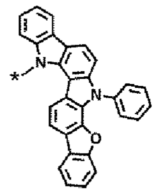
D128
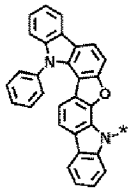
D129

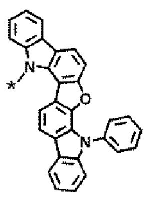
D130
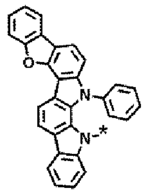
D131
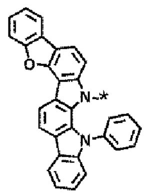
D132
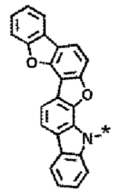
D133
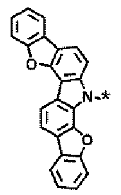
D134
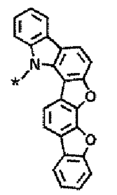
D135

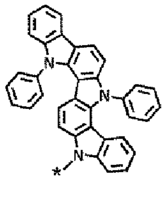
D136
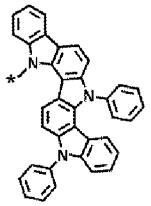
D137
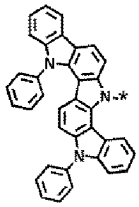
D138
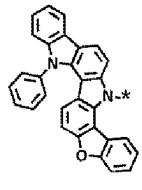
D139
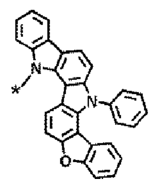
D140
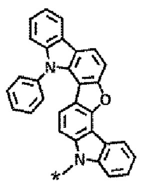
D141

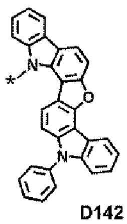
D142
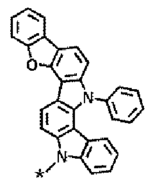
D143
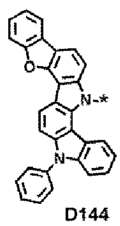
D144
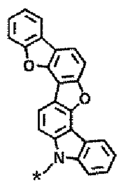
D145
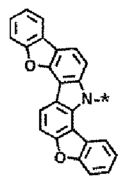
D146
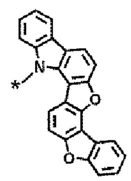
D147

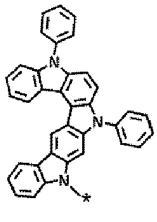
D148
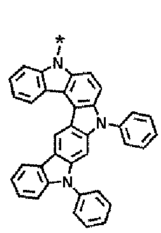
D149
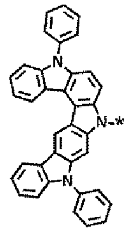
D150
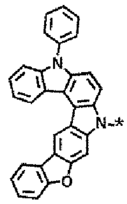
D151
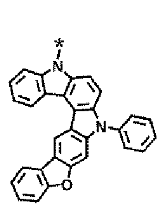
D152
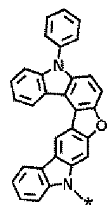
D153

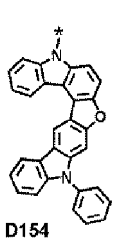
D154
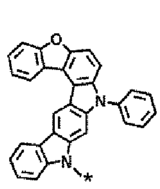
D155
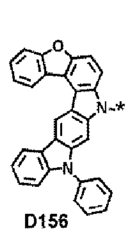
D156
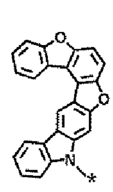
D157
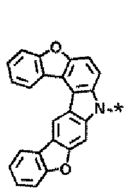
D158
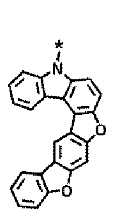
D159

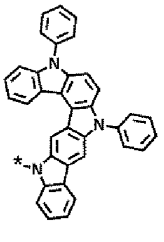
D160
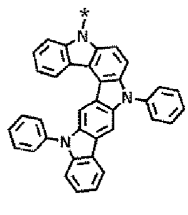
D161
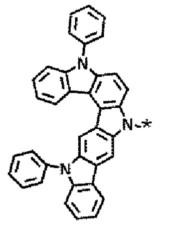
D162
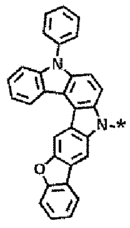
D163
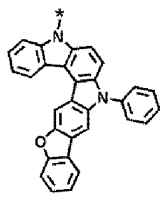
D164
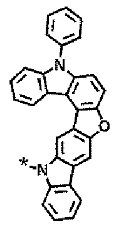
D165

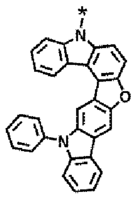
D166
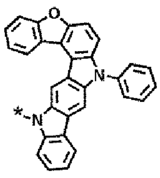
D167
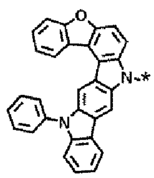
D168
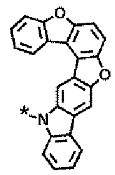
D169
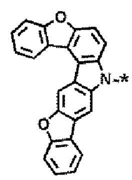
D170
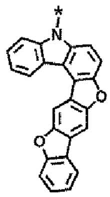
D171

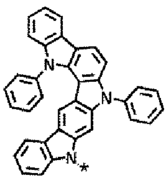
D172
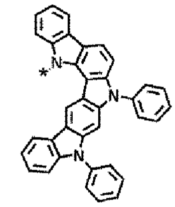
D173
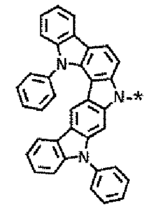
D174
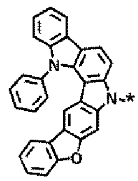
D175
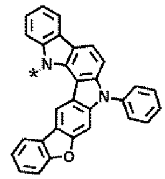
D176
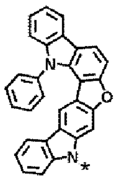
D177

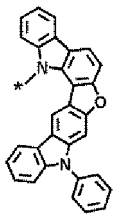
D178
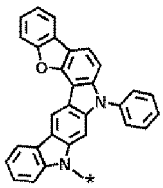
D179
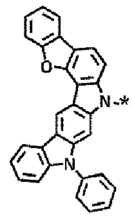
D180
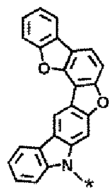
D181
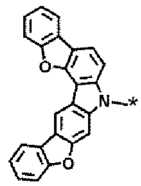
D182
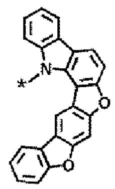
D183

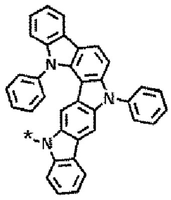
D184
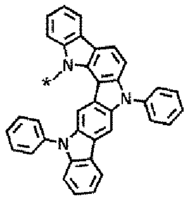
D185
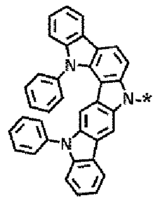
D186
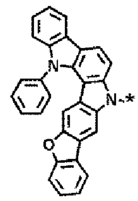
D187
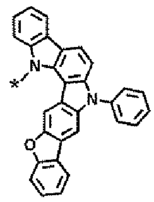
D188
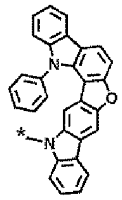
D189

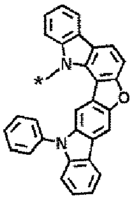
D190
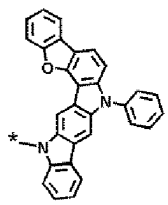
D191
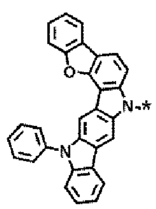
D192
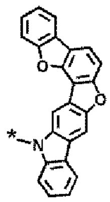
D193
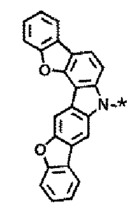
D194
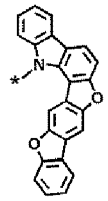
D195

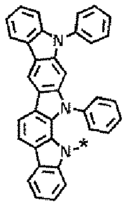
D196
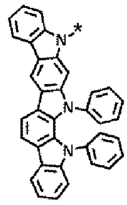
D197
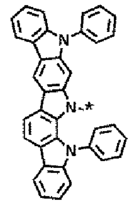
D198
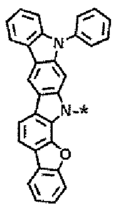
D199
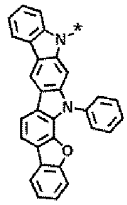
D200
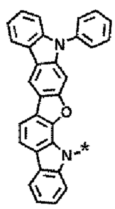
D201

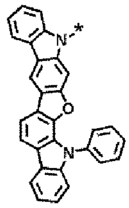
D202
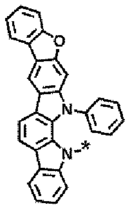
D203
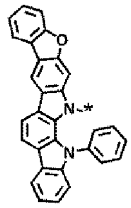
D204
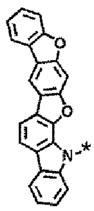
D205
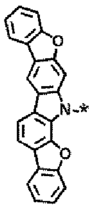
D206
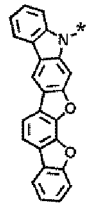
D207

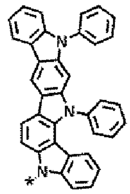
D208
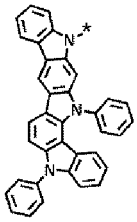
D209
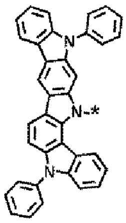
D210
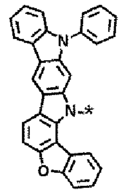
D211
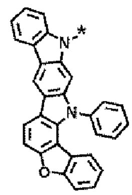
D212
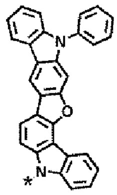
D213

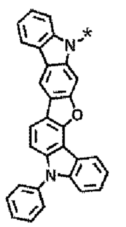
D214
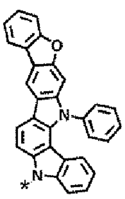
D215
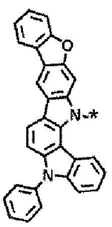
D216
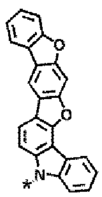
D217
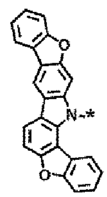
D218
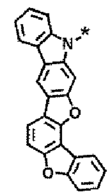
D219

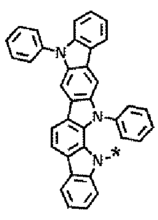
D220
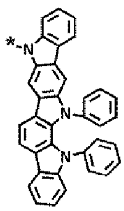
D221
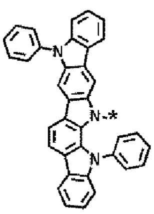
D222
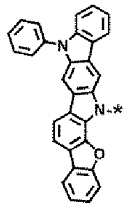
D223
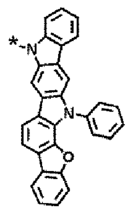
D224
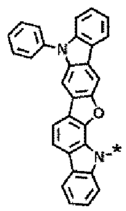
D225

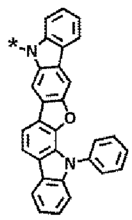
D226
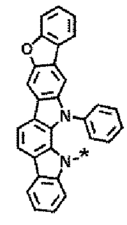
D227
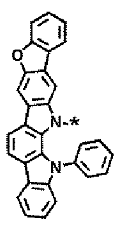
D228
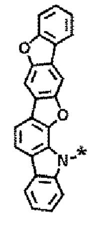
D229
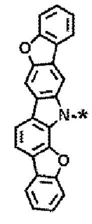
D230
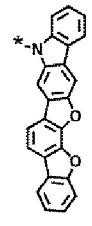
D231

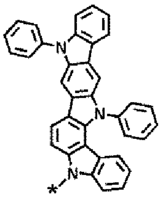
D232

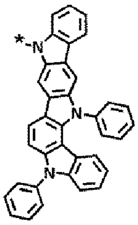
D233

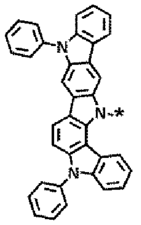
D234

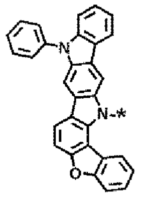
D235

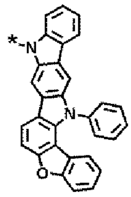
D236

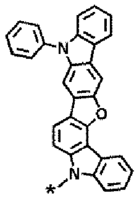
D237

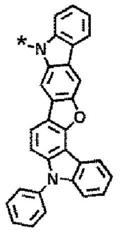
D238

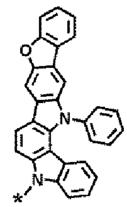
D239

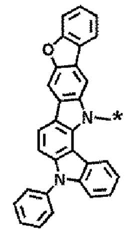
D240

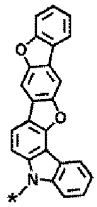
D241

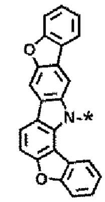
D242

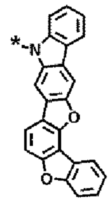
D243

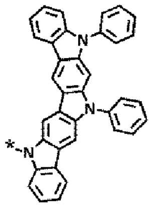
D244

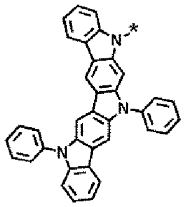
D245

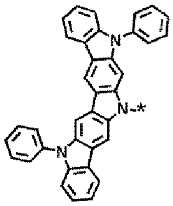
D246

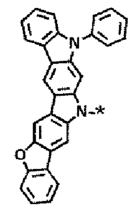
D247

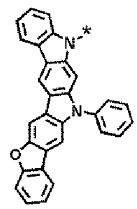
D248

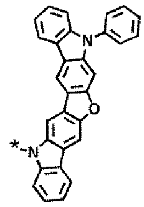
D249

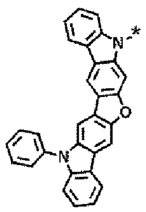
D250

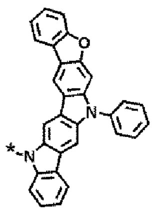
D251

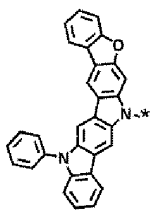
D252

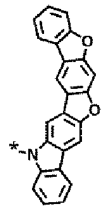
D253

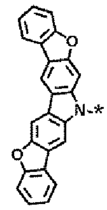
D254

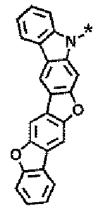
D255

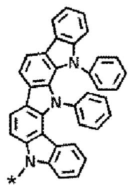
D256

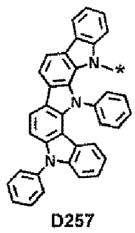
D257

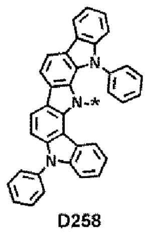
D258

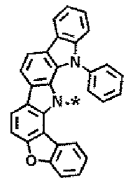
D259

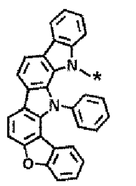
D260

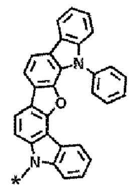
D261

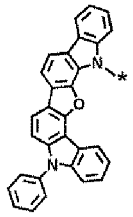
D262

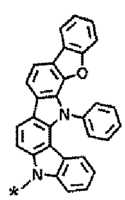
D263

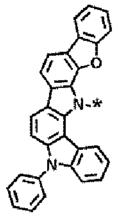
D264

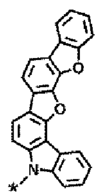
D265

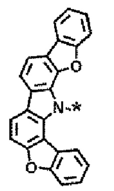
D266

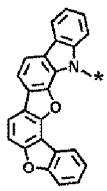
D267

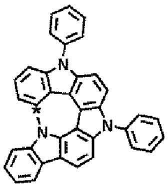
D268
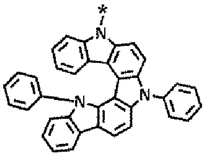
D269
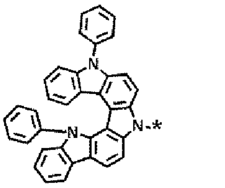
D270
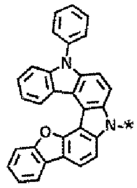
D271
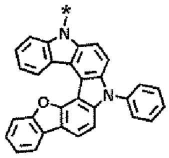
D272
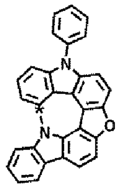
D273

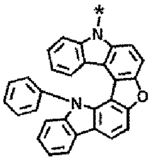
D274
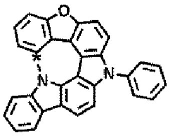
D275
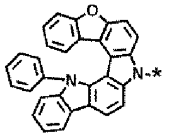
D276
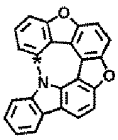
D277
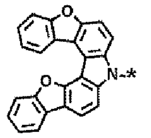
D278
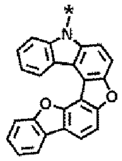
D279

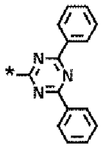
A1
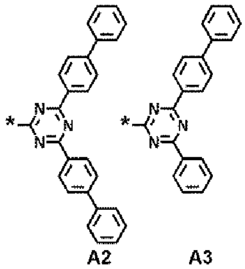
A2 A3
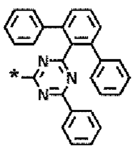
A4
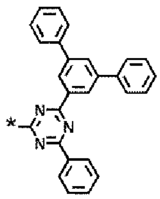
A5
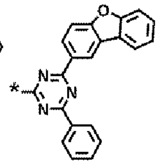
A6
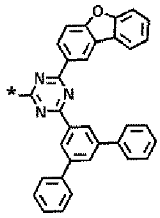
A7
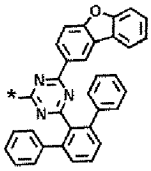
A8

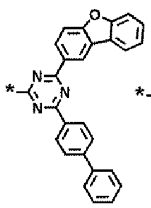
A9
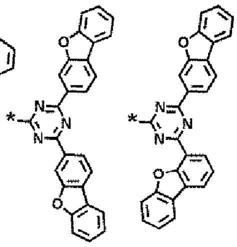
A10 A11
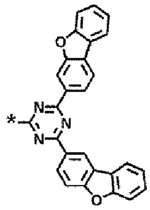
A12
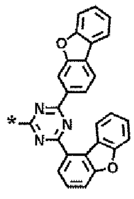
A13
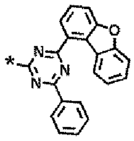
A14
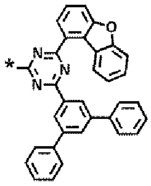
A15
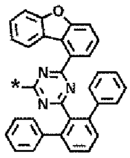
A16

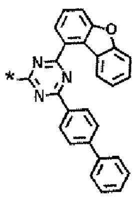
A17
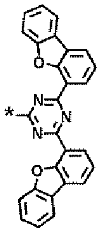
A18
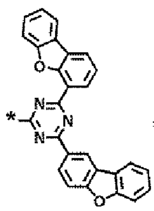
A19
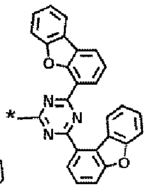
A20
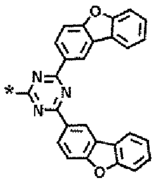
A21
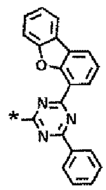
A22
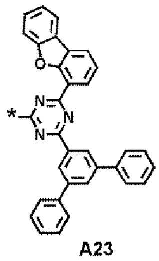
A23

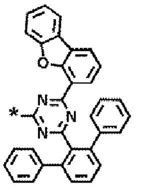
A24
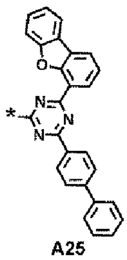
A25
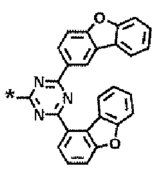
A26
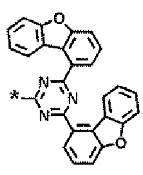
A27
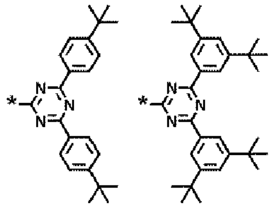
A28 A29
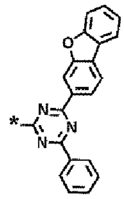
A30

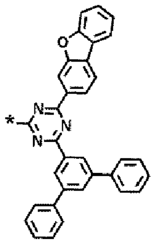
A31

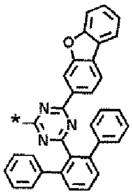
A32

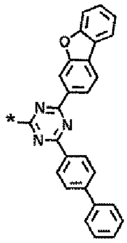
A33

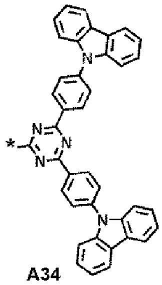
A34

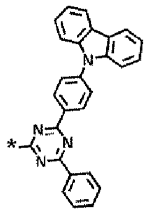
A35

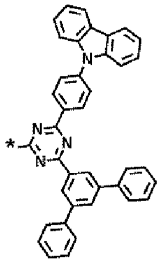
A36

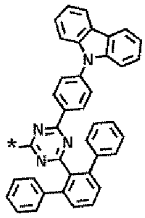
A37

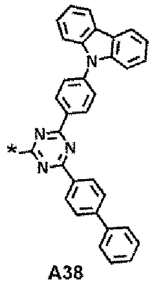
A38

,

wherein, in the formulae above, * and *' each indicate a binding site to a neighboring atom.

**2.** An organic light-emitting device comprising:

a first electrode,
a second electrode, and
an organic layer disposed between the first electrode and the second electrode,
wherein the organic layer comprises an emission layer, and the organic layer comprises at least one heterocyclic compound of claim 1 represented by Formula 1.

**3.** The organic light-emitting device of claim 2, wherein

the first electrode is an anode,
the second electrode is a cathode,
the organic layer comprises a hole transport region disposed between the first electrode and the emission layer and an electron transport region disposed between the emission layer and the second electrode,
wherein the hole transport region comprises a hole injection layer, a hole transport layer, an electron blocking layer, a buffer layer, or any combination thereof, and
wherein the electron transport region comprises a hole blocking layer, an electron transport layer, an electron injection layer, or any combination thereof.

**4.** The organic light-emitting device of claims 2 or 3, wherein
the emission layer comprises the heterocyclic compound represented by Formula 1.

**5.** The organic light-emitting device of claim 4, wherein

the emission layer comprises a host and a dopant,
the dopant comprises the heterocyclic compound represented by Formula 1, and
an amount of the host is larger than an amount of the dopant; and/or
wherein the emission layer emits blue light; and/or
wherein a ratio of a delayed fluorescence component emitted from the heterocyclic compound of the emission layer to a total emission component emitted from the emission layer is 90 % or more.

**6.** The organic light-emitting device of claim 5, wherein

the host comprises at least one selected from a first material and a second material,
the first material comprises at least one π electron-rich cyclic group and does not comprise an electron transport moiety,
the second material comprises at least one π electron-rich cyclic group and at least one electron transport moiety, and
the electron transport moiety is selected from a cyano group, a π electron-depleted nitrogen-containing cyclic group, and a group represented by one selected from the following formulae:

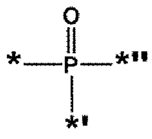 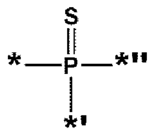 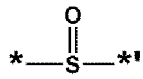 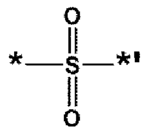,

wherein *, *', and *" in the formulae each indicate a binding site to a neighboring atom;
preferably wherein
the first material comprises a cyano group-free benzene group and a cyano group-free carbazole group, and
the second material comprises at least one selected from a cyano group-containing benzene group and a cyano group-containing carbazole group.

**Patentansprüche**

**1.** Heterocyclische Verbindung dargestellt durch Formel 1:

Formel 1

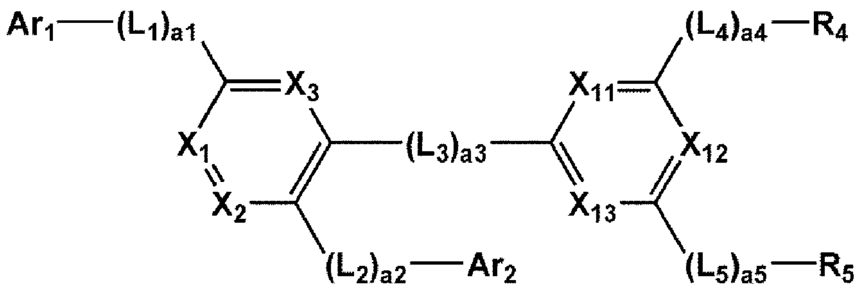,

wobei in Formel 1

$X_1$ N oder $C(R_1)$ ist, $X_2$ N oder $C(R_2)$ ist und $X_3$ N oder $C(R_3)$ ist, wobei i) eines ausgewählt aus $X_1$ bis $X_3$ N ist und die anderen davon nicht N sind; oder ii) jedes von $X_1$ bis $X_3$ nicht N ist,
$R_1$ bis $R_3$ jeweils unabhängig Wasserstoff, Deuterium, eine $C_1$-$C_{20}$-Alkylgruppe, eine $C_1$-$C_{20}$-Alkylgruppe substituiert mit zumindest einem Deuterium, eine $C_1$-$C_{20}$-Alkoxygruppe oder eine $C_1$-$C_{20}$-Alkoxygruppe substituiert mit zumindest einem Deuterium sind,
$L_1$ und $L_2$ eine Einzelbindung sind,
$X_{11}$ bis $X_{13}$ jeweils unabhängig N sind,
$Ar_1$ und $Ar_2$ jeweils unabhängig eine Gruppe dargestellt durch eines ausgewählt aus den Formeln D'-1 bis D'-7 und D002 bis D005, D015 bis D0027, D0029 bis D0032 und D0034 bis D279 sind,
eine Gruppe dargestellt durch *-$(L_3)_{a3}$-*' eine Einfachbindung ist,
eine Gruppe dargestellt durch

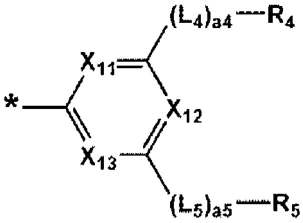

eine Gruppe dargestellt durch eines ausgewählt aus Formel A1 bis A38 ist,
* in einer Gruppe dargestellt durch

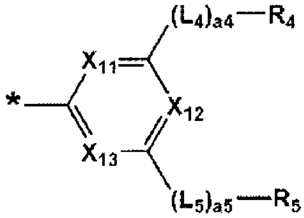

eine Bindungsstelle an ein benachbartes Atom angibt, und
in einer Gruppe dargestellt durch $*-(L_3)_{a3}-*'$ * eine Bindungsstelle an einen 6-gliedrigen Ring auf der linken Seite in Formel 1 angibt und *' eine Bindungsstelle an einen 6-gliedrigen Ring auf der rechten Seite in Formel 1 angibt:

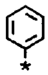
D'-1
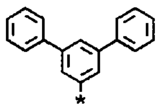
D'-2
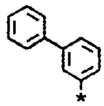
D'-3
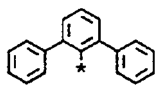
D'-4
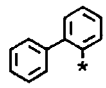
D'-5
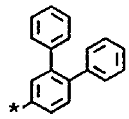
D'-6
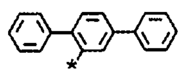
D'-7

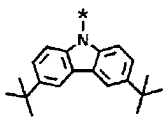
D002
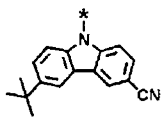
D003
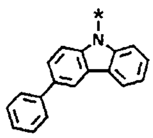
D004
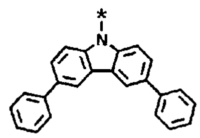
D005

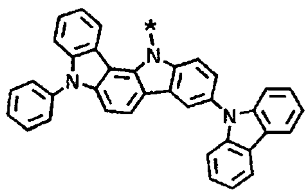
D015
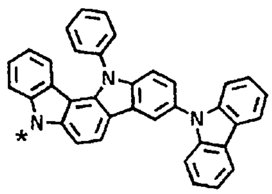
D016
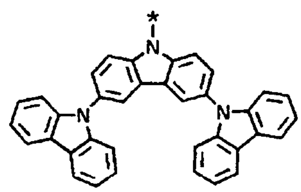
D017

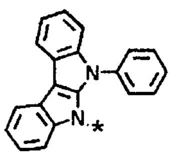
D018
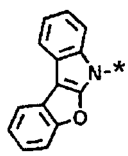
D019
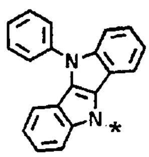
D020
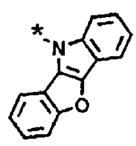
D021
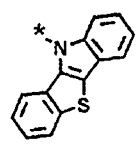
D022
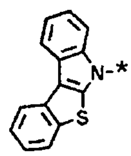
D023

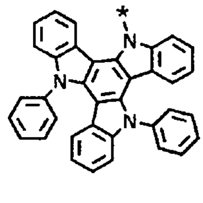
D024
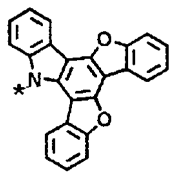
D025
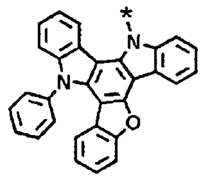
D026
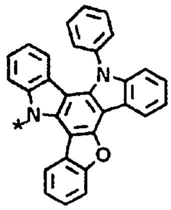
D027

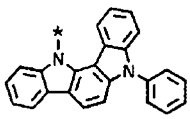
D029
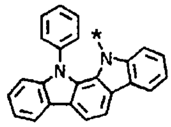
D030
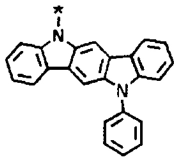
D031
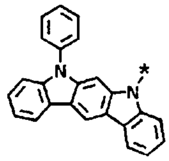
D032

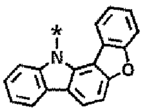
D034
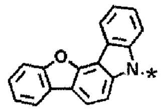
D035
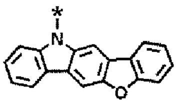
D036
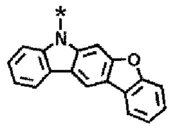
D037
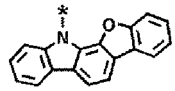
D038
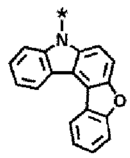
D039

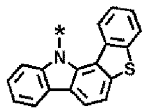
D040
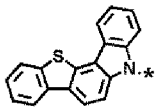
D041
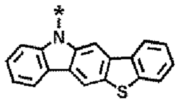
D042
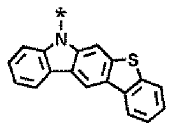
D043
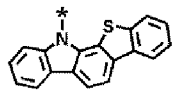
D044
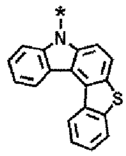
D045

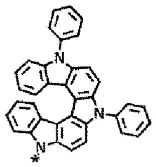
D046
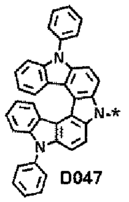
D047
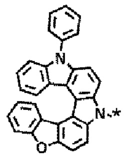
D048
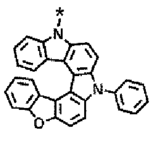
D049
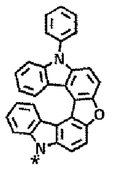
D050
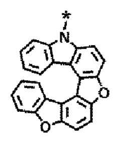
D051
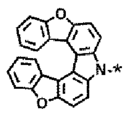
D052

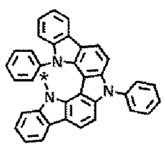
D053
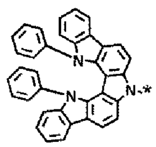
D054
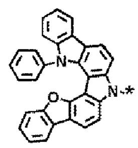
D055
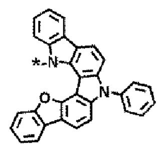
D056
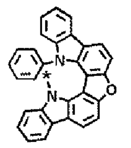
D057
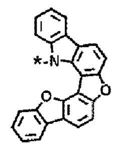
D058
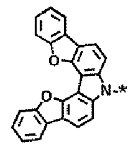
D059

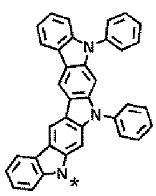
D060
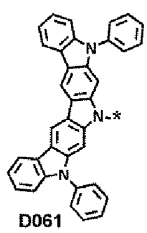
D061
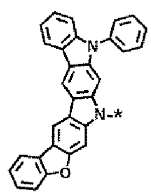
D062
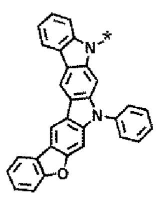
D063
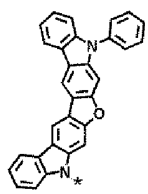
D064
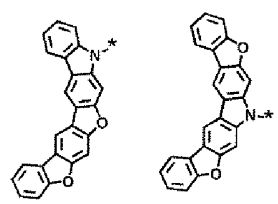
D065
D066

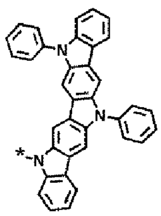
D067

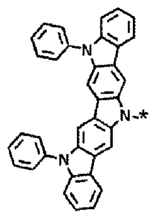
D068

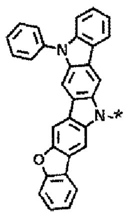
D069

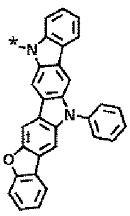
D070

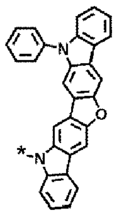
D071

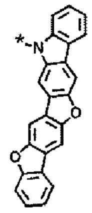
D072

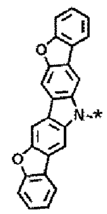
D073

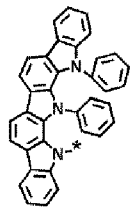
D074

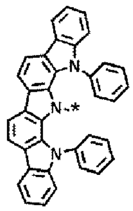
D075

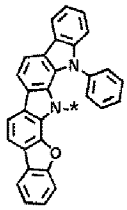
D076

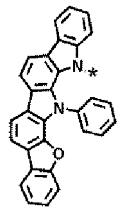
D077

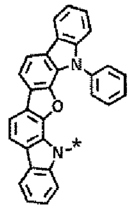
D078

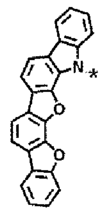
D079

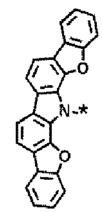
D080

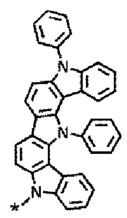
D081

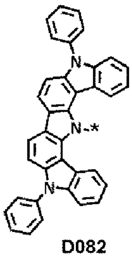
D082

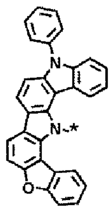
D083

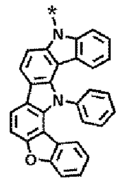
D084

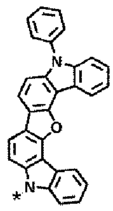
D085

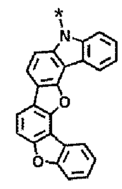
D086

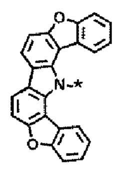
D087

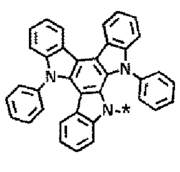
D088

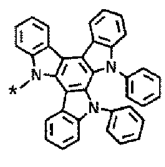
D089

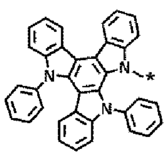
D090

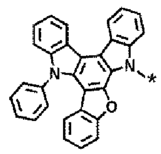
D091

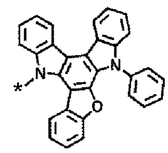
D092

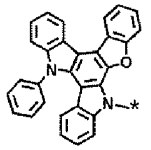
D093

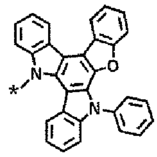
D094

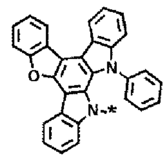
D095

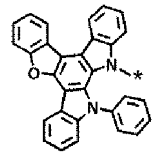
D096

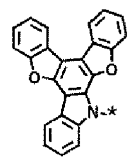
D097

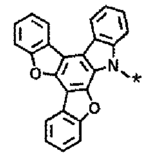
D098

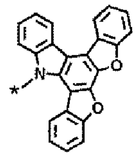
D099

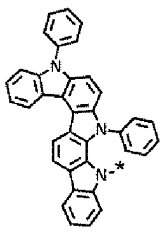
D100

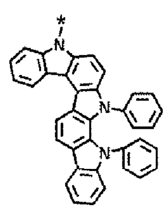
D101

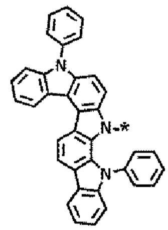
D102

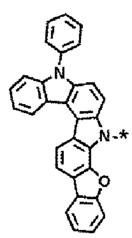
D103

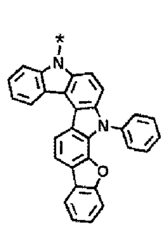
D104

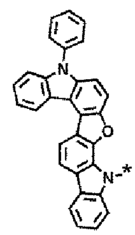
D105

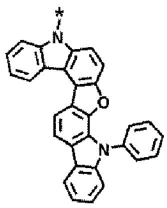
D106

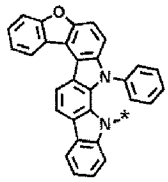
D107

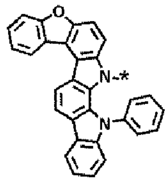
D108

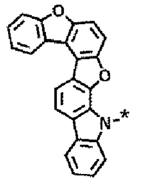
D109

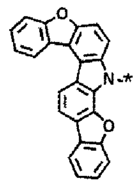
D110

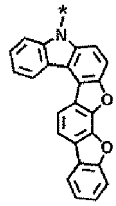
D111

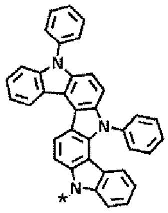
D112

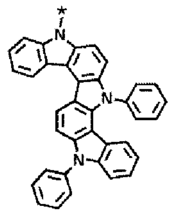
D113

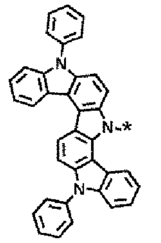
D114

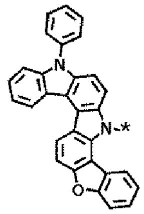
D115

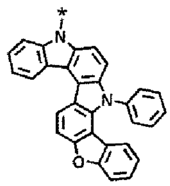
D116

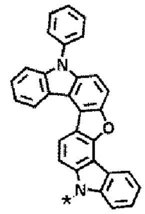
D117

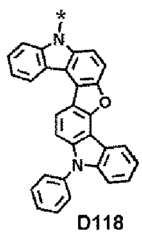
D118

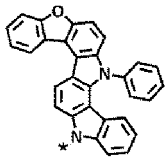
D119

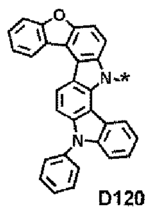
D120

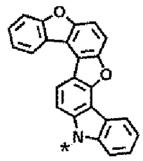
D121

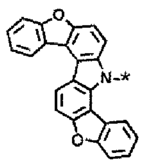
D122

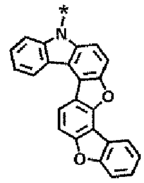
D123

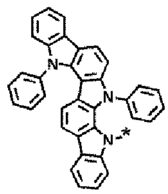
D124

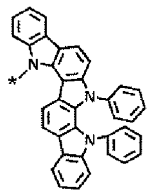
D125

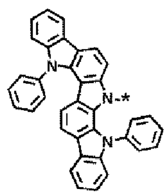
D126

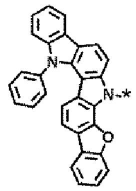
D127

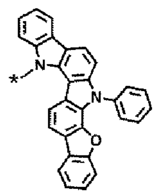
D128

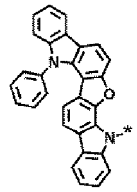
D129

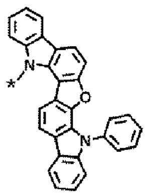
D130

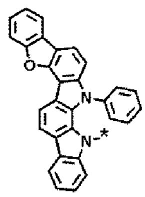
D131

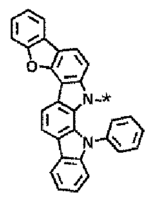
D132

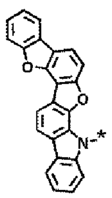
D133

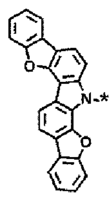
D134

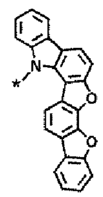
D135

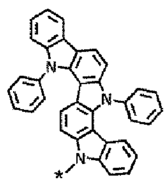
D136

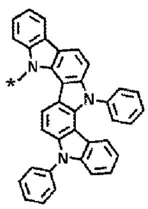
D137

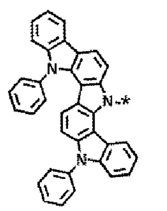
D138

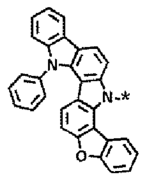
D139

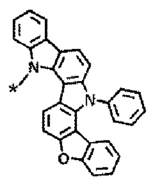
D140

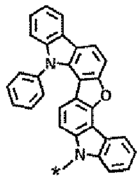
D141

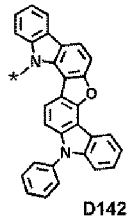
D142

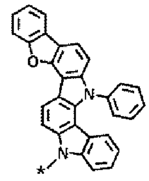
D143

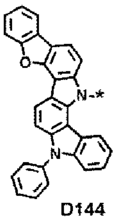
D144

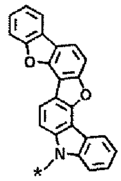
D145

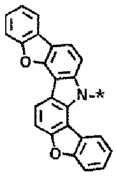
D146

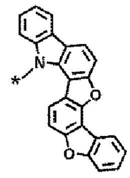
D147

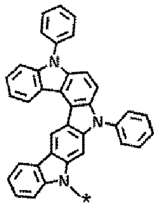
D148

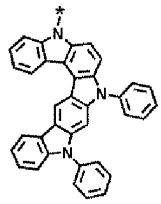
D149

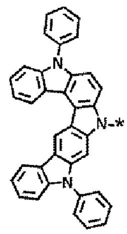
D150

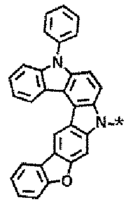
D151

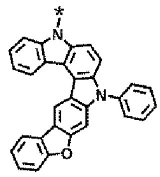
D152

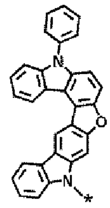
D153

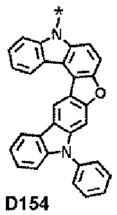
D154

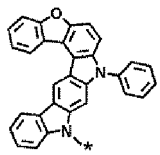
D155

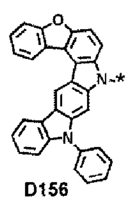
D156

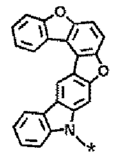
D157

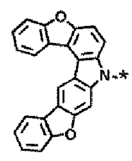
D158

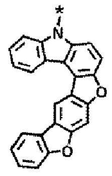
D159

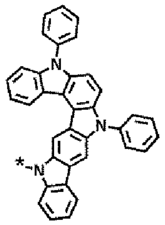
D160

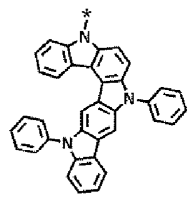
D161

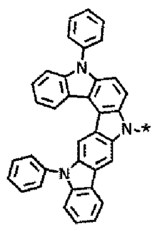
D162

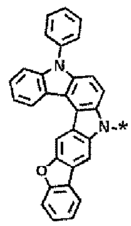
D163

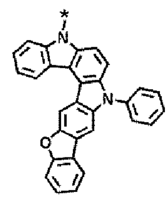
D164

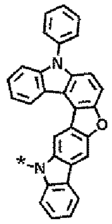
D165

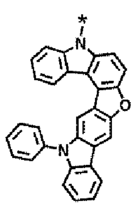
D166

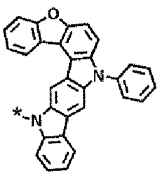
D167

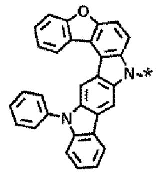
D168

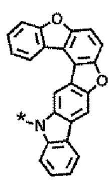
D169

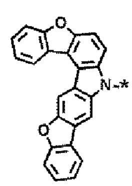
D170

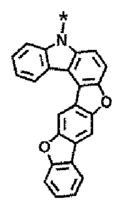
D171

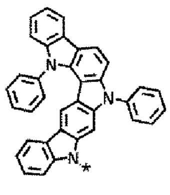
D172

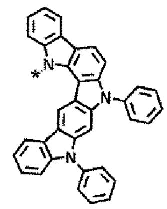
D173

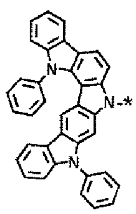
D174

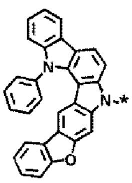
D175

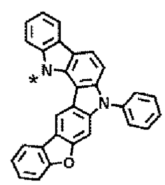
D176

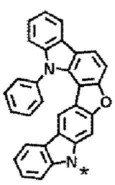
D177

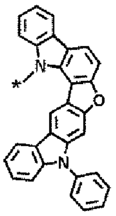
D178

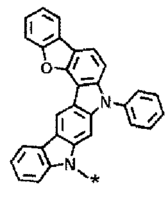
D179

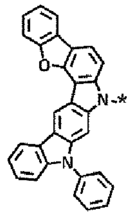
D180

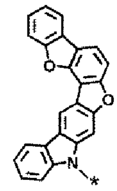
D181

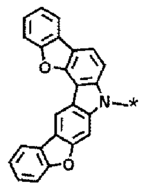
D182

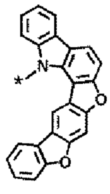
D183

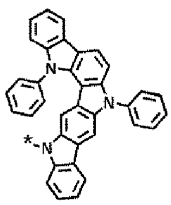
D184

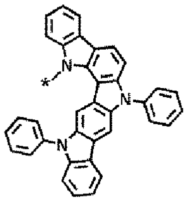
D185

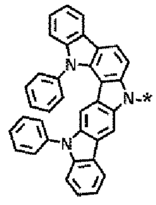
D186

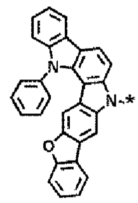
D187

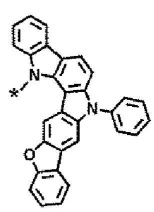
D188

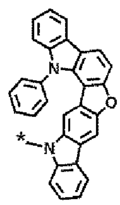
D189

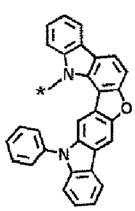
D190

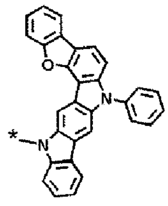
D191

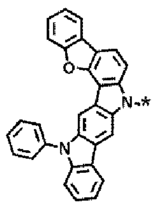
D192

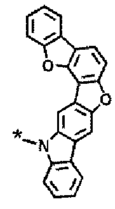
D193

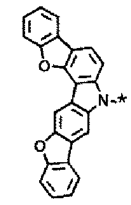
D194

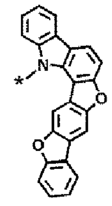
D195

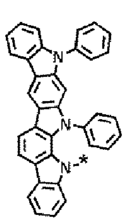
D196

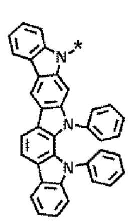
D197

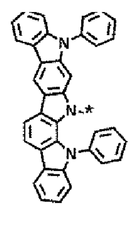
D198

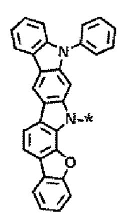
D199

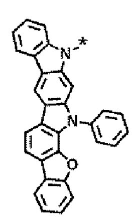
D200

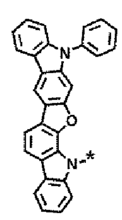
D201

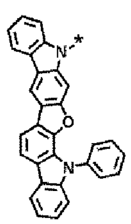
D202

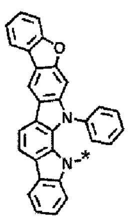
D203

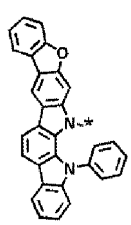
D204

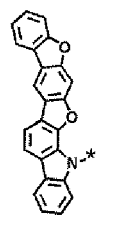
D205

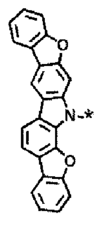
D206

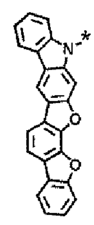
D207

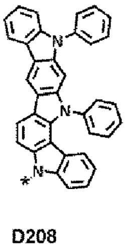
D208

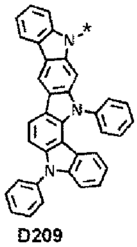
D209

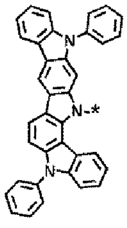
D210

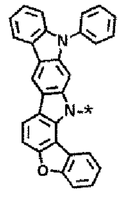
D211

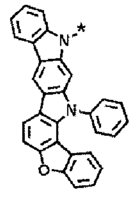
D212

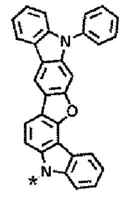
D213

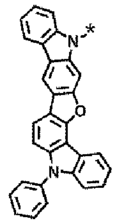
D214

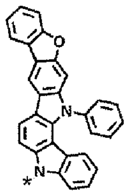
D215

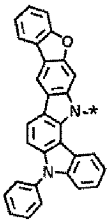
D216

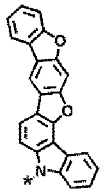
D217

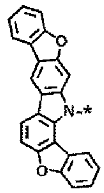
D218

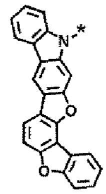
D219

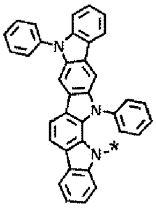
D220

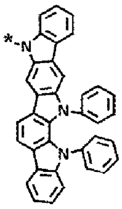
D221

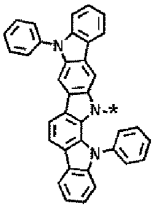
D222

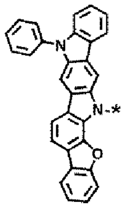
D223

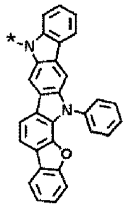
D224

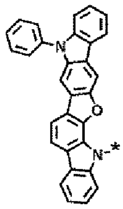
D225

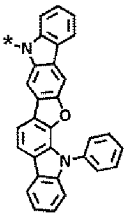
D226

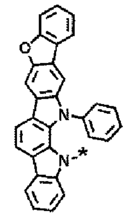
D227

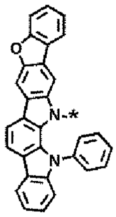
D228

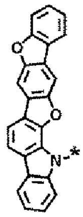
D229

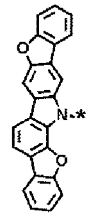
D230

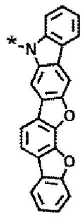
D231

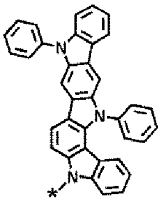
D232

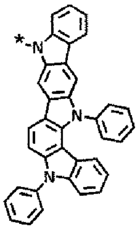
D233

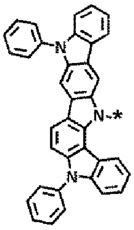
D234

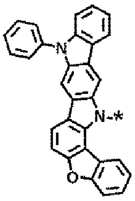
D235

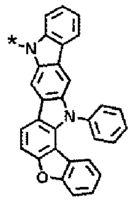
D236

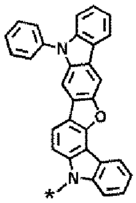
D237

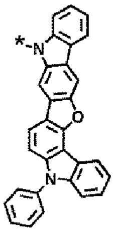
D238

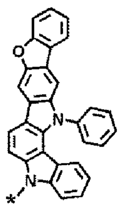
D239

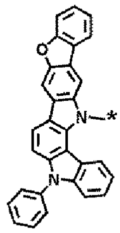
D240

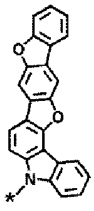
D241

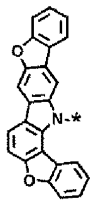
D242

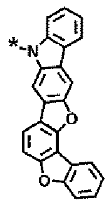
D243

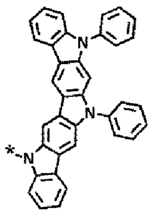
D244

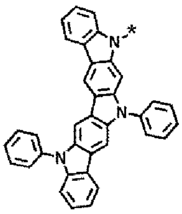
D245

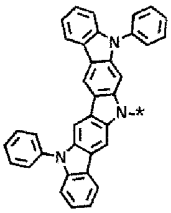
D246

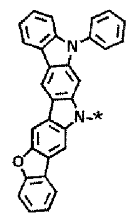
D247

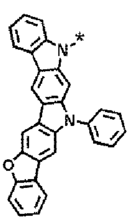
D248

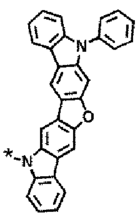
D249

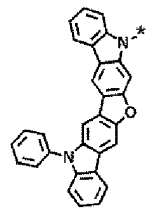
D250

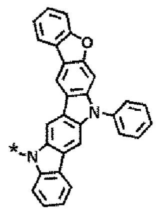
D251

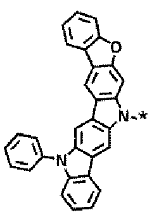
D252

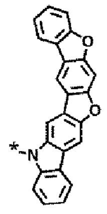
D253

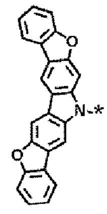
D254

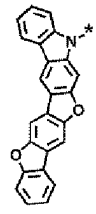
D255

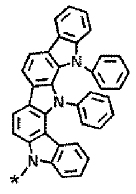
D256

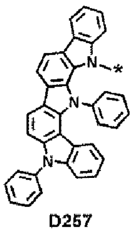
D257

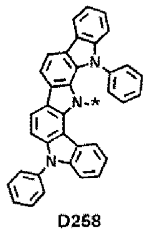
D258

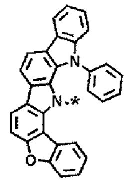
D259

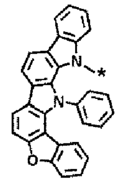
D260

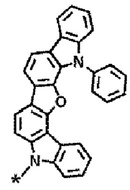
D261

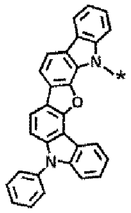
D262

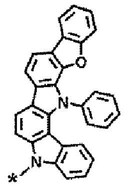
D263

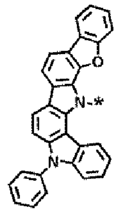
D264

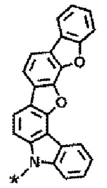
D265

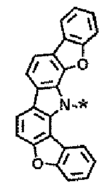
D266

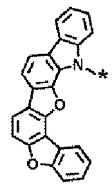
D267

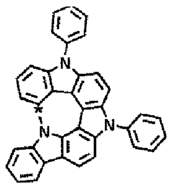
D268

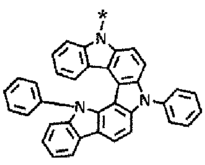
D269

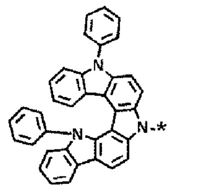
D270

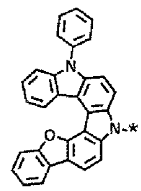
D271

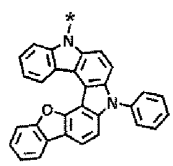
D272

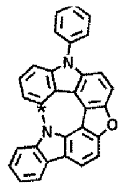
D273

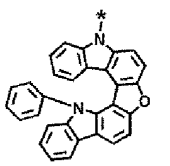
D274

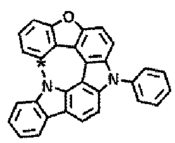
D275

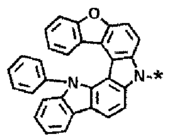
D276

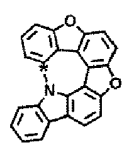
D277

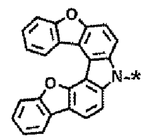
D278

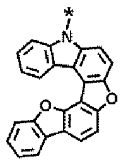
D279

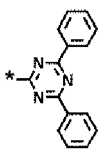
A1

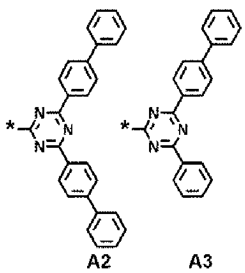
A2 A3

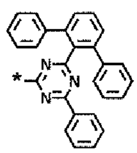
A4

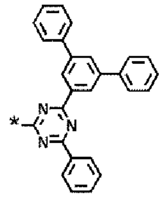
A5

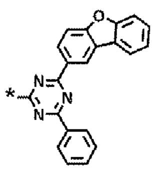
A6

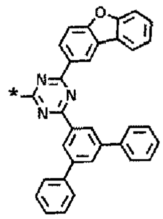
A7

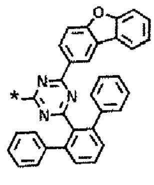
A8

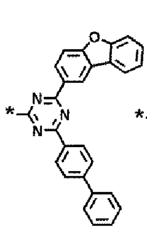
A9
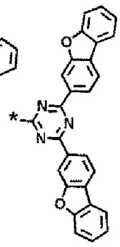
A10
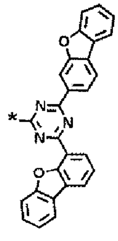
A11
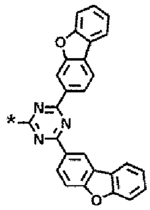
A12
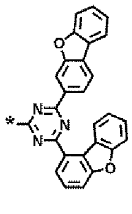
A13
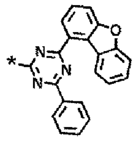
A14
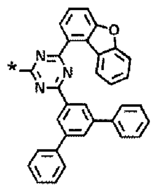
A15
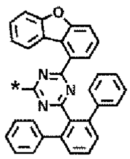
A16

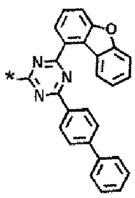
A17
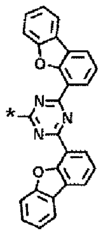
A18
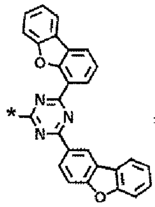
A19
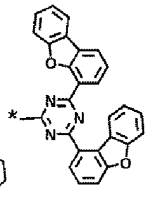
A20
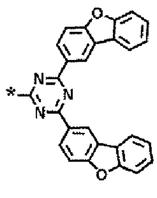
A21
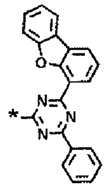
A22
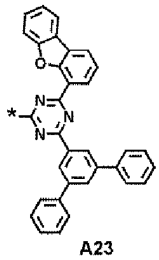
A23

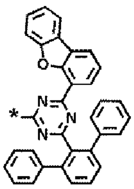
A24
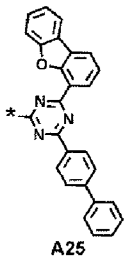
A25
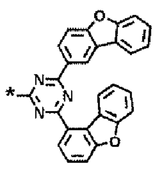
A26
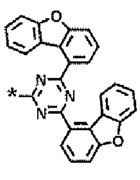
A27
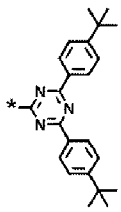
A28
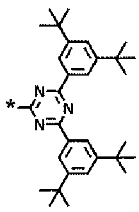
A29
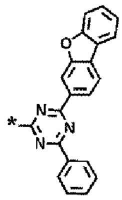
A30

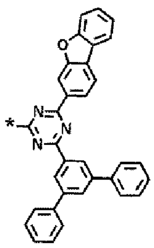
A31
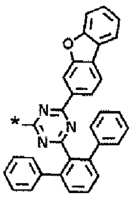
A32
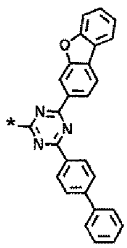
A33
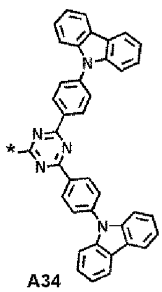
A34
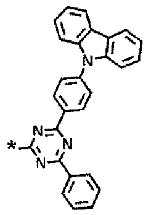
A35
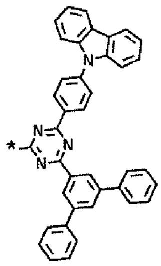
A36
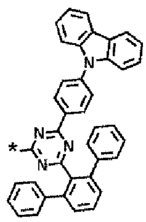
A37

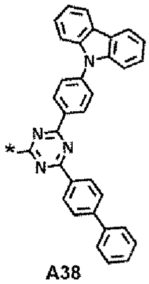
A38

wobei in den obigen Formeln * und *' jeweils eine Bindungsstelle an ein benachbartes Atom angeben.

**2.** Organische lichtemittierende Vorrichtung, umfassend:

eine erste Elektrode,
eine zweite Elektrode, und
eine organische Schicht, die zwischen der ersten Elektrode und der zweiten Elektrode angeordnet ist,
wobei die organische Schicht eine Emissionsschicht umfasst und die organische Schicht zumindest eine

heterocyclische Verbindung nach Anspruch 1 dargestellt durch Formel 1 umfasst.

**3.** Organische lichtemittierende Vorrichtung nach Anspruch 2, wobei

die erste Elektrode eine Anode ist,
die zweite Elektrode eine Kathode ist,
die organische Schicht eine Lochtransportregion, die zwischen der ersten Elektrode und der Emissionsschicht angeordnet ist, und eine Elektronentransportregion, die zwischen der Emissionsschicht und der zweiten Elektrode angeordnet ist, umfasst,
wobei die Lochtransportregion eine Locheinspritzschicht, eine Lochtransportschicht, eine Elektronenblockierschicht, eine Pufferschicht oder eine beliebige Kombination davon umfasst, und
wobei die Elektronentransportregion eine Lochblockierschicht, eine Elektronentransportschicht, eine Elektroneneinspritzschicht oder eine beliebige Kombination davon umfasst.

**4.** Organische lichtemittierende Vorrichtung nach Anspruch 2 oder 3, wobei die Emissionsschicht die heterocyclische Verbindung dargestellt durch Formel 1 umfasst.

**5.** Organische lichtemittierende Vorrichtung nach Anspruch 4, wobei

die Emissionsschicht einen Wirt und einen Dotierstoff umfasst,
der Dotierstoff die heterocyclische Verbindung dargestellt durch Formel 1 umfasst, und
eine Menge des Wirts größer als eine Menge des Dotierstoffes ist; und/oder
wobei die Emissionsschicht blaues Licht emittiert; und/oder
wobei ein Verhältnis einer Komponente verzögerter Fluoreszenz, die von der heterocyclischen Verbindung der Emissionsschicht emittiert wird, zu einer Gesamtemissionskomponente, die von der Emissionsschicht emittiert wird, 90 % oder mehr ist.

**6.** Organische lichtemittierende Vorrichtung nach Anspruch 5, wobei

der Wirt zumindest eines ausgewählt aus einem ersten Material und einem zweiten Material umfasst,
das erste Material zumindest eine π-elektronenreiche cyclische Gruppe umfasst und keinen Elektronentransportanteil umfasst,
das zweite Material zumindest eine π-elektronenreiche cyclische Gruppe und zumindest einen Elektronentransportanteil umfasst, und
der Elektronentransportanteil ausgewählt ist aus einer Cyanogruppe, einer an π-Elektronen verarmten stickstoffhaltigen cyclischen Gruppe und einer Gruppe dargestellt durch eines ausgewählt aus den folgenden Formeln:

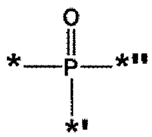
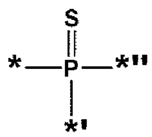
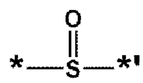
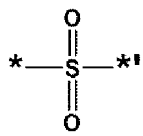

wobei *, *' und *" in den Formeln jeweils eine Bindungsstelle an ein benachbartes Atom angeben;
wobei bevorzugt
das erste Material eine cyanogruppenfreie Benzolgruppe und eine cyanogruppenfreie Carbazolgruppe umfasst, und
das zweite Material zumindest eines ausgewählt aus einer cyanogruppenhaltigen Benzolgruppe und einer cyanogruppenhaltigen Carbazolgruppe umfasst.

## Revendications

**1.** Composé hétérocyclique représenté par la Formule 1 :

Formule 1

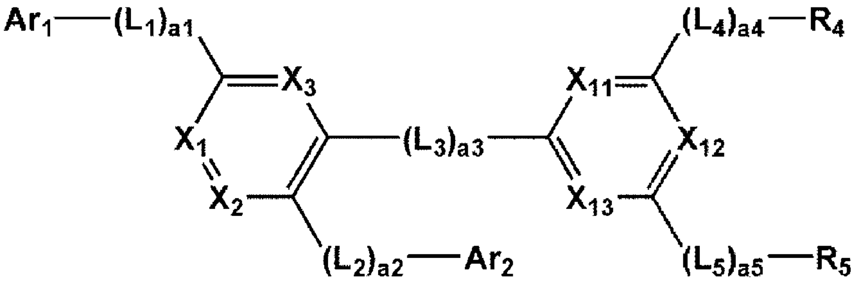

dans lequel, dans la Formule 1,

$X_1$ est N ou $C(R_1)$, $X_2$ est N ou $C(R_2)$, et $X_3$ est N ou $C(R_3)$, dans lequel i) l'un choisi parmi $X_1$ à $X_3$ est N, et les autres ne sont pas N ; ou ii) chacun de $X_1$ à $X_3$ n'est pas N,

$R_1$ à $R_3$ sont chacun indépendamment un hydrogène, un deutérium, un groupe alkyle en $C_1$-$C_{20}$, un groupe alkyle en $C_1$-$C_{20}$ substitué par au moins un deutérium, un groupe alcoxy en $C_1$-$C_{20}$ ou un groupe alcoxy en $C_1$-$C_{20}$ substitué par au moins un deutérium,

$L_1$ et $L_2$ sont une liaison simple,

$X_{11}$ à $X_{13}$ sont chacun indépendamment N,

$Ar_1$ et $Ar_2$ sont chacun indépendamment un groupe représenté par l'une choisie parmi les Formules D'-1 à D'-7 et D002 à D005, D015 à D0027, D0029 à D0032 et D0034 à D279,

un groupe représenté par *-$(L_3)_{a3}$-*' est une liaison simple,

un groupe représenté par

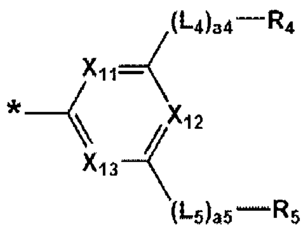

est un groupe représenté par l'une choisie parmi les Formules A1 à A38,

* dans un groupe représenté par

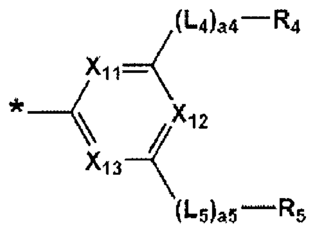

indique un site de liaison à un atome voisin, et

dans un groupe représenté par *-$(L_3)_{a3}$-*', * indique un site de liaison à un cycle à 6 chaînons sur le côté gauche dans la Formule 1, et *' indique un site de liaison à un cycle à 6 chaînons sur le côté droit dans la Formule 1 :

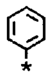
D'-1

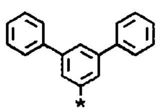
D'-2

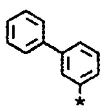
D'-3

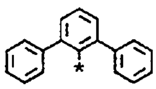
D'-4

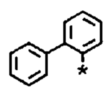
D'-5

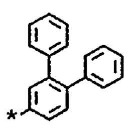
D'-6

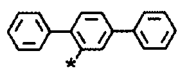
D'-7

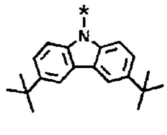
D002
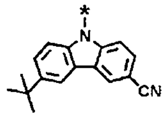
D003
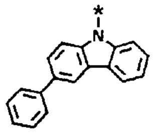
D004
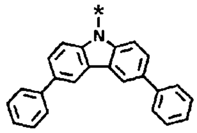
D005

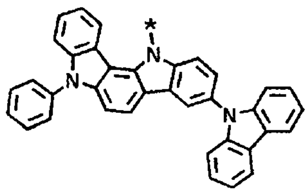
D015
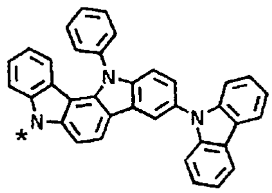
D016
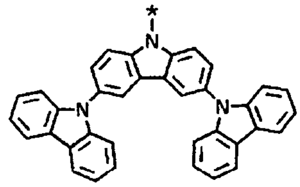
D017

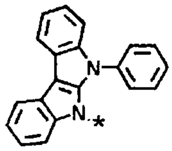
D018
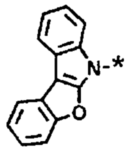
D019
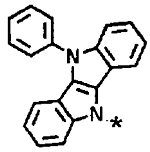
D020
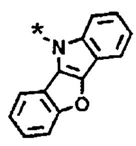
D021
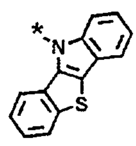
D022
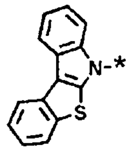
D023

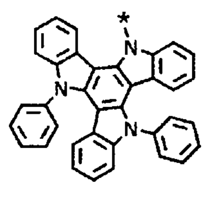
D024
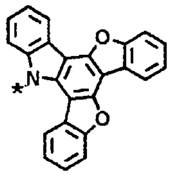
D025
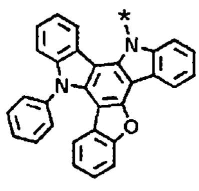
D026
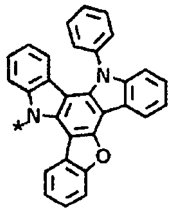
D027

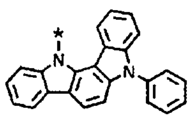
D029
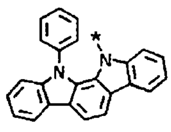
D030
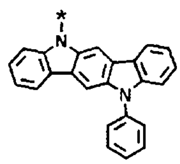
D031
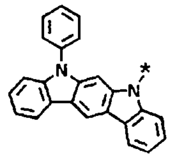
D032

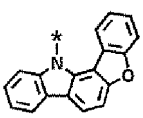
D034
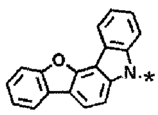
D035
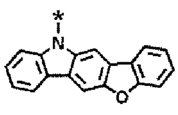
D036
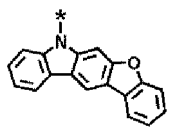
D037
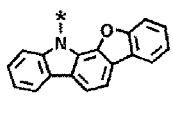
D038
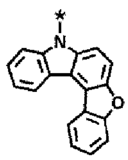
D039

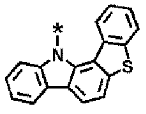
D040
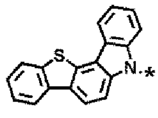
D041
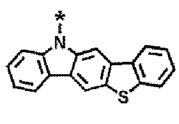
D042
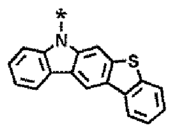
D043
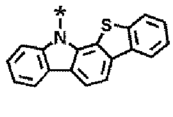
D044
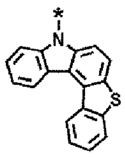
D045

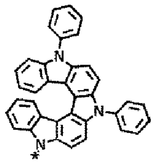
D046

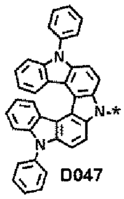
D047

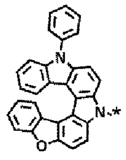
D048

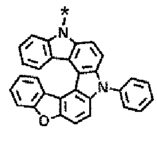
D049

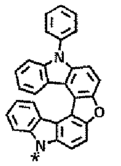
D050

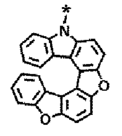
D051

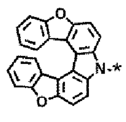
D052

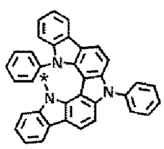
D053

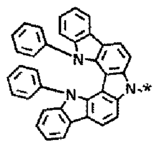
D054

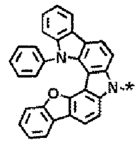
D055

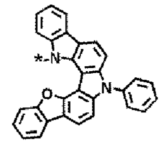
D056

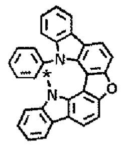
D057

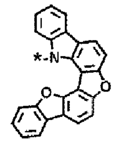
D058

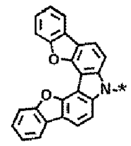
D059

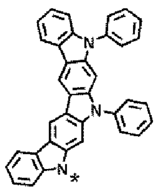
D060

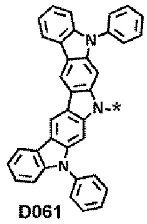
D061

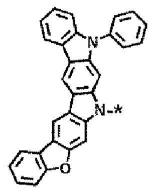
D062

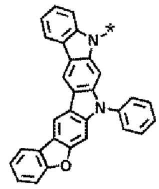
D063

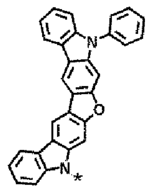
D064

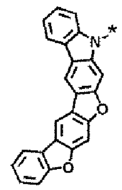
D065

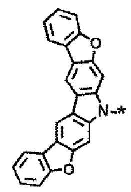
D066

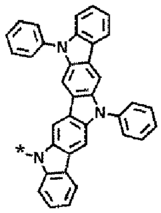
D067

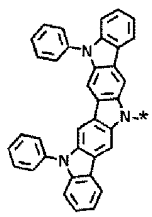
D068

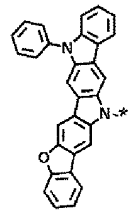
D069

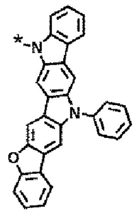
D070

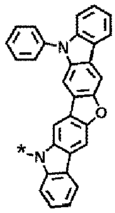
D071

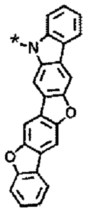
D072

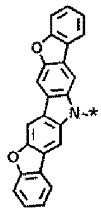
D073

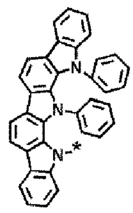
D074

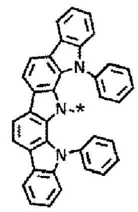
D075

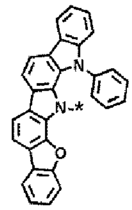
D076

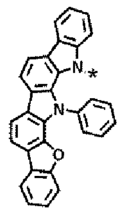
D077

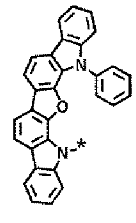
D078

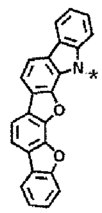
D079

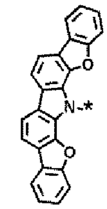
D080

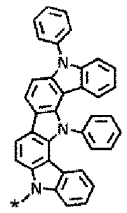
D081

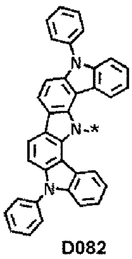
D082

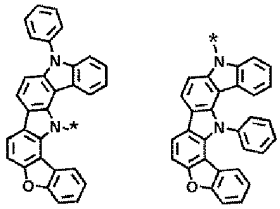
D083 D084

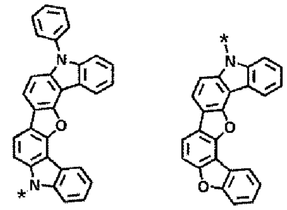
D085 D086

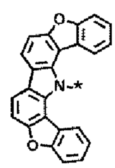
D087

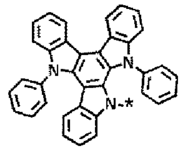
D088
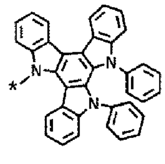
D089
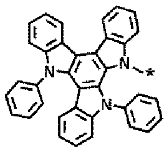
D090
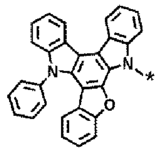
D091
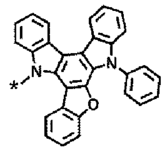
D092
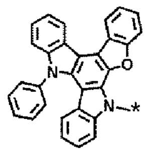
D093

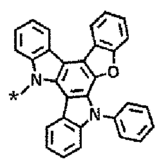
D094
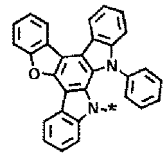
D095
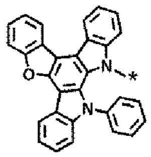
D096
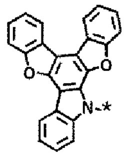
D097
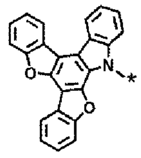
D098
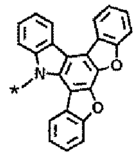
D099

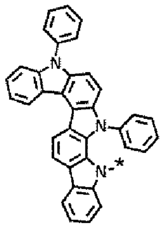
D100
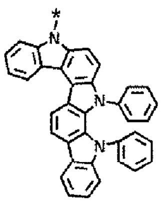
D101
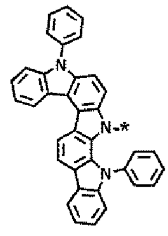
D102
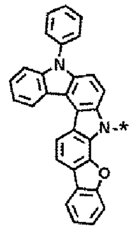
D103
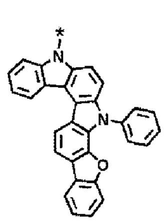
D104
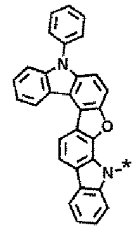
D105

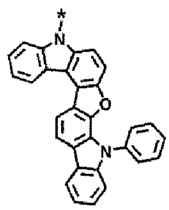
D106
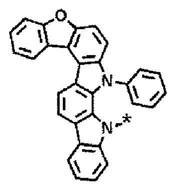
D107
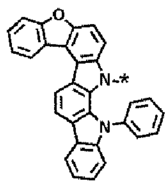
D108
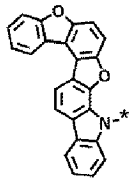
D109
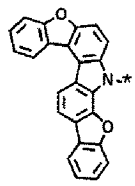
D110
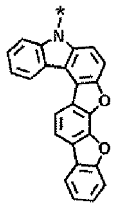
D111

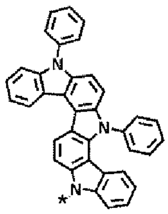
D112
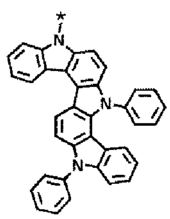
D113
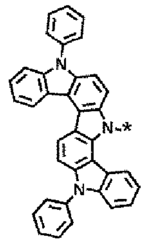
D114
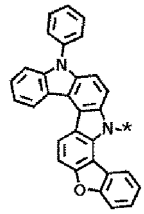
D115
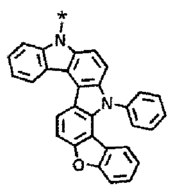
D116
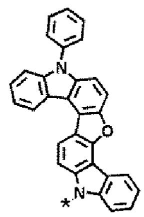
D117

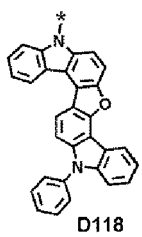
D118
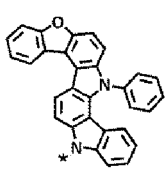
D119
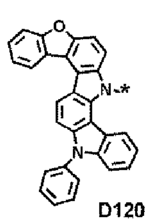
D120
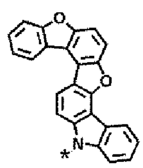
D121
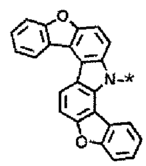
D122
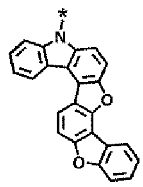
D123

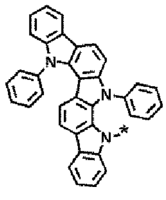
D124

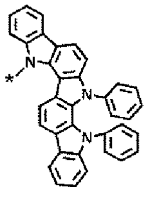
D125

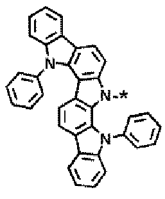
D126

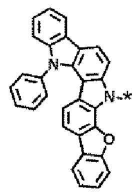
D127

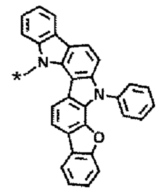
D128

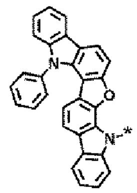
D129

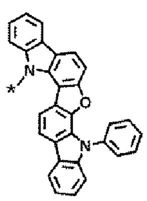
D130

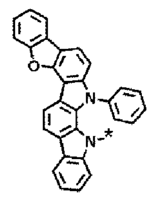
D131

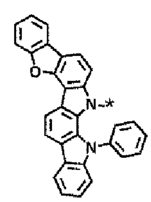
D132

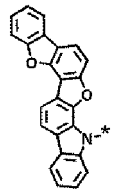
D133

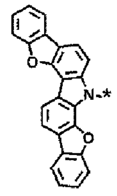
D134

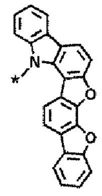
D135

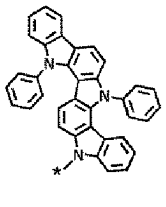
D136

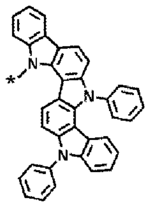
D137

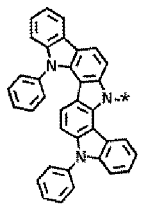
D138

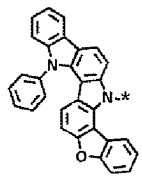
D139

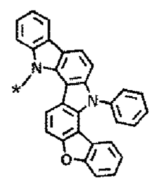
D140

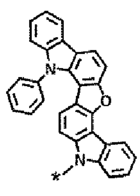
D141

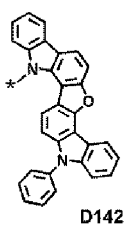
D142

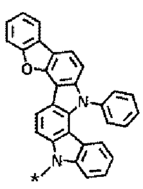
D143

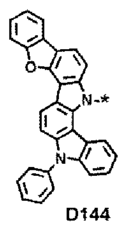
D144

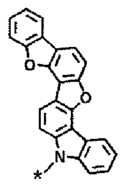
D145

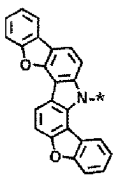
D146

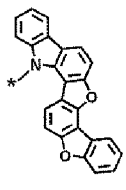
D147

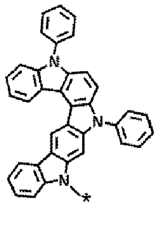
D148

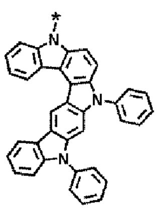
D149

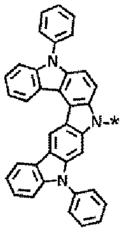
D150

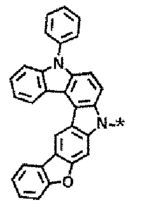
D151

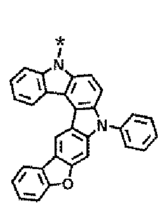
D152

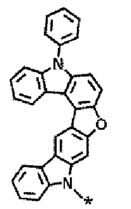
D153

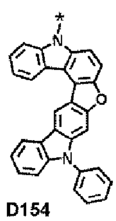
D154

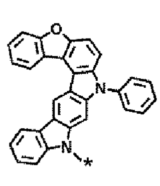
D155

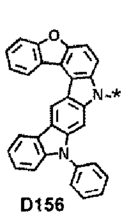
D156

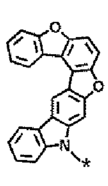
D157

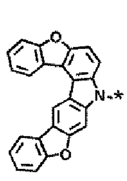
D158

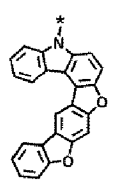
D159

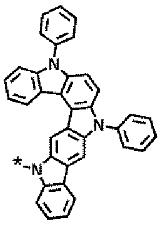

D160

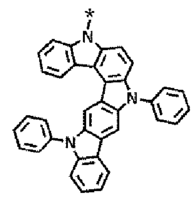

D161

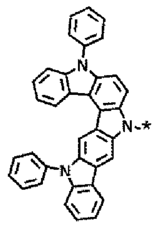

D162

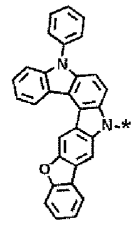

D163

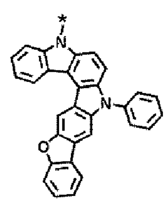

D164

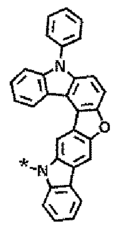

D165

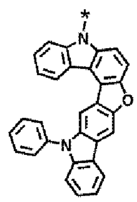

D166

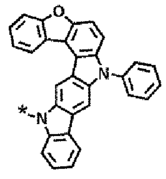

D167

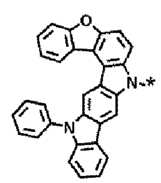

D168

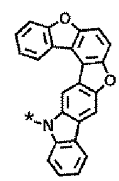

D169

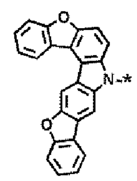

D170

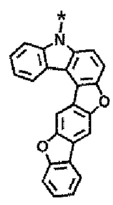

D171

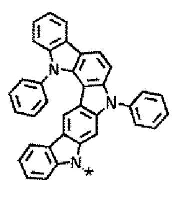

D172

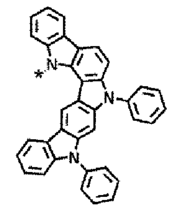

D173

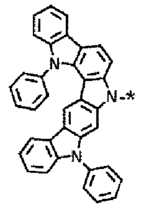

D174

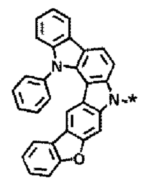

D175

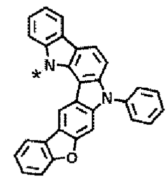

D176

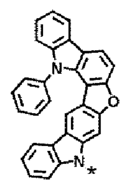

D177

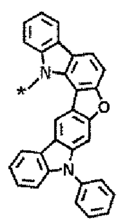

D178

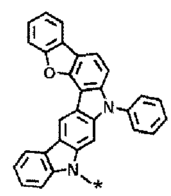

D179

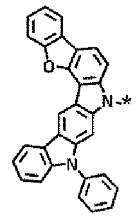

D180

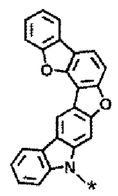

D181

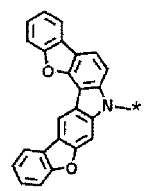

D182

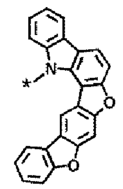

D183

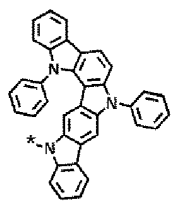

D184

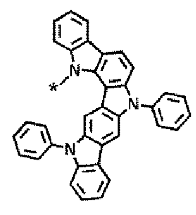

D185

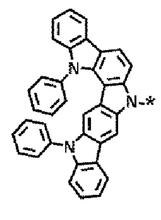

D186

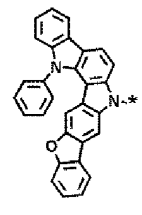

D187

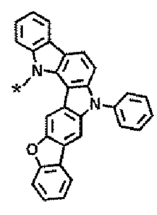

D188

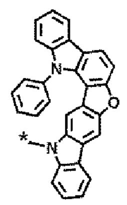

D189

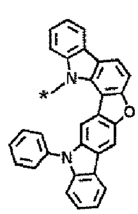

D190

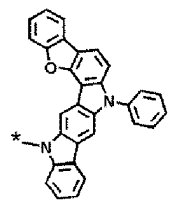

D191

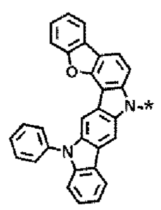

D192

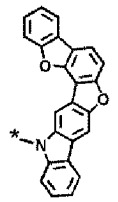

D193

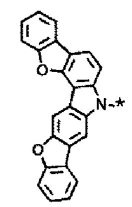

D194

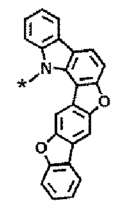

D195

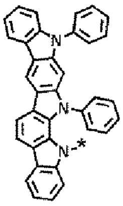
D196

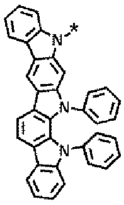
D197

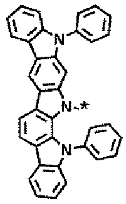
D198

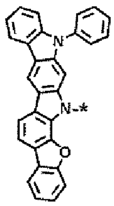
D199

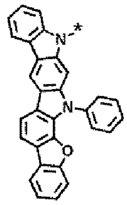
D200

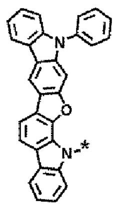
D201

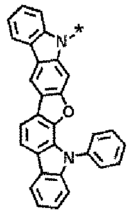
D202

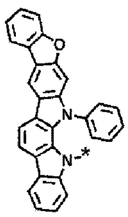
D203

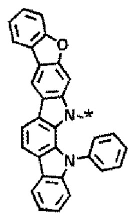
D204

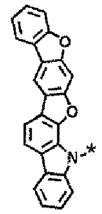
D205

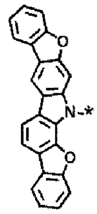
D206

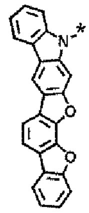
D207

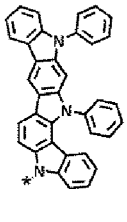
D208

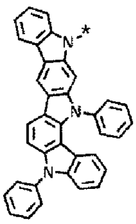
D209

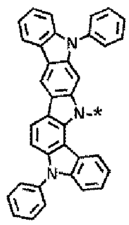
D210

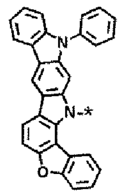
D211

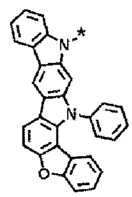
D212

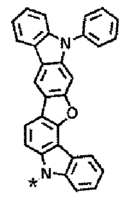
D213

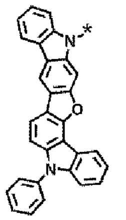
D214

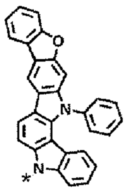
D215

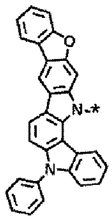
D216

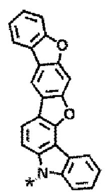
D217

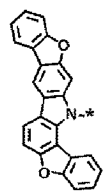
D218

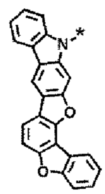
D219

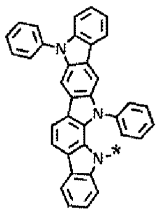
D220

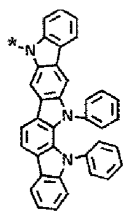
D221

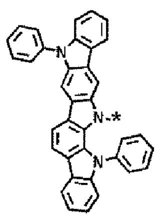
D222

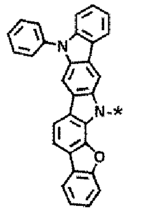
D223

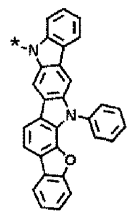
D224

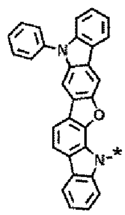
D225

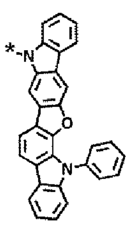
D226

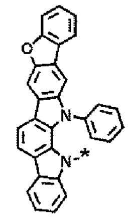
D227

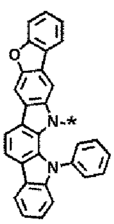
D228

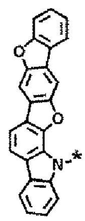
D229

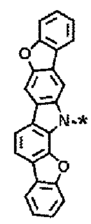
D230

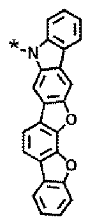
D231

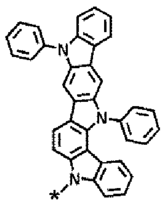
D232
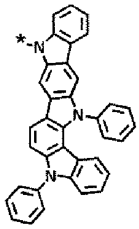
D233
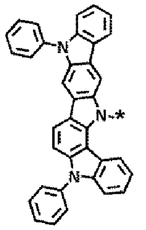
D234
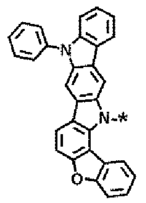
D235
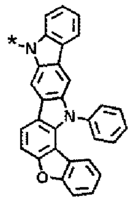
D236
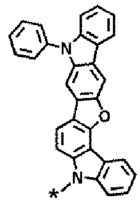
D237

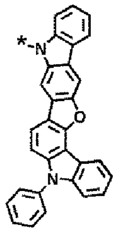
D238
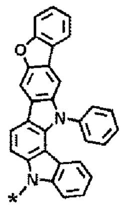
D239
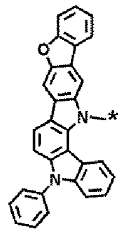
D240
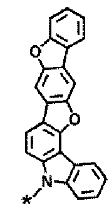
D241
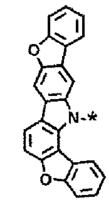
D242
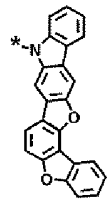
D243

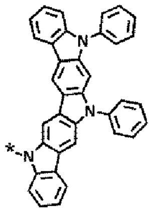
D244
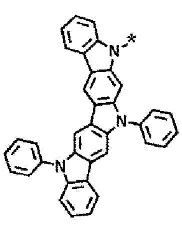
D245
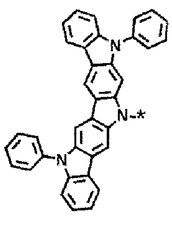
D246
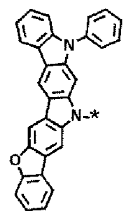
D247
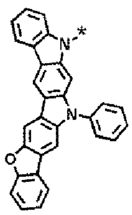
D248
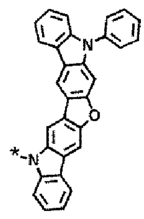
D249

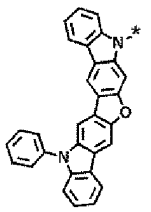
D250
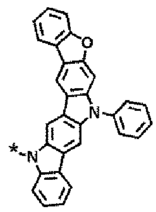
D251
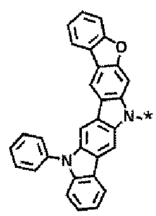
D252
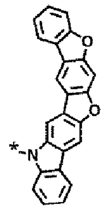
D253
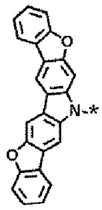
D254
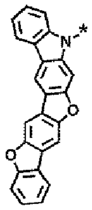
D255

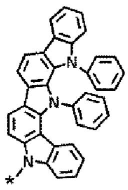
D256
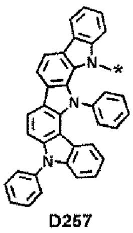
D257
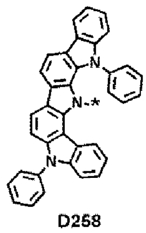
D258
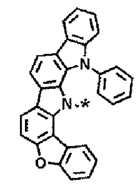
D259
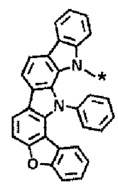
D260
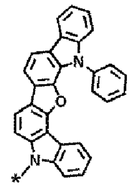
D261

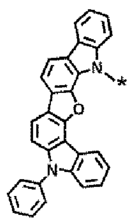
D262
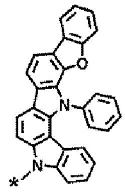
D263
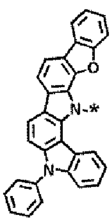
D264
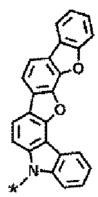
D265
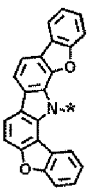
D266
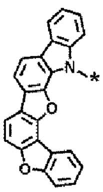
D267

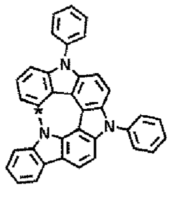
D268
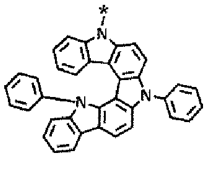
D269
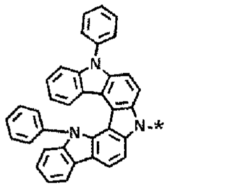
D270
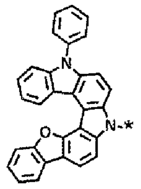
D271
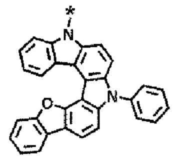
D272
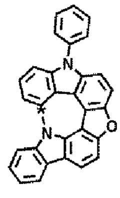
D273

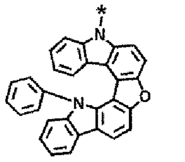
D274
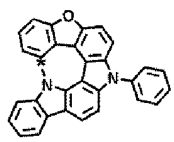
D275
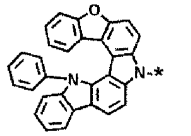
D276
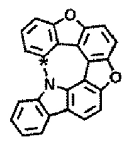
D277
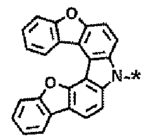
D278
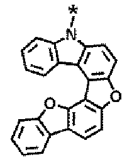
D279

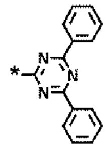
A1
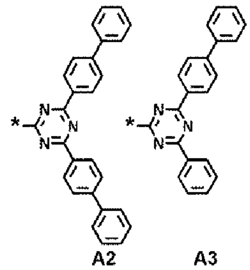
A2 A3
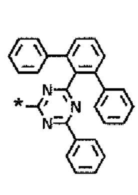
A4
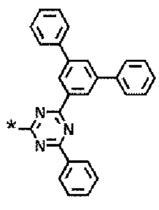
A5
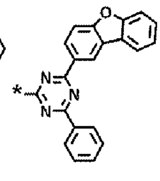
A6
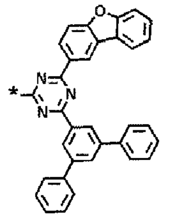
A7
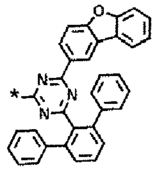
A8

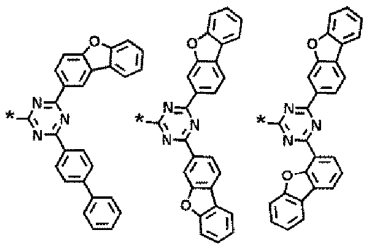
A9 A10 A11
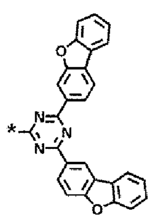
A12
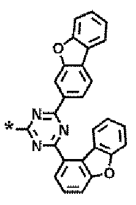
A13
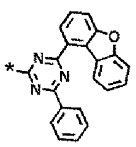
A14
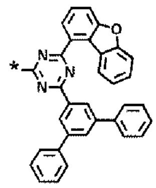
A15
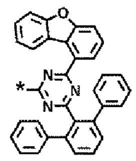
A16

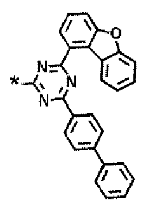
A17
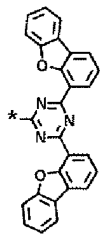
A18
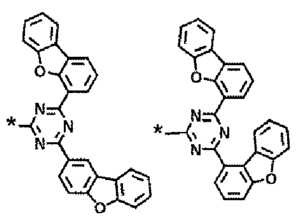
A19 A20
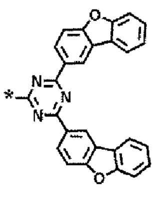
A21
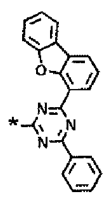
A22
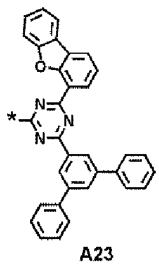
A23

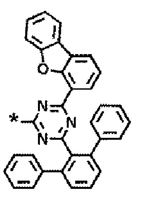
A24
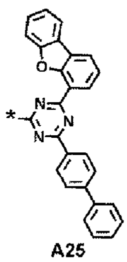
A25
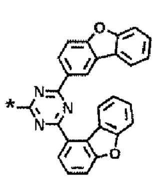
A26
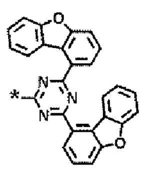
A27
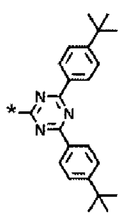
A28
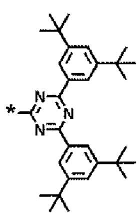
A29
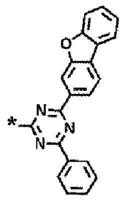
A30

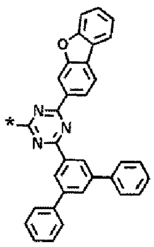
A31

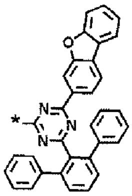
A32

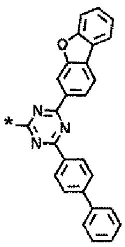
A33

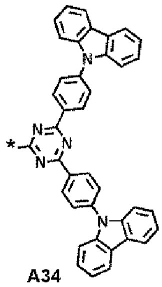
A34

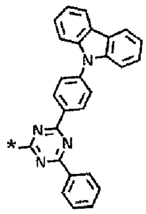
A35

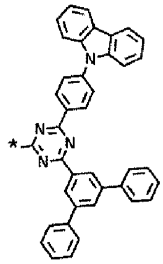
A36

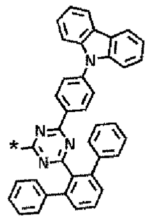
A37

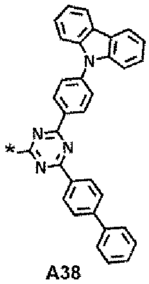
A38

dans lequel, dans les formules ci-dessus, * et *' indiquent chacun un site de liaison à un atome voisin.

**2.** Dispositif électroluminescent organique, comprenant :

une première électrode,
une seconde électrode, et
une couche organique disposée entre la première électrode et la seconde électrode,
dans lequel la couche organique comprend une couche d'émission, et la couche organique comprend au moins un composé hétérocyclique de la revendication 1 représenté par la Formule 1.

**3.** Dispositif électroluminescent organique de la revendication 2, dans lequel

la première électrode est une anode,
la seconde électrode est une cathode,
la couche organique comprend une région de transport de trous disposée entre la première électrode et la couche d'émission et une région de transport d'électrons disposée entre la couche d'émission et la seconde électrode,
dans lequel la région de transport de trous comprend une couche d'injection de trous, une couche de transport de trous, une couche de blocage d'électrons, une couche tampon, ou une combinaison de celles-ci, et
dans lequel la région de transport d'électrons comprend une couche de blocage de trous, une couche de transport d'électrons, une couche d'injection d'électrons ou toute combinaison de celles-ci.

**4.** Dispositif électroluminescent organique de l'une des revendications 2 ou 3, dans lequel la couche d'émission comprend le composé hétérocyclique représenté par la Formule 1.

**5.** Dispositif électroluminescent organique de la revendication 4, dans lequel

la couche d'émission comprend un hôte et un dopant,
le dopant comprend le composé hétérocyclique représenté par la Formule 1, et
une quantité de l'hôte est supérieure à une quantité du dopant ; et/ou
dans lequel la couche d'émission émet une lumière bleue ; et/ou
dans lequel un rapport entre une composante de fluorescence retardée émise par le composé hétérocyclique de la couche d'émission et une composante d'émission totale émise par la couche d'émission est de 90 % ou plus.

**6.** Dispositif électroluminescent organique de la revendication 5, dans lequel

l'hôte comprend au moins l'un choisi parmi un premier matériau et un second matériau,
le premier matériau comprend au moins un groupe cyclique riche en électrons π et ne comprend pas de fragment

de transport d’électrons,
le second matériau comprend au moins un groupe cyclique riche en électrons π et au moins un fragment de transport d’électrons, et
le fragment de transport d’électrons est choisi parmi un groupe cyano, un groupe cyclique contenant de l’azote appauvri en électrons π et un groupe représenté par l’une choisie parmi les formules suivantes :

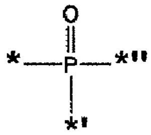 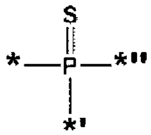 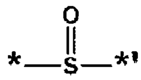 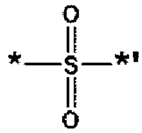

dans lequel *, *’ et *" dans les formules indiquent chacun un site de liaison à un atome voisin ;
de préférence dans lequel
le premier matériau comprend un groupe benzène dépourvu de groupe cyano et un groupe carbazole dépourvu de groupe cyano, et
le second matériau comprend au moins l’un choisi parmi un groupe benzène contenant un groupe cyano et un groupe carbazole contenant un groupe cyano.

# FIGURE

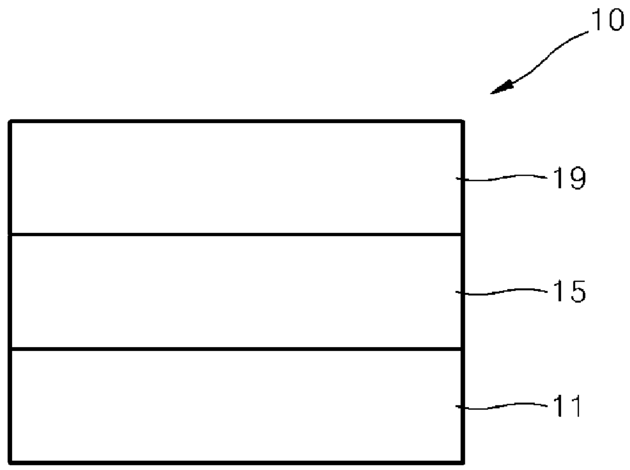

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1829871 A
- CN 107987009
- CN 107954922
- US 2017186962 A
- US 2018145262 A
- KR 20170113808

### Non-patent literature cited in the description

- **Y. WANG et al.** A series of new bipolar CBP derivatives with introduction of a electron-deficient moiety for efficient green organic light-emitting diodes. *Organic Electronics*, October 2018, vol. 61, 142-150